# EUROPEAN PATENT APPLICATION

(11) **EP 4 361 267 A1**
(43) Date of publication of application: **01.05.2024**
(21) Application number: 22828547.4
(22) Date of filing: 24.06.2022
(51) Int. Cl.: C12N 15/11, C07K 14/705, C07K 14/725, C12Q 1/68, C40B 40/10, G01N 33/53

(54) **METHOD FOR IDENTIFYING T-CELL EPITOPE SEQUENCE, AND APPLICATION OF SAME**

(30) Priority: 25.06.2021 JP 2021105709
(71) Applicant: Repertoire Genesis Incorporation, Ibaraki-shi, Osaka 567-0085 (JP)
(72) Inventor: SUZUKI, Ryuji, Ibaraki-shi, Osaka 567-0085 (JP); SHIN-I, Tadasu, Sunto-gun, Shizuoka 411-0943 (JP); NAKAMURA, Yukio, Ibaraki-shi, Osaka 567-0085 (JP); SASAMURA, Takeshi, Ibaraki-shi, Osaka 567-0085 (JP)
(74) Representative: J A Kemp LLP
(86) International application number: PCT/JP2022/025376
(87) International publication number: WO 2022/270631

(57) **Abstract**

According to the present disclosure, there is provided a method for identifying an epitope sequence.

The present disclosure provides a method for identifying an epitope sequence associated with a specific physiological state of a test subject, the method including the steps of: A) obtaining BCR and/or TCR repertoire data associated with the specific physiological state of the test subject; B) specifying a TREM sequence (repertoire specific TREM sequence) from the BCR and/or TCR repertoire data; C) obtaining a reference TREM sequence associated with the test subject; D) specifying a repertoire-reference common specific TREM sequence from the reference TREM sequence and the repertoire specific TREM sequence; and E) determining, based on the repertoire-reference common specific TREM sequence, whether a peptide sequence including the repertoire-reference common specific TREM sequence is an epitope sequence associated with the specific physiological state of the test subject.

## Description

### Technical Field

The present disclosure relates to a method for identifying a T-cell epitope and application thereof.

### Background Art

In the immune mechanism of organisms, T cells play a central role in both humoral immunity and cellular immunity. In humoral immunity, T cells (CD4⁺T cells) are known to recognize epitopes presented on MHC II, promote production of antibodies by B cells, and serve to activate CD8⁺T cells in cellular immunity (immunity by MHC II and CD4⁺T cells). Meanwhile, in cellular immunity, T cells themselves (CD8⁺T cells) recognize epitopes presented on MHC I of target cells, and act to eliminate non-self (immunity by MHC I and CD8⁺T cells) .

Main cells expressing MHC II are called antigen-presenting cells, and dendritic cells, macrophages, and B cells are known. Meanwhile, MHC I is expressed on all cells (including antigen-presenting cells) except for some cells such as red blood cells. There is an accommodating groove to which a peptide serving as an epitope binds on MHC, and the length of the peptide bound via the accommodating groove varies depending on the class of MHC. A peptide usually composed of about 9 amino acids is bound on MHC I, and a peptide usually composed of about 15 amino acids is bound on MHC II. It is known that MHC (HLA in human, H-2 in mouse) shows a high degree of polymorphism, and the peptide sequence capable of binding depends on the type and type of MHC (MHC binding affinity). T cells are restricted by their own type of MHC to recognize peptides, which is referred to as "MHC restriction".

It is known that B cells as antigen-presenting cells not only fragment antigen peptides captured by the BCR but also fragment their own BCR (idiotope), and present the peptides to T cells (Non Patent Literatures 1 and 2) (Idiotope-driven T-B collaboration). B cells function in the construction of diverse populations of T cells via sIg and antibody-derived idiotope sequences (Non Patent Literature 3) (formation of T cell populations by B cells).

According to the immune network theory of Jerne, a living body has epitopes corresponding to non-self as an internal image in an idiotope on the BCR, and an interaction between an idiotope and an anti-idiotype antibody forms a B cell network (Jerne's Immune network theory, idiotype network theory). Through the use of the immune network, the immune system in the living body performs a defense function against antigens from the outside and non-self foreign substances generated inside.

### Citation List

### Patent Literatures

Non Patent Literature 1: Weiss andBogen, (1989) PNAS,86, (1) :282-286
Non Patent Literature 2: Weiss and Bogen,(1991) Cell,64(4):767-776
Non Patent Literature 3: Joao et al.(2004) J Immunol,172 (8) , 4709-4716

### Summary of Invention

### Technical Problem

The present disclosures have found an internal image of an antigen epitope from the inside of the Idiotope of BCR, and have considered that an antigen epitope capable of inducing T cells according to MHC restriction can be identified. As a result of intensive studies, they have found a method for identifying an epitope sequence associated with a specific physiological state of a test subject based on BCR repertoire and/or TCR repertoire data associated with the specific physiological state of the test subject.

Therefore, the present disclosure provides the followings.

### (Item 1)

A method for identifying an epitope sequence associated with a specific physiological state of a test subject,
the method including the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence; and
E) determining, based on the repertoire-reference common specific TREM sequence, whether a peptide sequence including the repertoire-reference common specific TREM sequence is an epitope sequence associated with the specific physiological state of the test subject.

### (Item 2)

The method according to the above item, in which the step of obtaining BCR repertoire and/or TCR repertoire data includes a step of obtaining a full-length sequence of BCR and/or TCR.

### (Item 3)

The method according to any one of the preceding items, further including a step of determining a full-length sequence of an immunoglobulin variable section of BCR or TCR clone specific to the physiological state.

### (Item 4)

The method according to any one of the preceding items, in which the BCR repertoire and/or TCR repertoire data is obtained by analyzing a specimen obtained from the test subject.

### (Item 5)

The method according to any one of the preceding items, in which the BCR repertoire and/or TCR repertoire data is obtained from an existing database.

### (Item 6)

The method according to any one of the preceding items, further including a step of analyzing BCR and/or TCR repertoire in the specimen from the test subject to determine a clone specifically reacting to the specific physiological state.

### (Item 7)

The method according to any one of the preceding items, further including a step of determining a gene region in which the repertoire-specific TREM sequence is present.

### (Item 8)

The method according to any one of the preceding items, in which the gene region is selected from the group consisting of an L region, a V region, a D region, a J region or a C region, FR1, 2 or 3, and CDR1, 2 or 3.

### (Item 9)

The method according to any one of the preceding items, further including a step of producing an about 8- to about 10-mer and/or about 13- to about 17-mer amino acid sequence from the sequence of BCR or TCR clone specific to the physiological state and extracting the TREM sequence.

### (Item 10)

The method according to any one of the preceding items, further including a step of producing an about 9- and/or about 15-mer amino acid sequence from the sequence of BCR or TCR clone specific to the physiological state and extracting the TREM sequence.

### (Item 11)

The method according to any one of the preceding items, in which, in a 9-mer amino acid sequence, the TREM sequence includes at least one of positions 4, 5, 6, 7 and 8 from the N-terminus of the 9-mer amino acid sequence.

### (Item 12)

The method according to any one of the preceding items, in which, in a 15-mer amino acid sequence, the TREM sequence includes at least one of positions 5, 6, 8, 10 and 11, or at least one of positions 3, 6, 8, 10 and 11 from the N-terminus of the 15-mer amino acid sequence.

### (Item 13)

The method according to any one of the preceding items, further including a step of determining MHC I/II binding affinity of an about 8- to about 10-mer and/or about 13- to about 17-mer amino acid sequence including the TREM sequence.

### (Item 14)

The method according to any one of the preceding items, further including a step of specifying a variant TREM.

### (Item 15)

The method according to any one of the preceding items, further including a step of evaluating and specifying a peptide with B-cell epitope properties.

### (Item 16)

The method according to any one of the preceding items, in which the step of determining whether a peptide sequence is an epitope sequence associated with the specific physiological state includes testing HLA (MHC) binding affinity of the peptide sequence including the repertoire-reference common specific TREM sequence to determine whether the peptide sequence is an epitope sequence associated with the specific physiological state of the test subject.

### (Item 17)

The method according to any one of the preceding items, in which the method includes, in the test of HLA (MHC) binding affinity, a step of selecting an epitope sequence with an IC50 of about 5000 or less as the epitope sequence associated with the specific physiological state of the test subject.

### (Item 18)

The method according to any one of the preceding items, in which the test of HLA (MHC) binding affinity includes estimating the HLA (MHC) binding affinity.

### (Item 19)

The method according to any one of the preceding items, in which the reference TREM sequence is a TREM sequence of one selected from the group consisting of a cancer-associated protein, an infection-associated protein, an autoimmunity-associated protein, an allergy-associated protein, a microbiome and an alloreaction/xenoreaction-associated protein.

### (Item 20)

The method according to any one of the preceding items, in which the reference TREM sequence is a TREM sequence of one selected from the group consisting of a variant protein, a neoantigen, a tumor protein, a proteome in an inflamed area, a virus-associated protein, a bacteria-associated protein, a fungus-associated protein, an alloantigen, a xenoantigen, an allergen protein and a microbiome.

### (Item 21)

The method according to any one of the preceding items, in which the reference TREM sequence is obtained from the specimen from the test subject.

### (Item 22)

The method according to any one of the preceding items, in which the reference TREM sequence is obtained from an existing database.

### (Item 23)

The method according to any one of the preceding items, in which the reference TREM sequence contains an artificial peptide.

### (Item 24)

The method according to any one of the preceding items, further including a step of comparing the sequence of the BCR or TCR clone with any reference sequence to analyze whether an identical TREM sequence is present.

### (Item 25)

The method according to any one of the preceding items, in which the any reference sequence includes a reference sequence unique to the physiological state.

### (Item 26)

The method according to any one of the preceding items, including a step of determining the about 9-mer and/or about 15-mer amino acid sequence with a common TREM sequence between the sequence of the BCR or TCR clone and the reference sequence, as a candidate T-cell antigen epitope to be presented to T cells.

### (Item 27)

The method according to any one of the preceding items, in which the candidate T-cell antigen epitope is determined by evaluating identity between the repertoire specific TREM sequence and the reference TREM sequence, extracting the repertoire specific TREM sequence having TREM with identity and/or an about 9-mer or about 15-mer peptide sequence in the reference TREM sequence, and calculating HLA (MHC) binding affinity of the extracted peptide sequence. (Item 28)

The method according to any one of the preceding items, including a step of extracting a reference sequence peptide in which the TREM matches with the BCR repertoire and/or TCR repertoire.

### (Item 29)

The method according to any one of the preceding items, further including a step of narrowing down peptides based on the HLA type to determine an HLA-specific peptide with a common TREM between the BCR and/or TCR repertoire and the reference TREM sequence.

### (Item 30)

The method according to any one of the preceding items, in which the step of determining whether a peptide sequence is an epitope sequence includes a step of evaluating binding affinity for a plurality of different MHCs.

### (Item A1)

A method for producing an antigen containing an epitope sequence associated with a specific physiological state of a test subject,
the method including the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence;
E) determining, based on the repertoire-reference common specific TREM sequence, whether a peptide sequence including the repertoire-reference common specific TREM sequence is an epitope sequence associated with the specific physiological state of the test subject; and
F) generating a peptide containing the epitope sequence.

### (Item A2)

The method according to the above item, in which the step of obtaining BCR repertoire and/or TCR repertoire data includes a step of obtaining a full-length sequence of BCR and/or TCR.

### (Item A3)

The method according to any one of the preceding items, further including a step of determining a full-length sequence of an immunoglobulin variable section of BCR or TCR clone specific to the physiological state.

### (Item A4)

The method according to any one of the preceding items, in which the BCR repertoire and/or TCR repertoire data is obtained by analyzing a specimen obtained from the test subject.

### (Item A5)

The method according to any one of the preceding items, in which the BCR repertoire and/or TCR repertoire data is obtained from an existing database.

### (Item A6)

The method according to any one of the preceding items, further including a step of analyzing BCR and/or TCR repertoire in the specimen from the test subject to determine a clone specifically reacting to the specific physiological state.

### (Item A7)

The method according to any one of the preceding items, further including a step of determining a gene region in which the repertoire-specific TREM sequence is present.

### (Item A8)

The method according to any one of the preceding items, in which the gene region is selected from the group consisting of an L region, a V region, a D region, a J region or a C region, FR1, 2 or 3, and CDR1, 2 or 3.

### (Item A9)

The method according to any one of the preceding items, further including a step of producing an about 8- to about 10-mer and/or about 13- to about 17-mer amino acid sequence from the sequence of BCR or TCR clone specific to the physiological state and extracting the TREM sequence.

### (Item A10)

The method according to any one of the preceding items, further including a step of producing an about 9- and/or about 15-mer amino acid sequence from the sequence of BCR or TCR clone specific to the physiological state and extracting the TREM sequence.

### (Item A11)

The method according to any one of the preceding items, in which, in a 9-mer amino acid sequence, the TREM sequence includes at least one of positions 4, 5, 6, 7 and 8 from the N-terminus of the 9-mer amino acid sequence.

### (Item A12)

The method according to any one of the preceding items, in which, in a 15-mer amino acid sequence, the TREM sequence includes at least one of positions 5, 6, 8, 10 and 11, or at least one of positions 3, 6, 8, 10 and 11 from the N-terminus of the 15-mer amino acid sequence.

### (Item A13)

The method according to any one of the preceding items, further including a step of determining MHC I/II binding affinity of an about 8- to about 10-mer and/or about 13- to about 17-mer amino acid sequence including the TREM sequence.

### (Item A14)

The method according to any one of the preceding items, further including a step of specifying a variant TREM.

### (Item A15)

The method according to any one of the preceding items, further including a step of evaluating and specifying a peptide with B-cell epitope properties.

### (Item A16)

The method according to any one of the preceding items, in which the step of determining whether a peptide sequence is an epitope sequence associated with the specific physiological state includes testing HLA (MHC) binding affinity of the peptide sequence including the repertoire-reference common specific TREM sequence to determine whether the peptide sequence is an epitope sequence associated with the specific physiological state of the test subject.

### (Item A17)

The method according to any one of the preceding items, in which the method includes, in the test of HLA (MHC) binding affinity, a step of selecting an epitope sequence with an IC50 of about 5000 or less as the epitope sequence associated with the specific physiological state of the test subject.

### (Item A18)

The method according to any one of the preceding items, in which the test of HLA (MHC) binding affinity includes estimating the HLA (MHC) binding affinity.

### (Item A19)

The method according to any one of the preceding items, in which the reference TREM sequence is a TREM sequence of one selected from the group consisting of a cancer-associated protein, an infection-associated protein, an autoimmunity-associated protein, an allergy-associated protein, a microbiome and an alloreaction/xenoreaction-associated protein.

### (Item A20)

The method according to any one of the preceding items, in which the reference TREM sequence is a TREM sequence of one selected from the group consisting of a variant protein, a neoantigen, a tumor protein, a proteome in an inflamed area, a virus-associated protein, a bacteria-associated protein, a fungus-associated protein, an alloantigen, a xenoantigen, an allergen protein and a microbiome.

### (Item A21)

The method according to any one of the preceding items, in which the reference TREM sequence is obtained from the specimen from the test subject.

### (Item A22)

The method according to any one of the preceding items, in which the reference TREM sequence is obtained from an existing database.

### (Item A23)

The method according to any one of the preceding items, in which the reference TREM sequence contains an artificial peptide.

### (Item A24)

The method according to any one of the preceding items, further including a step of comparing the sequence of the BCR or TCR clone with any reference sequence to analyze whether an identical TREM sequence is present.

### (Item A25)

The method according to any one of the preceding items, in which the any reference sequence includes a reference sequence unique to the physiological state.

### (Item A26)

The method according to any one of the preceding items, including a step of determining the about 9-mer and/or about 15-mer amino acid sequence with a common TREM sequence between the sequence of the BCR or TCR clone and the reference sequence, as a candidate T-cell antigen epitope to be presented to T cells.

### (Item A27)

The method according to any one of the preceding items, in which the candidate T-cell antigen epitope is determined by evaluating identity between the repertoire specific TREM sequence and the reference TREM sequence, extracting the repertoire specific TREM sequence having TREM with identity and/or an about 9-mer or about 15-mer peptide sequence in the reference TREM sequence, and calculating HLA (MHC) binding affinity of the extracted peptide sequence. (Item A28)

The method according to any one of the preceding items, including a step of extracting a reference sequence peptide in which the TREM matches with the BCR repertoire and/or TCR repertoire.

### (Item A29)

The method according to any one of the preceding items, further including a step of narrowing down peptides based on the HLA type to determine an HLA-specific peptide with a common TREM between the BCR and/or TCR repertoire and the reference TREM sequence.

### (Item A30)

The method according to any one of the preceding items, in which the step of determining whether a peptide sequence is an epitope sequence includes a step of evaluating binding affinity for a plurality of different MHCs.

### (Item B1)

A program for causing a computer to execute processing of a method for identifying an epitope sequence associated with a specific physiological state of a test subject,
the method including the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence; and
E) causing the computer to determine, based on the repertoire-reference common specific TREM sequence, whether a peptide sequence including the repertoire-reference common specific TREM sequence is an epitope sequence associated with the specific physiological state of the test subject.

### (Item B2)

A recording medium that stores a program for causing a computer to execute processing of a method for identifying an epitope sequence associated with a specific physiological state of a test subject,
the method including the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence; and
E) causing the computer to determine, based on the repertoire-reference common specific TREM sequence, whether a peptide sequence including the repertoire-reference common specific TREM sequence is an epitope sequence associated with the specific physiological state of the test subject.

### (Item B3)

A system for identifying an epitope sequence associated with a specific physiological state of a test subject,
the system including:
A) means for obtaining BCR and/or TCR repertoire data associated with the specific physiological state of the test subject;
B) means for specifying a TREM sequence (repertoire specific TREM sequence) from the BCR and/or TCR repertoire data;
C) means for obtaining a reference TREM sequence associated with the test subject;
D) means for specifying a repertoire-reference common specific TREM sequence from the reference TREM sequence and the repertoire specific TREM sequence; and
E) means for determining, based on the repertoire-reference common specific TREM sequence, whether a peptide sequence including the repertoire-reference common specific TREM sequence is an epitope sequence associated with the specific physiological state of the test subject.

### (Item C1)

A computer program for causing a computer to execute processing of a method for producing an antigen containing an epitope sequence associated with a specific physiological state of a test subject,
the method including the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence;
E) causing the computer to determine, based on the repertoire-reference common specific TREM sequence, whether a peptide sequence including the repertoire-reference common specific TREM sequence is an epitope sequence associated with the specific physiological state of the test subject; and
F) causing the computer to generate a peptide containing the epitope sequence.

### (Item C2)

A recording medium that stores a computer program for causing a computer to execute processing of a method for producing an antigen containing an epitope sequence associated with a specific physiological state of a test subject,
the method including the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence;
E) causing the computer to determine, based on the repertoire-reference common specific TREM sequence, whether a peptide sequence including the repertoire-reference common specific TREM sequence is an epitope sequence associated with the specific physiological state of the test subject; and
F) causing the computer to generate a peptide containing the epitope sequence.

### (Item C3)

A system for producing an antigen containing an epitope sequence associated with a specific physiological state of a test subject,
the system including:
A) means for obtaining BCR and/or TCR repertoire data associated with the specific physiological state of the test subject;
B) means for specifying a TREM sequence (repertoire specific TREM sequence) from the BCR and/or TCR repertoire data;
C) means for obtaining a reference TREM sequence associated with the test subject;
D) means for specifying a repertoire-reference common specific TREM sequence from the reference TREM sequence and the repertoire specific TREM sequence;
E) means for determining, based on the repertoire-reference common specific TREM sequence, whether a peptide sequence including the repertoire-reference common specific TREM sequence is an epitope sequence associated with the specific physiological state of the test subject; and
F) means for generating a peptide containing the epitope sequence.

### (Item D1)

A method for diagnosing a disease history, physiological state or pathological condition of a test subject by analyzing a repertoire-reference common specific TREM sequence obtained by the method described herein, or a part thereof.

### (Item D2)

A method for synthesizing a peptide using a repertoire-reference common specific TREM sequence obtained by the method described herein, or a part thereof; the method including: isolating, identifying or creating reactive T cells; or producing a recognizable antibody; and treating a disease in a test subject using the peptide, the reactive T cells, or the antibody.

### (Item D3)

A peptide including an epitope sequence identified by the method described in any one of the preceding items.

### (Item D4)

An antigen peptide produced by the method described in any one of the preceding items.

### (Item D5)

An antigen peptide associated with a specific physiological state of a test subject, including an amino acid sequence described herein or a portion thereof, or an amino acid sequence identified by the method described herein or a portion thereof.

### (Item D6)

An antibody or antigen-binding fragment thereof that specifically binds to an amino acid sequence described herein or a portion thereof, or an amino acid sequence identified by the method described herein or a portion thereof.

### (Item D7)

The antigen peptide according to the above item, in which the amino acid sequence includes a sequence set forth in any one of SEQ ID NOs: 1 to 485 or any combination thereof.

### (Item D8)

A peptide including a TREM sequence described herein or a portion thereof, or a TREM sequence identified by the method described herein or a portion thereof.

### (Item D9)

The peptide according to any one of the preceding items, in which the TREM sequence is a repertoire specific TREM sequence, a reference TREM sequence or a repertoire-reference common specific TREM sequence, or a combination thereof.

### (Item D10)

The peptide according to any one of the preceding items, in which the TREM sequence includes a sequence set forth in any one of SEQ ID NOs: 1 to 485 or any combination thereof.

### (Item E0)

A peptide including an amino acid sequence of a dengue virus antigen described herein.

### (Item E1)

A peptide including the amino acid sequence KRLEPSWASVKKDLI of the dengue virus antigen. (Item E2)
A peptide including the amino acid sequence HQLWATLLSLTFIKT of the dengue virus antigen. (Item E3)
A peptide including the amino acid sequence GLLGRSQVGVGVFQE of the dengue virus antigen. (Item E4)
A peptide including the amino acid sequence EDTMTYKCPRITETE of the dengue virus antigen. (Item E5)
A peptide including the amino acid sequence VTVHTGDQHQVGNES of the dengue virus antigen. (Item E6)
A peptide including the amino acid sequence QLGQIMLLILCTSQI of the dengue virus antigen. (Item E7)
A peptide including the amino acid sequence QDEKGVTQNGRLITA of the dengue virus antigen. (Item E8)
A peptide including the amino acid sequence EVTAKWLWGFLSRNK of dengue virus antigen. (Item E9)
A peptide including the amino acid sequence IIFILLMLVTPSMAM of the dengue virus antigen. (Item E10)
A peptide including the amino acid sequence GGVFTSVGKLVHQIF of the dengue virus antigen. (Item E11)
A peptide including the amino acid sequence RGPSLRTTTVTGKII of the dengue virus antigen. (Item E12)
A peptide including the amino acid sequence GIVSILLSSLLKNDV of the dengue virus antigen. (Item F1)
A peptide library including two or more of the amino acid sequences or peptides described in the above item or herein.

### (Item F2)

A peptide library including two or more peptides containing epitope sequences identified by the method described in item 1.

### (Item F3)

A peptide library including two or more antigen peptides produced by the method described in item A1.

### (Item F4)

A peptide library including two or more peptides containing the amino acid sequence of the dengue virus antigen described herein.

### (Item F5)

A method for generating a peptide library of peptides containing an epitope sequence associated with a specific physiological state of a test subject,
the method including the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence;
E) determining, based on the repertoire-reference common specific TREM sequence, whether a peptide sequence including the repertoire-reference common specific TREM sequence is an epitope sequence associated with the specific physiological state of the test subject;
F) generating a peptide containing the epitope sequence; and
G) combining two or more of the peptides generated by the step F to generate a peptide library.

### (Item G1)

A peptide including an epitope sequence identified by the method described in any one of the preceding items, for diagnosing a disease history, physiological state or pathological condition of a test subject.

### (Item G2)

A pharmaceutical composition for diagnosing a disease history, physiological state or pathological condition of a test subject, in which the composition contains a peptide including an epitope sequence identified by the method described in any one of the preceding items.

### (Item G3)

Use of a peptide including an epitope sequence identified by the method described in any one of the preceding items, for producing a pharmaceutical composition for diagnosing a disease history, physiological state or pathological condition of a test subject.

### (Item H1)

A peptide including an epitope sequence identified by the method described in any one of the preceding items, for treating a disease in a test subject.

### (Item H2)

A pharmaceutical composition for treating a disease in a test subject, in which the composition contains a peptide including an epitope sequence identified by the method described in any one of the preceding items.

### (Item H3)

Use of a peptide including an epitope sequence identified by the method described in any one of the preceding items, for producing a pharmaceutical composition for treating a disease in a test subject.

### (Item I1)

An antigen peptide produced by the method described in any one of the preceding items, for diagnosing a disease history, physiological state or pathological condition of a test subject.

### (Item I2)

A pharmaceutical composition for diagnosing a disease history, physiological state or pathological condition of a test subject, in which the composition contains an antigenic peptide produced by the method described in any one of the preceding items.

### (Item I3)

Use of an antigen peptide produced by the method described in to any one of the preceding items, for producing a pharmaceutical composition for diagnosing a disease history, physiological state or pathological condition of a test subject.

### (Item J1)

An antigenic peptide produced by the method described in any one of the preceding items, for treating a disease in a test subject.

### (Item H2)

A pharmaceutical composition for treating a disease in a test subject, in which the pharmaceutical composition contains an antigenic peptide produced by the method described in any one of the preceding items.

### (Item H3)

Use of an antigen peptide produced by the method described in any one of the preceding items, for producing a pharmaceutical composition for treating a disease in a test subject.

In the present disclosure, it is intended that the one or more features described above may be further combined, in addition to the specified combination. It should be noted that further embodiments and advantages of the present disclosure will be appreciated by those skilled in the art upon reading and understanding the following detailed description as needed.

Note that features and remarkable actions and effects of the present disclosure other than those described above will be apparent to those skilled in the art with reference to the following embodiments and drawings of the invention. Advantageous Effects of Invention

According to the method of the present disclosure, an epitope associated with a specific physiological state can be identified. Further, the epitope is a specific epitope and thus can also be evaluated as an epitope derived from a specific immune response. Furthermore, the method of the present disclosure can be used for antigen prediction. As described above, the method of the present disclosure enables a physiological state to be predicted, and can be applied in the development of various medical techniques (e.g. drug discovery and diagnostic agent development).

In addition, the method of the present disclosure can also be applied to elucidation of mechanisms of various diseases and pathological conditions and diagnosis thereof.

### Brief Description of Drawings

FIG. 1 is an outline diagram of an antigen prediction technique according to one embodiment of the present invention.
FIG. 2A is a schematic diagram illustrating an analysis flow of full-length analysis according to one embodiment of the present invention.
FIG. 2B is a schematic diagram illustrating an analysis flow of antigen prediction analysis according to one embodiment of the present invention.
FIG. 3 shows a graph and a photograph of results of ELISPOT assay in one embodiment of the present invention. The graph shows the results for all eight peptides (cell 1 and cell 2). The spot film shows assay results of peptides of #2 (left: no peptide is added, right: peptide is added).
FIG. 4 is a schematic diagram showing a comparison between the method of the present disclosure and a method using classification according to the frequency of TREM sequence use.
FIG. 5 is a schematic diagram illustrating a motif of a T cell epitope.
FIG. 6 is a schematic diagram illustrating a motif of a T cell epitope.
FIG. 7 is a flowchart showing a processing flow of a method for analyzing a TCR or BCR repertoire.
FIG. 8 is a schematic diagram illustrating an example of a configuration of a computer system 100 for realizing the present disclosure.
FIG. 9 illustrates a block diagram of a system according to one embodiment of the present disclosure.
FIG. 10 is a flowchart showing a processing flow of a method for analyzing the full-length of a clone from which TCR or BCR repertoire data is obtained.
FIG. 11 is a flowchart showing a processing flow of a method for performing antigen prediction analysis.
FIG. 12 is a schematic diagram illustrating a bio (in vitro) application example using the method of the present disclosure.
FIG. 13A is a schematic diagram illustrating a drug discovery application example (autoimmune disease) using the method of the present disclosure.
FIG. 13B is a schematic diagram illustrating a drug discovery application example (allograft) using the method of the present disclosure.
FIG. 14 is a schematic diagram illustrating a diagnostic application example using the method of the present disclosure.
FIG. 15 is a schematic diagram illustrating a therapeutic application example using the method of the present disclosure. Description of Embodiments

Hereinafter, the present disclosure will be described while showing the best mode of the present disclosure. Throughout the entire specification, a singular expression should be understood as encompassing the concept thereof in the plural form, unless specifically noted otherwise. Thus, singular articles (e.g. "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific-technical terms that are used herein have the same meaning as the general understanding of those skilled in the art to which the present disclosure pertains. In case of a contradiction, the present specification (including the definitions) takes precedence.

The definitions of the terms and/or the detailed basic technology that are particularly used herein are described hereinafter as appropriate.

As used herein, the term "about" means ± 10% of the subsequent numerical value.

As used herein, "physiological state" refers to a physical state of any subject, and includes the presence or absence of a disease, physical fatigue, physical stress or the like, or the degree, type or the like thereof. Examples of the physiological state include any state in a test subject, and represents, for example, the presence or absence of infections such as viruses, bacteria or fungi, cancer (including a variant protein, a neoantigen and a tumor protein), and autoimmune disease (proteome information such as inflamed area), allograft such as alloantigen, microbiome state observed in gut microbiota, and the presence or absence of allergy such as allergen protein.

As used herein, "epitope" refers to a site recognized by the immune system, and can be generally represented by an amino acid sequence. Examples of the epitope includes a T cell epitope recognized by a T cell involved in the activity control of a B cell or another cell responsible for producing antibodies in vivo and the elimination of an abnormal cell (cytotoxicity), and a B cell epitope recognized by the B cell and serving as an antibody binding site. In these epitopes, the B cell epitope refers to a part of the antigen recognized by the antibody, and refers to an antigenic determinant in the molecule, i.e. a part of the molecule recognized by the immune system (e.g. antibodies). For example, the B cell epitope is a three-dimensional site on the antigen recognized by the antibody. The epitope generally consists of chemically active surface groups of molecules such as amino acids or sugar side chains and generally have specific three-dimensional structural characteristics and specific charging characteristics. There are conformational and non-conformational epitopes. The epitope preferably includes a continuous or discontinuous portion, and is about 5 to about 100, preferably about 5 to about 50, more preferably about 8 to about 30, and most preferably about 10 to about 25 amino acids in length. For example, the epitope may preferably be about 8, about 9, about 10, about 11, about 12, about 13, about 14, about 15, about 16, about 17, about 18, about 19, about 20, about 21, about 22, about 23, about 24 or about 25 amino acids in length.

The T cell epitope requires a short peptide that binds to a class I or class II MHC molecule to form a ternary complex (MHC α, β or β-2 microglobulin and peptides). This ternary complex can be recognized by a T cell having a corresponding T cell receptor that binds with moderate affinity to the MHC/peptide complex. A peptide that binds to an MHC class I molecule is generally about 8 to about 15 amino acids in length, and most typically about 9 amino acids in length. Further, a peptide that binds to an MHC class II molecule is generally about 13 to about 25 amino acids in length, and most typically about 15 amino acids in length.

As used herein, "antibody" refers to a glycoprotein containing at least two heavy (H) chains and two light (L) chains interconnected by disulfide bonds, and includes any molecule containing an antigen-binding portion thereof. Examples of the "antibody" include human antibodies, humanized antibodies, chimeric antibodies, single-chain antibodies, e.g. scFvs, and antigen-binding antibody fragments, e.g. monoclonal antibodies including Fab and Fab' fragments, as well as antibody fragments or derivatives, and also include all recombinant forms of antibodies, non-glycosylated antibodies, and any antigen-binding antibody fragments and derivatives described herein. Each of the heavy chains is composed of a heavy chain variable region and a heavy chain constant region, and each of the light chains is composed of a light chain variable region and a light chain constant region. The VH and VL regions can be further subdivided into hypervariable regions called "complementarity determining regions (CDR)", interspersed with regions that are more conserved, called "framework regions (FR)". Each of the VH and VL regions is composed of three CDRs and four FRs arranged from amino- to carboxy-termini in the order FR1, CDR1, FR2, CDR2, FR3, CDR3 and FR4. The variable regions of the heavy and light chains contain a binding domain that interacts with an antigen.

As used herein, "T cell receptor (TCR)" also refers to a T cell receptor, a T cell antigen receptor or a T cell antigen receptor, and refers to a receptor (receptor) expressed on a cell membrane of a T cell responsible for the immune system, and recognizes an antigen. The α, β, γ, and δ chains are present and constitute a dimer of αβ or γδ. The TCR composed of the former combination is called αβTCR, the TCR composed of the latter combination is called γδTCR. T cells having the respective TCRs are called αβT cells and γδT cells, respectively. The T cells are structurally very similar to Fab fragments of antibodies produced by B cells and recognizes antigen molecules bound to MHC molecules. Since the TCR gene possessed by mature T cells has undergone gene rearrangement, one individual has a TCR rich in diversity, and can recognize various antigens. The TCR further binds to an invariable CD3 molecule present in the cell membrane to form a complex. CD3 has an amino acid sequence called immunoreceptor tyrosine-based activation motif (ITAM) in the intracellular region, and this motif is considered to be involved in intracellular signaling. Each TCR chain is composed of a variable section (V) and a constant section (C), and the constant section has a short cytoplasmic portion penetrating the cell membrane. The variable section is present extracellularly and binds to an antigen-MHC complex. The variable section has three regions called hypervariable sections or complementarity determining regions (CDR), and these regions bind to the antigen-MHC complex. The three CDRs are referred to as CDR1, CDR2 and CDR3, respectively. In the case of a TCR, it is considered that CDR1 and CDR2 bind to an MHC, and CDR3 binds to an antigen. The gene rearrangement of the TCR is similar to the process of a B cell receptor known as an immunoglobulin. In the gene rearrangement of αβTCR, VDJ rearrangement of the β chain is first performed, followed by VJ rearrangement of the α chain. Since the gene of the δ chain is deleted from the chromosome when performing the rearrangement of the α chain, T cells having the αβTCR do not simultaneously have the γδTCR. Conversely, in T cells having the γδTCR, a signal through this TCR suppresses the expression of the β chain, and thus the T cells having the γδTCR do not simultaneously have the αβTCR.

As used herein, "B cell receptor (BCR)" is also called a B cell receptor, B cell antigen receptor or B cell antigen receptor, and refers to those composed of an Igα/Igβ (CD79a/CD79b) heterodimer (α/β) conjugated with a membrane-bound immunoglobulin (mIg) or a surface immunoglobulin (sIg) molecule. An mIg subunit binds to an antigen to induce aggregation of the receptors, while an α/β subunit transmits a signal to the inside of a cell. BCRs, when aggregated, are understood to quickly activate Lyn, Blk and Fyn of Src family kinases, similarly to Syk and Btk of tyrosine kinases. Results greatly differ depending on the complexity of BCR signaling, the results including survival, resistance (anergy; lack of hypersensitivity reaction to antigen) or apoptosis, cell division, differentiation into antibody-producing cell or memory B cell and the like. Several hundred million types of T cells with a different TCR variable region sequence are produced and several hundred million types of B cells with a different BCR (or antibody) variable region sequence are produced. Individual sequences of TCRs and BCRs vary due to an introduced mutation or rearrangement of the genomic sequence. Thus, it is possible to obtain a clue for antigen specificity of a T cell or a B cell by determining a genomic sequence of TCR/BCR or a sequence of an mRNA (cDNA).

As used herein, "V region" refers to a variable section (V) region of a variable region of a TCR chain or a BCR chain.

As used herein, "D region" refers to a D region of a variable region of a TCR chain or a BCR chain.

As used herein, "J region" refers to a J region of a variable region of a TCR chain or a BCR chain.

As used herein, the term "C region" refers to a constant section (C) region of a TCR chain or a BCR chain.

As used herein, "repertoire" refers to a collection of TCRs or BCRs, or fragments thereof, and typically refers to a collection of V(D)J regions created in any manner by gene rearrangement in a TCR or BCR. Terms such as TCR repertoire and BCR repertoire are used, which are also called, for example, T cell repertoire or B cell repertoire in some cases. For example, "T cell repertoire" refers to a collection of lymphocytes characterized by expression of a T cell receptor (TCR) serving an important role in antigen recognition. A change in a T cell repertoire provides a significant indicator of an immune status in a physiological state and disease state. Thus, a T cell repertoire has been analyzed to identify an antigen specific T cell involved in the pathology of a disease and diagnosis of abnormality in T lymphocytes. Comparison of the variable region usage by fluorescence-activated cell sorter analysis which uses a larger panel of a TCR variable region specific antibody (van den Beemd R et al. (2000) Cytometry 40: 336 -345; MacIsaac C et al. (2003) J Immunol Methods 283: 9-15; Tembhare P et al. (2011) Am J Clin Pathol 135: 890-900; Langerak AWet al. (2001) Blood 98: 165-173), by polymerase chain reaction (PCR) using multiple primers (Rebai N et al. (1994) ProcNatl Acad Sci USA 91: 1529-1533), or by enzyme-linked immunosorbent assay based on PCR (Matsutani T et al. (1997) Hum Immunol 56: 57-69; Matsutani T et al. (2000) Br J Haematol 109: 759-769) have been extensively used to detect a change in a T cell repertoire. Analysis of a chain length distribution known as CDR3 spectrotyping is based on the addition of a non-template nucleotide in a V-(D)-J region and has been used to assess clonality and diversity of T cells (Matsutani T et al. (2007) Mol Immunol 44: 2378-2387; Matsutani T et al. (2011) Mol Immunol 48:623-629). To further identify the antigen specificity of a T cell, PCR cloning of a TCR clone type and subsequent sequencing of an antigen recognition region and CDR3 are required.

A used herein, "MAM (MHC agretope motif)" refers to a motif in a peptide (e.g. an antigen epitope) that is recognized and bound to a peptide accommodating groove of MHC. The motif can be determined with reference to Rudolph MG, et al. Annu Rev Immunol. 2006; 24: 419-66., Moise L, et al. Hum Vaccin Immunother. 2013 Jul; 9 (7): 1577-86., Calls JJ, et al. PLoS Comput Biol. 2012; 8 (3): e1002412. In addition, "agretope" refers to a site on an antigen recognized by an MHC molecule (Schwartz RH. et al. Annu Rev Immunol. 1985; 3: 237-61.).

As used herein, "TREM (TCR-recognized epitope motif)" refers to a moiety other than MAM in a peptide (e.g. an antigen epitope), and refers to a motif recognized by T cells. Amino acid positions per motif are described, for example, in Rudolph et al. (2006) Annu. Rev. Immunol. 2006. 24: 419-466.

As used herein, "repertoire specific TREM sequence" refers to a TREM sequence included in a repertoire (TCR or BCR) associated with a specific physiological state and is identified as an about 5-mer sequence extracted from about 8- to about 10-mer and/or about 13- to about 17-mer amino acid sequences generated from BCR repertoire and/or TCR repertoire data. A peptide of a length specific for HLA (MHC) I or HLA (MHC) II, including a repertoire specific TREM sequence, is antigen presented and has a function of inducing a T cell recognizing it. When the repertoire specific TREM sequence exhibits a TREM sequence associated with various physiological states, the sequence can control the activity of T cells that specifically respond to the physiological states.

As used herein, "reference TREM sequence" is a TREM sequence contained in a reference sequence referred to as being associated with various physiological states, and the TREM sequence specific to various physiological states is advantageously utilized. Examples of the reference TREM sequence may include, but are not limited to, TREM sequences of a cancer-associated protein, an infection-associated protein, an autoimmunity-associated protein, an allergy-associated protein, a microbiome, a transplantation alloantigen and the like. Specific examples thereof include TREM sequences of a variant protein, a neoantigen, a tumor protein, a proteome in an inflamed area, a virus-associated protein, a bacteria-associated protein, a fungus-associated protein, an alloantigen, a xenoantigen, an allergen protein, a microbiome and the like.

As used herein, "repertoire-reference common specific TREM sequence" is an about 5-mer TREM sequence common between a repertoire TREM sequence and a reference TREM sequence. For an about 9-mer or 15-mer peptide sequence including a repertoire-reference common specific TREM sequence, the HLA (MHC) binding affinity can be tested to determine whether it is an epitope sequence associated with a specific physiological state of a test subject. Whether "common" or not can be determined by confirming that about 5-mer TREM sequences are the same.

As used herein, "peptide", "protein", "polypeptide" and "oligopeptide" are used to have the same meaning, and the "peptide" refers to a series of amino acid residues of any length that are generally joined together by a peptide bond between the amino and carbonyl groups of adjacent amino acids. Such a peptide may be a straight chain, branched or cyclic. As used herein, "amino acid" may be a natural or non-natural or altered amino acid, as long as the objective of the present disclosure is met. The term may also encompass those assembled into a complex of a plurality of polypeptide chains. The term also encompasses natural or artificially altered amino acid polymers. Examples of such an alteration include disulfide bond formation, glycosylation, lipidation, acetylation, phosphorylation or any other manipulation or alteration (e.g. conjugation with a labeling component). The definition also encompasses, for example, a polypeptide including one or more analogs of an amino acid (e.g. including a non-natural amino acid, etc.), a peptide-like compound (e.g. peptoid), and other known alterations in the art.

As used herein, "polynucleotide", "oligonucleotide" and "nucleic acid" are used to have the same meaning and refer to a nucleotide polymer of any length. The term also encompasses "oligonucleotide derivative" and "polynucleotide derivative". "Oligonucleotide derivative" or "polynucleotide derivative" refers to an oligonucleotide or a polynucleotide, which has a bond between nucleotides that is not normal or includes a derivative of a nucleotide. Specific examples of such an oligonucleotide include 2'-O-methyl-ribonucleotide, oligonucleotide derivative with a phosphodiester bond in an oligonucleotide converted to a phosphorothioate bond, oligonucleotide derivative with a phosphodiester bond in an oligonucleotide converted to an N3'-P5' phosphoramidate bond, oligonucleotide derivative with a ribose and phosphodiester bond in an oligonucleotide converted to a peptide nucleic acid bond, oligonucleotide derivative with uracil in an oligonucleotide substituted with C-5 propynyl uracil, oligonucleotide derivative with uracil in an oligonucleotide substituted with C-5 thiazole uracil, oligonucleotide derivative with cytosine in an oligonucleotide substituted with C-5 propynyl cytosine, oligonucleotide derivative with cytosine in an oligonucleotide substituted with phenoxazine-modified cytosine, oligonucleotide derivative with ribose in a DNA substituted with 2'-O-propylribose, oligonucleotide derivative with ribose in an oligonucleotide substituted with 2'-methoxyethoxyribose and the like. Unless specifically noted otherwise, a specific nucleic acid sequence is further intended to encompass conservatively altered variants (e.g. degenerate codon substituted form) and complementary sequences thereof in addition to the explicitly shown sequences. Specifically, a degenerate codon substituted form can be obtained by creating a sequence in which the third position of one or more selected (or all) codons is substituted with a mixed base and/or deoxyinosine residue (Batzer et al., Nucleic Acid Res. 19: 5081 (1991); Ohtsuka et al., J.Biol. Chem. 260: 2605-2608 (1985); Rossolini et al., Mol. Cell. Probes 8: 91-98 (1994)). As used herein, "nucleic acid" is interchangeably used with gene, cDNA, mRNA, oligonucleotide, and polynucleotide. As used herein, "nucleotide" may be natural or non-natural.

As used herein, "gene" refers to a factor that defines a genotype. A "gene" may refer to a "polynucleotide", "oligonucleotide" or "nucleic acid".

In addition to the identified peptides, a variant thereof may be used in the present disclosure. Examples of such a variant include, but are not limited to, those that are homologous to the identified peptide.

As used herein, "homology" of genes refers to the level of identity of two or more gene sequences to one another. In general, "having homology" refers to having a high level of identity or similarity. Therefore, a high level of homology of two genes results in a higher level of identity or similarity of sequences thereof. It is possible to examine whether 2 types of genes are homologous by direct comparison of sequences or by hybridization under stringent conditions for nucleic acids. When directly comparing two gene sequences, the genes are homologous typically when DNA sequences between the gene sequences are at least about 50% identical, preferably at least about 70% identical, more preferably at least about 80%, about 90%, about 95%, about 96%, about 97%, about 98% or about 99% identical. Thus, as used herein, "homolog" or "homologous gene product" refers to a protein in another species, preferably a mammal, which exerts the same biological function as a protein constituent element of a complex further described herein. Such a homolog is also called "ortholog gene product". It is understood that such a homolog, homologous gene product, ortholog gene product, and the like can also be used as long as they align with the objective of the present disclosure.

An amino acid may be mentioned herein by a commonly known three letter symbol thereof or a one letter symbol recommended by IUPAC-IUB Biochemical Nomenclature Commission. Similarly, a nucleotide may be mentioned by a commonly recognized one letter code. Herein, comparison of similarity, identity and homology of amino acid sequences and base sequences is performed by comparing using sequences to be compared as a string of letters. Alternatively, the comparison can be calculated by using a default parameter with a sequence analysis tool BLAST. Identity can be searched by using, for example, NCBI's BLAST 2.2.28 (published on Apr. 2, 2013). As the value of identity herein, a value obtained by using the above-described BLAST to align sequences under default conditions can be used. However, when a higher value is output by changing a parameter, the highest value is considered the value of identity. When identity is assessed in a plurality of regions, the highest value thereamong is considered the value of identity. Similarity is a numerical value calculated based on similar amino acids, in addition to identity.

In one embodiment of the present disclosure, "several" may be, for example, 10, 8, 6, 5, 4, 3 or 2, or may be equal to or less than any one of these values. It is known that a polypeptide with a deletion, addition, insertion or other amino acid substitutions of one to several amino acid residues maintains its biological activity (Market al., ProcNatl Acad Sci USA. 1984 Sep;81(18): 5662-5666., Zoller et al., Nucleic Acids Res. 1982 Oct 25; 10(20): 6487-6500., Wang et al., Science. 1984 Jun 29; 224 (4656): 1431-1433.). An antibody with a deletion or the like can be produced, for example, by site-directed mutagenesis, random mutagenesis, biopanning using an antibody phage library or the like. For example, KOD-Plus-Mutagenesis Kit (TOYOBO CO., LTD.) can be used for site-directed mutagenesis. An antibody with the same activity as the wildtype can be selected from mutant antibodies introduced with a deletion or the like by performing various characterizations such as FACS analysis or ELISA.

In one embodiment of the present disclosure, "90% or greater" may be, for example, 90, 95, 96, 97, 98, 99 or 100% or greater, or may be within the range of any two of these values. For the above-described "homology", the percentage of the number of homologous amino acids in two or a plurality of amino acid sequences may be calculated in accordance with a known method in the art. Before calculating the percentage, amino acid sequences of a group of amino acid sequence to be compared are aligned. A space is introduced in a portion of amino acid sequences when necessary to maximize the percentage of the same amino acids. An alignment method, method of calculating the percentage, comparison method, and computer programs associated therewith have been well known in the art (e.g. BLAST and GENETYX). As used herein, "homology" can represent a value measured by BLAST of NCBI unless specifically noted otherwise. Blastp can be used in the default setting for an algorithm for comparing amino acid sequences with BLAST. Results of measurement are expressed in a numerical form as Positives or Identities.

As used herein, "polynucleotide that hybridizes under stringent conditions" refers to a well-known condition commonly used in the art. Such a polynucleotide can be obtained by using colony hybridization, plaque hybridization, southern blot hybridization or the like while using a polynucleotide selected from the polynucleotides of the present disclosure as a probe. Specifically, such a polynucleotide refers to a polynucleotide which can be identified by hybridization at 65°C in the presence of 0.7-1.0 M NaCl using a filter with immobilized DNA derived from a colony or a plaque, and then using a 0.1 to 2-fold concentration SSC (saline-sodium citrate) solution (composition of an SSC solution with 1-fold concentration is 150 mM sodium chloride and 15 mM sodium citrate) to wash the filter under the condition of 65°C. For "stringent conditions", for example, the following conditions can be adopted. (1) low ionic strength and a high temperature are used for washing (e.g. 0.015 M sodium chloride/0.0015 M sodium citrate/0.1% sodium dodecyl sulfate at 50°C), (2) a denaturing agent such as formamide is used in hybridization (e.g. 50% (v/v) formamide, 0.1% bovine serum albumin/0.1% ficoll/0.1% polyvinyl pyrrolidone/50 mM sodium phosphate buffer with a pH of 6.5, 750 mM sodium chloride, and 75 mM sodium citrate at 42°C), or (3) a solution containing 20% formamide, 5×SSC, 50 mM sodium phosphate (pH 7.6), 5×Denhardt's solution, 10% dextran sulfate, and 20 mg/ml denatured sheared salmon sperm DNA, is incubated overnight at 37°C and then a filter is washed with 1×SSC at about 37-50°C. The formamide concentration may be 50% or greater. Washing time may be 5, 15, 30, 60, 120 minutes or greater. A plurality of elements is considered to affect stringency in a hybridization reaction such as temperature, salt concentration and the like. Ausubel et al., Current Protocols in Molecular Biology, Wiley

Interscience Publishers, (1995) can be referred for details. "Highly stringent condition", for example, is 0.0015 M sodium chloride, 0.0015 M sodium citrate, 65-68°C, or 0.015 M sodium chloride, 0.0015 M sodium citrate, 50% formamide and 42°C. Hybridization is performed in accordance with the method described in experimental publications, such as Molecular Cloning 2nd ed., Current Protocols in Molecular Biology, Supplement 1-38, DNA Cloning 1: Core Techniques, A Practical Approach, Second Edition, Oxford University Press (1995). Here, a sequence including only an A sequence or only a T sequence is preferably excluded from a sequence that hybridizes under stringent conditions. A moderately stringent condition can be can be readily determined by those skilled in the art based on, for example, the length of a DNA and is shown in Sambrook et al., Molecular Cloning: A Laboratory Manual, Third Ed., Vol. 1, 7.42-7.45 Cold Spring Harbor Laboratory Press, 2001, including, for a nitrocellulose filters, hybridization conditions of a prewash solution of 1.0 mM EDTA (pH 8.0), 5×SSC, 0.5% SDS, and about 50% formamide and 2×SSC-6×SSC at about 40-50°C (or other similar hybridization solutions such as a Stark's solution in about 50% formamide at about 42°C) and washing conditions of 0.5×SSC, 0.1% SDS at about 60°C. Thus, the polypeptides used in the present disclosure encompass polypeptides encoded by a nucleic acid molecule that hybridizes under highly or moderately stringent conditions to a nucleic acid molecule encoding a polypeptide particularly described in the present disclosure.

As the peptide of the present disclosure or the like, purified and/or isolated peptide may be advantageously used. As used herein, "purified" substance or biological agent (e.g. nucleic acid, protein or the like) refers to a substance or biological agent having at least some of the agents that are naturally accompanied therewith removed. Thus, purity of a biological agent in a purified biological agent is higher than that of a normal condition of the biological agent (i.e. concentrated). The term "purified" as used herein preferably refers to the presence of at least 75 wt%, more preferably at least 85 wt%, still more preferably at least 95 wt%, and the most preferably at least 98 wt% of biological agents of the same type. A substance or biological agent used in the present disclosure is preferably a "purified" substance. An "isolated" substance or biological agent (e.g. nucleic acid, protein or the like) as used herein refers to a substance or biological agent having agents that are naturally accompanied therewith substantially removed. The term "isolated" as used herein varies depending on the objective. Thus, the term does not necessarily need to be represented by purity. However, when necessarily, the term refers to preferably the presence of at least 75 wt%, more preferably at least 85 wt%, still more preferably at least 95 wt% and the most preferably at least 98 wt% of biological agents of the same type. A substance used in the present disclosure is preferably an "isolated" substance or biological agent.

As used herein, "fragment" refers to a polypeptide or polynucleotide with a sequence length of 1 to n-1 with respect to the full length polypeptide or polynucleotide (with length n). The length of a fragment can be appropriately changed in accordance with the objective. Examples of the lower limit of such a length include 3, 4, 5, 6, 7, 8, 9, 10, 15, 20, 25, 30, 40, 50 and more amino acids for a polypeptide. Lengths represented by an integer that is not specifically listed herein (e.g. 11 and the like) also can be suitable as a lower limit. Further, examples of length include 5, 6, 7, 8, 9, 10, 15, 20, 25, 30, 40, 50, 75, 100 and more nucleotides for a polynucleotide. Lengths represented by an integer that is not specifically listed herein (e.g. 11 and the like) also can be suitable as a lower limit. As used herein, such a fragment is understood to be within the scope of the present disclosure, for example, when a full-length version functions as a marker or a target molecule, as along as the fragment itself also functions as a marker or a target molecule.

As used herein, "functional equivalent" refers to any entity having the same function of interest but a different structure relative to the original target entity. A functional equivalent can be found by searching a database or the like. As used herein, "search" refers to utilizing a certain nucleic acid base sequence electronically, biologically, or by another method to find another nucleic acid base sequence having a specific function and/or property. Electronic search can be performed by normal computer commands, treating the sequences as a string of letters and using an exact match or pattern match of the string. Alternatively, electronic search can also be performed using BLAST (Altschul et al., J. Mol. Biol. 215: 403-410 (1990)), FASTA (Pearson & Lipman, Proc. Natl. Acad. Sci., USA 85: 2444-2448 (1988)), Smith and Waterman method (Smith and Waterman, J. Mol. Biol. 147: 195-197 (1981)), Needleman and Wunsch method (Needleman and Wunsch, J. Mol. Biol. 48: 443-453 (1970)) and the like. Examples of biological search include, but are not limited to, stringent hybridization, a macroarray with a genomic DNA applied to a nylon membrane or the like or a microarray with a genomic DNA applied to a glass plate (microarray assay), PCR, in situ hybridization and the like. Herein, a gene used in the present disclosure is intended to include corresponding genes identified by such electronic search or biological search.

As a functional equivalent of the present disclosure, it is possible to use an amino acid sequence with one or more amino acid insertions, substitutions or deletions, or addition to one or both ends. As used herein, "one or more amino acid insertions, substitutions or deletions, or addition to one or both ends" in an amino acid sequence refers to an alteration with a substitution of multiple amino acids or the like to the extent that can occur naturally by a well-known technical method such as site-directed mutagenesis or a natural mutation. An altered amino acid sequence of a molecule can have, for example, 1-30, preferably 1-20, more preferably 1-9, still more preferably 1-5, and especially preferably 1-2 amino acid insertion, substitution or deletion or addition to one or both ends. Here, "conservative substitution" refers to a substitution of one or more amino acid residues with another chemically similar amino acid residue so as not to substantially alter a function of a protein. Examples thereof include cases where a hydrophobic residue is substituted with another hydrophobic residue, cases where a polar residue is substituted with another polar residue having the same charge and the like. Functionally similar amino acids that can be substituted in this manner are known in the art for each amino acid. Specific examples of nonpolar (hydrophobic) amino acids include alanine, valine, isoleucine, leucine, proline, tryptophan, phenylalanine, methionine and the like. Examples of polar (neutral) amino acids include glycine, serine, threonine, tyrosine, glutamine, asparagine, cysteine and the like. Examples of positively charged (basic) amino acids include arginine, histidine, lysine and the like. Examples of negatively charged (acidic) amino acids include aspartic acid, glutamic acid and the like.

As used herein, "agent" is used broadly and interchangeably, and may be any substance or other elements (e.g. energy such as light, radiation, heat or electricity) as long as the intended objective can be accomplished. Examples of such a substance include, but are not limited to, protein, polypeptide, oligopeptide, peptide, polynucleotide, oligonucleotide, nucleotide, nucleic acid (including, for example, DNAs such as cDNA and genomic DNA, and RNAs such as mRNA), polysaccharide, oligosaccharide, lipid, organic small molecule (e.g. hormone, ligand, information transmitting substance, organic small molecule, molecule synthesized by combinatorial chemistry, small molecule that can be used as pharmaceutical product (e.g. small molecule ligand and the like) and a complex molecule thereof).

Typical examples of an agent "specific" to a polynucleotide as used herein include, but are not limited to, a polynucleotide (e.g. primer) having complementarity with a certain sequence homology (e.g. 70% or greater sequence identity) to a sequence of the polynucleotide, a peptide having a sequence specific to HLA (MHC) I or II and the like.

As used herein, "therapy" refers to the prevention of worsening, preferably maintaining the current condition, more preferably alleviation, and still more preferably disappearance of a disease or disorder in case of such a condition, including being able to exert a prophylactic effect or an effect of improving one or more symptoms accompanying the disease. Preliminary diagnosis with suitable therapy is referred to as "companion therapy", and a diagnostic agent therefor may be referred to as "companion diagnostic agent".

As used herein, "therapeutic agent" broadly refers to all agents capable of treating a condition of interest. In one embodiment of the present disclosure, "therapeutic agent" may be a pharmaceutical composition including an active ingredient and one or more pharmacologically acceptable carriers. A pharmaceutical composition can be produced by mixing an effective ingredient and the above-described carriers by any method known in the technical field of pharmaceuticals.
Further, usage form of a therapeutic agent is not limited as long as it is used for therapy. A therapeutic agent may be an active ingredient alone or a mixture of an active ingredient and any ingredient. Further, the shape of the above-described carriers is not particularly limited. For example, the carrier may be a solid or liquid (e.g. buffer solution).

As used herein, "prevention" refers to the action of taking a measure against a disease or disorder from being in such a condition prior to being in such a condition. For example, it is possible to use the agent of the present disclosure to perform diagnosis, and optionally use the agent of the present disclosure to prevent or take measures for prevention.

As used herein, "prophylactic agent" broadly refers to all agents capable of treating a condition of interest.

As used herein, "test subject" refers to a target subjected to the method of the present disclosure, and examples thereof include humans, non-human primates or other animals, particularly, mammals such as cows, horses, pigs, sheep, goats, dogs and cats, or rodents such as mice and rats.

As used herein, "specimen" refers to any substance or component (body fluid such as blood or the like) obtained from a test subject or the like, or an organ or cell extracted from a subject or the like. Those skilled in the art can appropriately select a preferred specimen based on the descriptions herein.

As used herein, "gene region" refers to each of V region, D region, J region and C region, or a region such as FR1, 2 or 3, or CDR1, 2 or 3. Such gene regions are known in the art and can be appropriately determined by referring to a database or the like. As used herein, "CDR" refers to a complementarity determining region (CDR); CDR is a region that directly contacts with an antigen and undergoes a particularly large change among variable regions, and is referred to as a hypervariable region. Each variable region of a light chain and a heavy chain has three CDRs (CDR1-CDR3) and 4 FRs (FR1-FR4) surrounding the three CDRs. Since a CDR3 region is considered to be present across V region, D region and J region, it is considered as an important key for a variable region, and is thus used as a subject of analysis.

As used herein, "database" refers to any database related to genes and particularly to a database including T cell receptor and B cell receptor repertoires in the present disclosure. Examples of such a database include, but are not limited to, IMGT (the international ImMunoGeneTics information system, www.imgt.org) database, DNA Data Bank of Japan (DDBJ, DNA Data Bank of Japan, www.ddbj.nig.ac.jp) database, GenBank (National Center for Biotechnology Information. www.ncbi.nlm.nih.gov/genbank/) database, ENA (EMBL (European Molecular Biology Laboratory), www.ebi.ac.uk/ena) database, GENCODE (https://www.gencodegenes.org/) database and the like.

As used herein, "genetic sequence analysis" refers to analysis of a constituent nucleic acid sequence and/or amino acid sequence of a gene. "Genetic sequence analysis" includes any analysis associated with a gene such as determination of a base or residue, determination of homology, determination of a domain, or determination of a latent function.

As used herein, "instruction" is a document with an explanation of the method of use of the present disclosure for a physician or other users. The instruction describes the detection method of the present disclosure, the method of use of a diagnostic agent, or sentences instructing administration of a medicine or the like. Further, an instruction may describe a sentence instructing oral administration or administration to the esophagus (e.g. by injection or the like) as a site of administration. The instruction is prepared in accordance with a format defined by an authority of the country in which the present disclosure is practiced (e.g. Ministry of Health, Labour and Welfare in Japan or Food and Drug Administration (FDA) in the U.S. or the like), with an explicit description showing approval by the authority. The instruction is a so-called package insert and is typically provided in, but not limited to, paper media. The instructions may also be provided in a form such as electronic media (e.g. websites provided on the Internet or emails).

### (Preferred Embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description in the present specification. In addition, the following embodiments of the present disclosure can be used alone or in combination thereof.

### (Method for Identifying Epitope Sequence)

In one aspect of the present disclosure, there is provided a method for identifying an epitope sequence associated with a specific physiological state of a test subject, the method including the steps of: A) obtaining BCR and/or TCR repertoire data associated with the specific physiological state of the test subject; B) specifying a TREM sequence (repertoire specific TREM sequence) from the BCR and/or TCR repertoire data; C) obtaining a reference TREM sequence associated with the test subject; D) specifying a repertoire-reference common specific TREM sequence from the reference TREM sequence and the repertoire specific TREM sequence; and E) determining, based on the repertoire-reference common specific TREM sequence, whether a peptide sequence including the repertoire-reference common specific TREM sequence is an epitope sequence associated with the specific physiological state of the test subject. This method can be mainly implemented by inputting data to a computer system and performing calculation with an appropriate program. Further, according to the method, the idiotype network theory (N. K. Jerne (1974) Ann. Immunol. (Inst. Pasteur), 125C, 373-389) is developed. Among the antigen epitopes found in the Idiotope of the BCR, an antigen epitope that can induce a T cell according to MHC restriction can be identified.

The step of A) obtaining BCR and/or TCR repertoire data associated with the specific physiological state of the test subject in the present disclosure can utilize, for example, but not limited to, the technique described in WO2015/075939 by the same applicant.

In such a technique, a sample may be prepared by a method for preparing a sample for quantitatively analyzing a repertoire in a variable region of a T cell receptor (TCR) or B cell receptor (BCR). Gene analysis is performed using such a sample, and the identification may be performed using a next-generation sequencing technique based on bioinformatics for quantitatively analyzing a repertoire in a variable region of a T cell receptor (TCR) or B cell receptor (BCR).

The method for preparing a sample may include the steps of: (1) synthesizing complementary DNA using an RNA sample derived from a target cell as a template; (2) synthesizing a double-stranded complementary DNA using the complementary DNA as a template; (3) synthesizing an adaptor-added double-stranded complementary DNA by adding a common adaptor primer sequence to the double-stranded complementary DNA; (4) performing a first PCR amplification reaction by using the adaptor-added double-stranded complementary DNA, a common adaptor primer consisting of the common adaptor primer sequence, and a C-region-specific primer of a first TCR or BCR, where the C-region-specific primer of the first TCR or BCR is sufficiently specific to a C region of interest of the TCR or BCR and contains a sequence having no homology to other gene sequences, and is designed to contain a mismatching base between subtypes when amplified; (5) performing a second PCR amplification reaction by using a PCR amplicon of (4), the common adaptor primer, and a second TCR or BCR C region specific primer, where the second TCR or BCR C region specific primer is designed to have a sequence that is a complete match with the TCR or BCR C region in a sequence downstream the sequence of the first TCR or BCR C region specific primer, but include a sequence that is not homologous with other gene sequences, and include a mismatching base between subtypes downstream when amplified; 6) performing a third PCR amplification reaction by using a PCR amplicon of (1-5), an added common adaptor primer in which a nucleic acid sequence of the common adaptor primer includes a first additional adaptor nucleic acid sequence, and an adaptor-added third TCR or BCR C region specific primer in which a second additional adaptor nucleic acid sequence and a molecule identification (MID Tag) sequence are added to a third TCR or BCR C region specific sequence; where the third TCR or BCR C region specific primer is designed to have a sequence that is a complete match with the TCR or BCR C region in a sequence downstream to the sequence of the second TCR or BCR C region specific primer, but include a sequence that is not homologous with other gene sequences, and include a mismatching base between subtypes downstream when amplified, the first additional adaptor nucleic acid sequence is a sequence suitable for binding to a DNA capturing bead and for an emPCR reaction, the second additional adaptor nucleic acid sequence is a sequence suitable for an emPCR reaction, and the molecule identification (MID Tag) sequence is a sequence for imparting uniqueness such that an amplicon can be identified.

The method for analyzing a TCR or BCR repertoire may include, for example, the following steps: (1) providing a reference database for each gene region including at least one of a V region, a D region, a J region, and optionally a C region; (2) providing an input sequence set which is optionally trimmed and optionally extracted to have a suitable length; (3) searching for homology of the input sequence set with the reference database for the each gene region and recording an alignment with an approximate reference allele and a sequence of the reference allele; (4) assigning the V region and the J region for the input sequence set and extracting a nucleic acid sequence in the V region (preferably, assigning the V region and the J region for the input sequence set, and extracting a CDR3 sequence, with the front of CDR3 on a reference V region and end of CDR3 on reference J as guides); (5) translating the nucleic acid sequence of the V region into an amino acid sequence and classifying a D region by utilizing the amino acid sequence (preferably translating the nucleic acid sequence of CDR3 into an amino acid sequence and classifying the D region utilizing the amino acid sequence); and (6) calculating a frequency of appearance of each of the V region, the D region, the J region and optionally the C region, or a frequency of appearance of a combination thereof based on the classifying in (5) to derive the TCR or the BCR repertoire.

The step of B) identifying a TREM sequence (repertoire specific TREM sequence) from the BCR repertoire and/or TCR repertoire data in the present disclosure can be identified based on HLA (MHC) I or HLA (MHC) II information, and specifically, the step can be performed by calculating an IC50 value as binding affinity for a designated MHC type using any peptide sequence. For example, since a 9-mer peptide has MHC class I binding affinity and a 15-mer peptide has MHC class II binding ability, the respective binding affinities can be specified using ANN, NetMHCpan, SMM, SMMPMBEC, PickPocket, NetMHCcons, NetMHCstabpan or the like in the case of MHC class I, and using NetMHCIIpan, NN_align, SMM_align, or the like in the case of MHC class II.

In the present disclosure, the step of C) obtaining the reference TREM sequence associated with the test subject can be realized by various methods. For example, the reference sequence may be obtained by extracting a sequence from an agent associated with a target physiological state, or may be obtained from a protein sequence in a broad database.

In the step D) specifying a repertoire-reference common specific TREM sequence from the reference TREM sequence and the repertoire specific TREM sequence in the present disclosure, it is determined that the 5-mer TREM sequences are identical. In a case where the sequences are identical, each of the sequences is specified as the repertoire-reference common specific TREM sequence. Any means can be used to determine whether the sequences are identical or common.

In the step E) determining, based on the repertoire-reference common specific TREM sequence, whether a peptide sequence including the repertoire-reference common specific TREM sequence is an epitope sequence associated with the specific physiological state of the test subject in the present disclosure can be realized by various methods. In one embodiment, the HLA (MHC) binding affinity of the peptide sequence including the repertoire-reference common specific TREM sequence can be tested as needed to determine whether it is an epitope sequence associated with a specific physiological state of the test subject.

In one embodiment of the present disclosure, when obtaining BCR and/or TCR repertoire data associated with the specific physiological state, the full-length sequence of the BCR and/or TCR may be obtained. In another embodiment, the full-length sequence need not be used, and a part of the sequence of the BCR and/or TCR can also be used. Preferably, a partial sequence including the TREM can be used. In still another embodiment, a full-length sequence of an immunoglobulin variable section of BCR or TCR clone specific to the physiological state can also be determined.

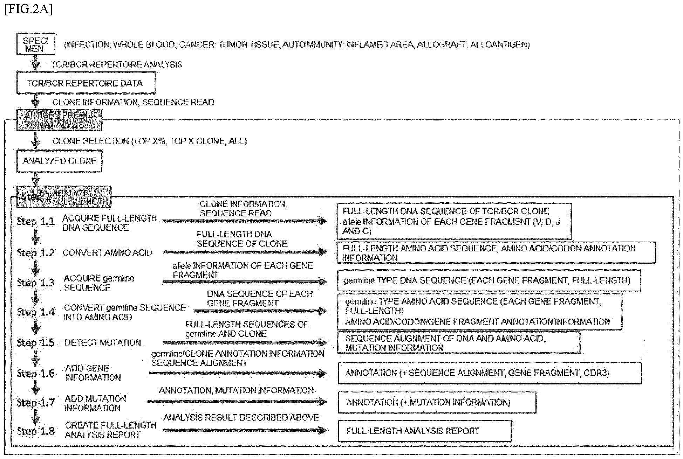

When the total length is analyzed, a procedure as shown in FIG. 2A is exemplified. For example, Step 1.1: full-length DNA sequence acquisition is performed, Step 1.2: amino acid conversion is performed, Step 1.3: germline sequence acquisition is performed, Step 1.4: germline sequence amino acid conversion is performed, Step 1.5: mutation detection is performed, Step 1.6: gene information addition is performed, Step 1.7: mutation information addition is performed, and Step 1.8: full-length analysis report creation is performed.

In the full-length DNA sequence acquisition, the clone information of the repertoire analysis result (the V, D, J and C gene determination result and the CDR3 sequence) and sequence reads are used to acquire sequence reads corresponding to each clone, and the reads are combined to reconstruct one sequence information, thereby acquiring the full-length DNA sequence of each clone. Further, allele information of gene fragments (V, D, J and C) for each clone can be acquired from the clone information of the repertoire analysis result. In addition to this, the information can be acquired, for example, from a database such as NCBI. Such operation can be realized by, for example, software such as repertoire analysis such as IgBLAST or MiXCR, or mapping in various NGS analyses.

In the amino acid conversion, the full-length DNA sequence of the clone obtained in Step 1.1 is converted into an amino acid sequence based on codon conversion, and a full-length amino acid sequence is acquired. Further, annotation can be performed to associate each converted amino acid with codon information and the base position of the full-length DNA sequence. In another embodiment, the amino acid sequence of the repertoire gene can be obtained by directly reading the protein sequence of TCR or BCR with a peptide sequence such as MS.

In the acquisition of the germline sequence, sequence information of each gene fragment is acquired based on the obtained allele information of each gene fragment, and these gene fragment sequences can be combined to construct a germlinetype full-length sequence. In addition to this, for example, the sequences of each gene fragment can be combined based on genome information or gene information to construct a sequence.

In the conversion of the germline sequence into an amino acid, the obtained germline full-length sequence is converted into an amino acid sequence based on codon conversion, and a full-length amino acid sequence is acquired. Further, annotation can be performed to associate each converted amino acid with codon information and gene fragment information. In another embodiment, the germline type amino acid sequence can also be obtained by directly reading the protein sequence of TCR or BCR with a peptide sequence such as MS and acquiring the amino acid sequence of the repertoire gene.

In the mutation detection, the full-length germline sequence and the full-length clonal sequence can be used to create sequence alignments of the DNA sequence and the amino acid sequence. Here, for creating the sequence alignments, an existing sequence alignment program: Clustal Omega can be used, but another software such as ClustalW or BLAST can also be used.

It is possible to identify a difference between two sequences on sequence alignment and detect this difference as a somatic hypermutation occurring in the clone. Such a step is not particularly limited as long as it can identify a difference between sequences and execute a program that can recognize a mutation.

In the gene information addition, information addition can be performed in association with the annotated germline full-length sequence, gene fragment information and CDR3 information, similarly to the resulting annotated clone full-length sequence, based on the resulting sequence alignment. In another embodiment, the same annotation as described above can be performed by searching a database containing gene fragment information for the full-length sequence of the clone obtained as a query sequence.

In the mutation information addition, mutation information on DNA and amino acid levels in the mutation site specified above is added to the resulting annotated clone full-length sequence information. Here, "1" can be output when there is a mutation, whereas "0" can be output when there is no mutation. However, the present disclosure is not limited thereto, and any means may be used as long as the means can add mutation information by detecting a difference between the sequences and outputting the difference.

The full-length analysis report can be created by any reporting method, but is preferably reported in the form of an electronic medium.

As the step of specifying the repertoire-reference common specific TREM sequence from the reference TREM sequence and the repertoire specific TREM sequence, the following analysis flow is exemplified.

For example, analysis can be completed by performing Step 2: extraction of the repertoire TREM sequence, Step 3: calculation of the MHC class binding affinity, Step 4: assessment of B-cell epitope properties, Step 5: comparison of a TREM sequence with a reference sequence, Step 6: calculation of the MHC class binding affinity of a reference peptide sequence, Step 7: narrowing down of peptides based on MHC type (thereby generating a peptide list based on MHC type), and Step 8: narrowing down based on commonality. Consequently, a peptide list can be generated based on commonality.

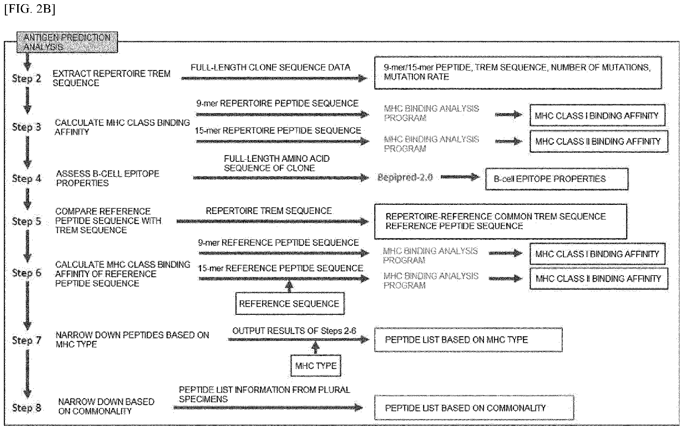

In the extraction of the repertoire TREM sequence, as illustrated in Step 2 of FIG. 2B, continuous 9-mer and 15-mer peptide sequences are created from the obtained TCR/BCR full-length sequence, TREMI/IIa/IIb is extracted, mutation search is performed, and the mutation rate can be assessed. Although exemplified in Example 2, the method is not particularly limited as long as the mutation rate in the peptide can be calculated.

For calculation of the MHC class binding affinity, the MHC binding affinity was calculated as illustrated in Step 3 of FIG. 2B. Specifically, in the calculation of MHC binding affinity, IC50 for MHC class I can be calculated for the 9-mer, and IC50 for MHC class II was calculated for the 15-mer. The calculation of IC50 is exemplified in Example 3. As software that can be used for the calculation of the IC50 value indicating MHC binding affinity, for example, ANN, NetMHCpan, SMM, SMMPMBEC, PickPocket, NetMHCcons, NetMHCstabpan and the like can be used in the case of MHC class I. For example, NetMHCIIpan, NN_align, SMM_align and the like can be used in the case of MHC class II.

In the assessment of B-cell epitope properties, as illustrated in Step 4 of FIG. 2B, a peptide with B-cell epitope properties was assessed using BepiPred-2.0. The B-cell epitope properties of the full-length amino acid sequence of the clone obtained in Example 1 can be assessed to obtain a peptide with B-cell epitope properties. As exemplified in Example 4, in addition to this, the B-cell epitope properties can be analyzed using programs such as Chou and Fasman beta turn prediction, Emini surface accessibility scale, Karplus and Schulz flexibility scale, Kolaskar and Tongaonkar antigenicity scale, Parker Hydrophilicity Prediction, and Bepipred-1.0 Linear Epitope Prediction.

As illustrated in Step 5 of FIG. 2B, in comparison of a TREM sequence with a reference sequence, a repertoire specific TREM is compared with a reference sequence based on various reference sequences, the same TREM sequence of the repertoire clone sequence as the TREM sequence of the reference sequence as well as the reference sequence peptide can be extracted. In addition to the method exemplified in Example 5, the method is not particularly limited as long as the identity of the sequences can be confirmed.

In calculation of the MHC class binding affinity of the reference peptide sequence, IC50 for MHC class I can be calculated for the 9-mer, and IC50 for MHC class II can be calculated for the 15-mer, as illustrated in Step 6 of FIG. 2B. The calculation of IC50 is exemplified in Example 6. As software that can be used for the calculation of the IC50 value indicating MHC binding affinity, for example, ANN, NetMHCpan, SMM, SMMPMBEC, PickPocket, NetMHCcons, NetMHCstabpan and the like can be used in the case of MHC class I. For example, NetMHCIIpan, NN_align, SMM_align and the like can be used in the case of MHC class II.

As a result of narrowing down peptides based on the MHC type, as illustrated in Step 7 of FIG. 2B, a peptide list is generated based on the MHC type. Peptides based on the HLA type among the obtained peptide sequences can be narrowed down. As exemplified in Example 7, in addition to this, in the case of MHC class I, for example, ANN, NetMHCpan, SMM, SMMPMBEC, PickPocket, NetMHCcons, NetMHCstabpan and the like can be used for analysis. In the case of MHC class II, for example, NetMHCIIpan, NN_align, SMM_align and the like can be used for analysis.

As illustrated in Step 8 of FIG. 2B, the analysis by narrowing down based on commonality is capable of narrowing down peptides from a plurality of specimens and determining a highly versatile peptide sequence candidate. As exemplified in Example 8, in addition to this, the method is not particularly limited as long as peptides with high commonality can be narrowed down.

In one embodiment, the BCR repertoire and/or TCR repertoire data can be obtained from a specimen from a test subject or from an existing database.

In one embodiment, the physiological state includes the presence or absence of a disease, physical fatigue, physical stress or the like, or the degree, type or the like thereof. Examples of the physiological state include any state in a test subject, and represents, for example, the presence or absence of infections such as viruses or bacteria, cancer (including a variant protein, a neoantigen and a tumor protein), and autoimmune disease (proteome information such as inflamed area (obtained from RNA-seq analysis or the like)), allograft such as alloantigen (main alloantigen: MHC/HLA), and the presence or absence of allergy such as allergen protein. In one embodiment, in the case of infection, a certain degree of clinical information already identified, such as pathological diagnosis, can be used for the method of the present disclosure.

In one embodiment, it is possible to identify a clone that specifically reacts to a specific physiological state (e.g. suffering from cancer or infection). For determining whether the clone is a clone that specifically reacts with a specific physiological state, for example, when repertoire analysis is performed, a clone present at a frequency of, for example, about 0.5%, about 0.6%, about 0.7%, about 0.8%, about 0.9%, about 1.0%, about 1.1%, about 1.2%, about 1.5% or about 2.0% or greater, with respect to the entire clone obtained in a usual physiological state of a healthy person, i.e. compared with the normal physiological state, can be defined as the clone specifically reacting therewith.

In one embodiment, when extracting a TREM sequence, an about 8- to about 10-mer and/or about 13- to about 17-mer amino acid sequence is produced from the sequence of BCR or TCR clone specific to the physiological state, and the TREM sequence can be extracted from the sequence. The length of such an amino acid sequence is not particularly limited as long as the position of the TREM can be determined in the sequence. Preferably, about 9-mer and/or about 15-mer MHC binding peptides are generated from the sequence of BCR or TCR clone specific to the physiological state, and the position of the TREM is determined within the sequences.

In one embodiment, when specifying the position where a repertoire specific TREM sequence is present, it is also possible to determine where the gene region is present. In this case, examples of the gene region include, but are not limited to, an L region, a V region, a D region, a J region or a C region, FR1, 2 or 3, and CDR1, 2 or 3.

In one embodiment, in order to adjust the binding affinity of the MHC binding peptide, it is also possible to partially alter or modify the resulting about 8- to about 10-mer and/or about 13- to about 17-mer amino acid sequence. In still another embodiment, the strength of MHC binding affinity can be adjusted depending on the length of the amino acid sequence.

In one embodiment, a TREM sequence (repertoire specific TREM sequence) can be specified from BCR repertoire and/or TCR repertoire data using a known method. For example, in Rudolph MG, et al. Annu Rev Immunol. 2006; 24: 419-66, a TREM sequence in agreement with the TCEM sequence of the present disclosure is disclosed, from which position the TREM sequence can be appropriately specified.

In one embodiment, the MHC I/II binding affinity can be determined for an about 8- to about 10-mer and/or about 13- to about 17-mer amino acid sequence including a TREM sequence. In this case, for example, only the TREM sequence with common between a target repertoire sequence and a reference sequence is intended for the determination, the MHC binding affinity of both the sequences can be determined.

In one embodiment of the present disclosure, a variant TREM can also be identified. In a specific physiological state (e.g. suffering from cancer or infection), a mutation specific to the living body or the physiological state is generated. Thus, specifying such a mutation makes it possible to identify a TREM specific to the specific physiological state.

In one embodiment of the present disclosure, in the test of the HLA (MHC) binding affinity, the strength of MHC binding can be specified in order to determine whether the obtained peptide sequence functions in vivo. The binding affinity between MHC and peptide can be determined, for example, using the IC50 value, but the method is not particularly limited as long as the binding affinity can be assessed. In one embodiment, when the IC50 value is used for assessing the binding affinity, it can be assessed that the binding affinity is exhibited when the IC50 value is about 5000 or less, the binding affinity is moderate when the IC50 value is about 500 or less, and the binding is strong when the IC50 value is about 50 or less. Accordingly, in one embodiment, an epitope sequence having an IC50 of about 8000 or less, about 7000 or less, about 6000 or less, about 5000 or less, about 4000 or less, about 3000 or less, about 2000 or less, about 1000 or less, about 800 or less, about 500 or less, about 300 or less, about 100 or less, about 8 or less, about 50 or less, or about 30 or less can be selected as an epitope sequence associated with a specific physiological state of a test subject.

In one embodiment of the present disclosure, the binding affinity between MHC and peptide can also be obtained by estimation from a technique using any software, machine learning or other bioinformatics. For example, based on analysis data obtained from binding assay using HLA (MHC) and peptides, in software using machine learning such as NetMHCpan or MHCflurry, the HLA (MHC) binding affinity can be estimated using a method based on an artificial neural network or a support vector machine using the analysis data as learning data. Meanwhile, in software such as ProPred, SMM or MHCPred, the HLA binding affinity can be estimated using a position-specific scoring matrix.

In one embodiment of the present disclosure, B-cell epitope properties can also be analyzed in identifying sequences specific for HLA (MHC) I or HLA (MHC) II (repertoire specific TREM sequences). In the analysis of B-cell epitope properties, based on known protein information (such as hydrophobicity, polarity and accessibility of individual amino acid residues) including crystal structures of antibodies and proteins, ease of binding to an antibody is assessed using a primary sequence of amino acids as input information (http://tools.iedb.org/bcell/help/). In one embodiment, for example, in the case of using BepiPred-2.0, the threshold value of the score for ease of binding can be set to 0.5 or greater. In the Repetoire Genesis antigen prediction algorithm, when the score in the target sequence is 0.5 or greater, it can also be assessed that binding is easy.

In one embodiment of the present disclosure, the reference TREM sequence may include, but is not limited to, a TREM sequence selected from the group consisting of a viral protein, a bacterial protein, a cancer mutant protein, a neoantigen, a tumor protein, a human proteome in autoimmunity, a mouse proteome, a microbiome, a transplantation alloantigen, and an allergen protein. In another embodiment, the repertoire itself of the TCR or BCR can also be used as the reference TREM sequence.

In one embodiment, these reference TREM sequences can be obtained from a specimen from a test subject or from an existing database. In another embodiment, an artificial peptide can also be used as the reference TREM sequence.

In one embodiment of the present disclosure, when the repertoire-reference common specific TREM sequence is identified from the reference TREM sequence and the repertoire specific TREM sequence, the identification can be performed by comparing the sequence of the BCR or TCR clone with any reference sequence and analyzing whether or not the same TREM sequence is present. For the comparison with the reference sequence, for example, when the physiological state is an autoimmune disease, a TREM sequence of a human proteome, an inflamed area or the like is subjected to RNA-seq expression analysis, and sequence data thereof can be used as the reference TREM sequence. In one embodiment of the present disclosure, any reference sequence can also include a reference sequence unique to a physiological state. For example, when a human proteome is used as a reference sequence, this reference sequence can also be used in a predetermined disease.

In one embodiment of the present disclosure, the about 9-mer and/or about 15-mer amino acid sequence with a common TREM sequence between the sequence of the BCR or TCR clone and the reference sequence can be defined as a candidate T-cell antigen epitope to be presented to T cells. In one embodiment, the candidate T-cell antigen epitope is determined by evaluating identity between the repertoire specific TREM sequence and the reference TREM sequence, extracting the repertoire specific TREM sequence having TREM with identity and/or a 9-mer or 15-mer peptide sequence in the reference TREM sequence, and calculating HLA (MHC) binding affinity of the extracted peptide sequence. In one embodiment, the candidate T-cell antigen epitope can be determined by, for example: 1) narrowing down the TREM by evaluating the identity between the TREM of the specific TCR/BCR repertoire and the reference TREM; 2) extracting an about 9-mer and/or about 15-mer peptide sequence of the reference sequence having the narrowed TREM; and 3) calculating MHC/HLA binding affinity of the extracted peptide with respect to a preset set of MHC/HLA (not patient-unique, for example, HLA types of all dengue patients, those with a frequency of 1% or greater in Japanese, etc.), and then defining an about 9-mer and/or about 15-mer amino acid sequence with a common TREM sequence between the sequence of the BCR or TCR clone and the reference sequence as the candidate T-cell antigen epitope.

In one embodiment of the present disclosure, when determining the candidate T-cell antigen epitope, it is possible to extract a reference sequence peptide in which the TREM matches with the BCR repertoire and/or TCR repertoire. The 5-mer of the TREM is a sequence included in the 9-mer or 15-mer, and the position of the 5-mer amino acid is also determined. Therefore, a process of extracting a sequence other than the 5-mer (i.e. the remaining 4-mer for a 9-mer peptide, and the remaining 10-mer for a 15-mer peptide) and adding the sequence to the 5-mer of the TREM makes it possible to allow the sequence to return to the original 9-mer or 15-mer sequence.

In one embodiment of the present disclosure, narrowing down peptides based on the HLA type makes it possible to determine an HLA-specific peptide with a common TREM between the BCR and/or TCR repertoire and the reference TREM sequence. There are 3 types of MHC/HLA binding affinity assessments, one of which is performed on a certain predetermined level of MHC/HLA type. For example, the MHC/HLA binding affinity is assessed for the HLA type of all dengue patients, those with a frequency of 1% or greater in Japanese, and the like. The MHC/HLA binding affinity is also assessed in determining the HLA-specific peptide.

The second assessment of MHC/HLA binding affinity is performed on a patient- or specimen-unique MHC/HLA. The binding affinity of the patient or specimen for HLA class varies depending on one type (for homo) or two types (for hetero). For example, the binding affinity varies depending on HLA-A, HLA-B and DRB1. The assessment of the binding affinity here is performed when narrowing down and determining candidates for each patient or specimen. This assessment of binding affinity will be integrated for each clone with the data evaluated in the other steps (type of TREM (type I, type IIa, type IIb), gene region where TREM is present (L, V, D, J C), and clone information (type of V, D, J, C and CDR3), mutation rate, B-cell epitope properties).

The third assessment of MHC/HLA binding affinity is performed on all the MHC/HLA types in the MHC/HLA database list to assess peptide versatility. The MHC/HLA binding affinity is assessed after identification of a common TREM candidate between a patient or specimen and between clones.

In one embodiment of the present disclosure, based on a repertoire-reference common specific TREM sequence, it is possible to determine whether the peptide sequence including the repertoire-reference common specific TREM sequence is an epitope sequence associated with a specific physiological state of a test subject. For example, the HLA (MHC) binding affinity of the peptide sequence including the repertoire-reference common specific TREM sequence can be tested to determine whether it is an epitope sequence associated with a specific physiological state of a test subject. In this case, a 9-mer and/or 15-mer amino acid sequence with a common TCEM sequence between the sequence of the BCR or TCR clone and the reference sequence can be determined as a candidate T-cell antigen epitope to be presented to T cells. In one embodiment, when group analysis (e.g. analysis of different patients or specimens of the same case) is conducted, it is possible to determine a peptide candidate common among patients, specimens, and clones. For example, the following assessments can be performed to determine the peptide candidate.
1) Assessment of Commonality among Patients, Specimens and Clones. The peptide candidate with high commonality is a highly versatile peptide candidate. Conversely, the peptide candidate with low commonality is a specific (patient-unique) peptide candidate
2) Assessment of Presence or Absence of Mutations. In a case where a mutation is present in a TREM, it is assessed to be a TREM specifically generated by the immune response.
   Meanwhile, in a case where no mutation is present, it is assessed that since the TREM pattern originally present in the TCR/BCR is the same as a reference, it is detected in the immune response. Further, in a case where a mutation is present in an MAM, the peptide having the same TREM as the reference is a peptide specifically generated by the immune response.
3) Assessment of Presence of Identified TREM in Idiotope Such as V, D, J CDR3, Rather than Common Sequence Such as C Region on TCR/BCR.
4) Assessment of Presence of Identified TREM in Any Protein Functional Domain, Module, Motif or the Like in Reference Sequence.
5) Assessment of Commonality with Sequence Other than Intended Reference. For example, in the case of dengue, it is assessed whether the TREM sequence is commonly present in viruses of the same Flaviviridae family as that of the dengue. In the case of allograft, it is assessed whether a common TREM is also present in HLA other than an alloantigen of a donor.

In one embodiment of the present disclosure, the step of determining whether it is an epitope sequence can be accomplished by assessing binding affinity for a plurality of different MHCs. The binding affinity here corresponds to the third assessment of the MHC/HLA binding affinity described above, and the assessment is performed on all MHC/HLA information or a wide range of MHC/HLA types available in the database. Consequently, it is possible to narrow down versatile peptide candidates.

### (Antigen Containing Epitope Sequence)

In another aspect of the present disclosure, there is provided a method for producing an antigen containing an epitope sequence associated with a specific physiological state of a test subject, the method including the steps of: A) obtaining BCR and/or TCR repertoire data associated with the specific physiological state of the test subject; B) specifying a TREM sequence (repertoire specific TREM sequence) from the BCR and/or TCR repertoire data; C) obtaining a reference TREM sequence associated with the test subject; D) specifying a repertoire-reference common specific TREM sequence from the reference TREM sequence and the repertoire specific TREM sequence; E) determining, based on the repertoire-reference common specific TREM sequence, whether a peptide sequence including the repertoire-reference common specific TREM sequence is an epitope sequence associated with the specific physiological state of the test subject; and F) generating a peptide containing the epitope sequence.

Here, as described in a method for identifying an epitope sequence associated with a specific physiological state of a test subject, the method including the steps of: A) obtaining BCR and/or TCR repertoire data associated with the specific physiological state of the test subject; B) specifying a TREM sequence (repertoire specific TREM sequence) from the BCR and/or TCR repertoire data; C) obtaining a reference TREM sequence associated with the test subject; D) specifying a repertoire-reference common specific TREM sequence from the reference TREM sequence and the repertoire specific TREM sequence; and E) determining, based on the repertoire-reference common specific TREM sequence, whether a peptide sequence including the repertoire-reference common specific TREM sequence is an epitope sequence associated with the specific physiological state of the test subject, any embodiments thereof can be optionally combined and performed.

In the present disclosure, F) generating a peptide containing the epitope sequence can be performed using any biochemical, chemical or biotechnological method.

In one embodiment, the peptide can be synthesized, for example, by a known peptide synthesis method, such as a solid-phase synthesis method or a liquid-phase synthesis method. In the synthesis, a commercially available peptide synthesizer (e.g. PSSM-8, manufactured by Shimadzu Corporation) may be used.

In the case of the cyclic peptide, the disulfide bond in the amino acid sequence is not particularly limited, but can be formed by, for example, a DMSO oxidation method, an iodine oxidation method or the like. In this case, an intramolecular disulfide bond can be formed by treating a free sulfhydryl group or a sulfhydryl group protected by a protecting group with DMSO or iodine (I2), and as a result, a cyclic peptide can be obtained.

Examples of the protecting group include a 4-methylbenzyl group (Bzl (4Me)), a trityl group (Trt), a tert-butyl group, and an N-(acetyl)aminomethyl group (Acm) and the like. Deprotection can be performed by a suitable treatment corresponding to these protecting groups, for example, by treating a 4-methylbenzyl group with a strong acid and treating an N-(acetyl)aminomethyl group with iodine.

In a case where a peptide is synthesized by genetic engineering, the peptide can be synthesized by a known method, for example, a method as described in Sambrook J. et al., Molecular Cloning, A Laboratory Manual (4th edition) (Cold Spring Harbor Laboratory Press (2012)). For example, a polynucleotide (typically DNA) with a nucleotide sequence (including an ATG start codon) encoding the amino acid sequence of a desired synthetic peptide is synthesized. Then, a recombinant vector having a gene construct for expression including the synthesized polynucleotide (DNA) and various regulatory elements (including promoters, ribosome binding sites, terminators, enhancers, various cis-elements that control expression levels) for expressing the amino acid sequence in a host cell is constructed according to the host cell.

This recombinant vector is introduced into a predetermined host cell (e.g. yeast, insect cells, plant cells) by a general technique. Then, the host cell or a tissue or an individual containing the cell is cultured under predetermined conditions. This makes it possible to express and produce a target peptide in cells. Then, the peptide is isolated from the host cell (in the medium when secreted), and refolding, purification, and the like are performed as needed, whereby a target synthetic peptide can be obtained.

As a method for constructing a recombinant vector, a method for introducing the constructed recombinant vector into a host cell, and the like, a known method performed in the art can be adopted as it is.

Alternatively, a template DNA for a cell-free protein synthesis system (i.e. a synthetic gene fragment including a nucleotide sequence encoding an amino acid sequence of a synthetic peptide) is constructed, various compounds (ATP, RNA polymerase, amino acids, etc.) necessary for peptide synthesis are used, and a target polypeptide can be synthesized in vitro by adopting a so-called cell-free protein synthesis system. For the cell-free protein synthesis system, for example, the article of Shimizu et al., Nature Biotechnology, 19, 751-755(2001)), and the article of (Madin et al.,

Proc. Natl. Acad. Sci. USA, 97(2), 559-564(2000)) are useful references. Based on the techniques described in these articles, many companies have already performed contract production of polypeptides at the time of filing of the present application, and kits for cell-free protein synthesis (e.g. available from CellFree Sciences Co., Ltd.) are commercially available.

The fact that the target peptide has been obtained can be confirmed by a known method such as reverse-phase HPLC or mass spectrometry.

A single stranded or double stranded polynucleotide containing a nucleotide sequence encoding the synthetic peptide disclosed herein and/or a nucleotide sequence complementary to the nucleotide sequence can be easily produced (synthesized) by a known method in the related art. Hence, the nucleotide sequence corresponding to the amino acid sequence of the synthetic peptide is easily determined and provided by selecting the codon corresponding to each amino acid residue constituting the designed amino acid sequence. Once the nucleotide sequence is determined, a polynucleotide (single strand) corresponding to a desired nucleotide sequence can be easily obtained using a DNA synthesizer or the like. Further, the obtained single stranded DNA is used as a template, various enzymatic synthesis means (typically PCR) is used, as a result of which a target double stranded DNA can be obtained. The polynucleotide may be in the form of DNA or in the form of RNA (mRNA or the like). DNA may be provided in double stranded or single stranded form. When DNA is provided as a single strand, the strand may be a coding strand (sense strand) or a non-coding strand (antisense strand) having a sequence complementary thereto.

The polynucleotide thus obtained can be used as a material for constructing a recombinant gene (expression cassette) for synthetic peptide production in various host cells or in a cell-free protein synthesis system, as described above.

### (System program)

### (TCR/BCR Repertoire Analysis)

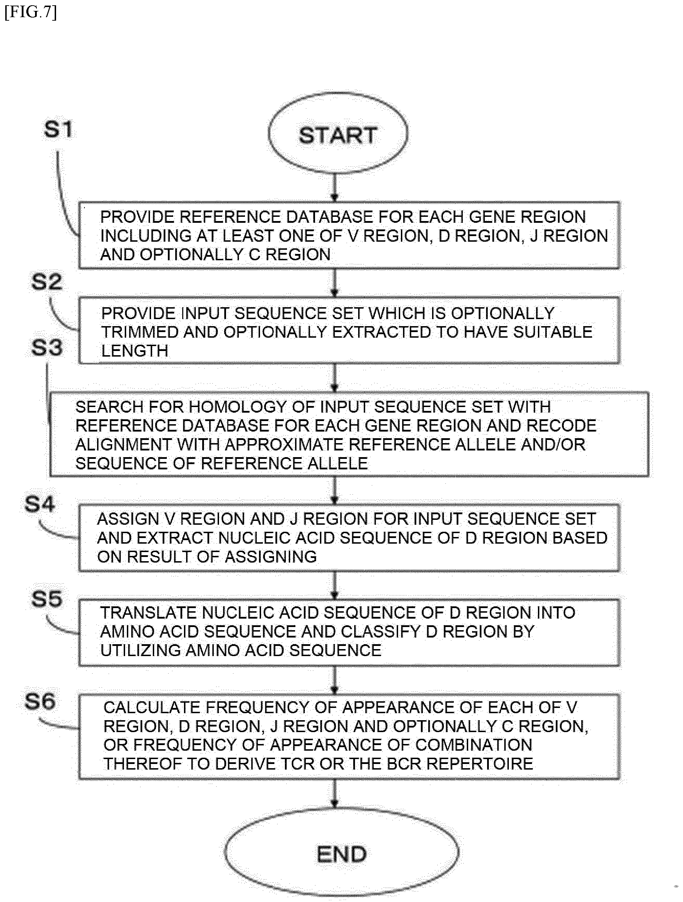

FIG. 7 illustrates an example of a flowchart of a computer program for realizing the present disclosure. FIG. 7 is a flowchart showing a processing flow of a method for analyzing a TCR or BCR repertoire. Each step is explained with specific operation of each system or device while referring to the flow chart. Further, each of the symbols S1-S6 in the Figure corresponds to each of (1)-(6) in the following explanation.

In the method of the present disclosure, it is possible to provide (1) a reference database for each gene region including at least one of a V region, a D region, a J region and optionally a C region. For example, in the case of the V region, this can be accomplished by appropriately selecting a database including information of the V region and providing the database.

In the method of the present disclosure, (2) providing an input sequence set which is optionally trimmed and optionally extracted to have a suitable length is accomplished. This is accomplished by optional trimming with a function of an appropriate software or the like and optionally providing an extracted input sequence set after appropriately selecting a length. An input sequence may be, for example, a set of amplicons amplified by a known method or a set of amplicons amplified by PCR with an unbiased method.

In the method of the present disclosure, (3) searching for homology of the input sequence set with the reference database for the each gene region and recording an alignment with an approximate reference allele and/or a sequence of the reference allele is performed. This is performed by appropriately using a software for performing homology search, for each gene region (e.g. the V region and the like), performing homology search with a reference database on the input sequence set, and recording alignment with an approximate reference allele and/or a sequence of the reference allele obtained as a result.

In the method of the present disclosure, (4) assigning the V region and the J region for the input sequence set and extracting a nucleic acid sequence of the D region based on a result of assigning. This can be accomplished by determining the V region and/or J region from the sequence alignment based on known information and the like. Such extraction is preferably accomplished by assigning the V region and the J region for the input sequence set and extracting a CDR3 sequence, with the front of CDR3 on a reference V region and end of CDR3 on reference J as guides.

In the method of the present disclosure, (5) translating the nucleic acid sequence of the D region into an amino acid sequence and classifying the D region by utilizing the amino acid sequence. This can be accomplished by translating into an amino acid using a known method in the art and picking out a sequence corresponding to the D region by homology search or the like on the amino acid sequence. Preferably, the nucleic acid sequence of CDR3 can be translated into an amino acid sequence, and the amino acid sequence can be used to classify the D region.

In the method of the present disclosure, (6) calculating a frequency of appearance of each of the V region, the D region, the J region and optionally the C region, or a frequency of appearance of a combination thereof based on the classifying in (5) to derive the TCR or the BCR repertoire. This can be can be accomplished by calculating a frequency of appearance of each of the V region, the D region, the J region and/or the C region calculated in the above-described step, for example, by organizing the frequencies into a list. As a result, the TCR or BCR repertoire can be derived.

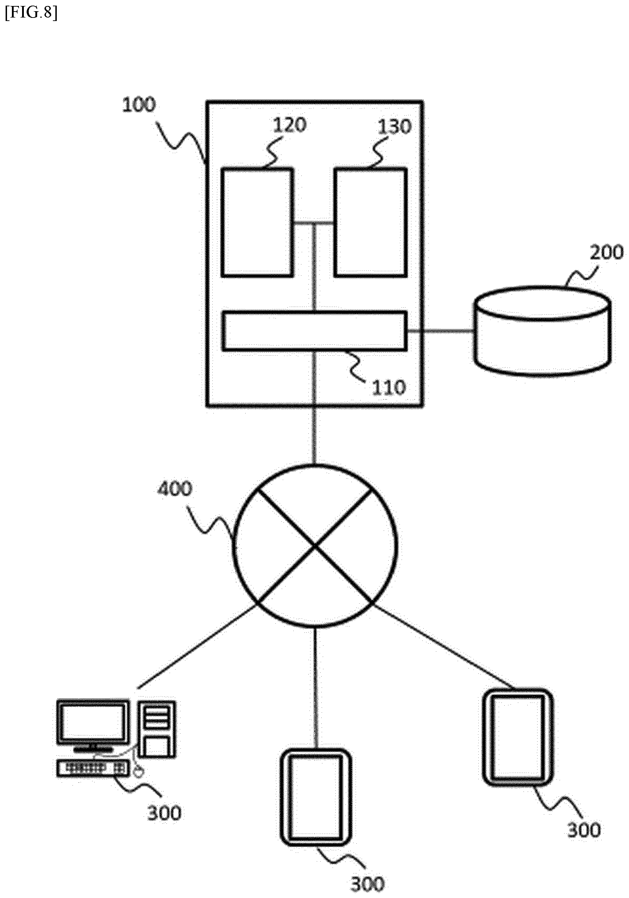

The above steps will be further described with reference to FIG. 8.

FIG. 8 illustrates an example of a configuration of the computer system 100 for realizing the present disclosure.

In the present example, the computer system 100 is connected to a database unit 200. Further, the computer system 100 is connected to at least one terminal device 300 via a network 400.

The network 400 can be any type of network. The network 400 may be, for example, the Internet or a LAN. The network 400 may be a wired network or a wireless network.

An example of the computer system 100 is a server device, but is not limited thereto. An example of the terminal device 300 is a terminal device held by a test subject, a terminal device installed in a laboratory or a hospital, or a terminal device installed in a public place, but is not limited thereto. Here, the server device and the terminal device may be any type of computer. For example, the terminal device may be a personal computer. The terminal device 300 preferably includes, for example, a means for analyzing BCR, TCR or the like.

The computer system 100 includes an interface unit 110, a processor unit 120, and a memory unit 130.

The interface unit 110 exchanges information with the outside of the computer system 100. The processor unit 120 of the computer system 100 can receive information from the outside of the computer system 100 via the interface unit 110, and can transmit information to the outside of the computer system 100. The interface unit 110 can exchange information in any format.

The interface unit 110 includes, for example, an input unit that enables information to be input to the computer system 100. It does not matter how the input unit enables information to be input to the computer system 100. For example, in a case where the input unit is a receiver, the receiver may receive information from the outside of the computer system 100 via the network 400 to perform input. Alternatively, in a case where the input unit is a data reading device, information may be input by reading information from a storage medium connected to the computer system 100. Alternatively, for example, in a case where the input unit is a touch panel, a user may input information by touching the touch panel. Alternatively, in a case where the input unit is a mouse, the user may input information by operating the mouse. Alternatively, in a case where the input unit is a keyboard, the user may input information by pressing a key of the keyboard. Alternatively, the input unit may be connected to the database.

The interface unit 110 includes, for example, an output unit that enables information to be output from the computer system 100. It does not matter how the output unit enables information to be output from the computer system 100. For example, when the output unit is a transmitter, the transmitter may transmit information to the outside of the computer system 100 via the network 400 to perform output. Alternatively, for example, in a case where the output unit is a data writing device, information may be output by writing the information in a storage medium connected to the computer system 100. Alternatively, in a case where the output unit is a display screen, information may be output to the display screen. Alternatively, in a case where the output unit is a speaker, information may be output by voice from the speaker.

For example, the output unit can output information such as a peptide estimated by the computer system 100 to the outside of the computer system 100.

The processor unit 120 executes a process of the computer system 100 and controls the operation of the entire computer system 100. The processor unit 120 reads a program stored in the memory unit 130 and executes the program. This enables the computer system 100 to function as a system that executes desired steps. The processor unit 120 may be implemented by a single processor or may be implemented by a plurality of processors.

The memory unit 130 stores a program required for executing a process of the computer system 100, data required for executing the program, and the like. Here, how to store the program in the memory unit 130 does not matter. For example, the program may be pre-installed in the memory unit 130. Alternatively, the program may be installed in the memory unit 130 by being downloaded via a network. Alternatively, the program may be stored in a computer-readable storage medium.

The memory unit 130 stores a program required for executing a process of the computer system 100, data required for executing the program, and the like. The memory unit 130 may store a program for a process of identifying an epitope and/or a program for a process of analyzing TCR/BCR and the like. Here, how to store the program in the memory unit 130 does not matter. For example, the program may be pre-installed in the memory unit 130. Alternatively, the program may be installed in the memory unit 130 by being downloaded via a network. Alternatively, the program may be stored in a computer-readable storage medium.

Information and data of the antigen prediction target may be stored in the database unit 200. Such data may be transmitted from the terminal device 300 to the database unit 200 (via the computer system 100), for example, or may be captured by a camera that can be included in the computer system 100, for example. Furthermore, the database unit 200 can store, for example, a calculation result output by the computer system 100.

In the example illustrated in FIG. 8, the database unit 200 is provided outside the computer system 100, but the present disclosure is not limited thereto. At least a part of the database unit 200 can also be provided inside the computer system 100. At this time, at least a part of the database unit 200 may be implemented by the same storage means as the storage means that implements the memory unit 130, or may be implemented by a storage means different from the storage means that implements the memory unit 130. In any case, at least a part of the database unit 200 is configured as a storage unit for the computer system 100. The configuration of the database unit 200 is not limited to a specific hardware configuration. For example, the database unit 200 may be configured by a single hardware component, or may be configured by a plurality of hardware components. For example, the database unit 200 may be configured as an external hard disk device of the computer system 100, or may be configured as a storage on a cloud connected via a network.

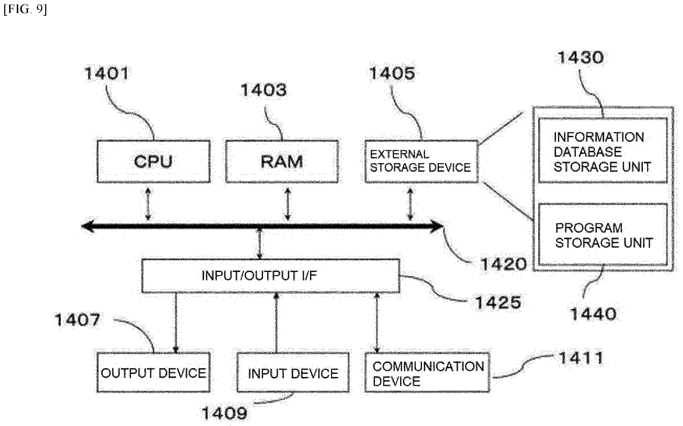

TCR and/or BCR repertoire analysis for use in the method of the present disclosure is further described with reference to FIG. 9. Hereinafter, steps (1) to (6) correspond to S1 to S6 in FIG. 7. In S1 (step (1)) of FIG. 7, a reference database is provided. This reference database may be a reference database stored in an external storage device 1405, but can generally be obtained as a database provided in public through a communication device 1411. Alternatively, the data may be input using an input device 1409 and recorded in a RAM 1403 or the external storage device 1405 as needed. Here, a database including a region of interest such as the V region is provided.

In S2 (step (2)), an input sequence set is provided. Here, a set of sequence information obtained from a set of amplicons amplified by, for example, a PCR amplification reaction is input using the input device 1409 or via the communication device 1411. Here, a device that receives the amplicons of the PCR amplification reaction and analyzes the gene sequences thereof may be connected. Such a connection is made through a system bus 1420 or through the communication device 1411. At this stage, trimming and/or extraction of a suitable length can be performed as needed. Such a process is performed by a CPU 1401. The program for trimming and/or extraction may be provided via each of the external storage device, communication device, or input device.

In S3 (step (3)), alignment is performed. Here, for the input sequence set, a homology search with the reference database is performed for each of the gene regions. In this homology search, a homology search program process is performed on the reference database obtained via the communication device 1411 or the like. This process is performed by the CPU 1401. Further, the results obtained as a result are analyzed and aligned with the sequence of an approximate reference allele and/or the sequence of the reference allele. This process is also performed by the CPU 1401. The program for executing these processes can be provided via each of the external storage device, communication device, or input device.

In S4 (step (4)), the nucleic acid sequence of the D information is detected. This process is also performed by the CPU 1401. The program for executing these processes can be provided via each of the external storage device, communication device, or input device. Here, for the input sequence set, the V region and the J region are assigned. The assigning process is also performed by the CPU 1401. The CPU 1401 also extracts the nucleic acid sequence of the D region based on a result of assigning. The program for the assigning and extracting processes may also be provided via each of the external storage device, communication device, or input device. Here, this can be preferably accomplished by determining the V region and/or J region from the sequence alignment based on known information and the like. The results can be stored in the RAM 1403 or the external storage device 1405. Such extraction is preferably accomplished by assigning the V region and the J region for the input sequence set and extracting a CDR3 sequence, with the front of CDR3 on a reference V region and end of CDR3 on reference J as guides. Such a process can also be performed in the CPU 1401. The program therefor may also be provided via each of the external storage device, communication device, or input device.

In S5 (step (5)), the D region is classified. A process of translating the nucleic acid sequence of the D region into an amino acid sequence and classifying the D region using the amino acid sequence is performed, and the process is also performed by the CPU 1401. The program for this process may also be provided via each of the external storage device, communication device, or input device. For the obtained amino acid sequence, a sequence corresponding to the D region may be extracted by homology search or the like. Such a process is also performed by the CPU 1401. The program for this process may also be provided via each of the external storage device, communication device, or input device. Preferably, the nucleic acid sequence of CDR3 can be translated into an amino acid sequence, and the amino acid sequence can be used to classify the D region. This process is also performed by the CPU 1401. The program for this process may also be provided via each of the external storage device, communication device, or input device.

In S6 (step (6)), a frequency of appearance of each of the V region, the D region, the J region and optionally the C region, or a frequency of appearance of a combination thereof is calculated based on the above-described classifying to derive the TCR or the BCR repertoire. The process for the calculation and derivation is also performed by the CPU 1401. The program for this process may also be provided via each of the external storage device, communication device, or input device.

In one preferred embodiment, the gene region used in the present disclosure includes all of the V region, the D region, the J region and optionally the C region.

In one embodiment, the reference database is a database with a unique ID assigned to each sequence. Uniquely assigning the ID makes it possible to analyze the sequence of the gene based on a simple index: ID.

In one embodiment, the input sequence set is an unbiased sequence set. The unbiased sequence set can be achieved by PCR amplification by the unbiased method as described herein. When the accuracy of the unbiased method is not required, a relatively low degree of "pseudo-unbiased method", such as the Smart method, may be used. Accordingly, the term "unbiased" used herein refers to unbiased with accuracy as accomplished by the method of the present disclosure, and a case where the accuracy does not reach that level refers to the "pseudo-unbiased method". The unbiased method as described herein may particularly be referred to as "accurate unbiased" upon being referred to in a distinguished manner. It is understood that, even in the absence of "accurate", it is at the level accomplished herein using the method described herein.

In another embodiment, the sequence set is trimmed. Performing trimming makes it possible to remove unnecessary or inappropriate nucleic acid sequences, and thus the efficiency of analysis is increased.

In a preferred embodiment, trimming is accomplished by a step of: deleting a low quality region from both ends of the read; deleting a region matching the adaptor sequence by 10 bp or more from both ends of the read; and using, as a sample of high quality for analysis, the resulting region when the remaining length is 200 bp or more (TCR), or 300 bp or more (BCR). Preferably, the low quality is that the 7 bp moving average of the QV values is less than 30.

In a preferred embodiment, the approximate sequence is the closest sequence. In a specific embodiment, the approximate sequence is determined by the ranking of 1. Number of matched bases, 2. Kernel length, 3. Score, and 4. Alignment length.

In another embodiment, the homology search is performed under a condition tolerating random mutations to be scattered throughout. Such a condition is often expressed by the following condition for BLAST/FASTA optimal parameters: tolerates a maximum mismatch of 33% across the full-length of an alignment; and tolerates a maximum nonconsecutive mismatch of 60% for any 30 bp therein. In one embodiment, the homology search includes at least one condition from (1) shortening of a window size, (2) reduction in a mismatch penalty, (3) reduction in a gap penalty, and (4) a top priority ranking of an indicator is a number of matching bases, compared with a default condition.

In another embodiment, the homology search is carried out under the following conditions in BLAST or FASTA:
V mismatch penalty = -1, shortest alignment length = 30, and shortest kernel length = 15;
D word length = 7 (for BLAST) or K-tup = 3 (for FASTA), mismatch penalty = -1, gap penalty = 0, shortest alignment length = 11, and shortest kernel length = 8;
J mismatch penalty = -1, shortest hit length = 18, and shortest kernel length = 10; and
C shortest hit length=30 and shortest kernel length = 15. This condition can also be used, for example, as long as it is a situation where a shorter (about 200 bp) sequence is used to classify only part of a region (situation that does not fall under the "preferred example"). This condition can also be used in a situation where an Illumina sequencer is used. In this case, possibility of using bwa or bowtie for homology search is considered.

In a specific embodiment, the D region is classified by a frequency of appearance of the amino acid sequence.

In a further embodiment, a combination of a result of search for homology with the nucleic acid sequence of CDR3 and a result of amino acid sequence translation is used as a classification result when there is a reference database for the D region in the step (5).

In another embodiment, only the frequency of appearance of the amino acid sequence is used for classification when there is no reference database for the D region in the step (5).

In a specific embodiment, the frequency of appearance is counted in a unit of a gene name and/or a unit of an allele.

In another embodiment, the step (4) includes assigning the V region and the J region for the input sequence set and extracting a CDR3 sequence, with the front of CDR3 on a reference V region and end of CDR3 on reference J as guides.

In a further embodiment, the step (5) includes translating the nucleic acid sequence of the CDR3 into an amino acid sequence and classifying a D region by utilizing the amino acid sequence.

The following is an example of estimation of TCR and/or BCR. cDNA synthesis, PCR amplification, and sequencing are performed using RNA derived from a specimen containing T cells and/or B cells to acquire sequence information (sequence reads) of the TCR and/or BCR. These sequence reads are used to classify or determine the above-described TCR and/or BCR, thereby specifying the types and proportions of V, D, J, C genes and CDR3 constituting the TCR and BCR clones contained in the specimen. For example, in a case where BCR is estimated using, as a specimen, blood from a certain virus-infected patient in a recovery stage, a BCR clone specifically reacting to the virus can be estimated by classifying or determining a human BCR gene for a sequence read obtained by amplification using a primer specific to the human BCR gene.

### (Analysis of Full-length)

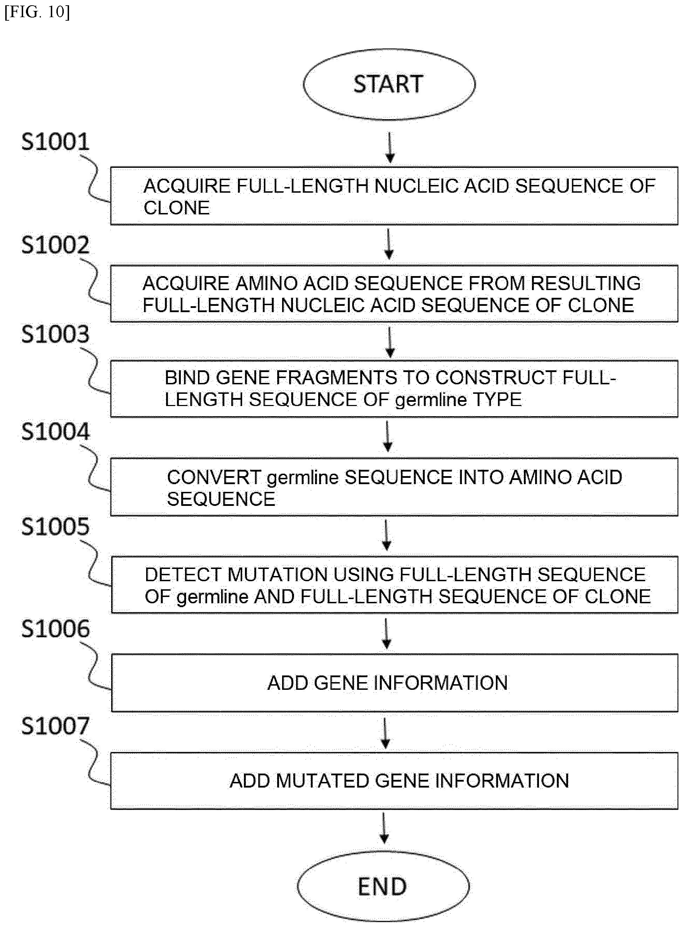

FIG. 10 is a flowchart showing a processing flow of a method for analyzing the full-length of a clone from which TCR or BCR repertoire data is obtained. Each step is explained with specific operation of each system or device while referring to the flow chart. Further, each of the symbols S1001-S1007 in the Figure corresponds to each of Steps 1.1-1.7 in the following explanation.

In the method of the present disclosure, the full-length DNA sequence acquisition in S1001 is accomplished by using the clone information of the repertoire analysis result (the V, D, J and C gene determination result and the CDR3 sequence) and sequence reads to acquire sequence reads corresponding to each clone, and combining the reads to reconstruct one sequence information to acquire the full-length DNA sequence of each clone (Step 1.1). Further, allele information of each gene fragment (V, D, J or C) for each clone can be acquired from the clone information of the repertoire analysis result and used.

In one embodiment, the combining and reconstructing of sequence reads is performed in the following manner. Specifically, a sequence alignment of sequence reads on the C region side is created, and a constant length sequence with the highest commonality in the 3' direction is obtained from the CDR3 sequence as a starting point. This is repeated up to the end of the lead, and the acquired sequence is bound and converted into one sequence (C region 3'-side read). Here, the constant length sequence has any length, but the length is typically 60 bp. Next, similarly, the CDR3 sequence is used as a starting point, and a constant length sequence with the highest commonality in the 5' direction is acquired. This is repeated up to the end of the lead, and the acquired sequence is bound and converted into one sequence (C region 5'-side read). Here, the constant length sequence has any length, but the length is typically 60 bp. Next, a 5' end sequence of the C region 5'-side read is acquired, and sequence reads on the V region side including the sequence is acquired to create a sequence alignment. In the sequence alignment of sequence reads on the V region side, the sequence or the CDR3 sequence is used as a starting point to acquire a constant length sequence with the highest commonality in the 5' direction. This is repeated up to the end of the lead, and the acquired sequence is bound and converted into one sequence (V region 5'-side read). Here, the constant length sequence has any length, but the length is typically 21 bp. Finally, the three obtained sequences are linked to form one full-length DNA sequence.

In the method of the present disclosure, amino acid conversion in S1002 can be accomplished by converting the full-length DNA sequence of the clone obtained in S1001 (Step 1.1) into an amino acid sequence based on codon conversion to obtain the full-length amino acid sequence (Step 1.2). In one embodiment, annotation may be performed to associate each converted amino acid with codon information and the base position of the full-length DNA sequence.

In the method of the present disclosure, based on the allele information of each gene fragment obtained in S1001 (Step 1.1), acquisition of the germline sequence in S1003 can be accomplished by acquiring sequence information of each gene fragment (Step 1.3). These gene fragment sequences can be combined to construct a full-length sequence of germline type.

In the method of the present disclosure, germline sequence amino acid conversion in S1004 can be accomplished by converting the germline full-length sequence obtained in S1003 (Step 1.3) into an amino acid sequence based on codon conversion to obtain the full-length amino acid sequence (Step 1.4). In one embodiment, annotation may be further performed to associate each converted amino acid with codon information and gene fragment information.

In the method of the present disclosure, mutation detection in S1005 can be accomplished by creating sequence alignments of the DNA sequence and the amino acid sequence, respectively, using the germline full-length sequence and the clone full-length sequence (Step 1.5). In one embodiment, sequence alignments may be created using an existing sequence alignment program: Clustal Omega. It is possible to identify a difference between two sequences on sequence alignment and detect this difference as a somatic hypermutation occurring in the clone.

In the method of the present disclosure, gene information addition in S1006 can be accomplished by adding information in association with the annotated germline full-length sequence, gene fragment information and CDR3 information obtained in S1004 (Step 1.4), similarly to the annotated clone full-length sequence obtained in S1002 (Step 1.2), based on the sequence alignment obtained in S1005 (Step 1.5) (Step 1.6).

In the method of the present disclosure, mutation information addition in S1007 can be accomplished by adding mutation information on DNA and amino acid levels in the mutation site specified above to the annotated clone full-length sequence information obtained in S1006 (Step 1.6) (Step 1.7). Here, "1" can be output when there is a mutation, whereas "0" can be output when there is no mutation.

In the method according to one embodiment of the present disclosure, the analysis results can be accumulated to form a full-length analysis report.

### (Antigen Prediction Analysis)

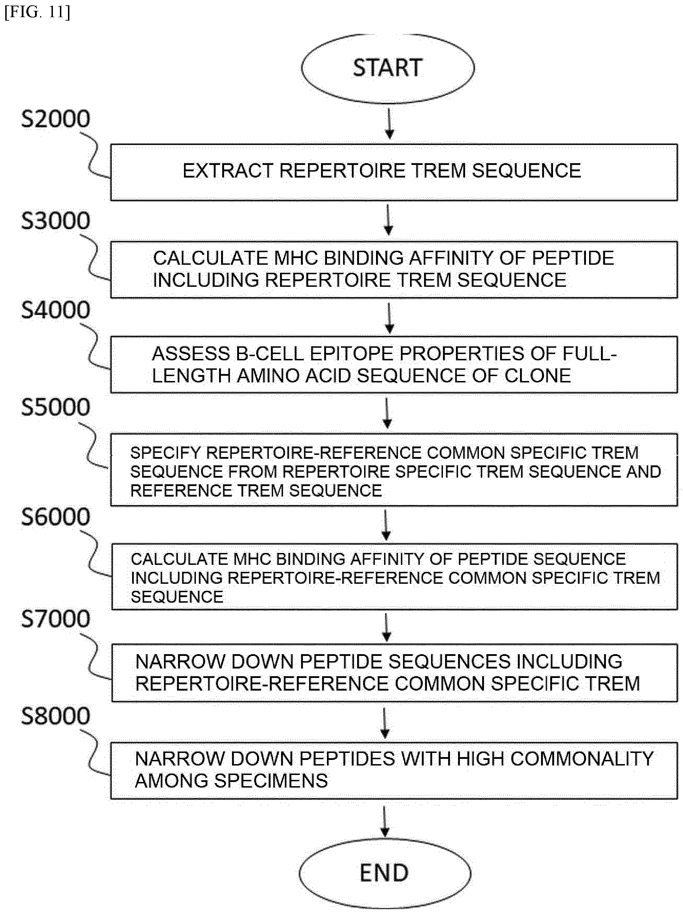

FIG. 11 is a flowchart showing a processing flow of a method for performing antigen prediction analysis. Each step is explained with specific operation of each system or device while referring to the flow chart. Further, each of the symbols S2000-S8000 in the Figure corresponds to each of Steps 2-8 in the following explanation.

In the method of the present disclosure, extraction of the repertoire TREM sequence in S2000 can be accomplished by creating continuous 9-mer and 15-mer peptide sequences from the TCR/BCR full-length sequence obtained in Step 1, extracting TREMI/IIa/IIb, performing mutation search, and assessing the mutation rate (Step 2).

In one embodiment, this step can be performed in the following manner. Specifically, 9-mer and 15-mer peptide sequences are prepared for the full-length amino acid sequences of both the germline type and the clone obtained in Step 1. Here, the 9-mer and 15-mer peptide sequences are a peptide sequence group obtained by moving one amino acid at a time from the N-terminal side to the C-terminal side of the full-length amino acid sequence and extracting consecutive 9 and 15 amino acids.

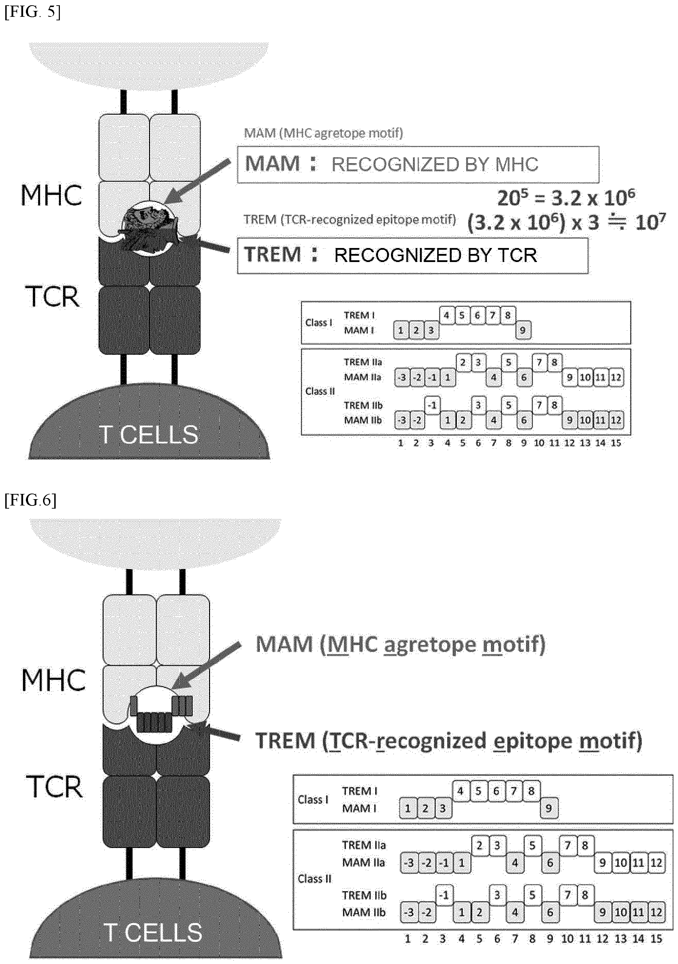

Next, a TREM sequence is extracted from each of the prepared 9-mer and 15-mer peptide sequences. Here, the TREM sequence is a continuous (TREMI) or discontinuous (TREMIIa, TREMIIb) motif sequence composed of 5-mer amino acids, is a sequence (TREMI) composed of amino acids present at positions P4, P5, P6, P7 and P8 (positions 4, 5, 6, 7 and 8 from N-terminus of 9-mer amino acid sequence) in the 9-mer amino acid sequence (with position numbers P1 to P9), and can include sequences (TREMIIa and TREMIIb, respectively) composed of amino acids present at positions P2, P3, P5, P7, and P8 (5, 6, 8, 10 and 11 positions from the N-terminus of the 15-mer amino acid sequence.) or positions P-1, P3, P5, P7 and P8 (3, 6, 8, 10 and 11 positions from the N-terminus of the 15-mer amino acid sequence) in the 15-mer amino acid sequence (with position numbers P-3 to P12) (FIGS. 5 and 6). The position of the motif sequence can be determined with reference to Rudolph MG, et al. Annu Rev Immunol. 2006; 24: 419-66., Moise L, et al. Hum Vaccin Immunother. 2013 Jul; 9 (7): 1577-86., Calls JJ, et al. PLoS Comput Biol. 2012; 8 (3): e1002412.

In one embodiment, the extracted germline type is compared with the TREM sequence and MAM sequence derived from the clone. In a case where there is a difference in the amino acid corresponding to the same position between both the sequences, it can be determined that a mutation occurs in the amino acid derived from the clone. In one embodiment, based on the mutation information annotated in Example 1, the number of mutations determined in each of the 9-mer, 15-mer and each TREM sequence is calculated as the number of mutations, and a ratio of the number of mutations to the length of each sequence can be calculated as the mutation rate. For example, when there are two mutations in the 5-mer TREM sequence, the mutation rate is 2/5 = 40.00 (%). At this time, the mutation rate as the 9-mer peptide sequence is calculated by 2/9 = 22.22 (%).

In the method of the present disclosure, calculation of the MHC binding affinity in S3000 can be accomplished by calculating IC50 for MHC class I with respect to the 9-mer and calculating IC50 for MHC class II with respect to the 15-mer (Step 3).

In one embodiment, IC50 values may be calculated as binding affinity for a designated MHC type using the 9-mer and 15-mer peptide sequences obtained from the clone sequences. Since the class of MHC type to which the 9-mer peptide binds is different from the class of MHC type to which the 15-mer peptide binds, the method for calculating the IC50 value vary according to the 9-mer peptide and the 15-mer peptide. The 9-mer peptide has MHC class I binding affinity, the 15-mer peptide has MHC class II binding ability, and different software is used for the program for analyzing these peptides for each MHC class. Thus, when the IC50 value is calculated, software for calculating the IC50 value and the MHC type are designated for each peptide length. Here, when the peptide length and the MHC type to be analyzed are the same, a plurality of peptide sequences can be processed by software at a time. Examples of software that can be used for calculating the IC50 value indicating MHC binding affinity in the case of MHC class I include, but are not limited to, ANN, NetMHCpan, SMM, SMMPMBEC, PickPocket, NetMHCcons and NetMHCstabpan. Examples of MHC class II include, but are not limited to, NetMHCIIpan, NN_align and SMM_align. Use of these software together makes it possible to improve reliability. Switching the reference database makes it possible to use the software in the case of humans, mice, chimpanzees, and the like.

In the method of the present disclosure, assessment of peptides with B-cell epitope properties in S4000 can be accomplished by assessing the B-cell epitope properties on the full-length amino acid sequence of the clone obtained by the full-length analysis (Step 4). In one embodiment, B-cell epitope properties may be assessed using the existing software BepiPred-2.0. A score is calculated for each amino acid residue. When the score is equal to or greater than a threshold value (default value was 0.5), it is determined that B-cell epitope properties are exhibited, and the score is output as "E".

In the method of the present disclosure, comparison of the TREM sequence with the reference sequence in S5000 can be accomplished by using a previously prepared reference sequence and searching for the presence of the same sequence as the TREM sequence present in the repertoire clone sequence in the reference sequence, thereby identifying a repertoire-reference common TREM sequence, and extracting a 9-mer or 15 mer peptide sequence derived from the reference sequence including the common TREM sequence (Step 5). As the reference sequence, 3 different types can be specified. The first reference sequence is a reference sequence unique to a specimen. Examples of the reference sequence include a protein sequence of pathogenic bacteria such as viruses and bacteria that have infected a patient with an infection, a mutated protein sequence in a patient with cancer, a protein sequence at an inflamed area of a patient with an autoimmune disease, an alloantigen protein sequence of a donor patient for allograft in the case where the specimen is a recipient patient, and the like. The type of the second reference sequence is a protein sequence specified when the reference sequence unique to the specimen is not present, a protein sequence to be compared with the reference sequence unique to the specimen or the like. Examples of the former include various proteome data available from a public database and the like in the case of cancer and autoimmune diseases. Further, examples of the latter include a protein sequence of a species related to pathogenic bacteria such as viruses and bacteria that have infected a patient with an infection, and the like. As the third reference sequence, all protein sequences to be compared can be specified, in addition to the first and second reference sequences. Further, in the setting of the above 3 types of reference sequences, a subordinate condition of each reference sequence group can be set. For example, these subordinate conditions are set, and thus it is possible to specify a protein functional domain, a module, a motif, a specific pathogenic species or the like in the reference sequence, and it is possible to specify at which site the identified TREM sequence is present.

In the method of the present disclosure, calculation of the MHC class binding affinity of the reference peptide sequence in S6000 can be accomplished by calculating IC50 for MHC class I with respect to the 9-mer and calculating IC50 for MHC class II with respect to the 15-mer, similarly to S3000 (Step 3) (Step 6) .

In the method of the present disclosure, narrowing down of peptides based on MHC type in S7000 can be accomplished by extracting peptide sequence information of 9-mer and 15-mer containing a TREM sequence common between a repertoire clone sequence and a reference sequence based on a designated HLA type (Step 7). In one embodiment, a threshold value of the IC50 value is specified, and thus peptide sequence information can be extracted based on the HLA type and the threshold value. The IC50 value (nM) is an alternative numerical value to indicate the strength of the binding force between the peptide and MHC. In general, when the IC50 value is small, the MHC binding affinity of the peptide is considered to be high. In the case of IC 50 < 50, the binding affinity is considered to be high. In the case of IC 50 < 500, the binding affinity is considered to be moderate. In the case of IC 50 < 5000, the binding affinity is considered to be low. Thus, a T-cell antigen epitope having an IC50 of greater than 5000 is not known. Here, as a typical analysis condition, the threshold value of the IC50 value for the designated HLA type can be set to 5000.

In the method of the present disclosure, narrowing down based on commonality in S 8000 can be accomplished by narrowing down peptides from a plurality of specimens and determining a highly versatile peptide sequence candidate (Step 8). In one embodiment, for the type of the obtained peptide candidate, peptides with high commonality among specimens can be narrowed down.

### (Peptide Obtained by Method of Present Disclosure, Nucleic Acid Encoding Same, Composition Related to Same, Medicine and Therapy Using Same)

A peptide obtained by the method of the present disclosure or a nucleic acid encoding the peptide can be used in various therapies, medicines, therapies, diagnoses or the like, and in particular, can be used in immunotherapy. This will be described below.

A peptide provided by the method of the present disclosure can be derived from antigens associated with tumorigenesis, and can be capable of binding sufficiently to MHC (HLA) class II molecules to activate an immune response of human leukocytes: a TH1-type immune response induced by, especially lymphocytes, especially T lymphocytes, especially CD4 positive T lymphocytes, especially CD4 positive T lymphocytes.

The present disclosure also provides a peptide derived from an antigen associated with tumorigenesis and having the ability to bind sufficiently to MHC (HLA) class I molecules to activate an immune response of human leukocytes, especially lymphocytes, especially T lymphocytes, especially CD8 positive cytotoxic T lymphocytes. The present disclosure also provides a combination of two peptides that are particularly useful as a vaccine of cancer patients.

The peptide of the present disclosure may be derived from a tumor-associated antigen, particularly a tumor-associated antigen with a function in, e.g. proteolysis, angiogenesis, cell growth, cell cycle regulation, cell division, transcriptional regulation and tissue invasion.

The peptide provided by the present disclosure can be used for immunotherapy, preferably immunotherapy for cancer, since the peptide is capable of chemical synthesis and can be used as an active pharmaceutical ingredient in pharmaceuticals.

The pharmaceutical composition of the present disclosure further includes an additional peptide and/or excipient to increase efficacy, which will be further described below.

The pharmaceutical composition of the present disclosure may include a peptide identified in the present disclosure, the peptide having a full-length of 8 to 100 amino acids, preferably from 8 to 30 amino acids, and most preferably 8 to 16 amino acids.

The peptide or a variant of the present disclosure can be chemically modified by a reaction with a specific amino acid before or after synthesis of the peptide used in the present disclosure. Examples of such modification are well known in the art. Examples thereof are summarized in R. Lundblad, Chemical Reagents for Protein Modification, 3rd ed. CRC Press, 2005 incorporated herein by reference. Examples of chemical modification of an amino acid include, but are not limited to, modifications by acylation, amidination, pyridoxylation of lysine, reductive alkylation, trinitrobenzylation of an amino group by 2,4,6-trinitrobenzenesulfonic acid (TNBS), amide modification of a carboxyl group and sulfhydryl modification by performic acid, oxidation from cysteine to cysteic acid, generation of mercury derivatives, generation of mixed disulfide with another thiol compound, reaction with maleimide, carboxymethylation with iodoacetic acid or iodoacetamide, and carbamoylation with cyanate at an alkaline pH. With regard to the above, those skilled in the art can reference a broader methodology related to chemical modification of a protein with Current Protocols In Protein Science, Eds. Coligan et al. (John Wiley& Sons NY 1995-2000). For example, modification of an arginine residue of a protein is often the formation of an adduct based on a reaction of adjacent dicarbonyl compounds such as phenylglyoxal, 2,3-butanedione, and 1,2-cyclohexanedione. Another example is a reaction of an arginine residue with methylglyoxal. Cysteine can be modified without simultaneous modification of another nucleophilic site such as lysine or histidine. Thus, numerous reagents can be utilized in cysteine modification. Information on specific reagents is provided by Pierce Chemical Company, Sigma-Aldrich, and other websites.

A disulfide bond in a protein used in the present disclosure is often selectively reduced. A disulfide bond can be formed and oxidized during heat treatment of a biomedicine. A specific glutamic acid residue can be modified by using Woodward's Reagent K. An intermolecular crosslink can be formed between a lysine residue and a glutamic acid residue by using N-(3-(dimethylamino)propyl)-N'-ethylcarbodiimide. For example, diethylpyrocarbonate is a reagent for modifying a histidine residue in a protein. Histidine can also be modified by using 4-hydroxy-2-nonenal. A reaction between a lysine residue and another α amino group is useful, for example, in a bond between a peptide and a surface or protein/peptide crosslink. Lysine is a site where poly(ethylene)glycol attaches and is a main site of modification in glycation of a protein. A methionine residue of a protein can be modified, for example, by iodoacetamide, bromoethylamine or chloramine-T. Tetranitromethane and N-acetylimidazole can be used in modification of a tyrosyl residue. A crosslink by formation of dityrosine can be accomplished with hydrogen peroxide/copper ions. N-bromosuccinimide, 2-hydroxy-5-nitrobenzyl bromide and 3-bromo-3-methyl-2-(2-nitrophenyl mercapto)-3H-indole (BPNS-skatole) were used in a recent study related to tryptophan modification. Suitable modification of a therapeutic protein and peptide with PEG often involves prolonging the circulation half-life. In addition, protein crosslink by glutaraldehyde, polyethylene glycol diacrylate and formaldehyde is used for preparation of a hydrogel. Chemical modification of an allergen for immunotherapy is often accomplished by carbamylation with potassium cyanate.

In general, the peptides and variants used in the present disclosure (at least those including a peptide link between amino acid residues) can be synthesized, for example, by using a Fmoc-polyamide form of solid phase peptide synthesis, as disclosed in Lu et al (1981) J. Org. Chem. 46, 3433 and a reference thereof. Purification can be performed by a combination of one or more methods such as recrystallization, size exclusion chromatography, ion exchange chromatography, hydrophobic interaction chromatography, and reversed-phase high-performance liquid chromatography that (generally) uses, for example, acetonitrile/water gradient separation. A peptide can be analyzed by using thin-layer chromatography, electrophoresis, particularly capillary electrophoresis, solid-phase extraction (CSPE), reversed-phase high-performance liquid chromatography, amino acid analysis after acid hydrolysis, fast atom bombardment (FAB) mass spectrometry, and MALDI and ESI-Q-TOF mass spectrometry.

In still another aspect of the present disclosure, a nucleic acid (e.g. polynucleotide) encoding the peptide of the present disclosure or a variant thereof is provided. For example, DNA, cDNA, PNA, CNA, RNA, single strand and/or double strand, or natural or stable form of a polynucleotide such as a polynucleotide having phosphorothioate backbone or a combination thereof can be such a polynucleotide. It is not essential to contain intron as long as a polynucleotide encodes the peptide. Naturally, only peptides including a naturally occurring amino acid residue bound by a naturally occurring peptide bond is encoded by a polynucleotide. Yet another aspect of the present disclosure provides an expression vector capable of expressing a polypeptide according to the present disclosure. Expression vectors of different cell types are well known in the art and can be selected without any special experimentation.

In general, a DNA is inserted into an expression vector such as a plasmid in a correct orientation with a correction reading frame for expression. If necessary, a DNA can be linked to a suitable transcription/translation regulating/managing nucleotide sequence which is recognized by a desired host. However, such a management function is generally in an expression vector. The vector is then introduced into a host by a standard method. In regards to this, Sambrook et al (1989) Molecular Cloning, A Laboratory Manual, Cold Spring Harbor Laboratory, Cold Spring Harbor, N.Y. can be referred.

A regimen with the optimal dose and optimal amount of the nucleic peptide contained in a vaccine can be determined by those skilled in the art without any special experimentation. For example, the peptide or a mutant form thereof can be prepared as an intravenous (i.v.) injection, subcutaneous (s.c.) injection, intradermal (i.d.) injection, intraperitoneal (i.p.) injection, or intramuscular (i.m.) injection. Preferred routes of administration for a peptide injection are s.c., i.d., i.p., i.m., and i.v.. Preferred routes of administration for a DNA injection are i.d., i.m., s.c., i.p., and i.v. For example, 1-500 mg, 50 µg-1.5 mg, preferably 125 µg-500 µg of peptide or DNA can be administered. The dose is dependent on each peptide or DNA. The dose in this range has been successfully used in clinical trials (Brunsvig P F, Aamdal S, Gjertsen M K, Kvalheim G, Markowski-Grimsrud C J, Sve I, Dyrhaug M, Trachsel S, Muller M, Eriksen J A, Gaudernack G; Telomerase peptide vaccination: a phase I/II study in patients with non-small cell lung cancer; Cancer Immunol Immunother. 2006; 55(12): 1553-1564; M. Staehler, A. Stenzl, P. Y. Dietrich, T. Eisen, A. Haferkamp, J. Beck, A. Mayer, S. Walter, H. Singh, J. Frisch, C. G. Stief; An open label study to evaluate the safety and immunogenicity of the peptide based cancer vaccine IMA901, ASCO meeting 2007; Abstract No 3017).

The present disclosure can be provided as a pharmaceutical composition. The selection, number, and/or amount of peptides in the pharmaceutical composition of the present disclosure can be made specific to tissue, cancer and/or patient in preparing the pharmaceutical composition. For example, a side effect can be avoided by deriving a correction selection of peptide by the expression pattern of a protein of tissue of a given patient. The selection may be dependent on the cancer type and condition of the disease specific to a patient receiving therapy, therapeutic regimen up to this point, immune status of the patient and naturally, the HLA haplotype of the patient. Furthermore, the vaccine according to the present disclosure can include an individualized constituent element depending on individual needs of a specific patient. Examples thereof include expression of related TAA, personal side effects due to allergy or other therapy of the individual, and different amounts of peptide in accordance with adjustments for secondary therapy after a series of initial therapeutic plans for a specific patient.

A peptide in which a parent protein is highly expressed in normal tissue is avoided, or is present at a low amount in the composition of the present disclosure. Meanwhile, when the tumor of a patient is known to highly express a specific protein, each pharmaceutical composition for the treatment of the cancer can be present at a high amount and/or include a plurality of peptides specific to the specific protein or route. Those skilled in the art can select a preferred combination of immunogenic peptides by testing T cell functionality, expansion, affinity and proliferation of a specific T cell against a specific peptide, overall presentation, in vitro T cell formation and efficacy thereof by, for example, analysis of IFN-γ (also see the following examples). Generally, the most efficient peptides are then combined as a vaccine for the aforementioned objective.

A suitable vaccine preferably contains 1-20 peptides, more preferably 2, 3, 4, 5, 6, 7, 8, 9, 10, 11, 12, 13, 14, 15, 16, 17, 18, 19 or 20 different peptides, still more preferably 6, 7, 8, 9, 10, 11, 12, 13 or 14 different peptides, and most preferably 14 different peptides. The length of the peptide used as a cancer vaccine may be any suitable peptide. Specifically, the length can be a suitable 9-mer peptide or a suitable 7-mer or 8-mer or 10-mer or 11-mer peptide, or a 12-mer, 13-mer, 14-mer or 15-mer peptide. A longer peptide is also suitable in some cases. A 9-mer or 10-mer peptide is preferred for an MHC class I peptide and a 12- to 15-mer is preferred for an MHC class II peptide.

The present disclosure can be provided as a peptide, and the peptide constitutes a tumor or cancer vaccine. The tumor or cancer vaccine can be administered directly to the organ with the disease or systemically into a patient, administered to the patient with a vaccine applied in vitro to a human cell line or a cell from the patient, or used in vitro to select a subpopulation from immune cells of a patient and re-administered into the patient.

The peptide can be substantially pure, combined with an immunostimulatory adjuvant, used in combination with an immunostimulatory cytokine, or co-administered with a suitable delivery system (e.g., liposome). The peptide can also be conjugated with a suitable carrier such as keyhole limpet hemocyanin (KLH) or mannan (see WO 95/18145 and Longenecker et al (1993) Ann. NY Acad. Sci. 690, 276-291). The peptide can also be tagged, or formed into a fusion protein or a hybrid molecule. A peptide with the given sequence in the present disclosure is expected to stimulate CD4 or CD8CTL. However, efficiency of stimulation is increased more by a positive T cell providing assistance to the opposite CD. Therefore, for an MHC class II epitope stimulating CD4CTL, a section of a hybrid molecule or a fusion partner thereof suitably provides an epitope stimulating a CD8 positive T cell. Meanwhile, for an MHC class I epitope stimulating CD8CTL, a section of a hybrid molecule or a fusion partner thereof suitably provides an epitope stimulating a CD4 positive T cell. CD4 and CD8 stimulating epitopes are well known in the art, including those specified in the present disclosure.

The composition of the present disclosure is used in parenteral administration such as subcutaneous, intradermal, or intramuscular administration or oral administration. Thus, the peptide and other selective molecules are dissolved or suspended in a pharmaceutically acceptable, preferably water-soluble carrier. In addition, the composition can include an excipient such as a buffer, binding agent, blasting agent, diluent, flavoring agent or lubricant. Further, the peptide can be co-administered with an immunostimulatory substance such as a cytokine. An extensive list of excipients that can be used in such a composition can be obtained from, for example, A. Kibbe, Handbook of Pharmaceutical Excipients, 3. Ed. 2000, American Pharmaceutical Association and pharmaceutical press. The composition can be used in tumor or cancer, preferably in CRC prevention and/or therapeutic method.

A cytotoxic T cell (CTL) recognizes a peptide shaped antigen bound to an MHC molecule, but not an original exogenous antigen itself. The MHC molecule itself is on a cell surface of an antigen-presenting cell. Therefore, CTL activation is only possible in the presence of APC, MHC molecule, and trimer complex of a peptide antigen. Therefore, CTL is not activated by using only a peptide. An immune response is increased by further adding APC with each MHC molecule. Therefore, in a preferred embodiment, the pharmaceutical composition of the present disclosure additionally includes at least one antigen-presenting cell.

An antigen-presenting cell (or a stimulator cell) generally has an MHC class I or II molecule on the surface thereof. In one embodiment, the MHC class I or II molecule having the selected antigen substantially cannot be loaded onto itself. As discussed below in detail, the selected antigen thereof can be readily loaded onto the MHC class I or II molecule in vitro.

In general, the pharmaceutical composition of the present disclosure including the nucleic acid of the present disclosure can be administered by the same method as a pharmaceutical composition including the peptide of the present disclosure, i.e. by intravenous, intraarterial, intraperitoneal, intramuscular, intradermal, intratumoral, oral, transdermal, trans-nasal cavity, trans-oral cavity, transrectal, or transvaginal administration, inhalation or topical administration.

Tumors often gain resistance to therapeutic agents by the mechanism of avoidance. The drug resistance occurs during therapy and in some cases appears as metastatic or recurrent tumor. To avoid such drug resistance, tumor therapy is generally administered by a combination of drugs. In many cases, a different combination is required for metastasis and tumor recurrence after an event-free period. Therefore, in one aspect of the present disclosure, the pharmaceutical composition is administered with a second anticancer agent. The second agent used in the present disclosure can be administered before, after or simultaneously with the pharmaceutical composition of the present disclosure. For example, if chemical properties are compatible, simultaneous administration can be performed by mixing the pharmaceutical composition of the present disclosure with the second anticancer agent. Another method of simultaneous administration is, for example, to inject the pharmaceutical composition of the present disclosure and orally administer a second anticancer agent so as to administer the composition and anticancer agent on the same day from independent routes of administration. The pharmaceutical composition and the second anticancer agent may also be administered through the same therapeutic course on different days and/or administered through separate therapeutic courses.

In another aspect of the present disclosure, a method of treating or preventing cancer in a patient is provided. The method has a step of administering any one of the pharmaceutical compositions of the present disclosure at a therapeutically effective amount to the patient. The therapeutically effective amount is an amount sufficient to elicit an immune response, especially to activate a subpopulation of CTL. Those skilled in the art can readily determine an effective amount by using a standard immunological method as provided in the Examples of the present specification. Another method of monitoring the effect of a specific amount of the pharmaceutical composition of the present disclosure is to observe the growth and/or recurrence of treated tumor.

In an especially preferred embodiment of the present disclosure, the pharmaceutical composition is used as an anticancer vaccine.

The composition including a peptide or a nucleic acid encoding the peptide of the present disclosure can constitute a tumor or cancer vaccine. The tumor or cancer vaccine can be administered directly to the organ with the disease or systemically into a patient, administered to the patient with a vaccine applied in vitro to a human cell line or a cell from the patient, or used in vitro to select a subpopulation from immune cells of a patient and re-administered into the patient.

The composition of the present disclosure can be used as a vaccine or as a method of treating cancer. The cancer is oral cavity or pharyngeal cancer, gastrointestinal cancer, colon, rectal, or anal cancer, airway cancer, breast cancer, uterine, vaginal, or vulvar cancer, endometrial or ovarian cancer, male genital tract cancer, urethral cancer, bone and soft tissue cancer, Kaposi's sarcoma, skin melanoma, eye melanoma, nonmelanoma eye cancer, brain or central nervous system cancer, thyroid and other endocrine gland cancer, Hodgkin's lymphoma, non-Hodgkin's lymphoma, or myeloma, and preferably renal cancer, colorectal carcinoma, lung cancer, breast cancer, pancreatic cancer, prostate cancer, gastric cancer, brain cancer, GIST or glioblastoma. According to the present disclosure, the preferred amount of peptide can vary between about 0.1-100 mg in 500 µl solution, preferably about 0.1-1 mg, and most preferably about 300 µg-800 µg. In this regard, the term "about" refers to +/-10 percentage of a given value unless specifically noted otherwise. Those skilled in the art would be able to adjust the actual amount of peptide to be used based on several factors such as the immune status of an individual patient and/or the amount of TUMAP presented in a specific type of cancer. The peptide of the present disclosure may be provided in a suitable shape (sterilized solution or the like) other than a freeze-thaw peptide.

The pharmaceutical composition of the present disclosure having the peptide and/or nucleic acid according to the present disclosure is administered to a patient with adenoma or cancerous disease associated with each corresponding peptide or antigen. An immune response mediated by a T cell is triggered thereby. Preferably, the amount of expression vector in the pharmaceutical composition of the present disclosure, in the peptide (especially peptide associated with tumor) or nucleic acid of the pharmaceutical composition of the present disclosure, or the composition of the present disclosure is specific to the tissue, cancer, and/or patient.

In another embodiment of the present disclosure, the present disclosure can be utilized as a vaccine, and the vaccine can be presented as a nucleic acid vaccine (DNA vaccine or RNA vaccine). It is well known that a T cell response is elicited by inoculation of a nucleic acid vaccine such as a DNA vaccine encoding a polypeptide. The tumor or cancer vaccine can be administered directly to the organ with the disease or systemically into a patient, administered to the patient with a vaccine applied in vitro to a human cell line or a cell from the patient, or used in vitro to select a subpopulation from immune cells of a patient and re-administered into the patient. When the nucleic acid is administered into a cell in vitro, it is useful in some cases to introduce cells such that an immunostimulatory cytokine such as interleukin-2 or GM-CSF is co-expressed. The nucleic acid can be substantially pure, combined with an immunostimulatory adjuvant, used in combination with an immunostimulatory cytokine, or co-administered with a suitable delivery system (e.g., liposome). The nucleic acid vaccine may be administered with an adjuvant described in relation to the above-described peptide vaccine. Preferably, the nucleic acid vaccine is administered without an adjuvant.

The polynucleotide of the present disclosure, in some cases, is substantially pure or included in a suitable vector or a delivery system. Suitable vectors and delivery systems included are viral, such as adenovirus, vaccinia virus, retrovirus, herpes virus, adeno-associated virus, or a plurality of virus element-containing hybrid based system. Non-viral delivery systems included are cationic lipids and cationic polymers well known in the technical field of DNA delivery. Physical delivery such as a "gene gun" can also be used. The peptide or a peptide encoded by the nucleic acid is in some cases a fusion protein, such as a fusion protein with an epitope from tetanus toxoid that simulates a CD4 positive T cell.

Suitably, all peptides administered to the patient are sterilized and free of pyogenic substance. A naked DNA can be administered by an intramuscular, intradermal, or subcutaneous injection. Conveniently, the nucleic acid vaccine can have any nucleic acid delivery means. Preferably, the nucleic acid, which is a DNA, can be delivered in a liposome or as a part of a viral vector delivery system.

It is preferable that a nucleic acid vaccine such as a DNA vaccine is administered into a muscle. A peptide vaccine is preferably administered s.c. or i.d. It is also preferable to administer the vaccine intradermally.

Expression of an encoded polypeptide and uptake of a nucleic acid by a professional antigen-presenting cell such as a dendritic cell are possibly a mechanism of priming of an immune response. Although there is a possibility that a dendritic cell is not introduced, it is still important as an expression peptide can be taken in from a cell introduced into the tissue ("cross-priming": Example: Thomas A M, Santarsiero L M, Lutz E R, Armstrong T D, Chen Y C, Huang L Q, Laheru D A, Goggins M, Hruban R H, Jaffee E M. Mesothelin-specific CD8(+) T cell responses provide evidence of in vivo cross-priming by antigen-presenting cells in vaccinated pancreatic cancer patients. J Exp Med. 2004 Aug. 2; 200(3): 297-306).

A cancer immune therapeutic method mediated by a polynucleotide is described in Conry et al (1996) Seminars in Oncology 23, 135-147; Condon et al (1996) Nature Medicine 2, 1122-1127; Gong et al (1997) Nature Medicine 3, 558-561; Zhai et al (1996) J. Immunol. 156, 700-710; Graham et al (1996) Int J. Cancer 65, 664-670; and Burchell et al (1996) 309-313 In: Breast Cancer, Advances in biology and therapeutics, Calvo et al (eds), John Libbey Eurotext, the entirety of all of which is incorporated herein by reference.

In the present disclosure, it is potentially useful to administer the peptide or nucleic acid ex vivo and to have the vaccine of the present disclosure target a specific cell population such as antigen-presenting cells by selective purification of a specific cell population from a patient or use of a delivery system, targeting vector and injection site (for example, as described in Zhou et al (1995) Blood 86, 3295-3301; Roth et al (1996) Scand. J. Immunology 43, 646-651, dendritic cells can be sorted). For example, a targeting vector can have a tissue or tumor specific promoter for directing expression of an antigen at a suitable location.

The vaccine of the present disclosure may be dependent on the cancer type and condition of the disease specific to a patient receiving therapy, therapeutic regimen up to this point, immune status of the patient and naturally, the HLA haplotype of the patient. Furthermore, the vaccine according to the present disclosure can include an individualized constituent element depending on individual needs of a specific patient. Examples thereof include expression of related TAA, personal side effects due to allergy or other therapy of the individual, and different amounts of peptide in accordance with adjustments for secondary therapy after a series of initial therapeutic plans for a specific patient.

The peptide of the present disclosure is not only useful in cancer therapy, but also in diagnosis. The peptide is generated from glioblastoma, and these peptides are identified to be absent from normal tissue. Thus, these peptides can be used to diagnose the presence of cancer.

A pathologist can use the presence of the peptide of the present disclosure in tissue biopsy to assist in the diagnosis of cancer. A pathologist can know whether the tissue is malignant, inflammatory or mostly affected by mass spectrometry or detection of a specific peptide of the present disclosure using an antibody, or another method well known in the art. The presence of a group of peptides of the present disclosure enables classification or sub-classification of affected tissue.

Detection of the peptide of the present disclosure in an affected tissue sample enables decision with respect to the benefits of a therapeutic method related to the immune system especially when it is known or anticipated that a T lymphocyte is associated with the action mechanism. Loss of MHC expression is a mechanism that is well understood, by which a malignant cell evades immune surveillance. Therefore, the presence of the peptide of the present disclosure indicates that this mechanism is not utilized by the analyzed cell.

The peptide of the present disclosure can be used in the analysis of a lymphocyte response to the peptide of the present disclosure. For example, it is possible to analyze an antibody response or T cell response to the peptide of the present disclosure or the peptide of the present disclosure which is a complex with an MHC molecule. These lymphocyte responses can be used as a prognosis marker for determining a further therapeutic step. These responses can also be used as a surrogate marker in an immunotherapeutic approach attempting to elicit a lymphocyte response by different means such as a protein, nucleic acid, endogenous substance, or lymphocyte immune transfer vaccination. Under the setting of gene therapy, a lymphocyte response to the peptide of the present disclosure can be considered in assessing a side effect. Monitoring a lymphocyte response is possibly useful in a follow-up test after transplantation therapy, such as detection of graft-versus-host and host-versus-graft diseases.

The peptides of the present disclosure can be used in generation and growth of antibodies specific to an MHC/peptide complex. They can be used in a therapeutic method to apply a toxin or a radioactive substance while targeting affected tissue. As another method of using such antibodies, the antibodies can be applied while targeting a radionuclide to affected tissue for use in an imaging method such as PET. This method of use can assist in detecting small metastasis or determining an accurate position and size of affected tissue. In addition, the peptides can be used in verification of diagnosis of cancer performed by a pathologist based on biopsy sample.

The present disclosure can be provided as a kit. As used herein, "kit" refers to a unit providing portions to be provided (e.g. testing agent, diagnostic agent, therapeutic agent, antibody, label, manual and the like) into two or more separate sections. This form of a kit is preferred when a composition that should not be provided in a mixed state and is preferably mixed immediately before use for stability or the like is intended to be provided. Such a kit advantageously includes an instruction or manual describing how the provided portions (e.g. testing agent, diagnostic agent or therapeutic agent) are used or how a reagent should be handled. When the kit is used herein as a reagent kit, the kit generally includes an instruction describing how to use a testing agent, diagnostic agent, therapeutic agent, antibody and the like.

In this manner, in a further aspect of the present disclosure, the present disclosure is directed to a kit, where the kit has (a) a container containing the pharmaceutical composition of the present disclosure in a solution form or a freeze-dried form, (b) selectively a second container containing a diluent or a reconstitution solution for the freeze-dried formulation, and (c) selectively, a manual directed to (i) use of the solution or (ii) reconstitution and/or use of the freeze-dried formulation. The kit further has one or more of (iii) buffer, (iv) diluent, (v) filter, (vi) needle, or (v) syringe. The container is preferably a bottle, vial, syringe or test tube, or may be a multi-purpose container. The pharmaceutical composition is preferably freeze-dried.

The kit of the present disclosure preferably has a manual directed to the reconstitution and/or use of the freeze-dried formulation of the present disclosure in a suitable container. Examples of a suitable container include bottles, vials (e.g. dual-chamber vials), syringes (such as dual-chamber syringes) and test tubes. The container can be formed from various materials such as glass or plastic. Preferably, the kit and/or container includes a manual, on the container or accompanying the container, showing the method of reconstitution and/or use. For example, the label can explain that the freeze-dried formulation is reconstituted to the above-described peptide concentration. The label can further explain that the formulation is useful for subcutaneous injection or is for a subcutaneous injection.

The container of the formulation may be a multi-purpose vial that can be used in repeated administration (e.g. 2 to 6 administrations). The kit can further have a second container with a suitable diluent (e.g. sodium bicarbonate solution).

The final peptide concentration of a reconstituted formulation made by mixing the diluent and the freeze-dried formulation is preferably at least 0.15 mg/mL/peptide (= 75 µg) and preferably 3 mg/mL/peptide (= 1500 µg) or less. The kit can further include other materials that are desirable for commercial purpose or for users (including other buffer, diluent, filter, needle, syringe, and package insert).

The kit of the present disclosure can have a single container containing a formulation of the pharmaceutical composition of the present disclosure with or without other constituent elements (e.g. other compounds or pharmaceutical composition of the other compounds). Alternatively, the kit can have separate containers for each constituent element.

Preferably, the kit of the present disclosure includes a formulation of the present disclosure packaged for use as a combination with combined administration of a second compound (adjuvant (e.g. GM-CSF), chemotherapeutic agent, natural product, hormone or antagonist, anti-angiogenic agent or angiogenesis inhibitor, apoptosis inducer, chelating agent or the like) or a pharmaceutical composition thereof. The constituent element of the kit can be a complex made in advance, or each constituent element can be placed in separate containers until administration to a patient. The constituent element of the kit can be provided as one or more liquid solutions, preferably as aqueous solutions, and more preferably sterilized aqueous solutions. The constituent elements of the kit can also be provided as a solid, which preferably can be converted into a liquid by adding a suitable solvent provided in another separate container thereto.

The container of a therapeutic kit can be a vial, test tube, flask, bottle, syringe or any other means for sealing a solid or a liquid. When there are a plurality of constituent elements, the kit generally includes a second vial or another container such that the constituent elements can be administered separately. The kit can also include another container for a pharmaceutically acceptable liquid. Preferably, a therapeutic kit includes an instrument (e.g. one or more needles, syringes, instillators, pipettes or the like) that enables administration of an agent of the present disclosure, which is the constituent element of the kit.

The pharmaceutical composition of the present disclosure is suitable for administering the peptide by any acceptable route such as oral (enteral), trans-nasal cavity, transocular, subcutaneous, intradermal, intramuscular, intravenous or transdermal route. The administration is preferably subcutaneous administration and most preferably intradermal administration. Administration can be performed with an infusion pump.

### (Therapeutic Method)

In another aspect of the present disclosure, the peptide identified by the method of the present disclosure can also be used to provide a method of treating a patient suffering from a specific disease. For example, a dendritic cell vaccine is produced by mixing and culturing a peptide identified by the method of the present disclosure and dendritic cells derived from a test subject, and thus the vaccine can be administered to the patient. This is also called a DC vaccine therapy.

For example, since an individualized peptide is created based on the sequence information obtained from a tumor cell derived from the patient in DC vaccine therapy, such therapy does not act on a normal cell but act more specifically to a tumor cell such that a high therapeutic effect can be expected. Since a peptide is used as an antigen, unlike proteins, there is an advantage in being able to readily perform chemical synthesis.

DC vaccine therapy can be used in, for example, hematologic cancer such as acute myeloid leukemia and related precursor hematologic neoplasms, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T-cell leukemia/lymphoma, diseases similar to leukemia such as multiple myeloma and myelodysplastic syndrome, autoimmune diseases such as rheumatoid arthritis, systemic lupus erythematosus, and type I diabetes, and used for patients with various infections as well as patients with terminal cancer, a refractory autoimmune disease or severe infection.

In another embodiment, the method of the present disclosure can include: mixing the peptide identified by the method of the present disclosure, the dendritic cell or the antigen-presenting cell derived from the test subject, and a CD8 + T cell derived from the test subject and culturing the mixture to produce a CD8+ T cell-dendritic cell/antigen presenting cell-peptide mixture; and mixing the peptide with the dendritic cell derived from the test subject and culturing the mixture to produce a dendritic cell-peptide mixture; and administering a patient. This is also called a patient autoimmune cell therapeutic method.

For example, CD8+ T cell is stimulated and activated with a peptide derived from the patient as in a CTL therapeutic method and peptide sensitization of a dendritic cell is performed in a patient autoimmune cell therapeutic method. Such a therapeutic method is characterized in that a synergistic effect of a sustained effect due to the dendritic cell utilized as the antigen-presenting cell and an acute effect due to CTL imparting specificity can be expected by introducing both the dendritic cell and the CD8+ cell derived from the patient into the patient.

The patient autoimmune cell therapeutic method can be used in, for example, hematologic cancer (leukemia or the like), such as acute myeloid leukemia and related precursor hematologic neoplasms, lymphoblastic leukemia/lymphoma, T lymphoblastic leukemia/lymphoma, chronic lymphocytic leukemia/small lymphocytic lymphoma, B-cell prolymphocytic leukemia, hairy cell leukemia, T-cell prolymphocytic leukemia, T-cell large granular lymphocyte leukemia, and adult T-cell leukemia/lymphoma, diseases similar to leukemia such as multiple myeloma and myelodysplastic syndrome, autoimmune diseases such as rheumatoid arthritis, systemic lupus erythematosus, and type I diabetes, and used for patients with various infections as well as patients with terminal cancer, a refractory autoimmune disease or severe infection.

### (Application as Diagnosis or Diagnostic Agent)

According to the method of the present disclosure, it is possible to obtain a specific epitope for a disease (e.g. cancer) caused by somatic mutation, for example, by performing repertoire analysis. Thus, it is possible to perform therapy or diagnosis such as immunotherapy or immune monitoring. As such an example, the method can be applied in, for example, a neoantigen technique based on the presence of an immune response targeting an individual gene mutation (unique antigen) and obtainment of an antitumor effect, or mutanome analysis that comprehensively analyzes the neoantigen. Other diseases for which the present disclosure may be directed may include, for example, autoimmune diseases due to autoreactive T cells. In this case, evidence has been shown that abnormalities in T cells are associated with etiology in many autoimmune diseases, and thus such information can be exploited. The present disclosure can be applied because it can easily identify and separate specific T cells that cause a specific disease, and also determine a recognition molecule (pathogenic antigen) thereof. For example, regarding the autoreactive T cells, rheumatoid arthritis, type I diabetes and multiple sclerosis are diseases caused by specific T cells against an antigen of an unknown joint. Thus, these diseases can be mentioned as target diseases. In autoimmune diseases, an autoantigen recognized by T cells in autoimmunity is identified, and the activation of autoreactive T cells is suppressed or the activation itself is inhibited, thereby suppressing the onset of diseases. It is considered that the breakdown of immune tolerance originally established for self is involved in the induction of autoimmunity. However, the present invention can identify not only known pathogenic antigens (epitopes) but also unknown pathogenic antigens (epitopes) by comprehensively examining and searching the presence or absence of somatic mutations on the antigen side that induces autoimmunity. Thus, the disease treatment and prevention can be carried out. The present invention can also be applied in diagnosis and prevention of the presence or absence of a pathogenic antigen of an autoimmune disease, and can also be applied in development of a therapeutic agent targeting the pathogenic antigen.

### (Application in Drug Discovery)

For the purpose of drug discovery, the present disclosure can be used as a tool for searching for an agent or a pharmaceutical composition for various pharmaceutical applications described herein.

As described elsewhere in the present disclosure, various medicines, therapeutic agents, diagnostic agents, and the like can be produced using the peptide identified by the method of the present disclosure. The formulation procedure as a medicine such as a diagnostic agent, a therapeutic agent or a prophylactic agent that can be used in the present disclosure is known in the art, and is described in, for example, the Japanese Pharmacopoeia, the U.S. Pharmacopoeia or the pharmacopoeia of other countries, or is described in other literatures. Therefore, those skilled in the art, given the description herein, can determine the amount to be used without undue experimentation.

### (General Techniques)

Molecular biological, biochemical, and microbiological methods used herein are well known and known techniques in the art that are described in, for example, Sambrook J. et al. (1989). Molecular Cloning: A Laboratory Manual, Cold Spring Harbor, and 3rd Ed. thereof (2001); Ausubel, F. M. (1987). Current Protocols in Molecular Biology, Greene Pub. Associates and Wiley-Interscience; Ausubel, F. M. (1989). Short Protocols in Molecular Biology: A Compendium of Methods from Current Protocols in Molecular Biology, Greene Pub. Associates and Wiley-Interscience; Innis, M. A. (1990). PCR Protocols: A Guideto Methods and Applications, Academic Press; Ausubel, F. M. (1992). Short Protocols in Molecular Biology: A Compendium of Methods from Current Protocols in Molecular Biology, Greene Pub. Associates; Ausubel, F. M. (1995). Short Protocols in Molecular Biology: A Compendium of Methods from Current Protocols in Molecular Biology, Greene Pub. Associates; Innis, M. A. et al. (1995). PCR Strategies, Academic Press; Ausubel, F. M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Gait, M. J. (1985). Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (1996). Bioconjugate Techniques, Academic Press, Bessatsu Jikken Igaku [Experimental Medicine, Supplemental Volume], Idenshi Donyu & Hatsugen Kaiseki Jikken Ho [Experimental Methods for Transgenesis & Expression Analysis], Yodosha, 1997, and the like. The relevant portions (which can be the entire document) of which are incorporated herein by reference.

Literatures of common technical knowledge commonly used in bioinformatics include, for example, Gibas C. et al. (2001). Developing Bioinformatics ComputerSkills, O'Reilly; Mount D. W., (2004). Bioinformatics: Sequence and Genome Analysis, CSHL Press; Pevzner P. et al. (2011). Bioinformatics for Biologist, Cambridge University Press; Sumio Kanno, et al. (2012) Cell Engineering Annex, "Advanced Methods for Next-generation Sequencers by Purpose", Shujun-sha. The relevant portions (which can be the entire document) of which are incorporated herein by reference.

Reference literatures cited herein, such as scientific literatures, patents, and patent applications, are hereby incorporated by reference in their entirety to the same extent as each was specifically described.

The present disclosure has been described above with reference to preferred embodiments for easy understanding. Hereinafter, the present disclosure will be described based on examples, but the above description and the following examples are provided by way of example only and are not meant to limit the present disclosure. Accordingly, the scope of the present disclosure is not limited to the embodiments or examples specifically described herein, but is limited only by the claims.

### Examples

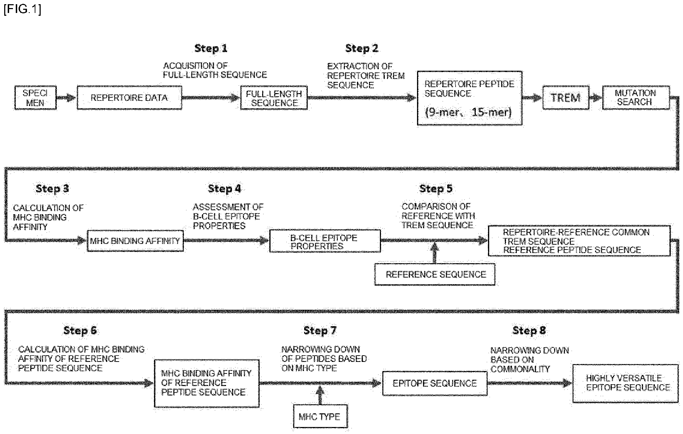

In the following Examples, the antigen prediction technique was performed according to the outline shown in FIG. 1.

It is understood that the calculations, algorithms, software to be used, programs, and applications exemplified in the Examples, and the present disclosure can be implemented by using other software or the like that realizes similar functions.

### (Example 1: TCR/BCR Repertoire Analysis/Full-length Sequence)

Whole blood (PBMC) was collected from 18 Vietnamese patients infected with dengue virus in both the acute and recovery stages (after 8 months and 1 year). Note that the identified information was used for the HLA type of each of the patients.

TCR/BCR repertoire analysis was performed using this specimen. Specifically, TCR/BCR repertoire analysis was performed in the following manner. RNA was extracted from the whole blood (PBMC) specimen, cDNA was synthesized, and then gene amplification targeting IgG, IgM, TRA and TRB was performed by an adaptor-ligation PCR method. The gene amplicons were subjected to paired-end sequencing by a sequencer MiSeq manufactured by Illumina Inc., Read 1 (C-region side read) and Read 2 (V-region side read) of 300 bp each were acquired, and repertoire analysis was performed with a repertoire analysis software: Repetoire Genesis.

The TCR/BCR repertoire data obtained from the TCR/BCR repertoire analysis was used to perform T-cell epitope identification analysis for each specimen. Specifically, as shown in Step 1 of FIG. 2A, the top 1% of clones were first selected from the specimens based on the clone information of the repertoire analysis result, and the full-length of each of the clones was analyzed. Full-length analysis was performed as a series of analyses including the following steps for each clone.
- Step 1.1: Acquire full-length DNA sequence
- Step 1.2: Convert amino acid
- Step 1.3: Acquire germline sequence
- Step 1.4: Convert germline sequence into amino acid
- Step 1.5: Detect mutation
- Step 1.6: Add gene information
- Step 1.7: Add mutation information
- Step 1.8: Create full-length analysis report

Hereinafter, the flow of each step will be specifically described.

### (Step 1.1)

### Acquire full-length DNA sequence

The clone information of the repertoire analysis result (the V, D, J and C gene determination result and the CDR3 sequence) and sequence reads were used to acquire sequence reads corresponding to each clone, and the reads were combined to reconstruct one sequence information, thereby acquiring the full-length DNA sequence of each clone. Further, allele information of each gene fragment (V, D, J or C) for each clone was acquired from the clone information of the repertoire analysis result.

The step of combining and reconstructing sequence reads was performed in the following manner. First, a sequence alignment of sequence reads on the C region side was created, and a constant length sequence with the highest commonality in the 3' direction was obtained from the CDR3 sequence as a starting point. This was repeated up to the end of the lead, and the obtained sequence was bound and converted into one sequence (C region 3'-side read). The constant length sequence was 60 bp. Next, similarly, the CDR3 sequence was used as a starting point, and a constant length sequence with the highest commonality in the 5' direction was acquired. This was repeated up to the end of the lead, and the acquired sequence was bound and converted into one sequence (C region 5'-side read). Here, the constant length sequence was 60 bp. Next, a 5' end sequence of the C region 5'-side read was acquired, and sequence reads on the V region side including the sequence was acquired to create a sequence alignment. In the sequence alignment of sequence reads on the V region side, the sequence was used as a starting point to acquire a constant length sequence with the highest commonality in the 5' direction. This was repeated up to the end of the lead, and the acquired sequence was bound and converted into one sequence (V region 5'-side read). Here, the constant length sequence was 21 bp. Finally, the three obtained sequences were linked to form one full-length DNA sequence.

### (Step 1.2)

### Convert amino acid

The full-length DNA sequence of the clone obtained in Step 1.1 was converted into an amino acid sequence based on codon conversion, and a full-length amino acid sequence was acquired. Further, annotation was performed to associate each converted amino acid with codon information and the base position of the full-length DNA sequence.

### (Step 1.3)

### Acquire germline sequence

Sequence information of each gene fragment was acquired based on the allele information of each gene fragment obtained in Step 1.1. These gene fragment sequences were combined to construct a full-length sequence of germline type.

### (Step 1.4)

### Convert germline sequence into amino acid

The germline full-length sequence obtained in Step 1.3 was converted into an amino acid sequence based on codon conversion, and a full-length amino acid sequence was acquired. Further, annotation was performed to associate each converted amino acid with codon information and gene fragment information.

### (Step 1.5)

### Detect mutation

The germline full-length sequence and the clone full-length sequence were used to create sequence alignments of the DNA sequence and the amino acid sequence. Here, each of the sequence alignments was created using an existing sequence alignment program: Clustal Omega. A difference between two sequences on sequence alignment was identified, and this difference was detected as a somatic hypermutation occurring in the clone.

### (Step 1.6)

### Add gene information

Information addition was performed in association with the annotated germline full-length sequence, gene fragment information and CDR3 information obtained in Step 1.4, similarly to the annotated clone full-length sequence obtained in Step 1.2, based on the sequence alignment obtained in Step 1.5.

### (Step 1.7)

### Add mutation information

Mutation information on DNA and amino acid levels in the mutation site specified above was added to the annotated clone full-length sequence information obtained in Step 1.6. Here, "1" was output when there was a mutation, whereas "0" was output when there was no mutation.

### (Step 1.8)

### Create full-length analysis report

The above-described analysis results were aggregated and output.

### (Example 2: Extraction of Repertoire TREM sequence)

Subsequently, in this example, as shown in Step 2 of FIG. 2B, continuous 9-mer and 15-mer peptide sequences were created from the TCR/BCR full-length sequence obtained in Step 1, TREMI/IIa/IIb was extracted, mutation search was performed, and the mutation rate was assessed.

Specifically, the process was performed in the following manner.

9-mer and 15-mer peptide sequences were prepared for the full-length amino acid sequences of both the germline type and the clone obtained in Step 1. Here, the 9-mer and 15-mer peptide sequences are a peptide sequence group obtained by moving one amino acid at a time from the N-terminal side to the C-terminal side of the full-length amino acid sequence and extracting consecutive 9 and 15 amino acids.

Next, a TREM sequence was extracted from each of the prepared 9-mer and 15-mer peptide sequences. Here, the TREM sequence is a continuous (TREMI) or discontinuous (TREMIIa, TREMIIb) motif sequence composed of 5-mer amino acids, is a sequence (TREMI) composed of amino acids present at positions P4, P5, P6, P7 and P8 in the 9-mer amino acid sequence (with position numbers P1 to P9), and include sequences (TREMIIa and TREMIIb, respectively) composed of amino acids present at positions P2, P3, P5, P7 and P8, or positions P-1, P3, P5, P7 and P8 in the 15-mer amino acid sequence (with position numbers P-3 to P12).

Next, the extracted germline type was compared with the TREM sequence and MAM sequence derived from the clone. In a case where there was a difference in the amino acid corresponding to the same position between both the sequences, it was determined that a mutation occurred in the amino acid derived from the clone. Based on the mutation information annotated in Example 1, the number of mutations determined in each of the 9-mer, 15-mer and each TREM sequence was calculated as the number of mutations, and a ratio of the number of mutations to the length of each sequence was calculated as the mutation rate. For example, when there were two mutations in the 5-mer TREM sequence, the mutation rate was 2/5 = 40.00 (%). At this time, the mutation rate as the 9-mer peptide sequence was 2/9 = 22.22 (%).

### (Results)

Some of the lists of the obtained sequential sequences: 9-mer and 15-mer peptides are shown in Tables 1-1 to 1-4 (the peptide sequences shown in the column clone_aa15 correspond to SEQ ID NOs: 1 to 86 in order from the top, and the peptide sequences shown in the column clone_aa9 correspond to SEQ ID NOs: 87 to 172 in order from the top).

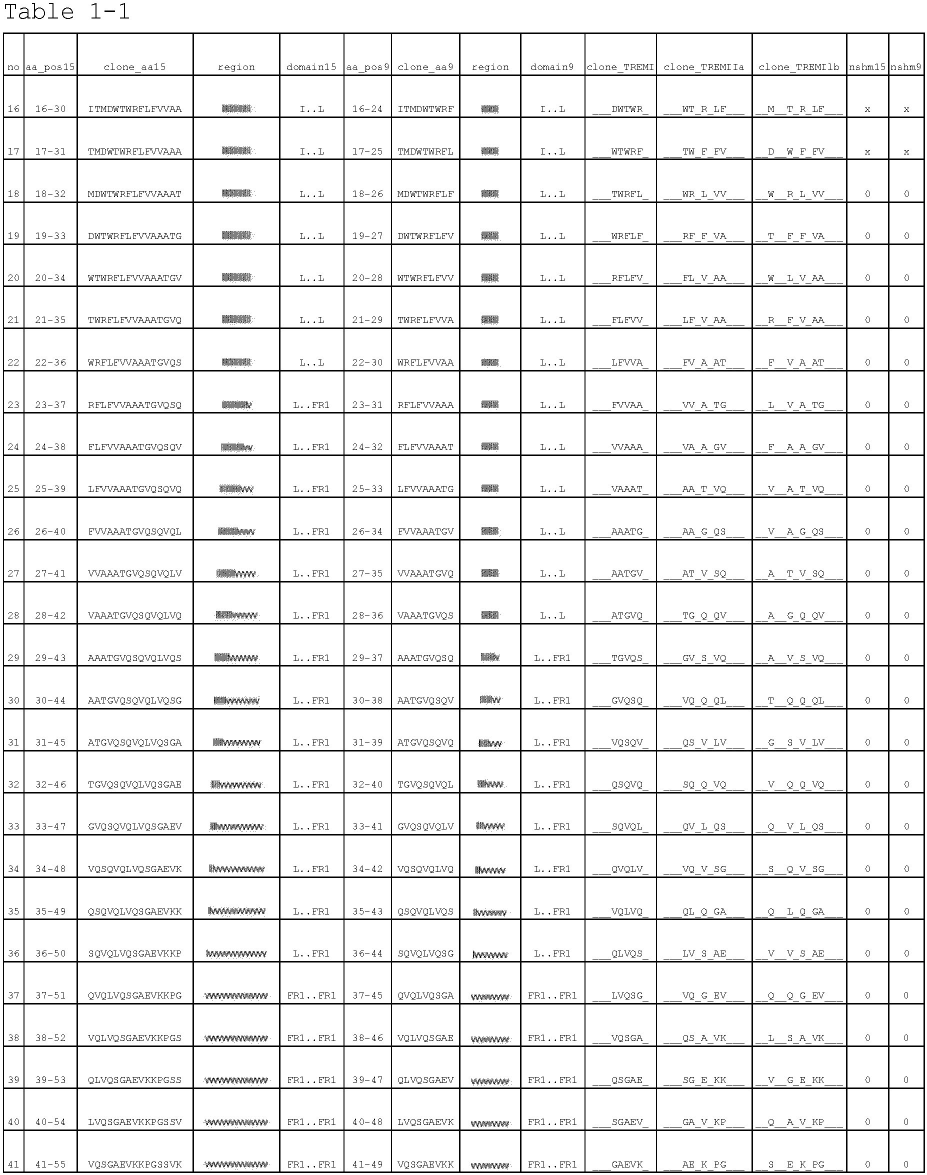

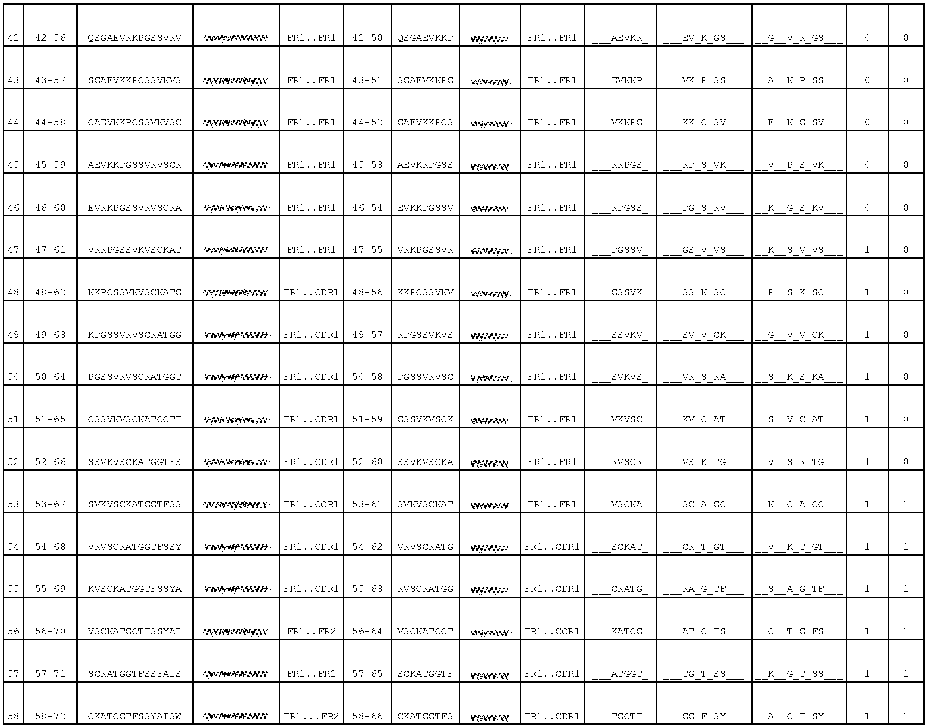

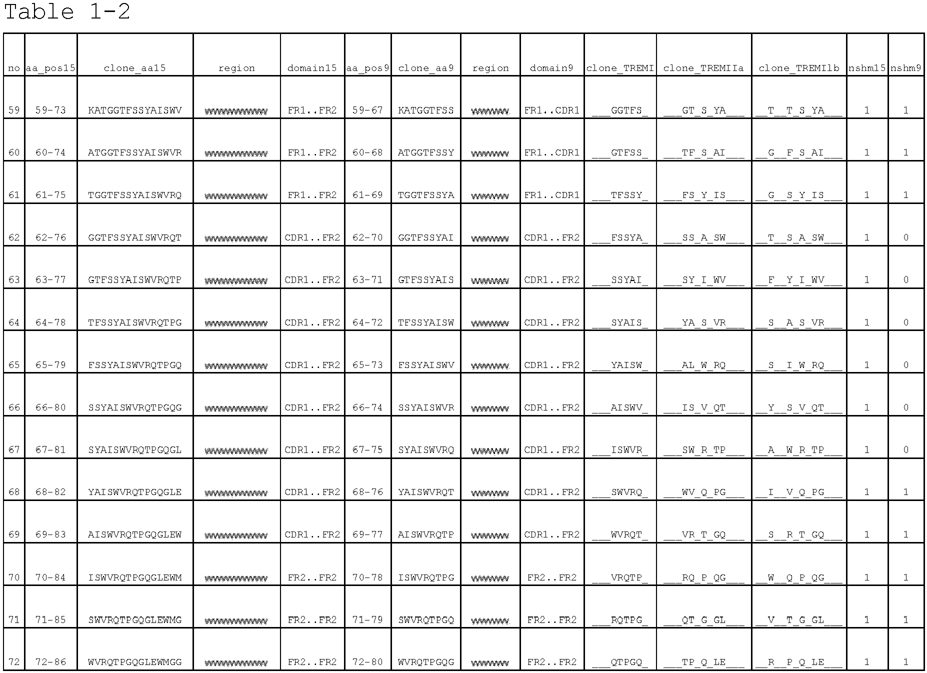

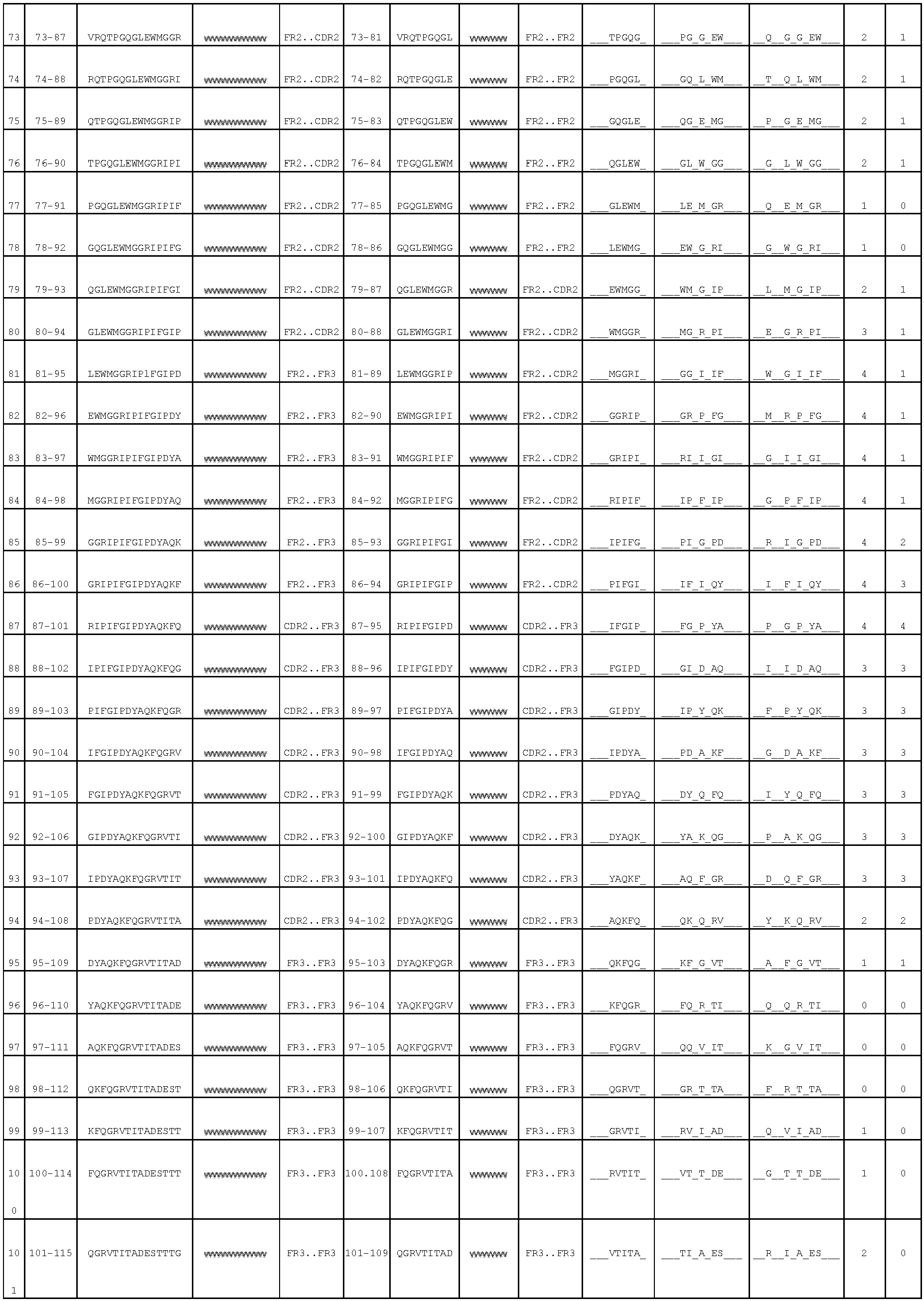

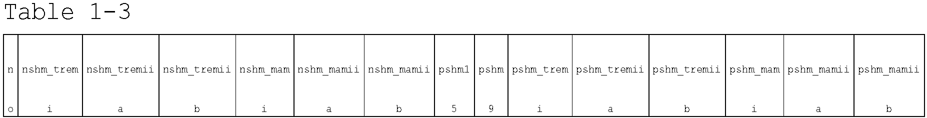

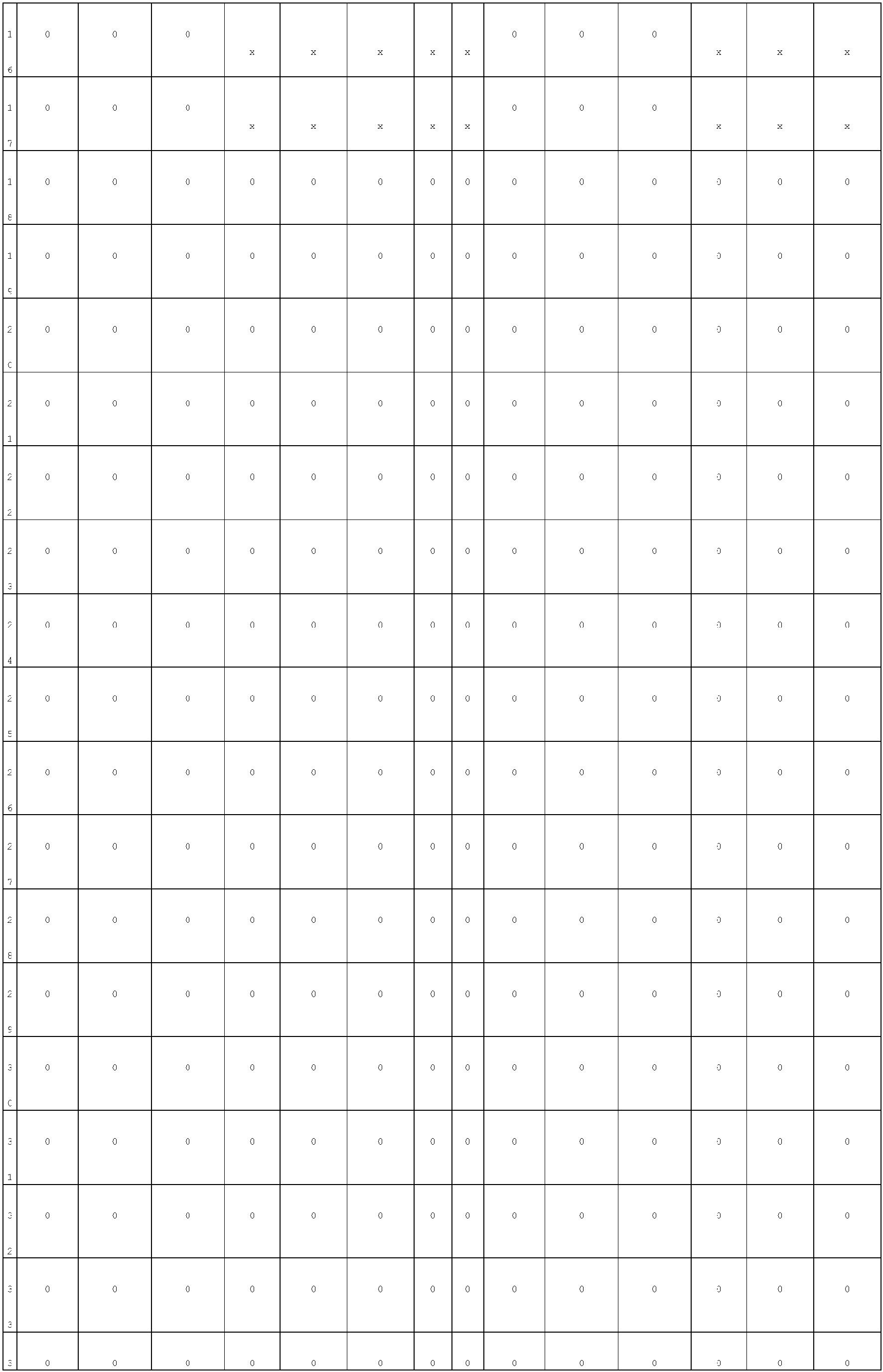

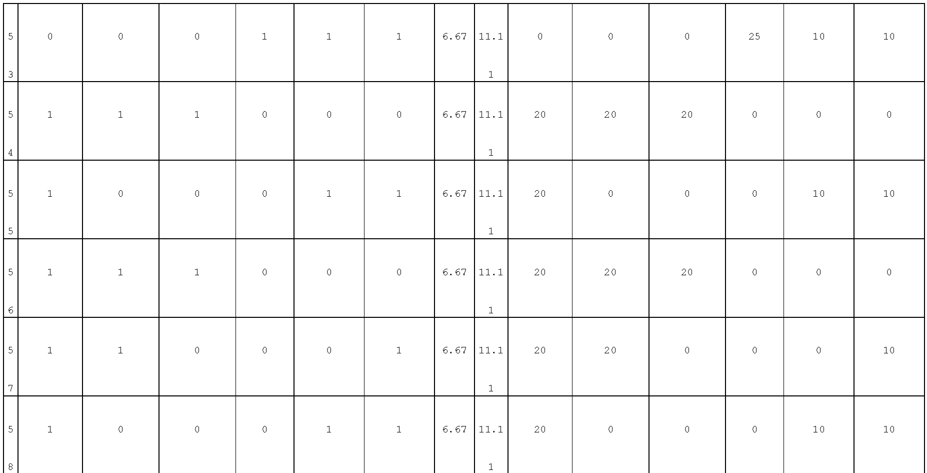

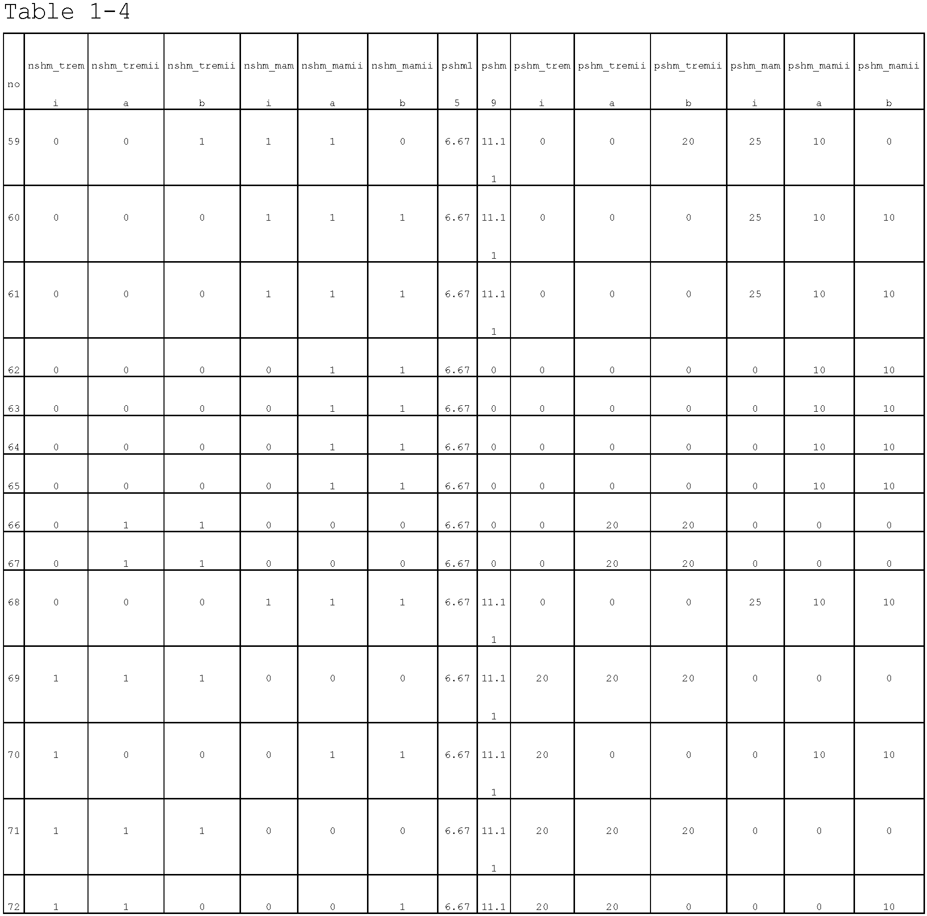

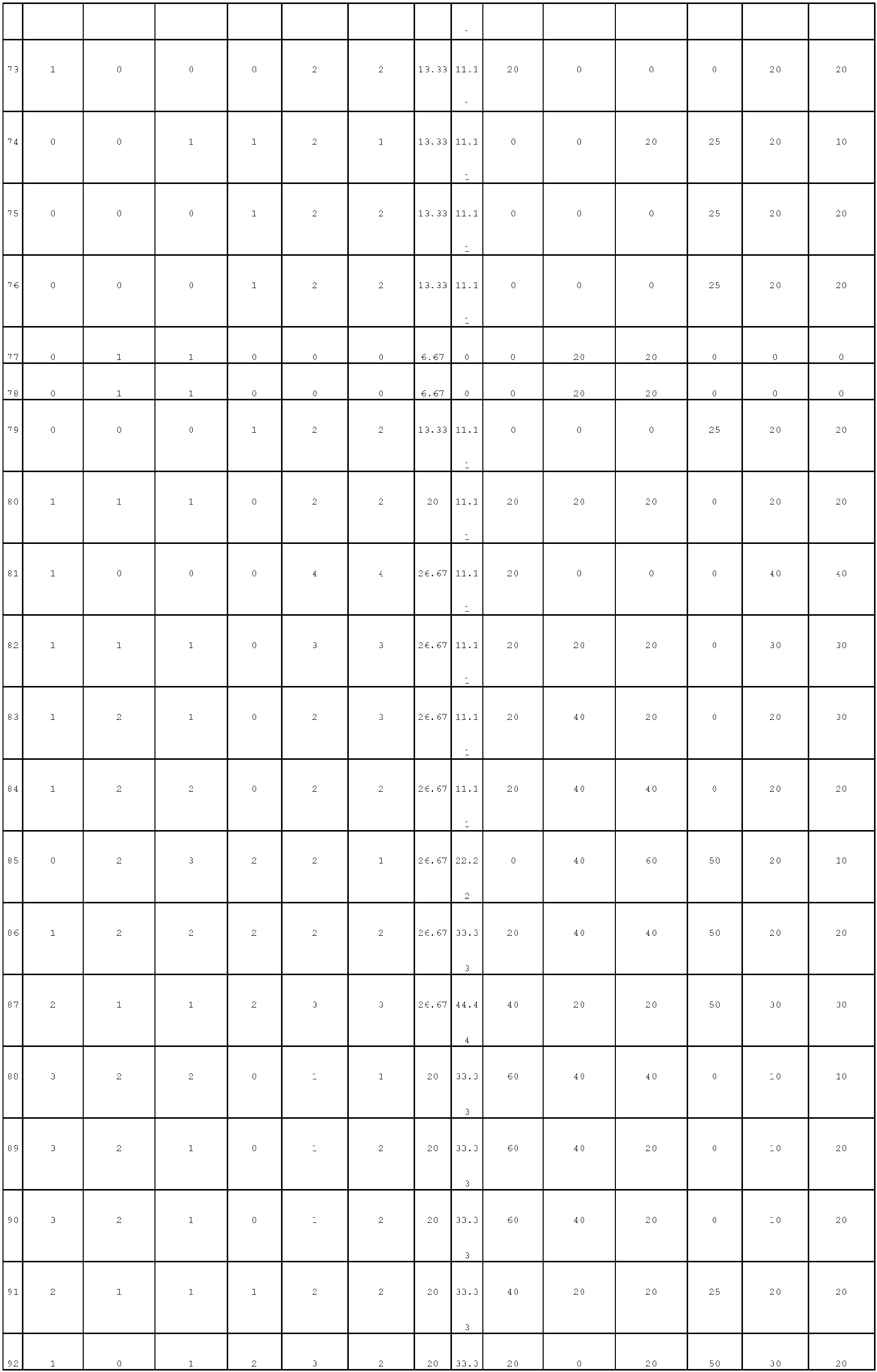

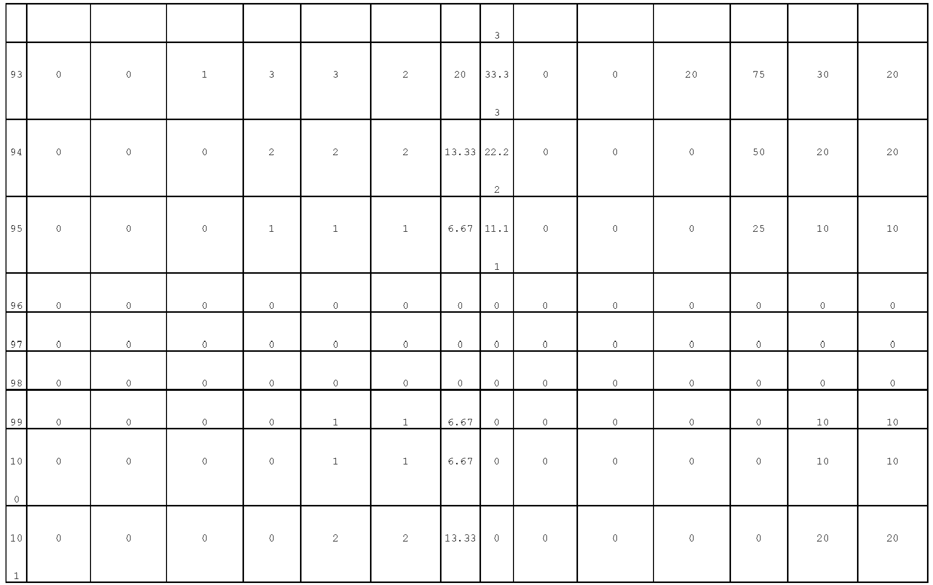

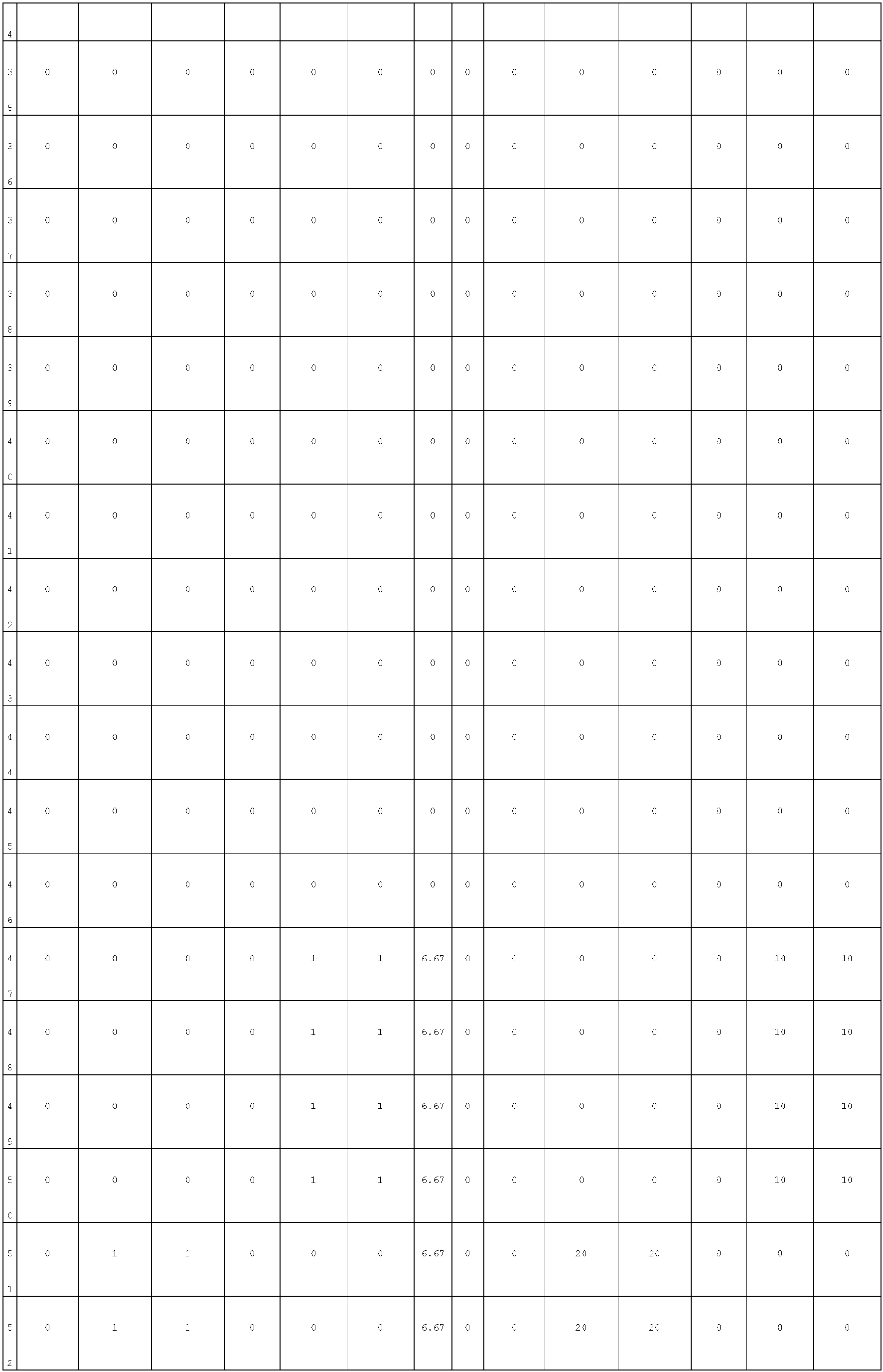

A part of the list of the obtained full-length sequences is shown in Table 2 below (the peptide sequences shown in the column sequence include SEQ ID NOs: 173 to 192 in order from the top) .

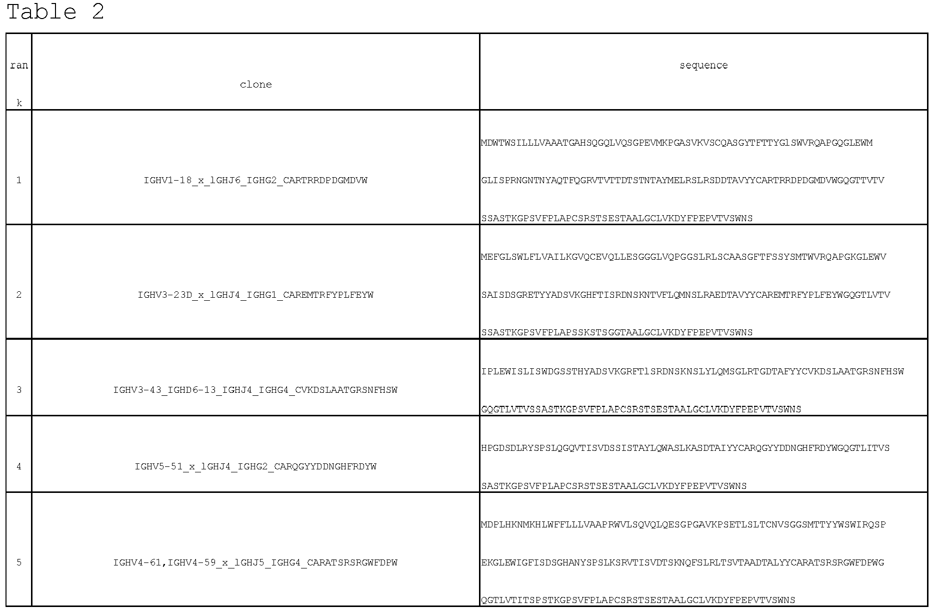

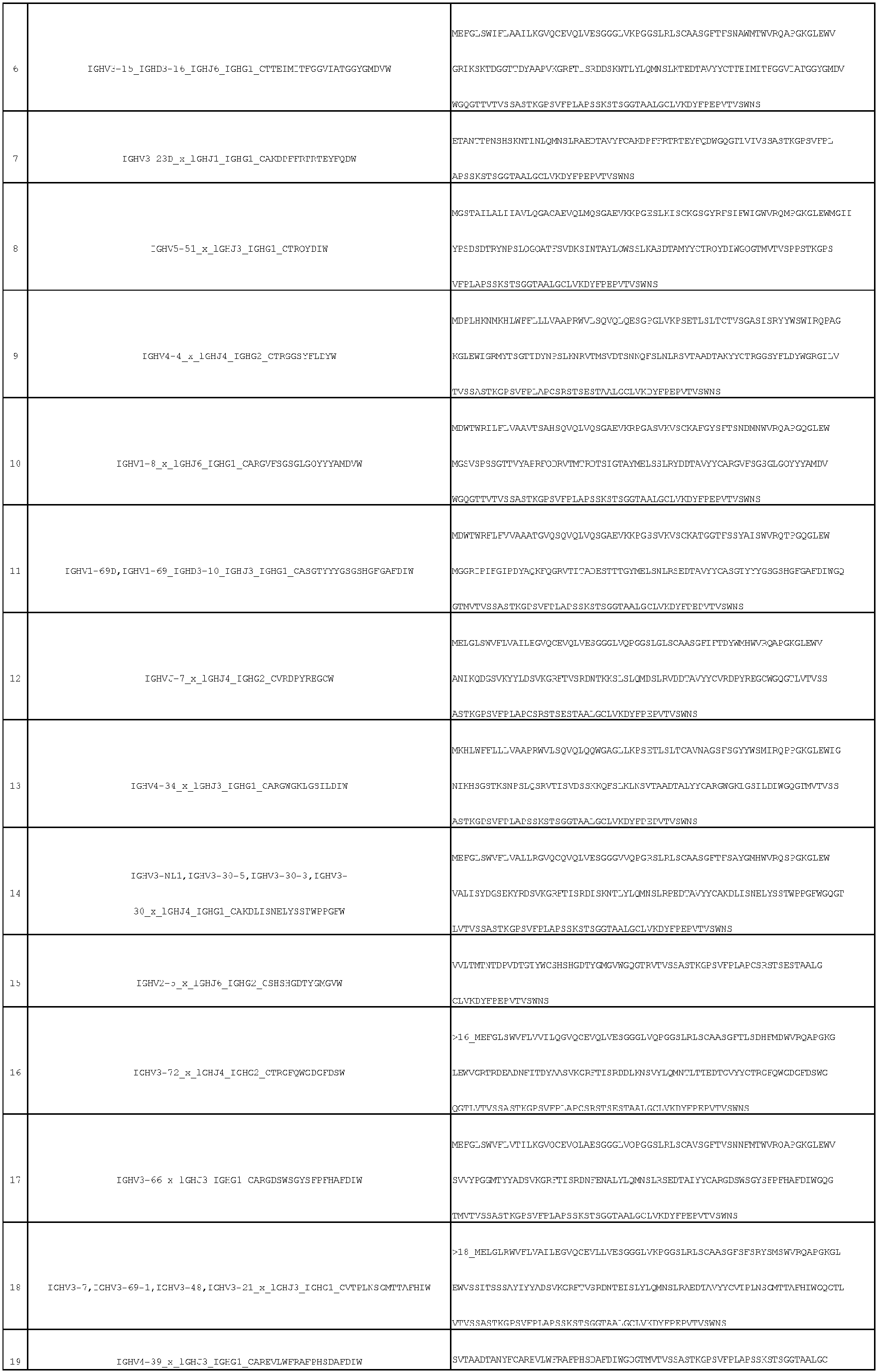

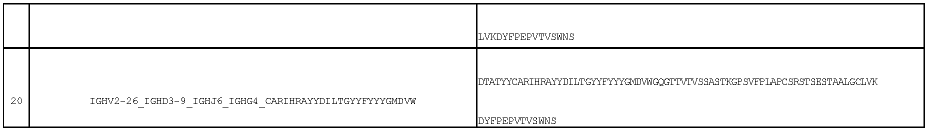

### (Example 3: Calculation of MHC Binding Affinity)

In this example, calculation of MHC binding affinity was conducted.

Thereafter, as shown in Step 3 of FIG. 2B, MHC binding affinity was calculated. Specifically, in the calculation of MHC binding affinity, IC50 for MHC class I was calculated for the 9-mer, and IC50 for MHC class II was calculated for the 15-mer.

MHC types using the 9-mer and 15-mer peptide sequences obtained from the clone sequence were used to calculate IC50 values as binding affinity for the designated MHC type. For the MHC type, HLA types of the group of 18 dengue-infected patients were used. In the calculation of the IC50 values showing MHC binding affinity, NetMHCpan was used for MHC class I, and NetMHCIIpan was used for MHC class II.

### (Results)

IC50 values of some of the obtained peptides were shown in Table 3-1 (class I) and Table 3-2 (class II) (SEQ ID NOs: 193 to 242 are assigned to the peptide sequences shown in the column mhc in Table 3-1 from the top, and SEQ ID NOs: 243 to 292 are assigned to the peptide sequences shown in the column mhc in Table 3-2 from the top).

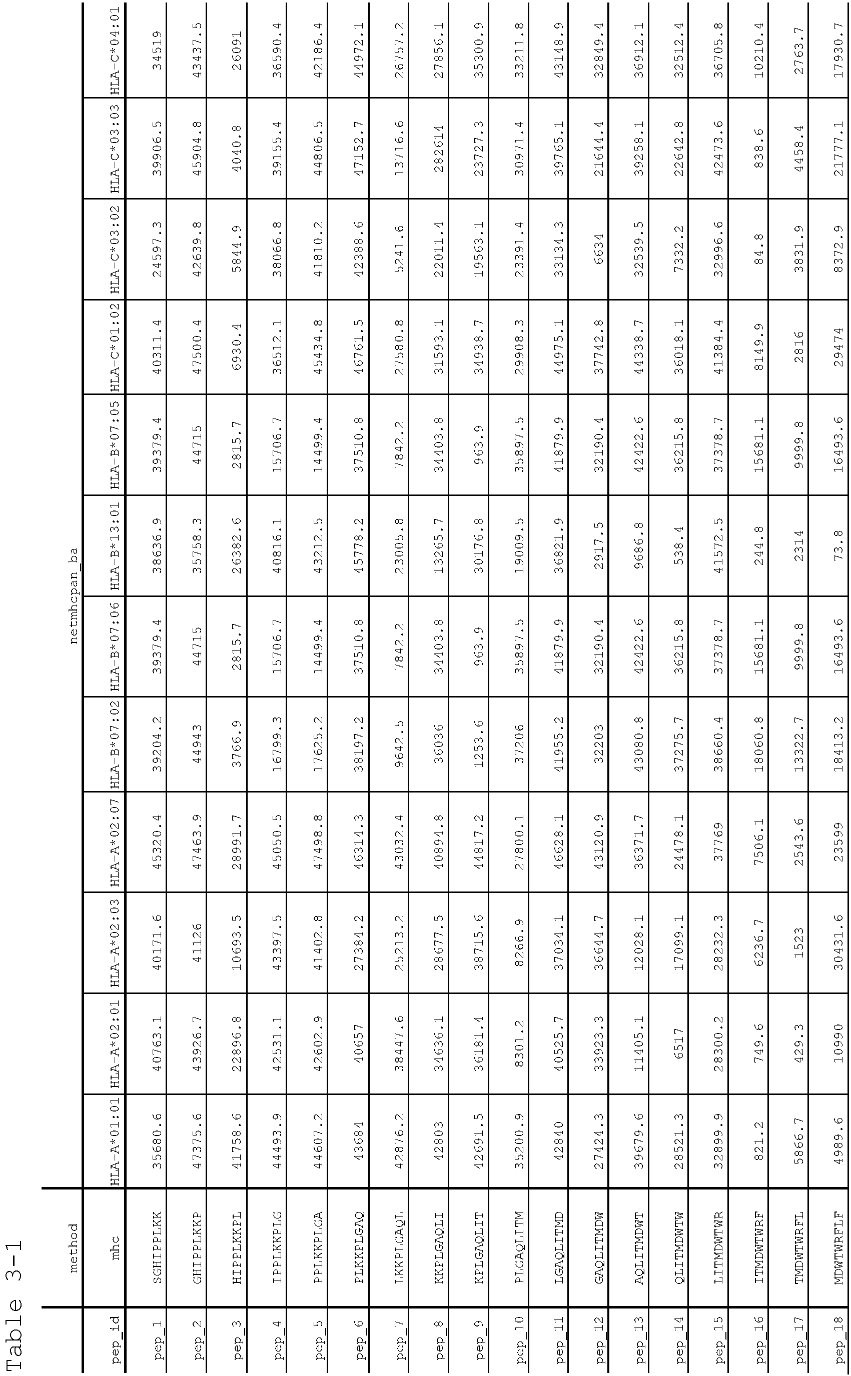

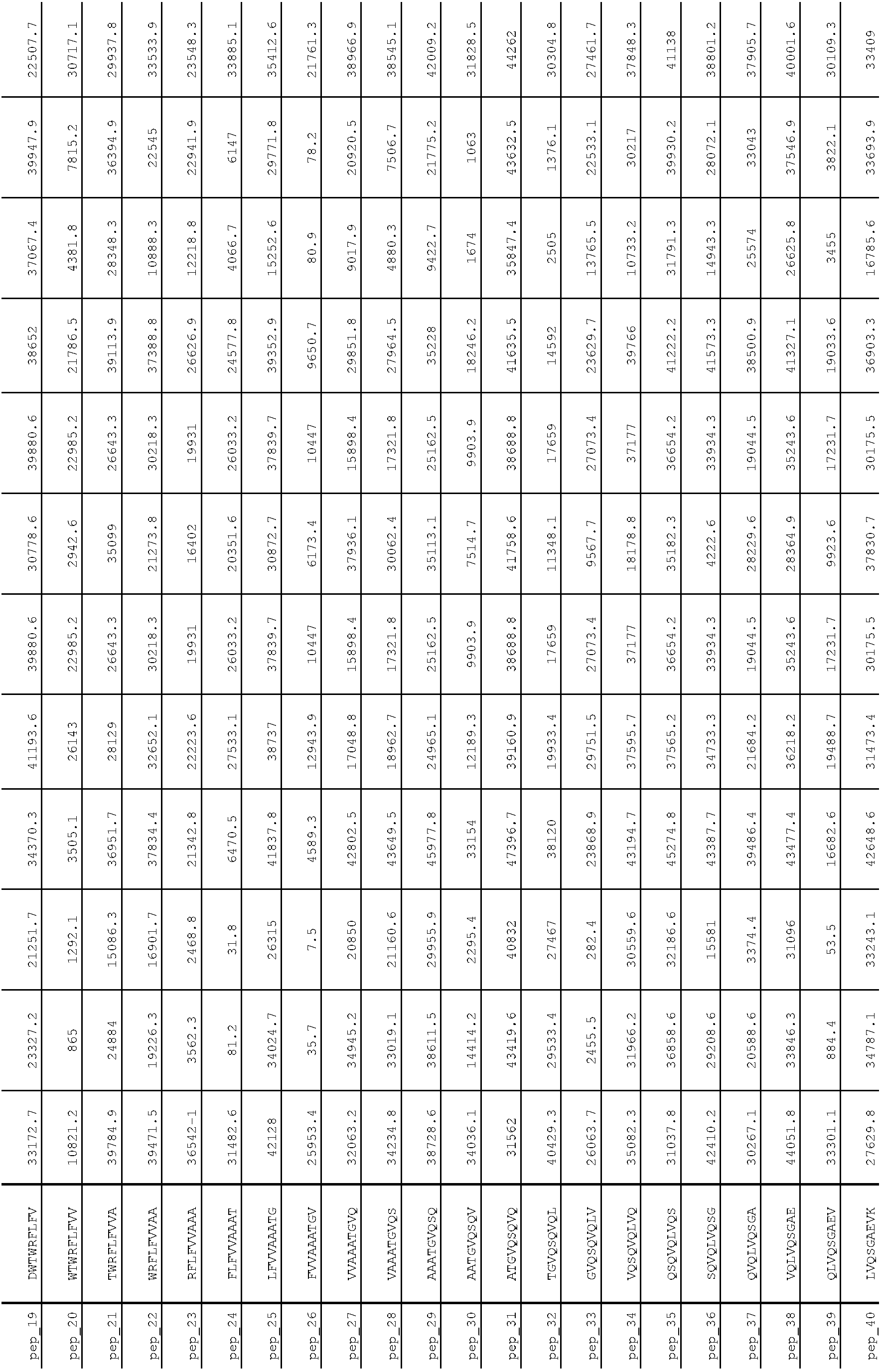

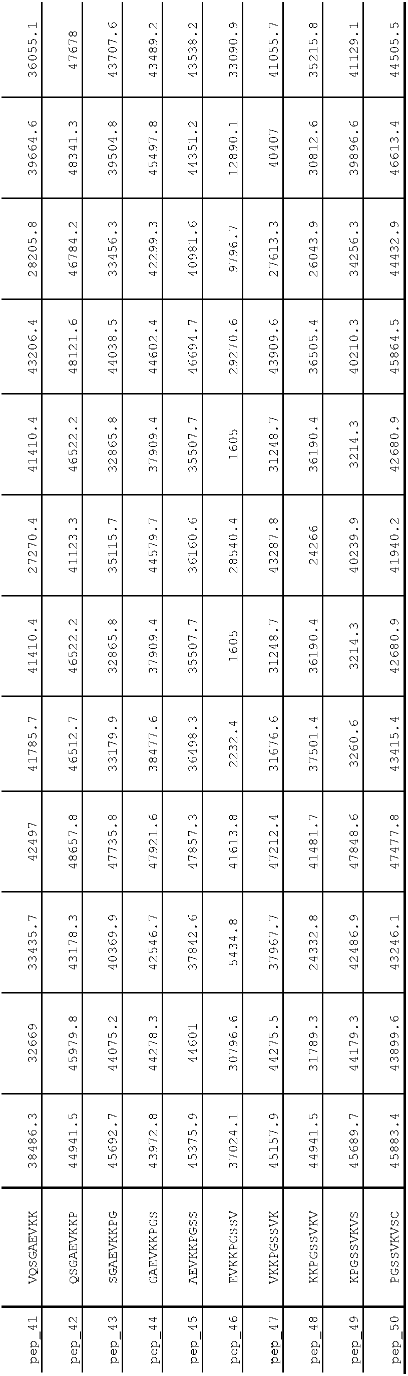

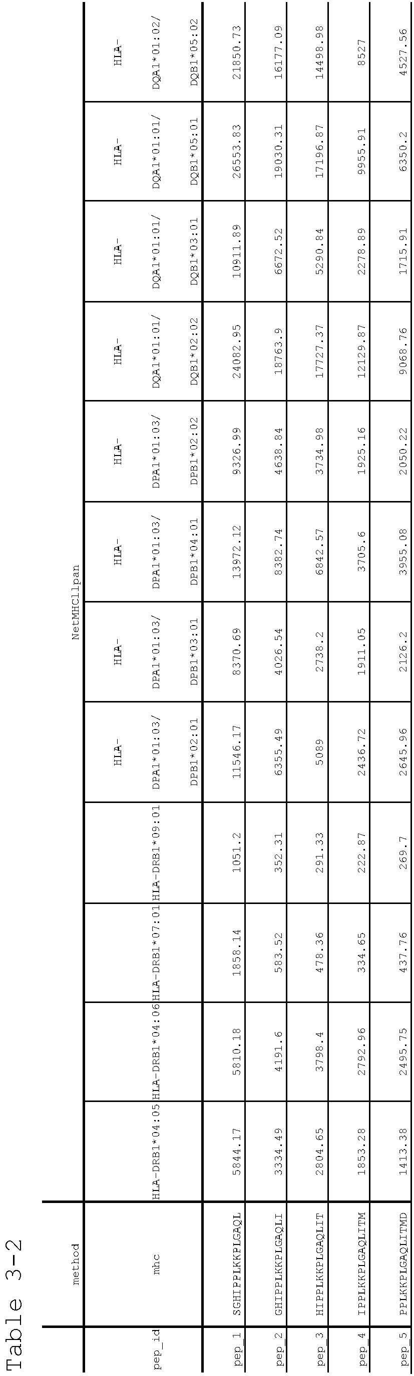

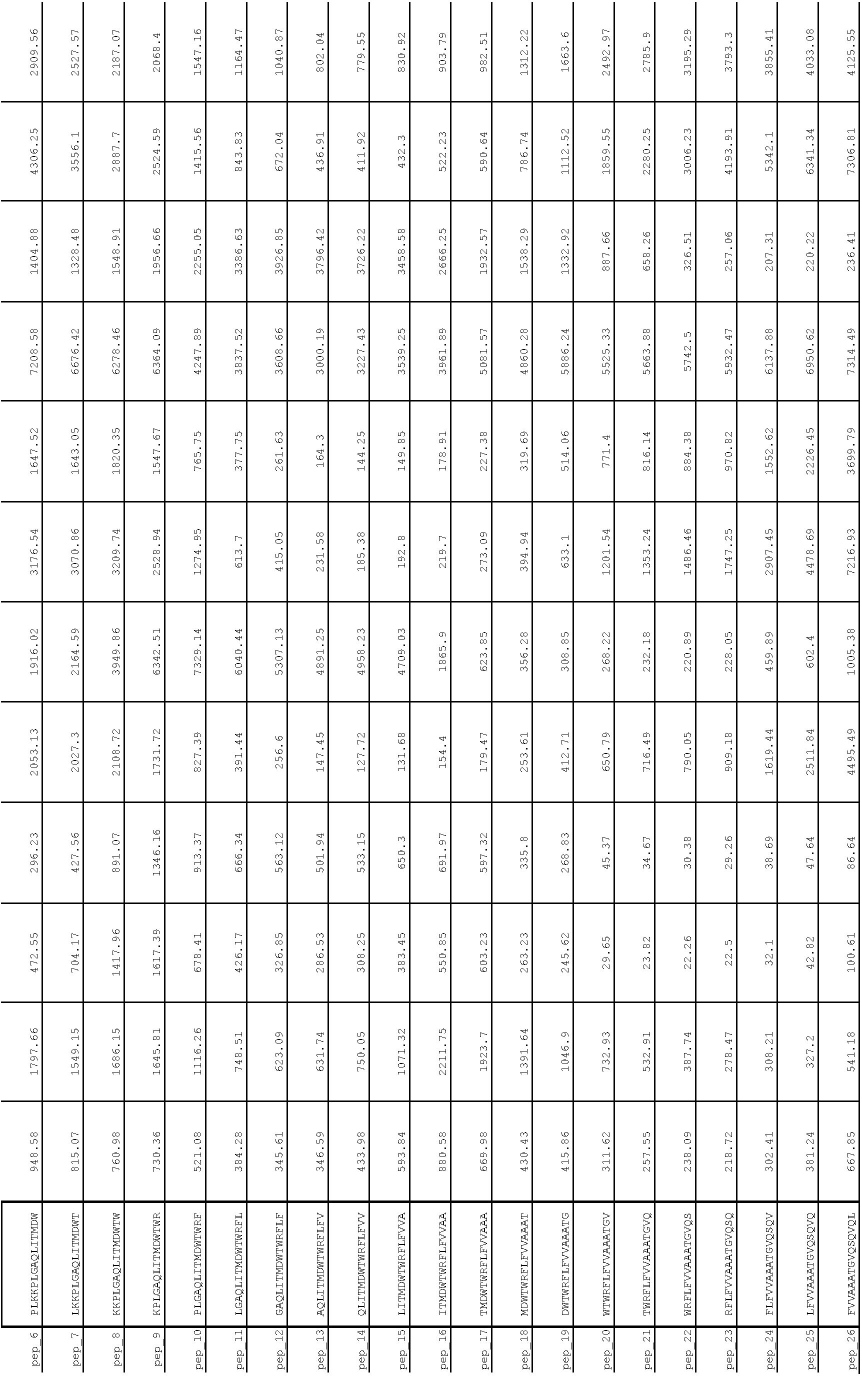

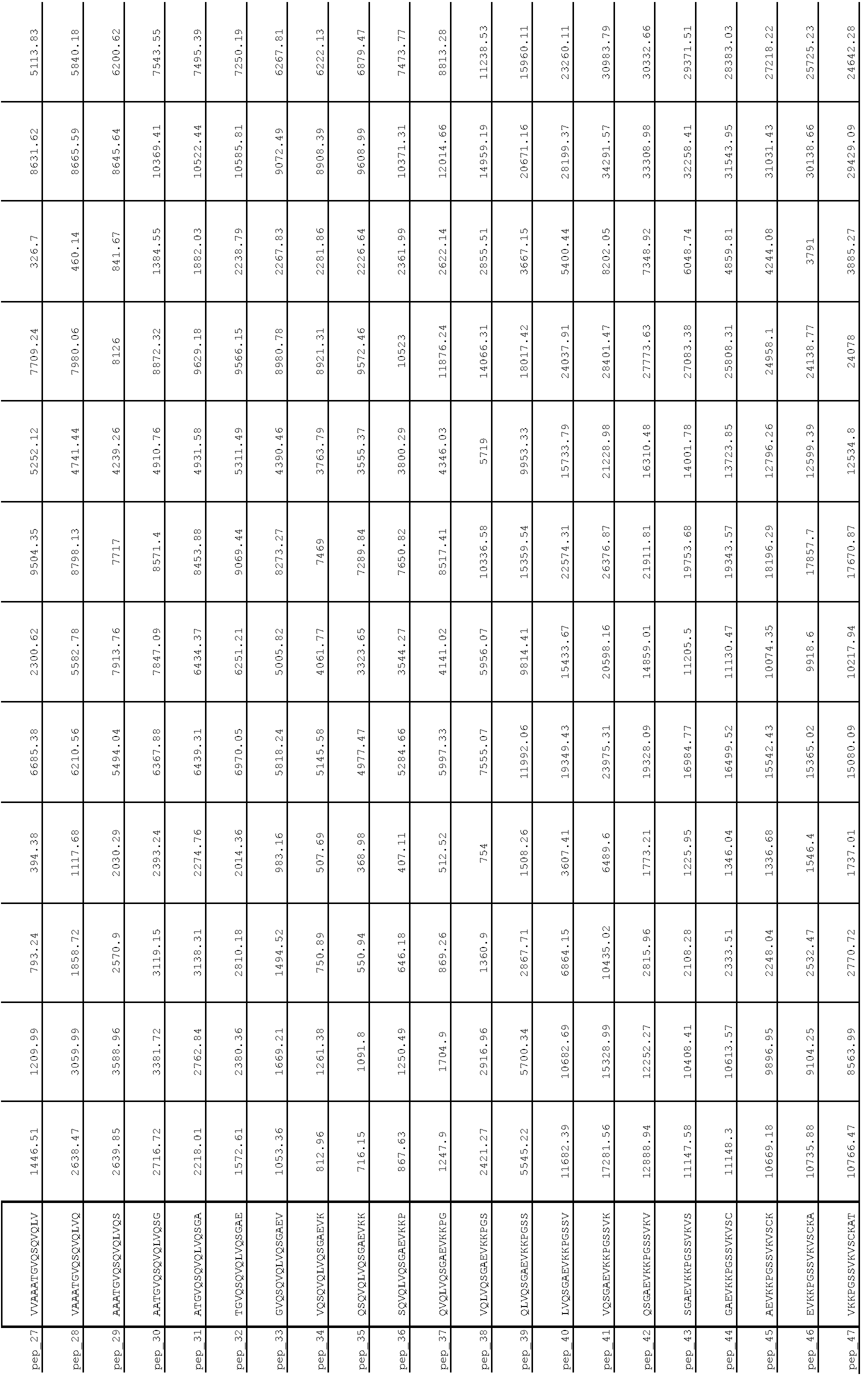

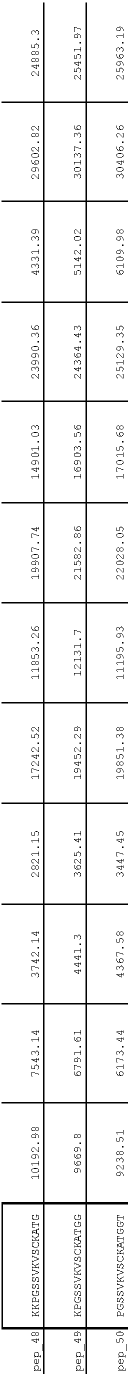

### (Example 4: Assessment of Peptides with B-Cell Epitope Properties)

In this example, peptides with B-cell epitope properties were assessed. The B-cell epitope properties of the full-length amino acid sequence of the clone obtained in Example 1 were assessed to obtain peptides with B-cell epitope properties (Step 4). The B-cell epitope properties were evaluated in the following manner.

The B-cell epitope properties were assessed on the full-length amino acid sequence of the clone obtained by the full-length analysis. Here, the B-cell epitope properties were assessed using the existing software BepiPred-2.0. A score was calculated for each amino acid residue. When the score was equal to or greater than a threshold value (default value was 0.5), it was determined that B-cell epitope properties were exhibited, and the score was output as "E".

### (Results)

The peptides with B-cell epitope properties obtained as described above are shown in Table 4 below.

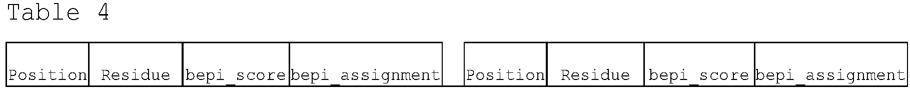

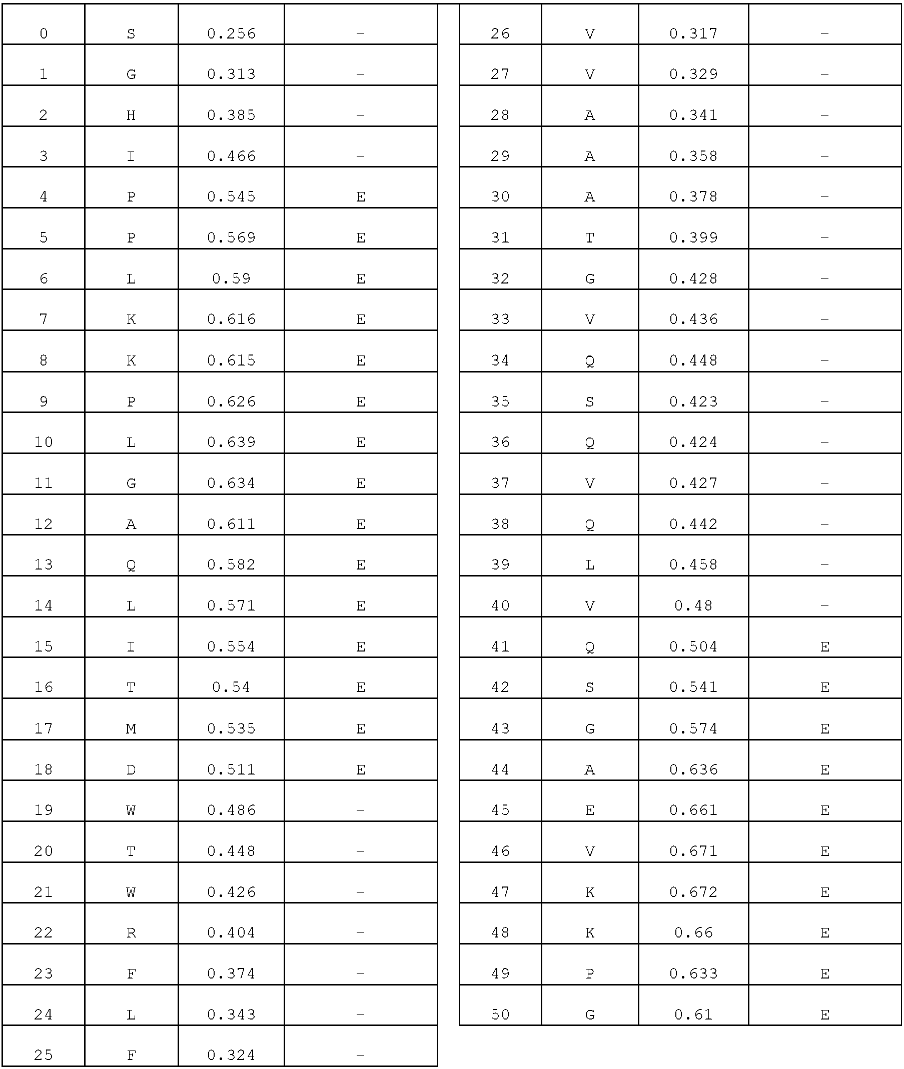

### (Example 5: Comparison with Various TREMs Depending on Physiological State)

In this example, a repertoire specific TREM derived from the clone was compared with a reference sequence, and the same TREM sequence of the repertoire clone sequence as the TREM sequence of the reference sequence as well as the reference sequence peptide were extracted. The repertoire specific TREM from the clone was compared with the reference sequence to identify the peptide sequence in the references with the same TREM.

### (5-1) Infection

A dengue fever infection was taken as an example, a dengue virus-infected patient's sequence was used as a reference sequence unique to a first specimen, a dengue virus sequence from a public database was used as a second reference sequence to be compared, and a protein sequence of another flavivirus belonging to the same Flaviviridae family as that of the dengue virus was used as a third reference sequence. Then, these sequences were compared with their TREMs.

### (Results)

As shown in Table 5 below, 9-mer and 15-mer peptide sequences derived from the reference sequence including the common TREM sequence between the repertoire clone sequence and the reference sequence were identified (the peptide sequences shown in the column clone_aa 15 correspond to SEQ ID NOs: 293 to 342 from the top, the peptide sequences shown in the column clone_aa 9 correspond to SEQ ID NOs: 343 to 392 from the top, the peptide sequence shown in the column TREMIIa:dbuniq:dbuniq/E:E:01-TN-081_envelope_protein corresponds to SEQ ID NO: 393, the peptide sequence shown in the column TREMIIb:dbuniq:dbuniq/NS3:NS3:01-TN-081_nonstructural_protein_3 corresponds to SEQ ID NO: 394, and the peptide sequence shown in the column TREMIIb:dbuniq:dbuniq/NS5:NS5:01-TN-081_nonstructural_protein_5 corresponds to SEQ ID NO: 395, the peptide sequence shown in the column TREMIIa:dbtype:microbiome/dengue.uniq/db.dengue/dengue_type _1/E:E:gi_209976732 corresponds to SEQ ID NO: 396; the peptide sequence shown in the column TREMIIa:dbtype:microbiome/dengue.uniq/db.dengue/dengue_type _3/E:E:gi_169143690 corresponds to SEQ ID NO: 397; the peptide sequence shown in the column TREMIIb:dbtype:microbiome/dengue.uniq/db.dengue/dengue_type _1/NS3:NS3:gi_332959458 corresponds to SEQ ID NO: 398; the peptide sequences shown in the column TREMI:dbother:microbiome/flavi/jev/WNV:WNV:gi_50872125_West _Nile_virus correspond to SEQ ID NOs: 399 to 400 in order from the top; the peptide sequence shown in the column TREMIIb:dbother:microbiome/flavi/jev/WNV:WNV:gi_50872125We st_Nile_virus corresponds to SEQ ID NO: 401; and the peptide sequence shown in the column TREMIIb:dbother:microbiome/flavi/YFV:YFV:gi_3851161_Yellow_ fever_virus corresponds to SEQ ID NO: 402.

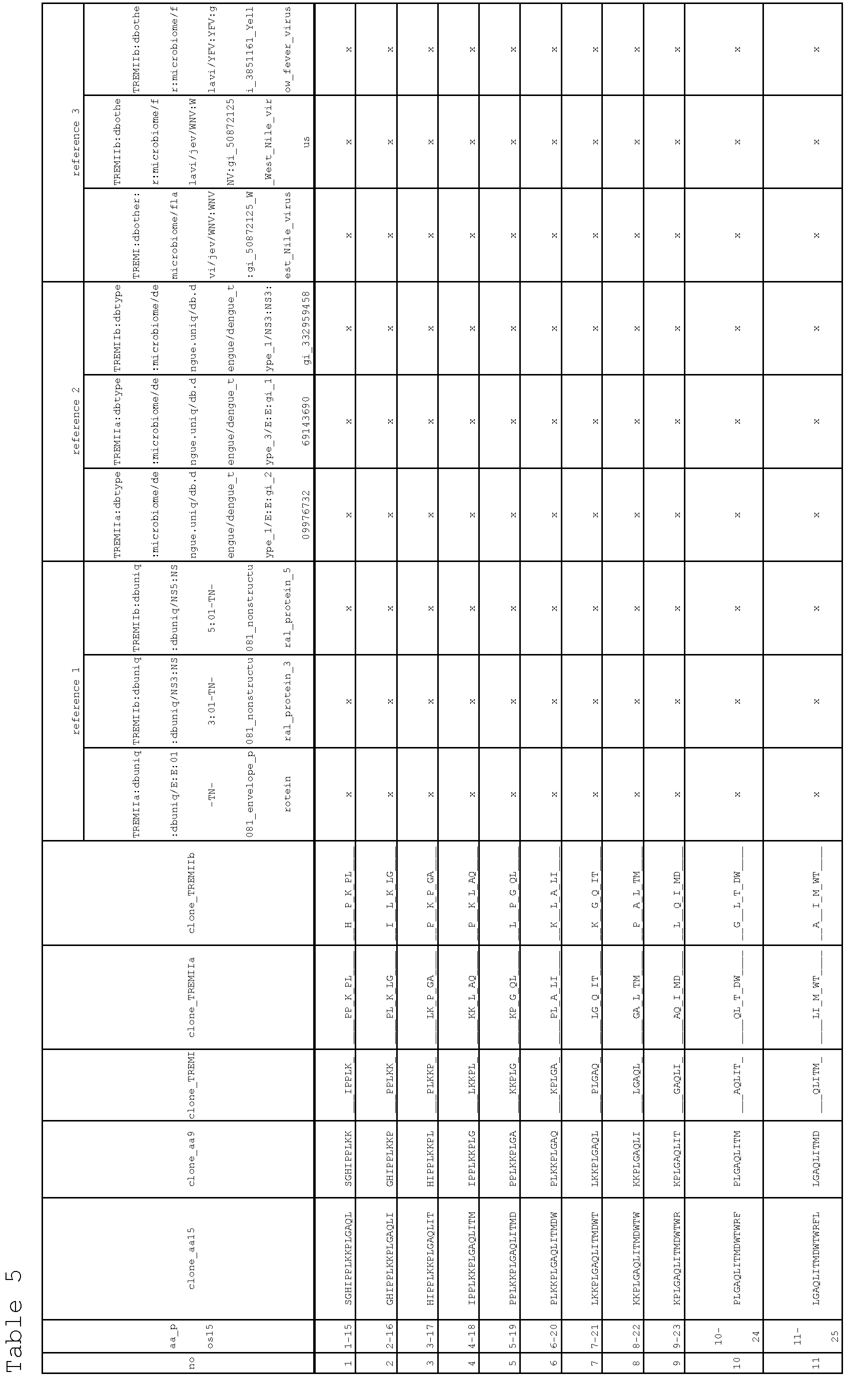

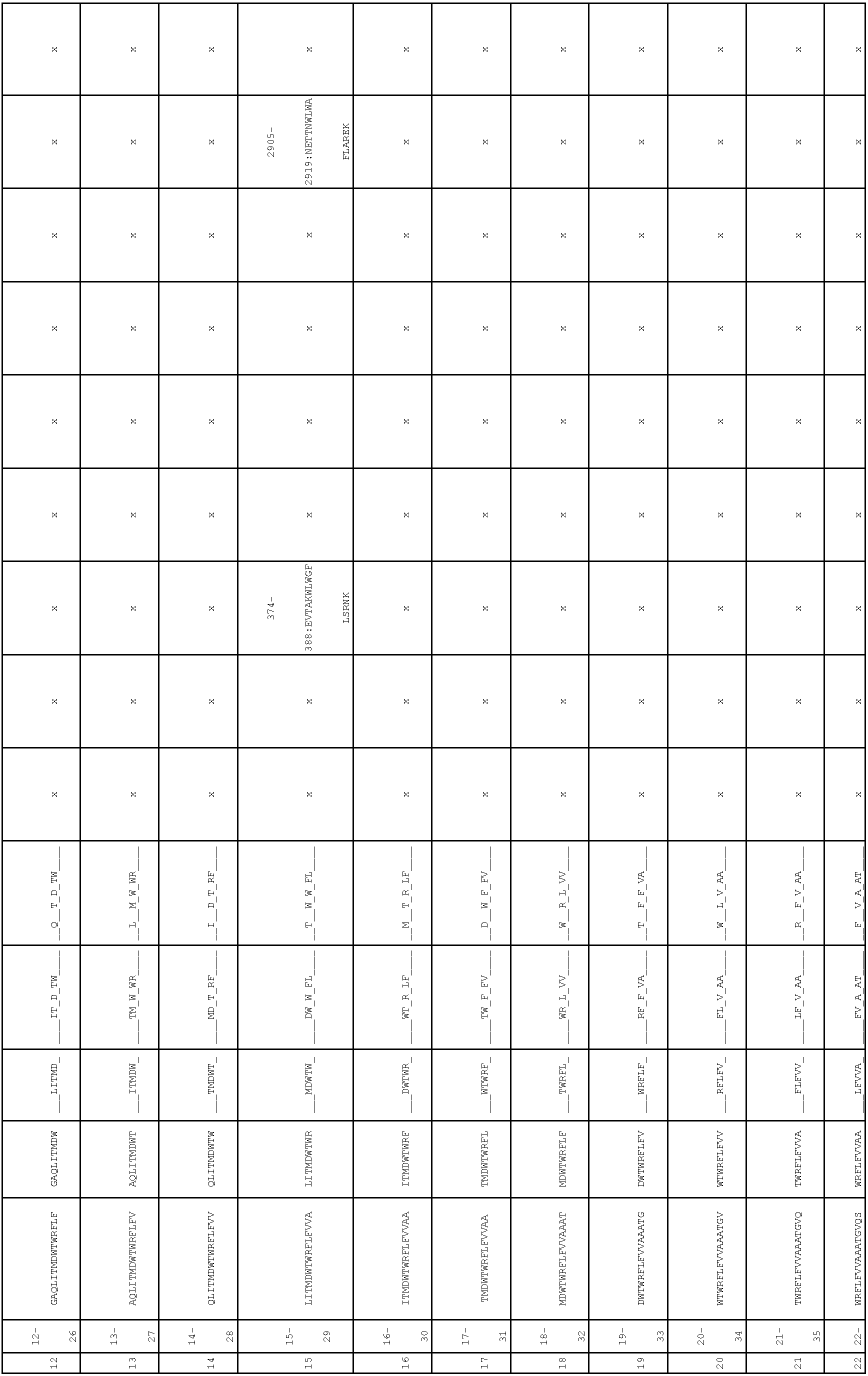

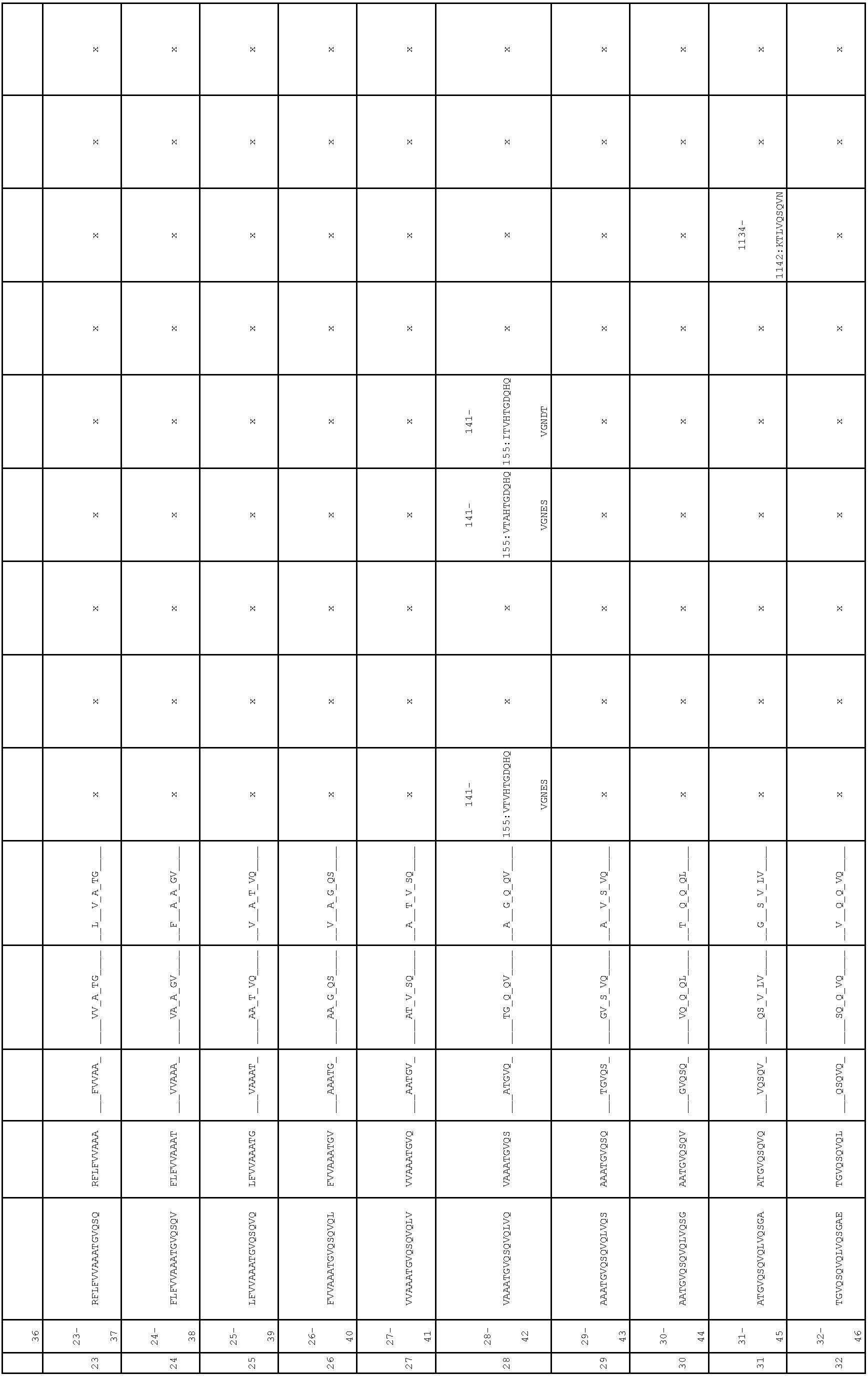

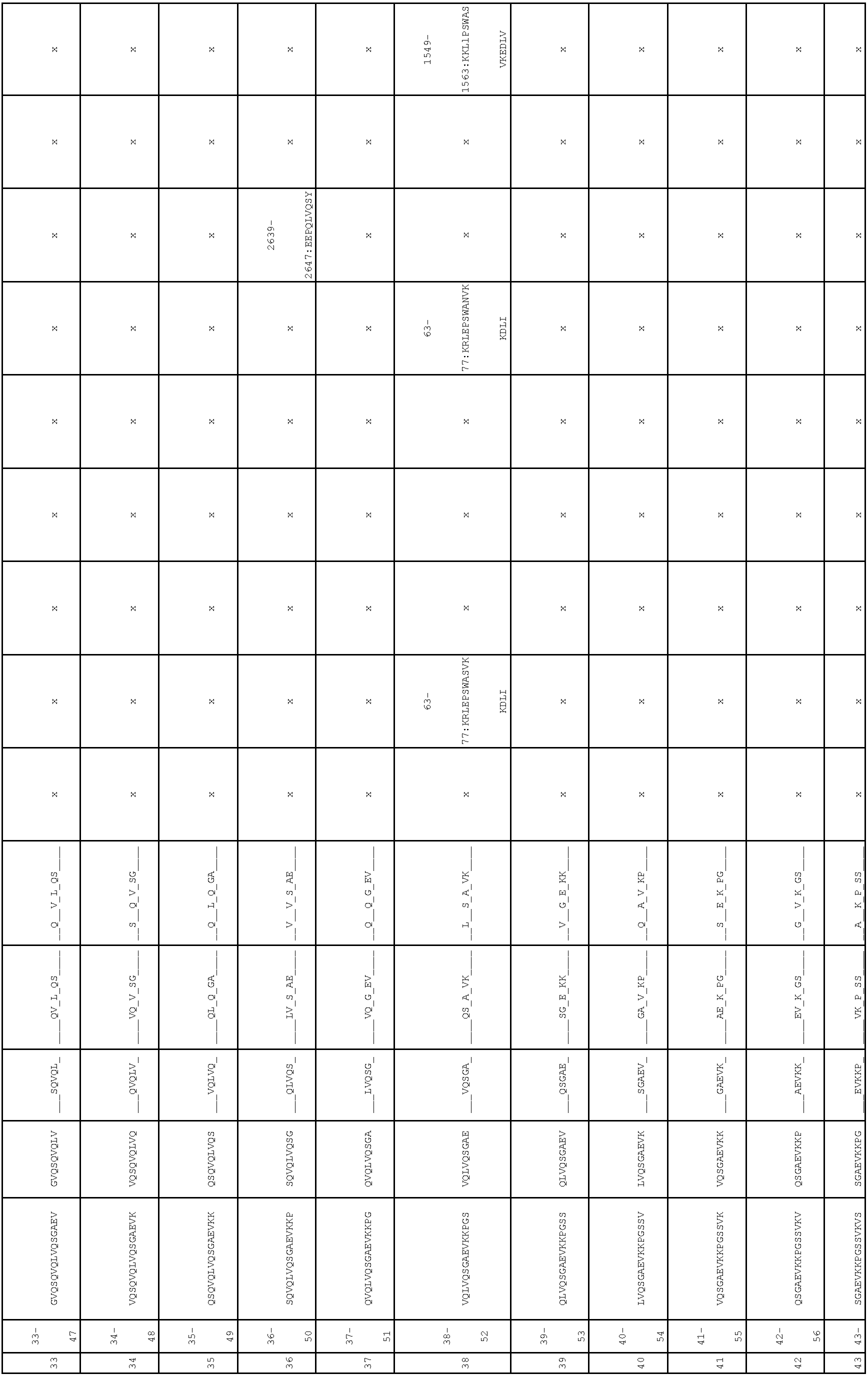

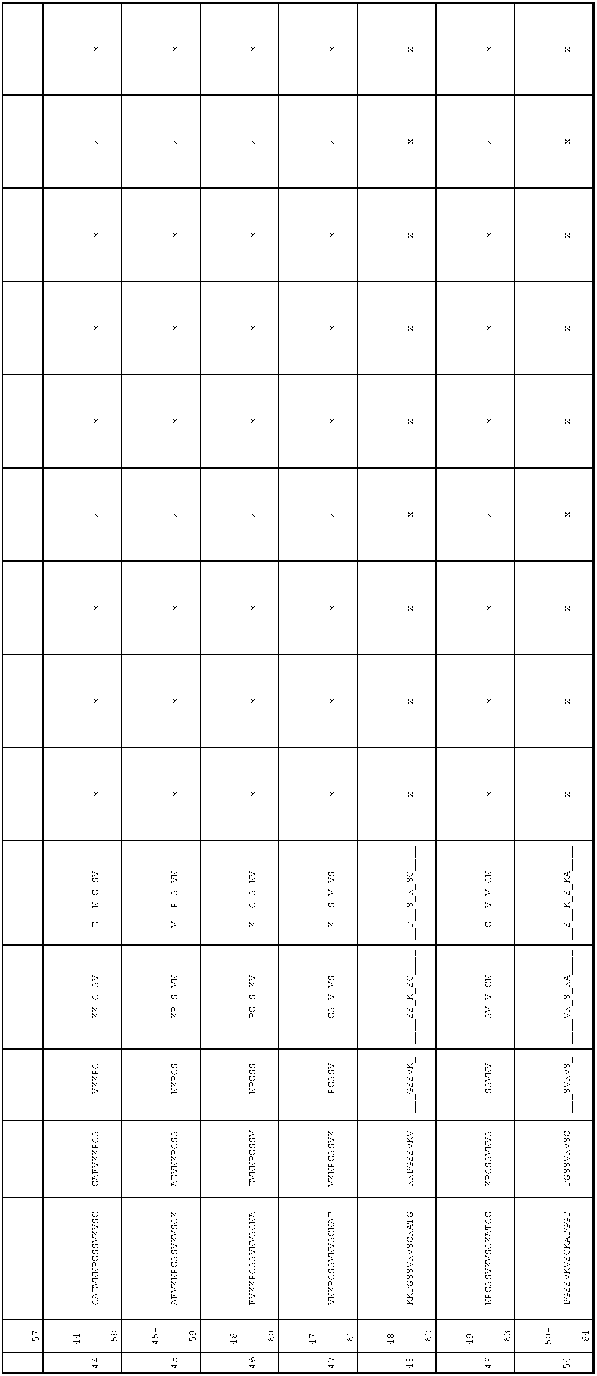

### (Example 6: Calculation of MHC Binding Affinity of Reference Sequence Peptide with Common TREM with BCR)

In this example, for the reference sequence in which this TREM sequence was identical to that of the repertoire clone, IC50 for MHC class I was calculated for the 9-mer and IC 50 for MHC class II was calculated for the 15-mer. Specifically, the measurement was performed in the following manner (the method was the same as that in Example 3).

IC50 values were calculated as binding affinity for the designated MHC types using the 9-mer and 15-mer peptide sequences obtained from the reference sequence including the TREM sequence identical to the repertoire clone. For the MHC type, HLA types of the group of 18 dengue-infected patients were used. In the calculation of the IC50 values showing MHC binding affinity, NetMHCpan was used for MHC class I, and NetMHCIIpan was used for MHC class II.

### (Results)

As shown in Tables 6-1 to 6-4 (all are class I) and Tables 6-5 to 6-8 (all are class II), the peptide sequences from the BCR and the reference matched with the TREM were obtained.

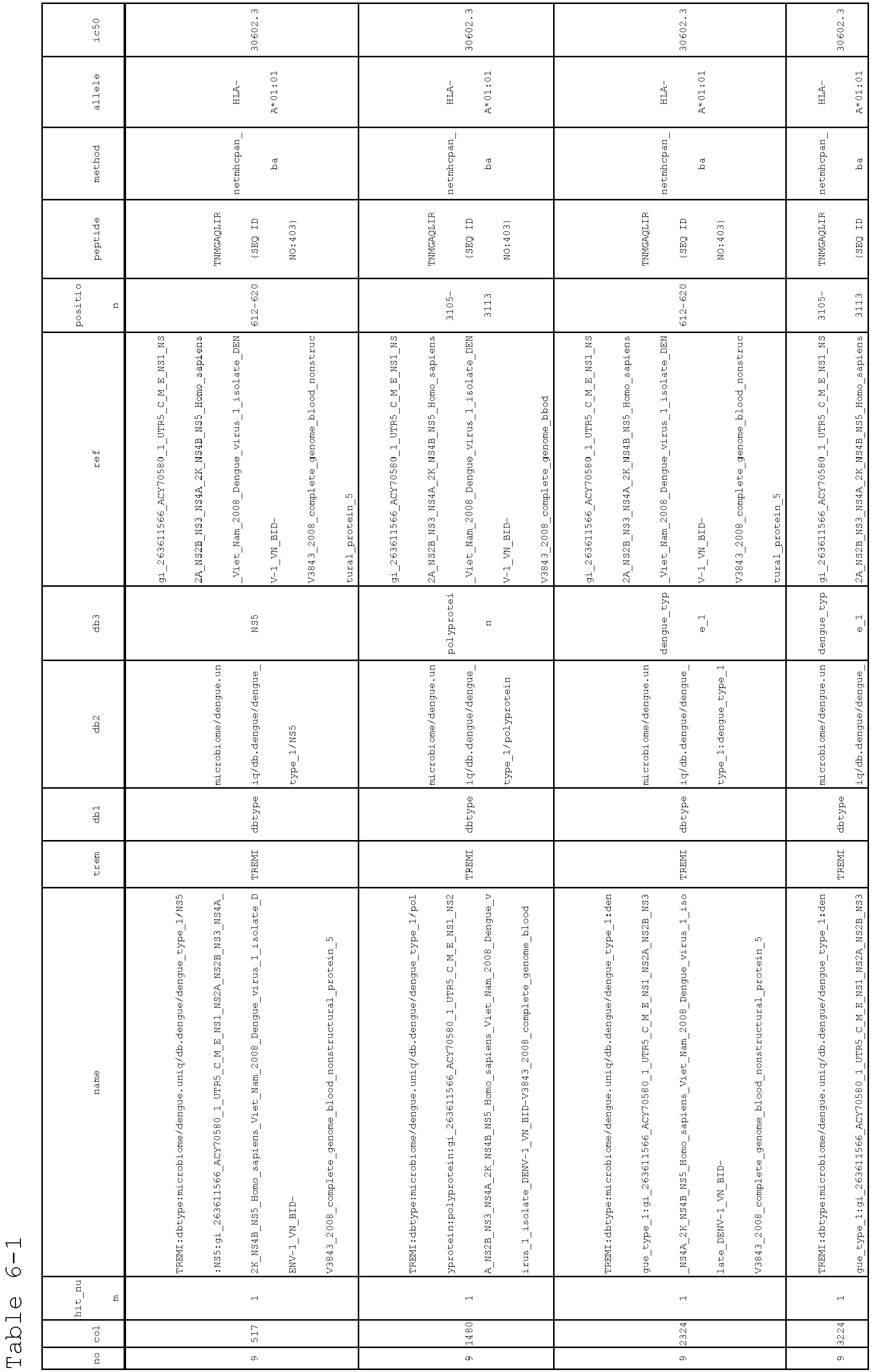

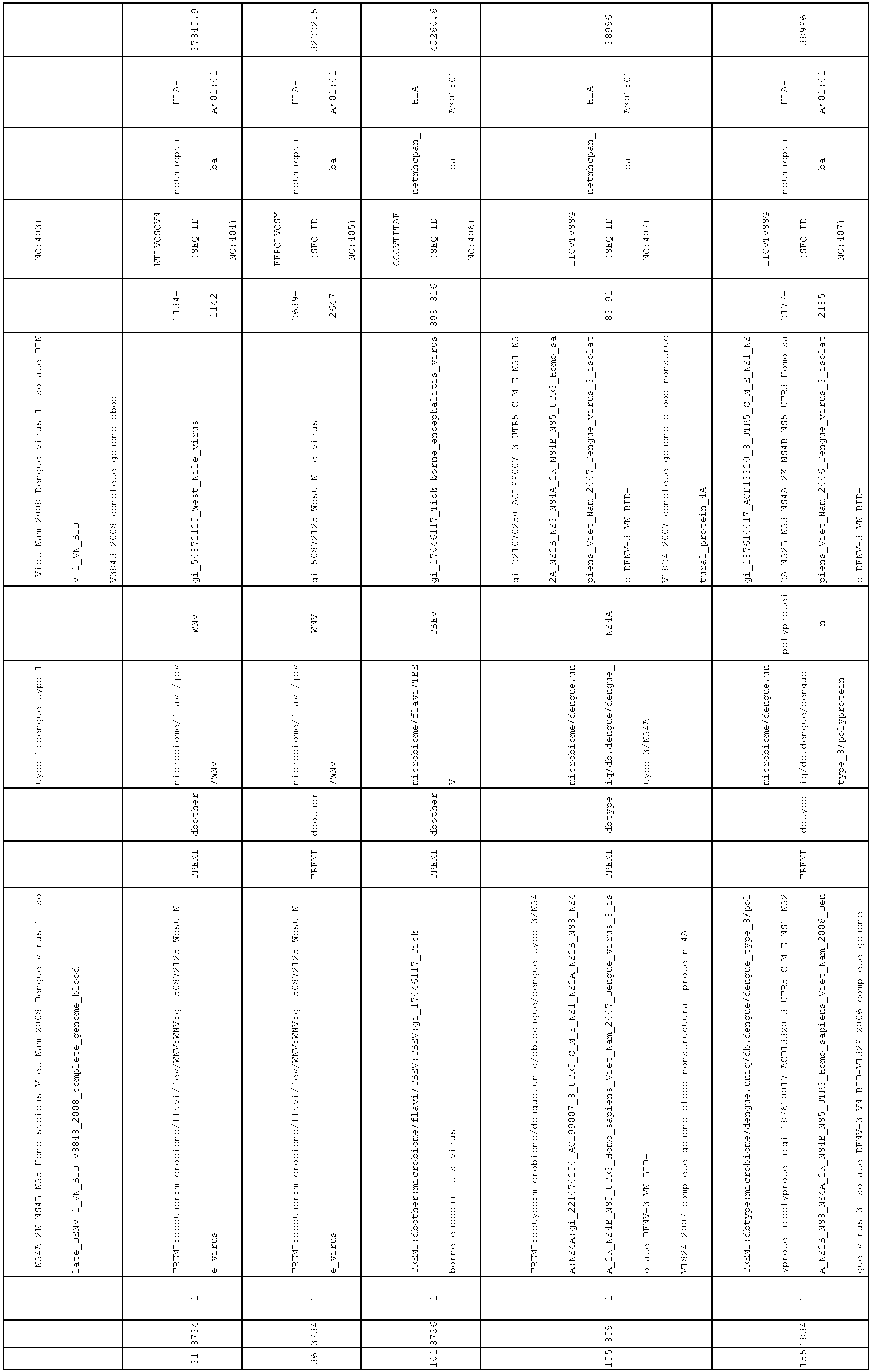

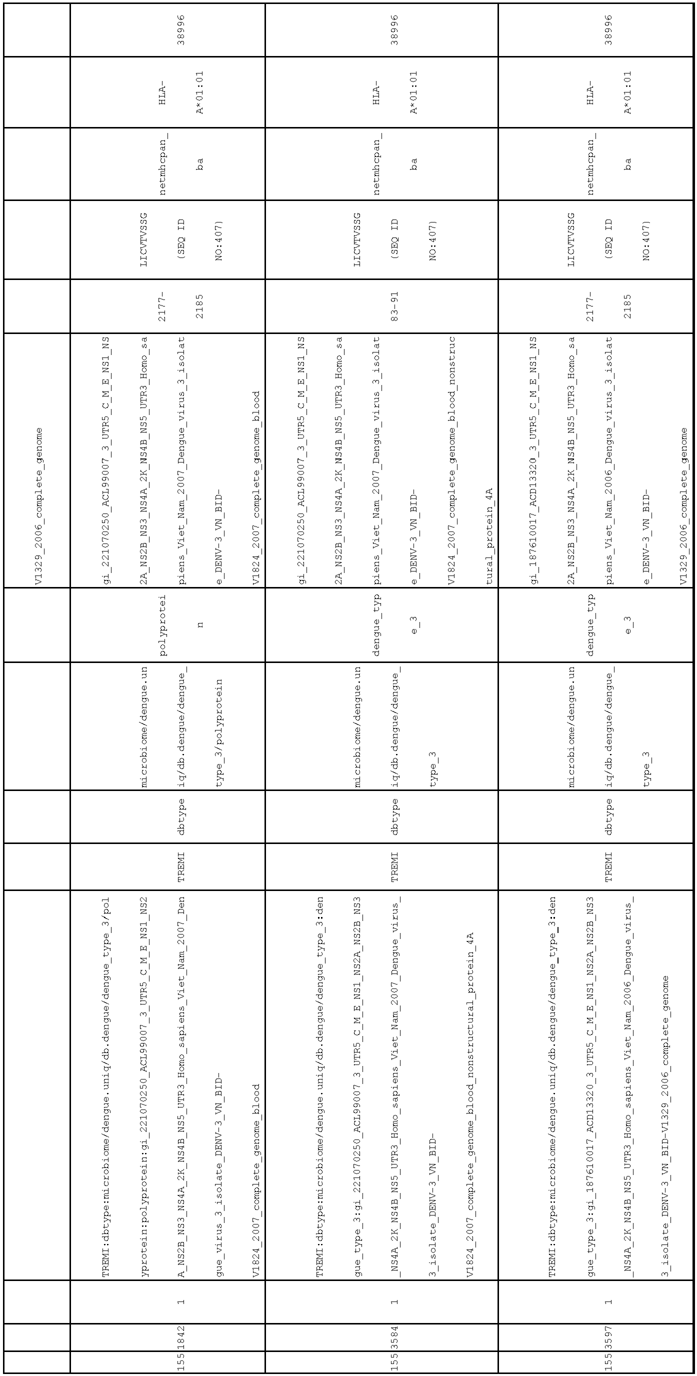

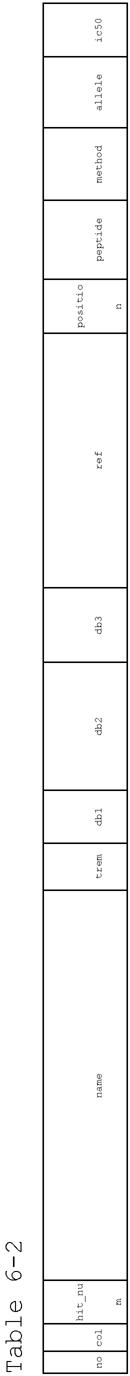

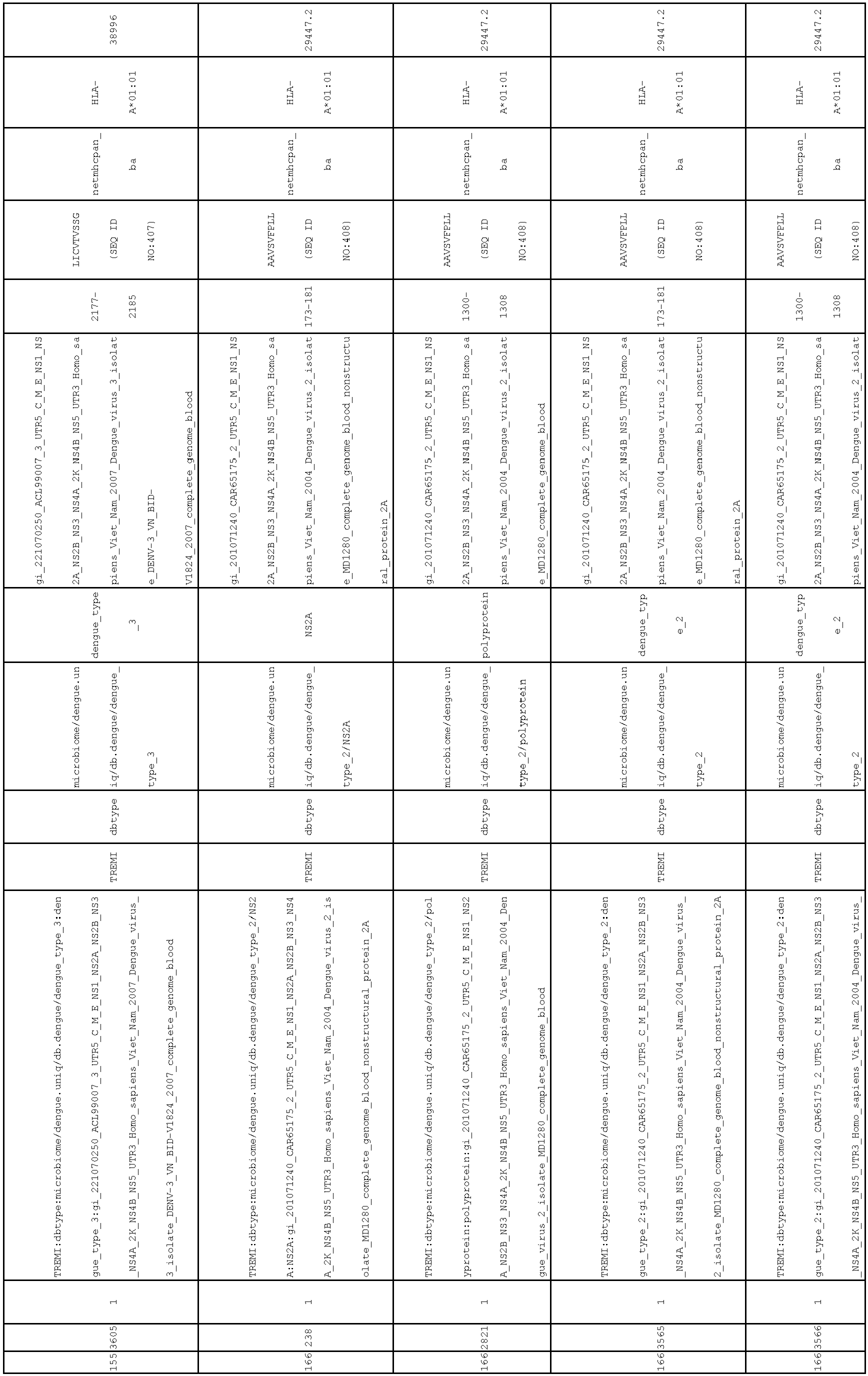

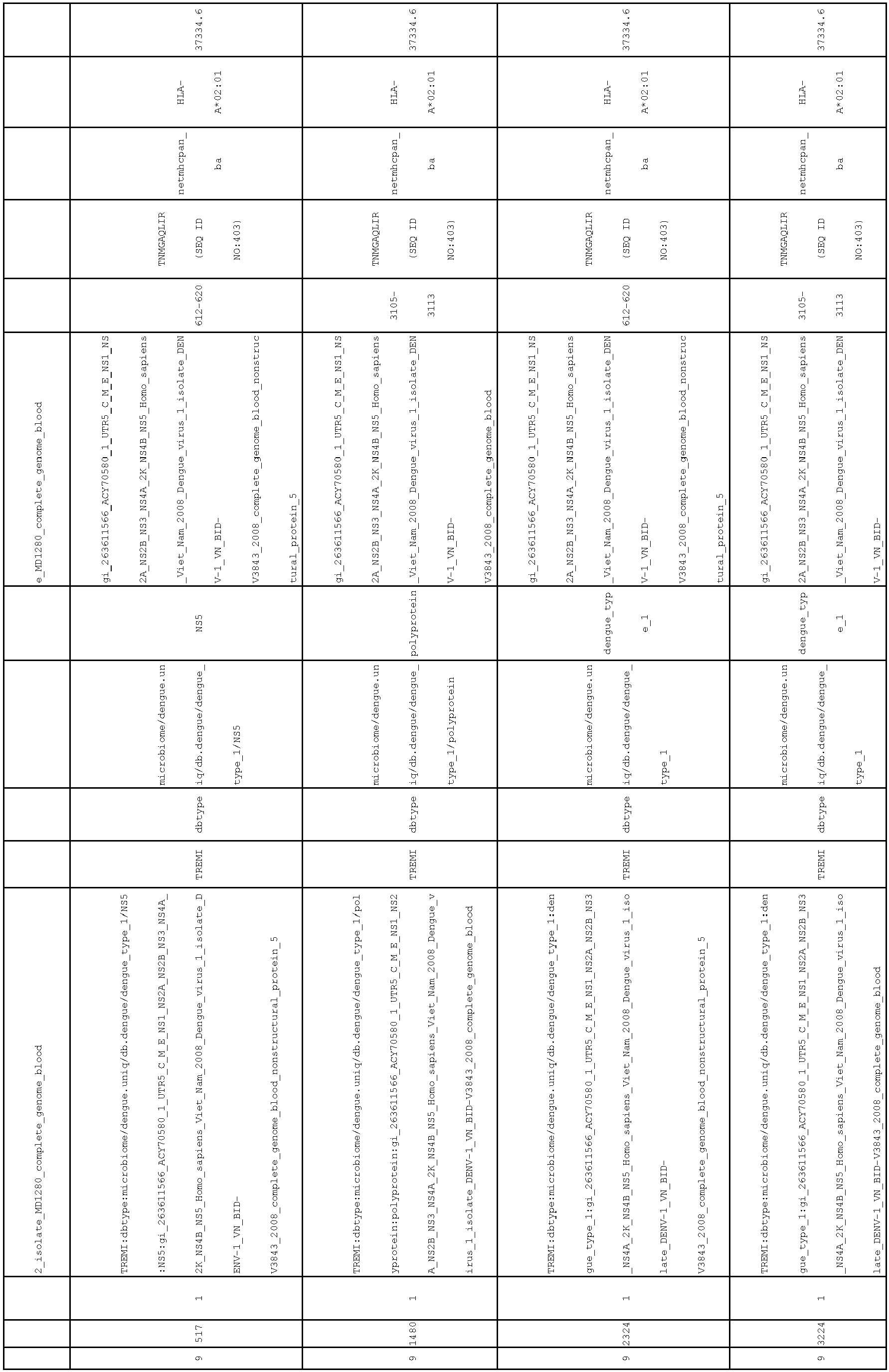

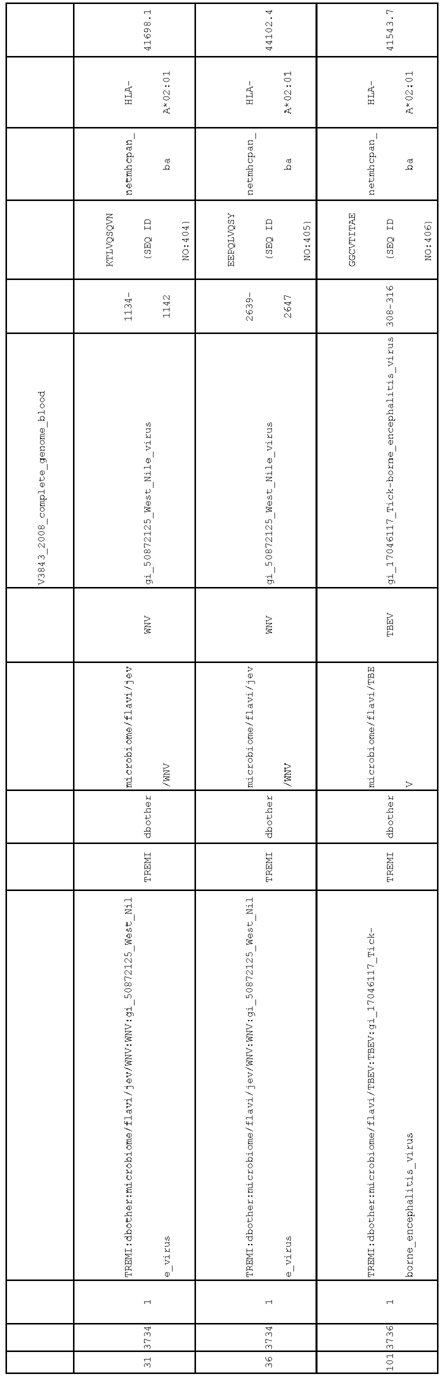

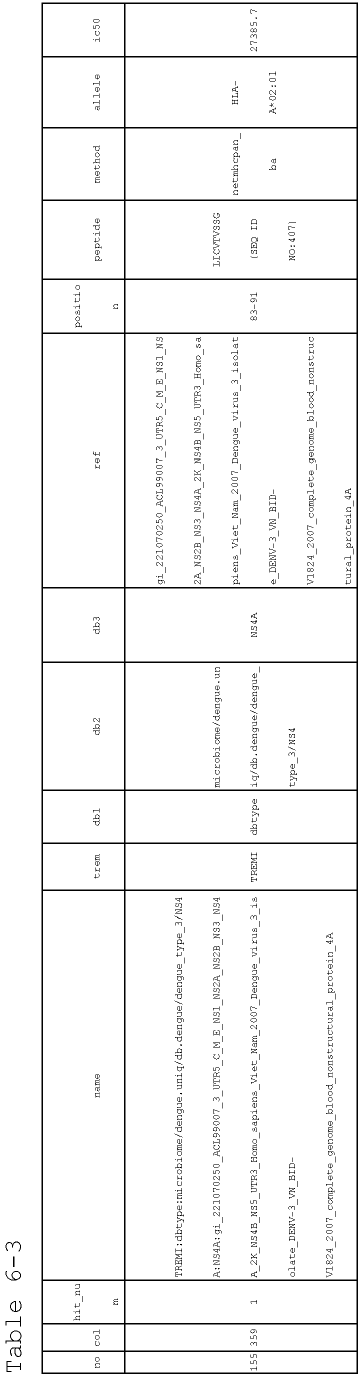

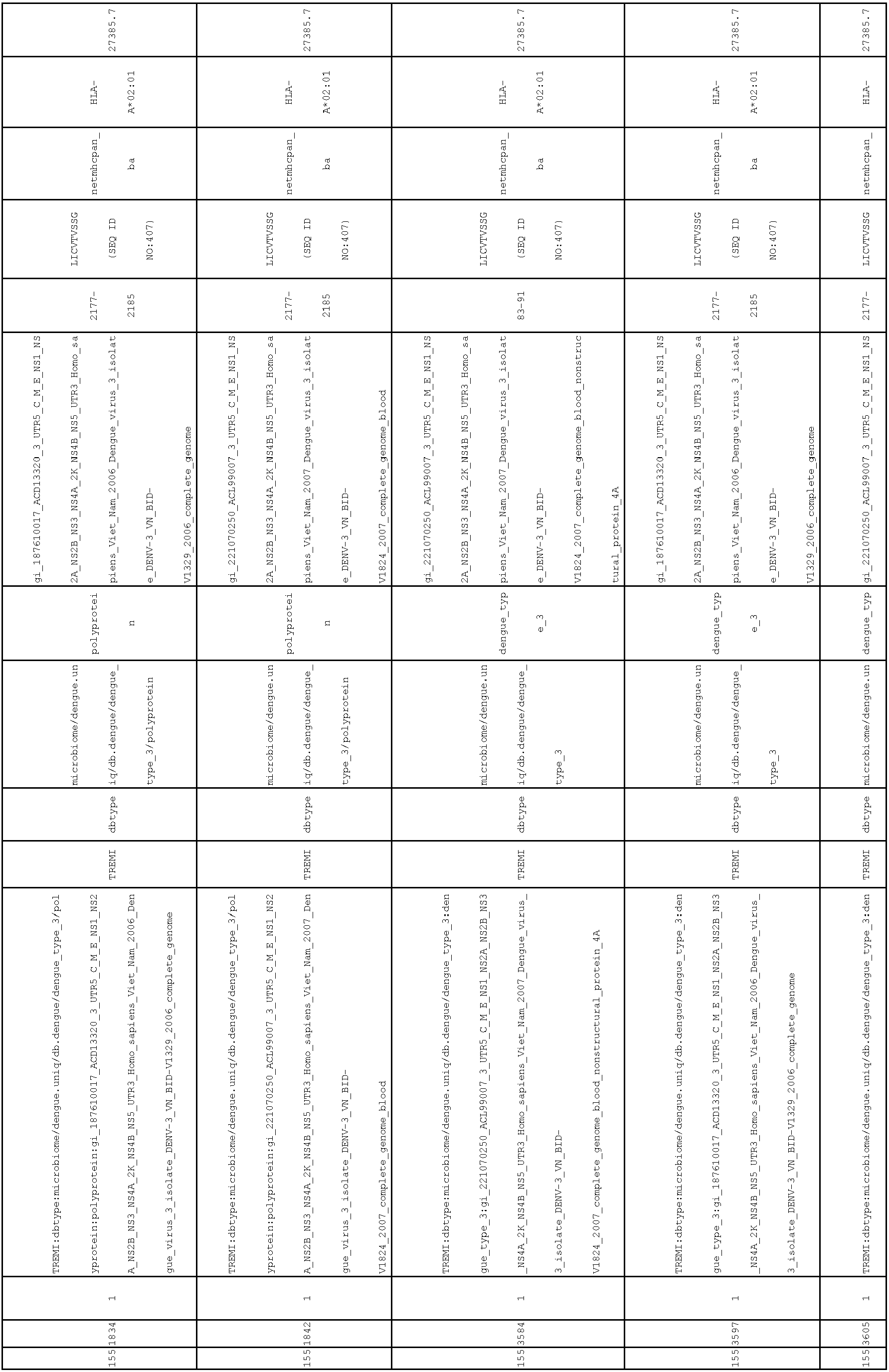

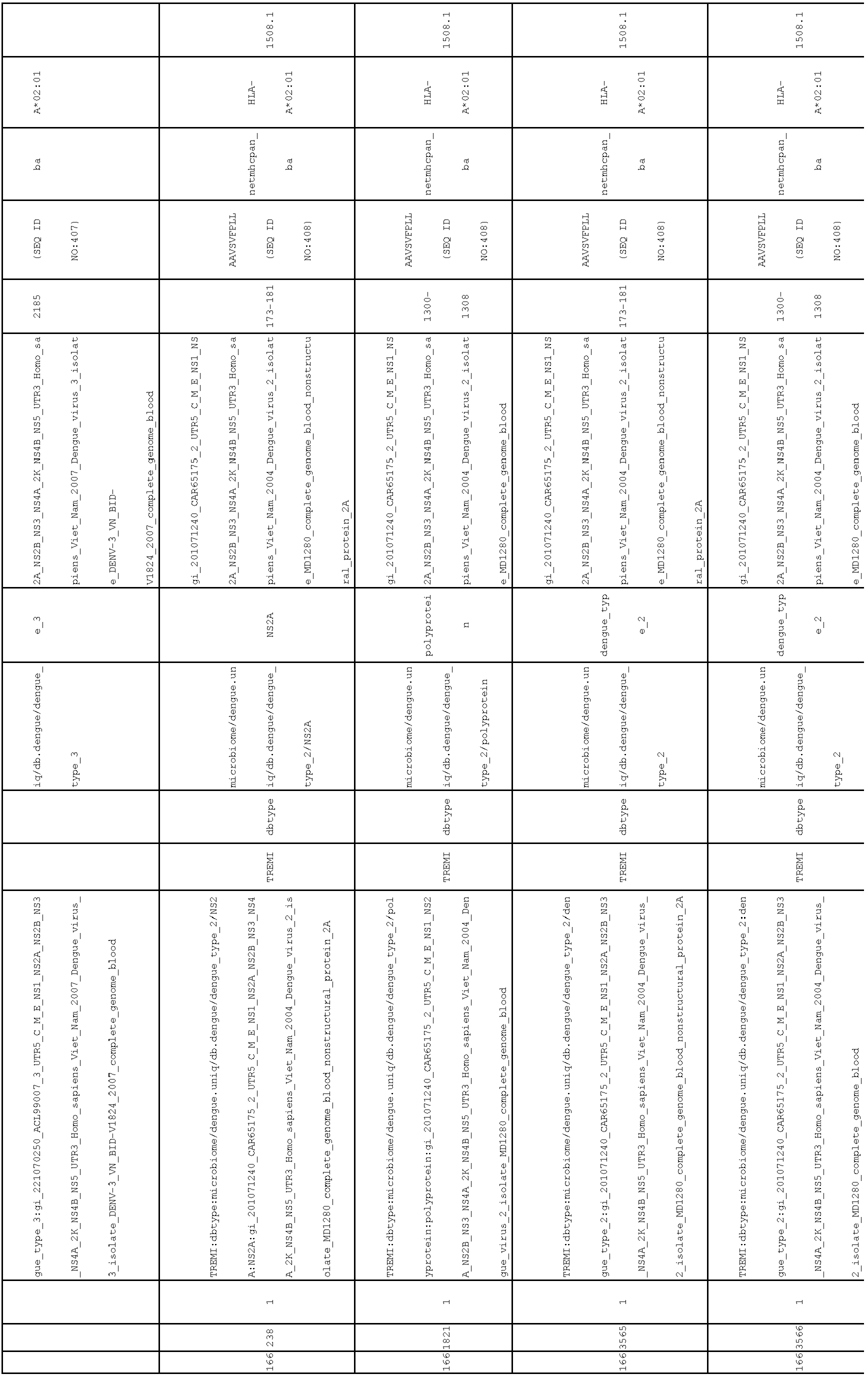

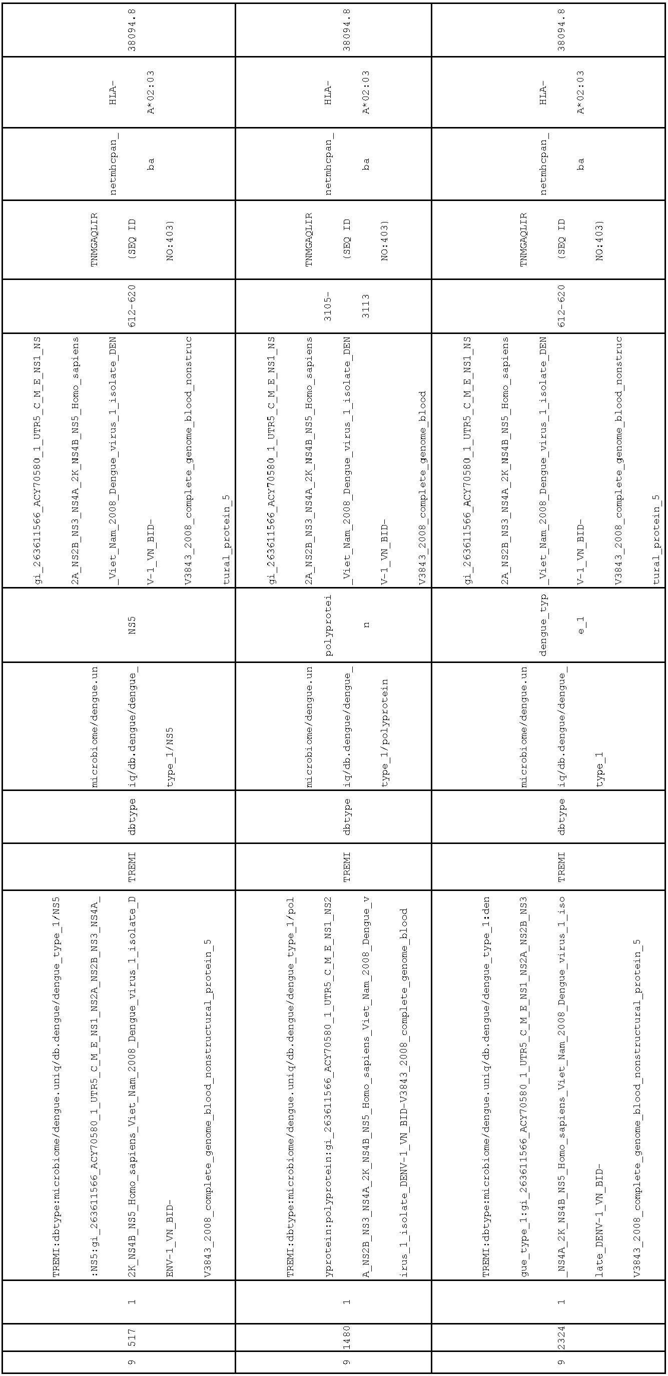

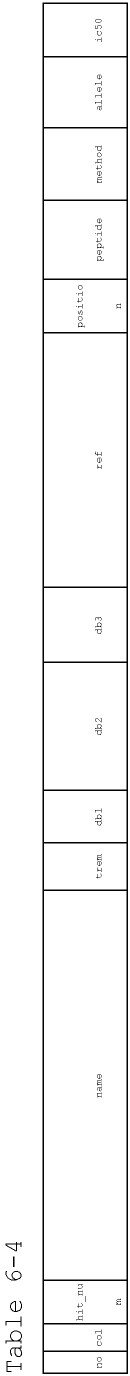

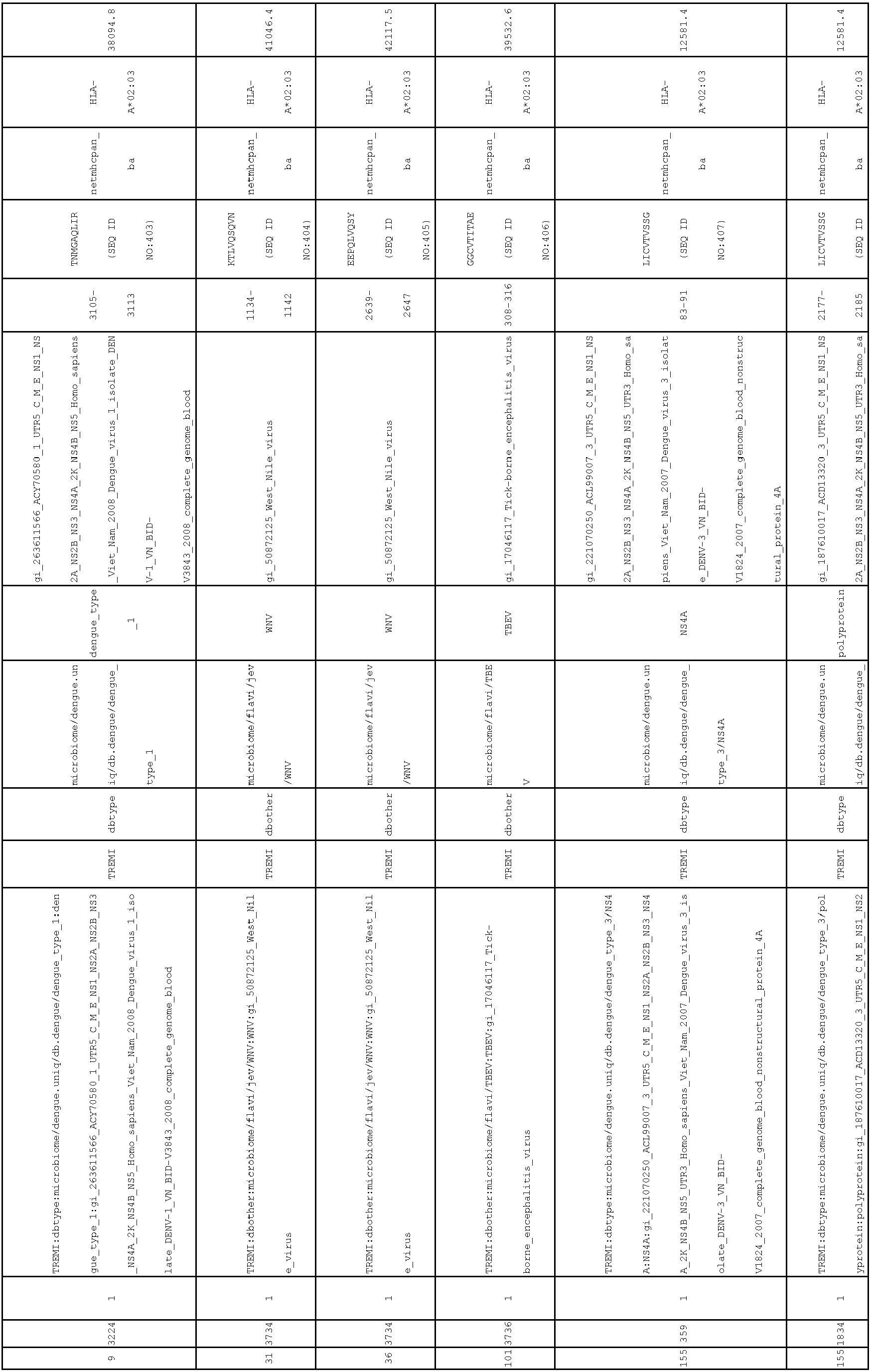

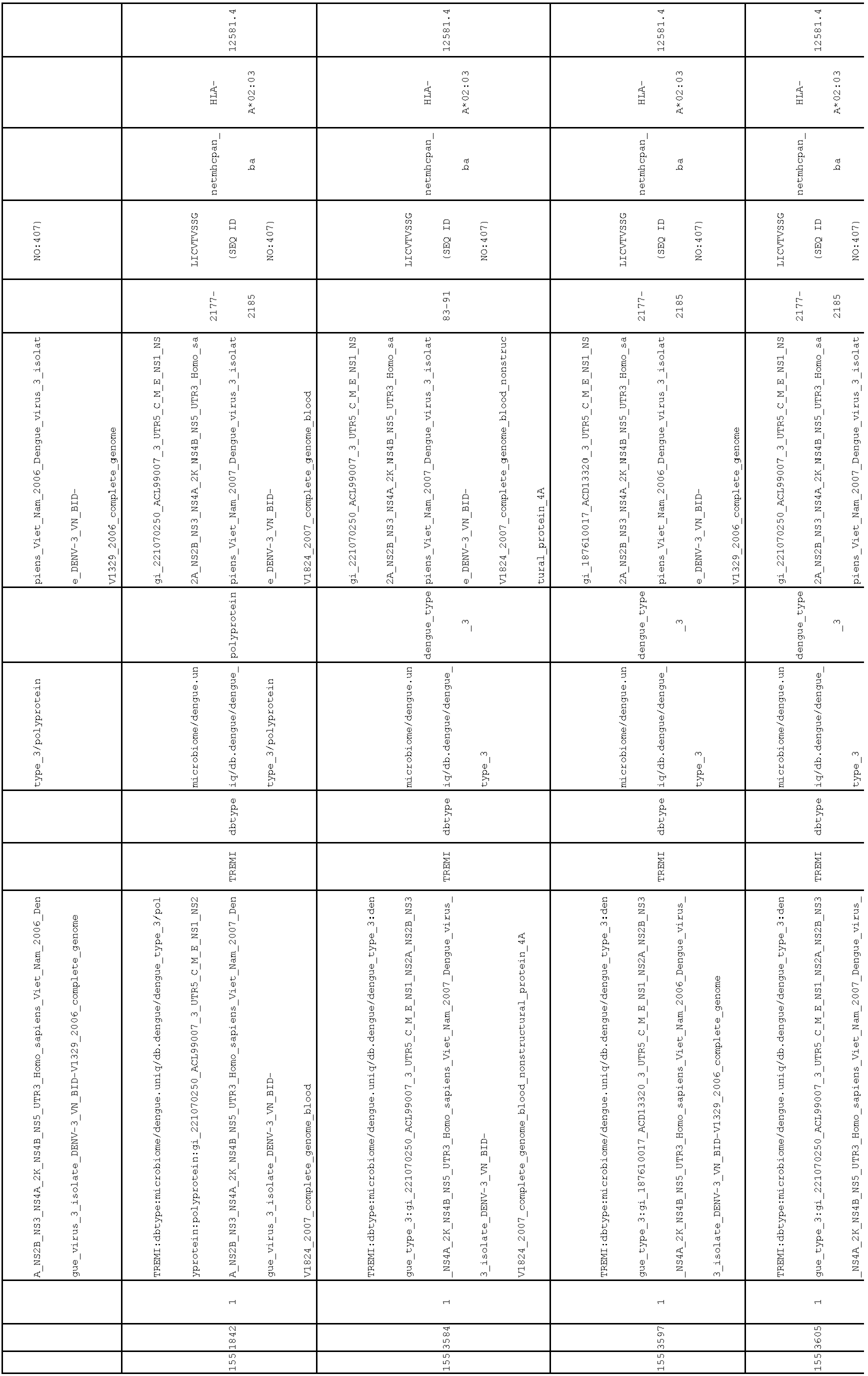

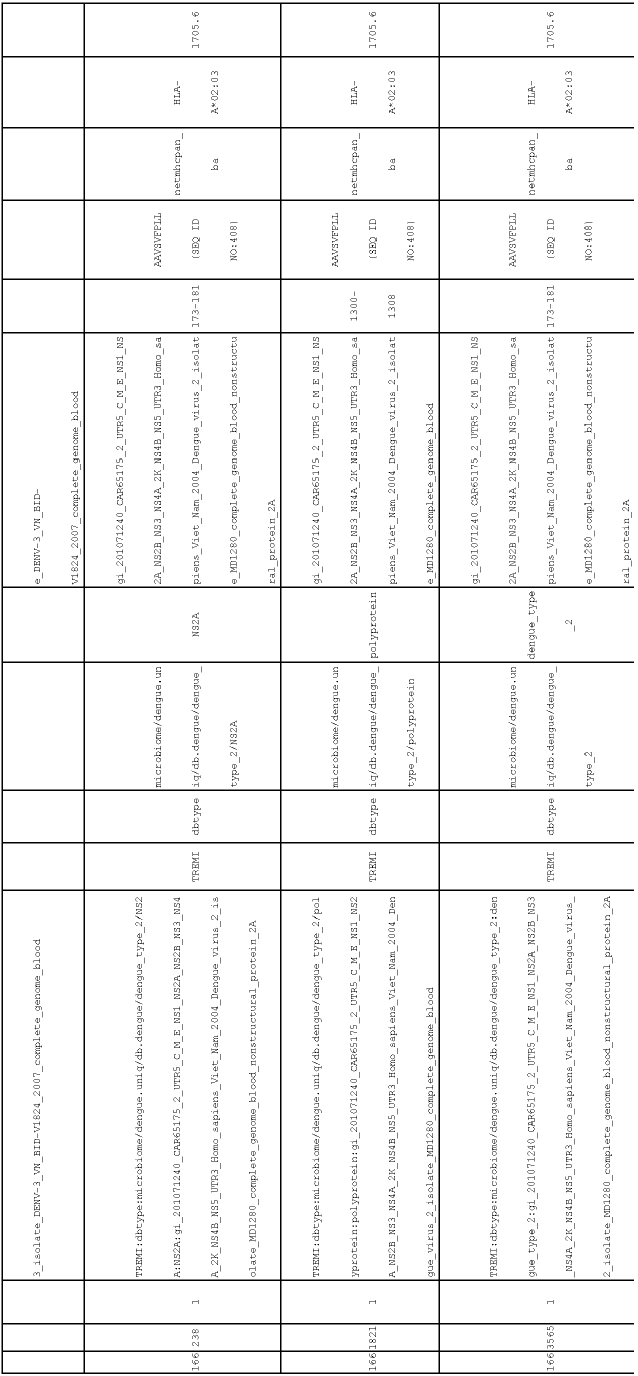

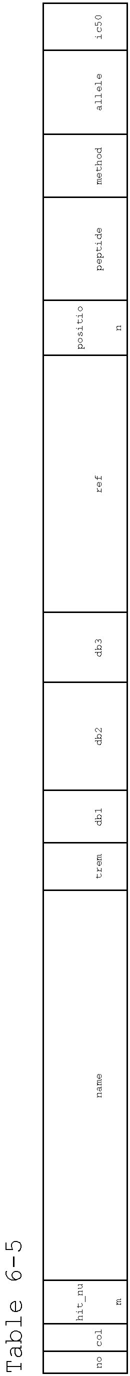

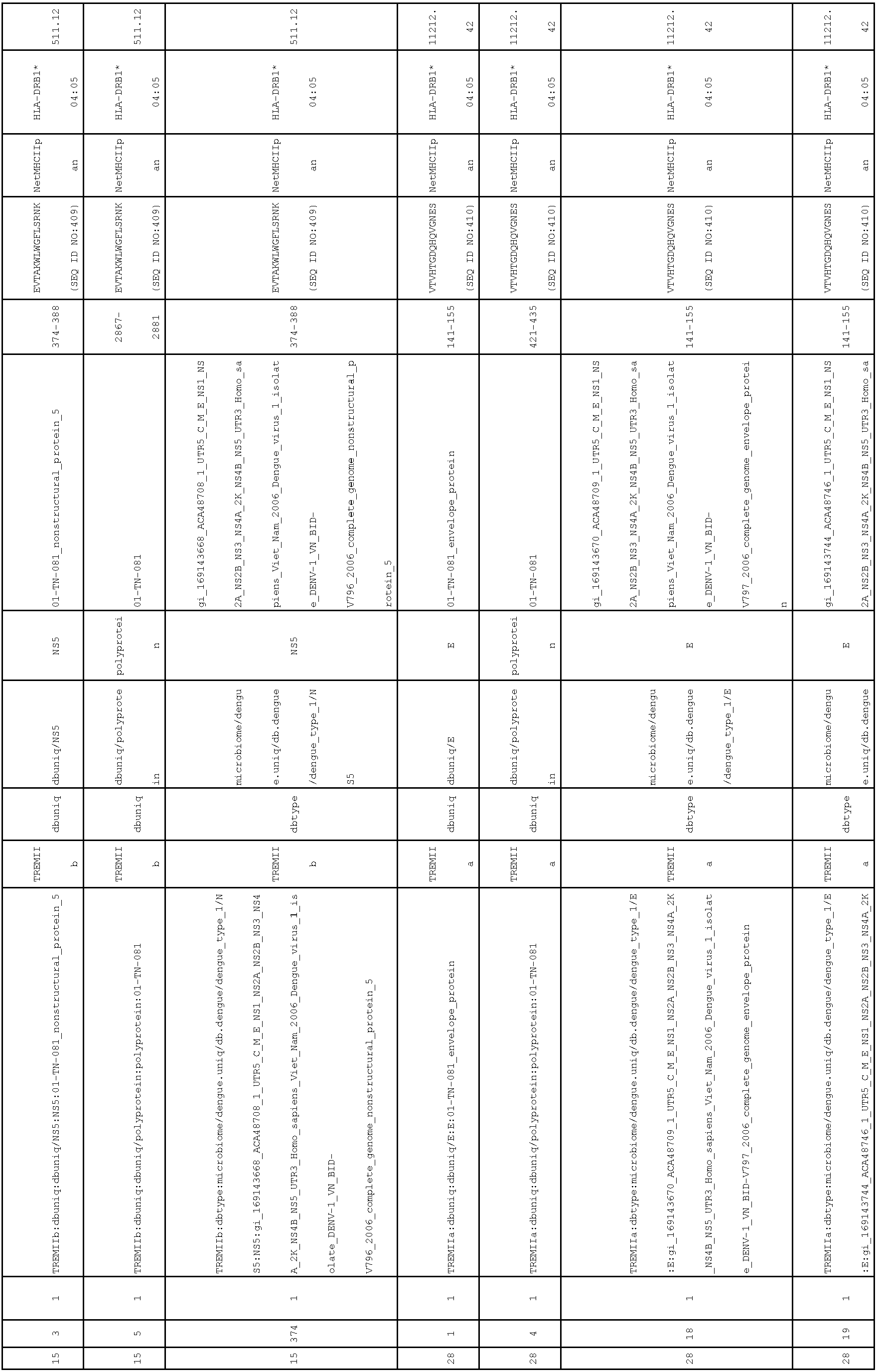

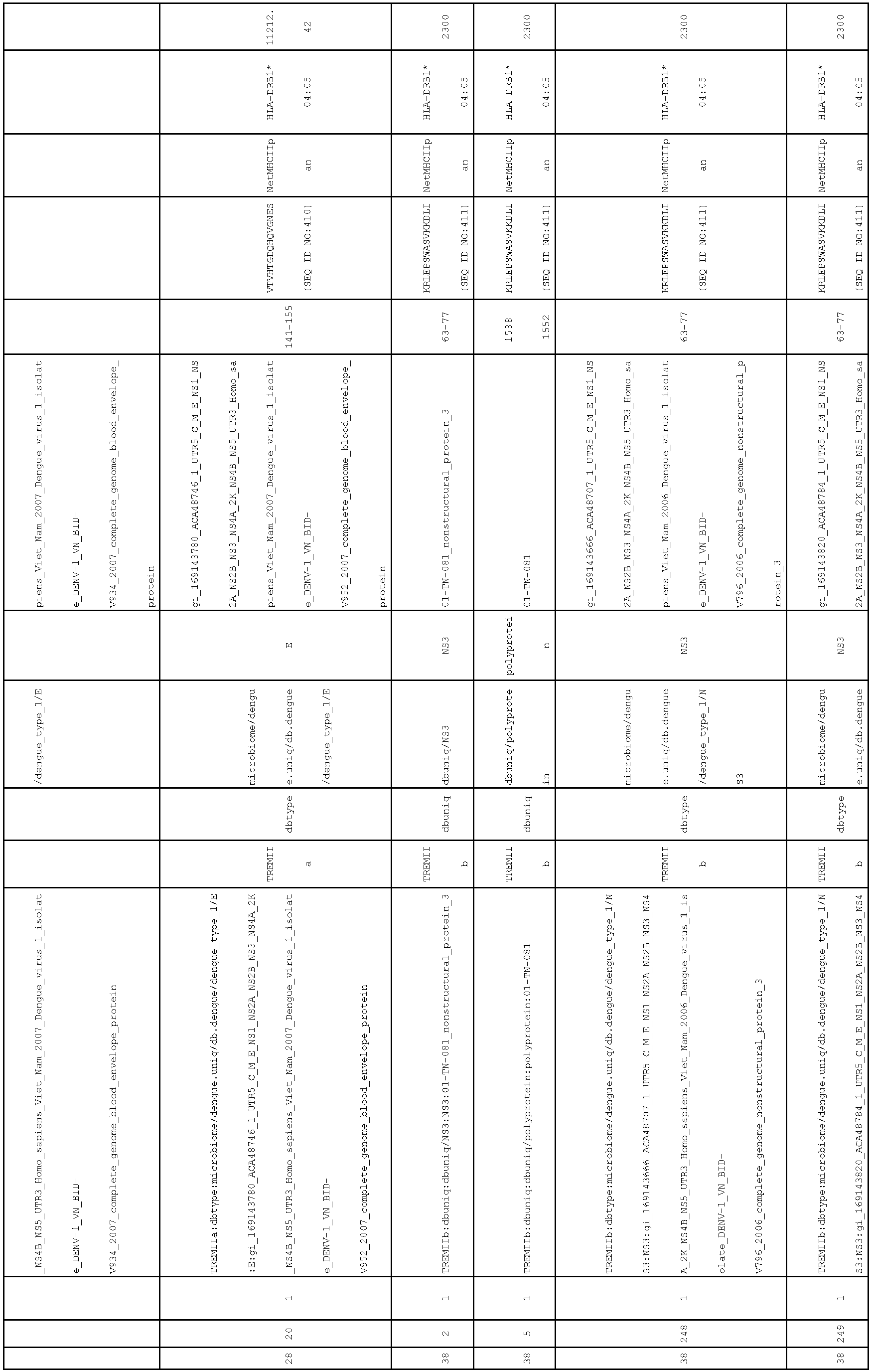

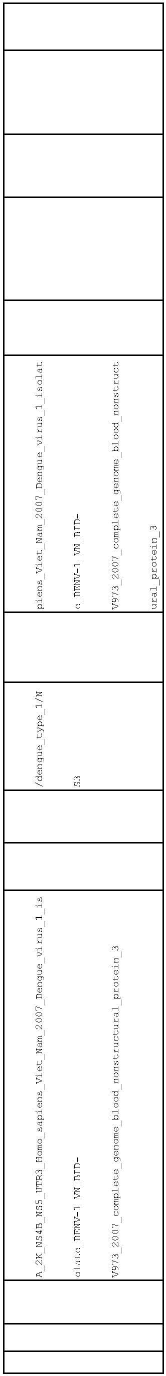

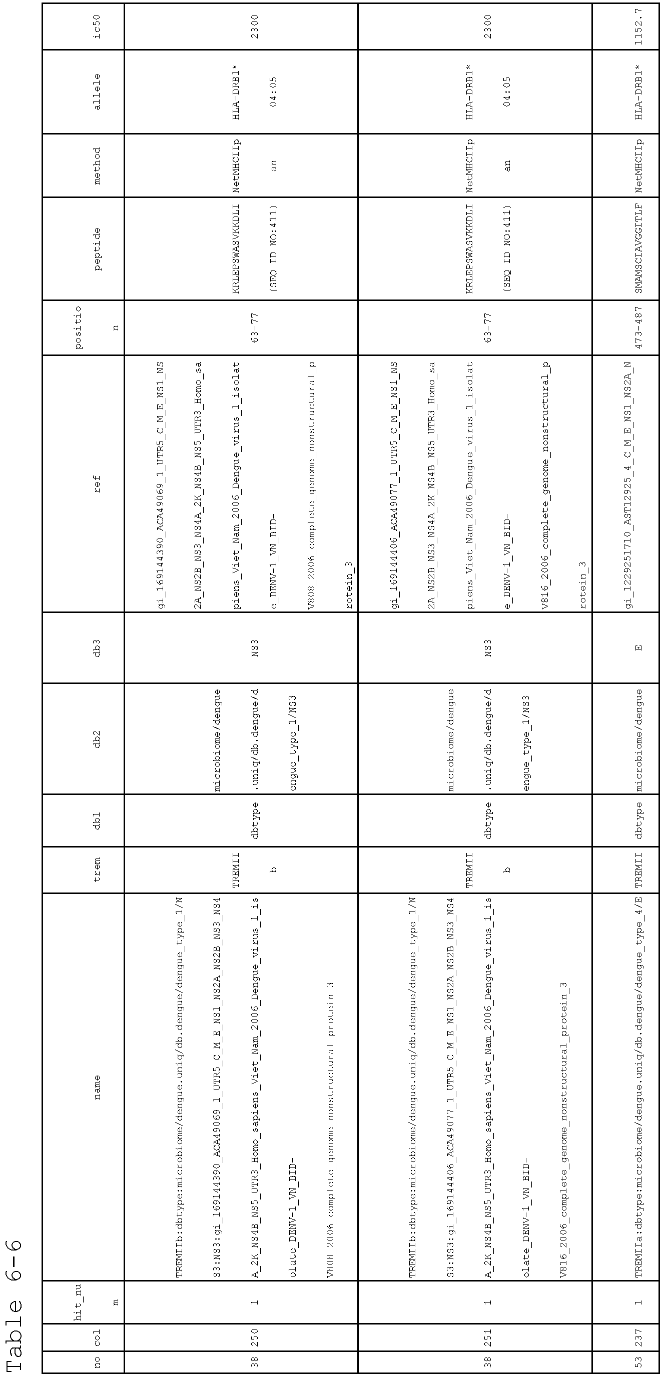

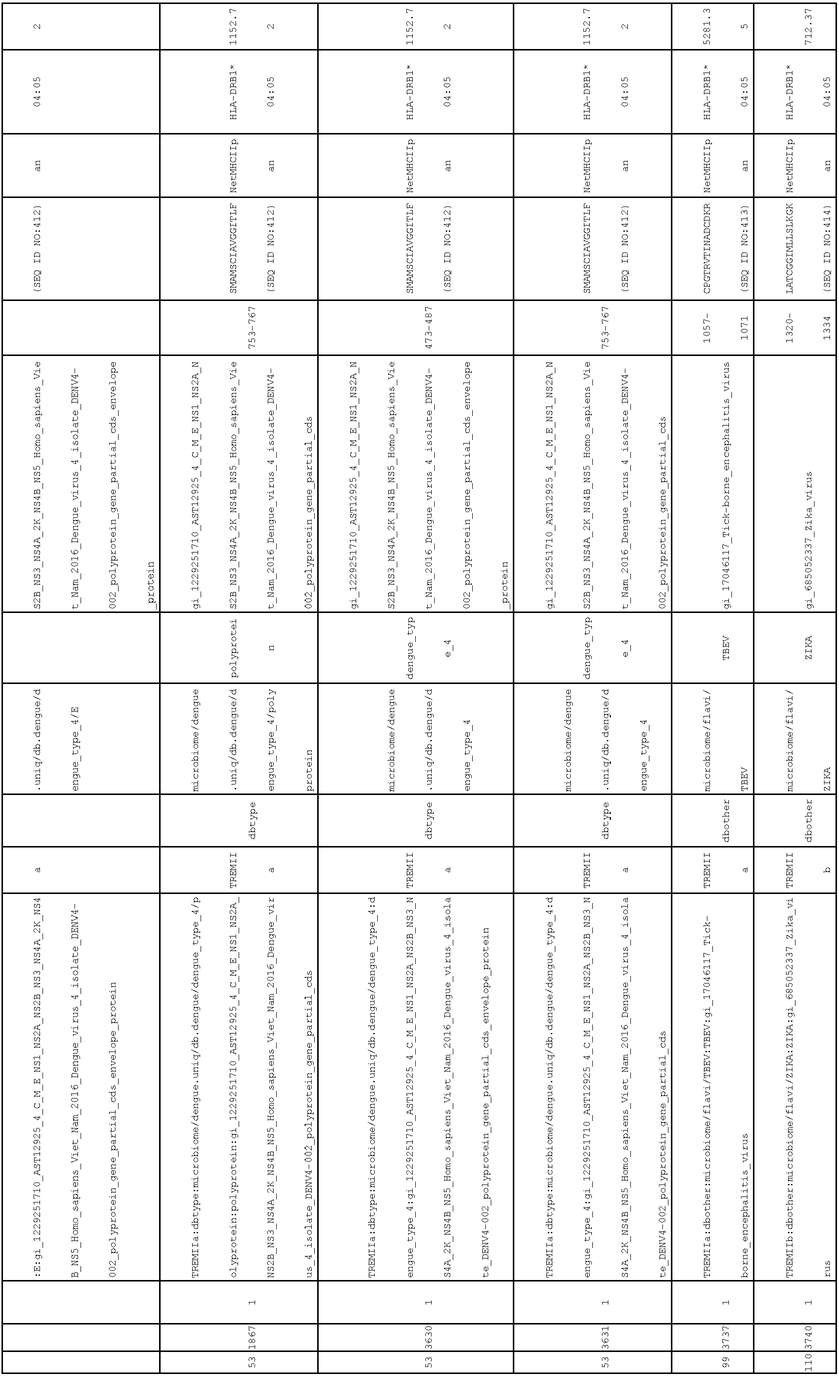

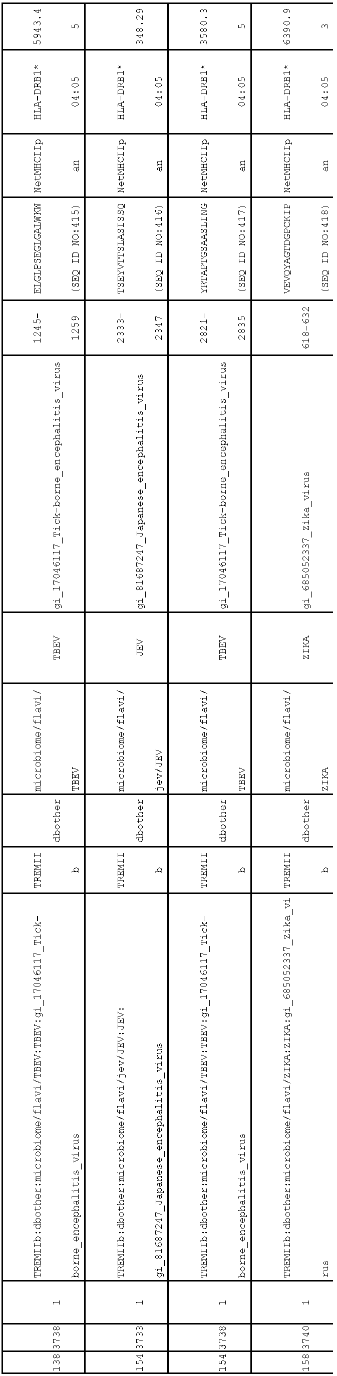

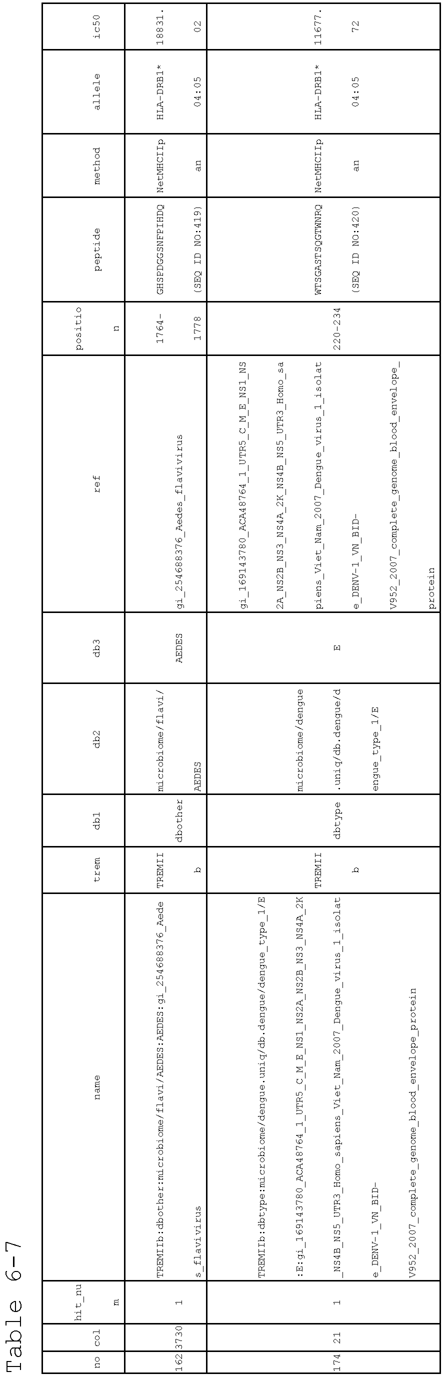

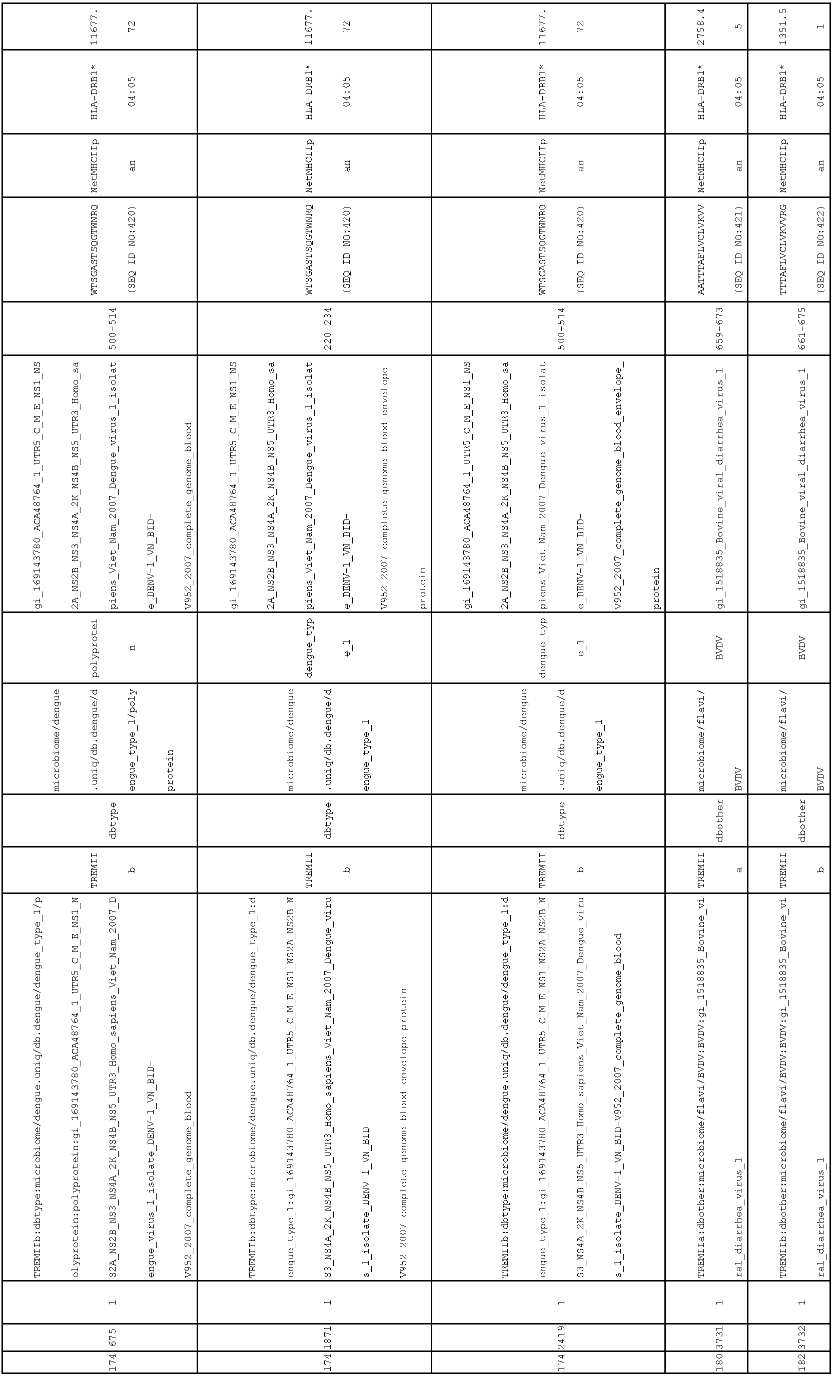

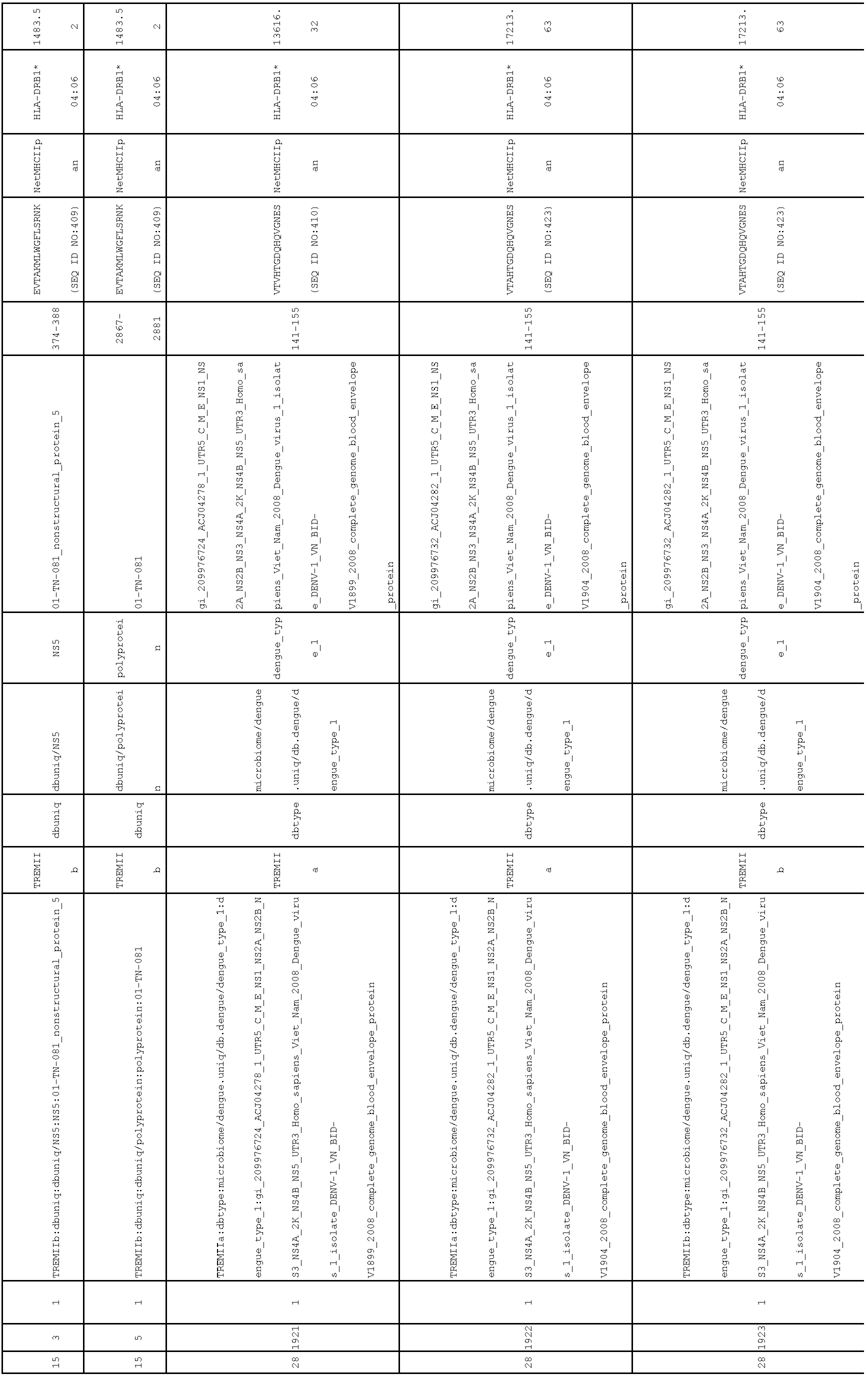

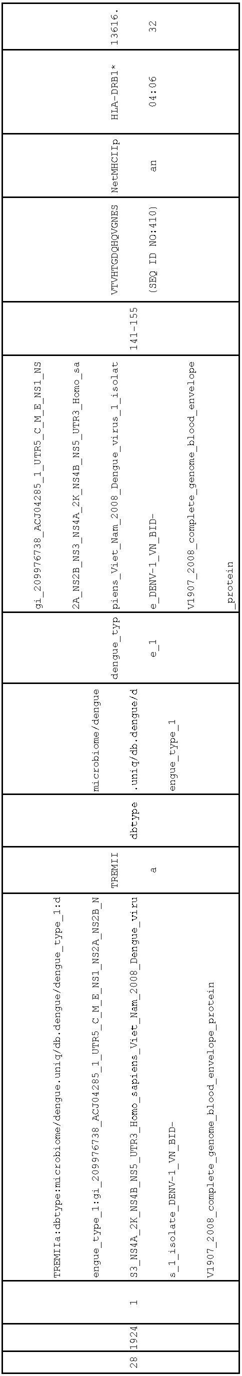

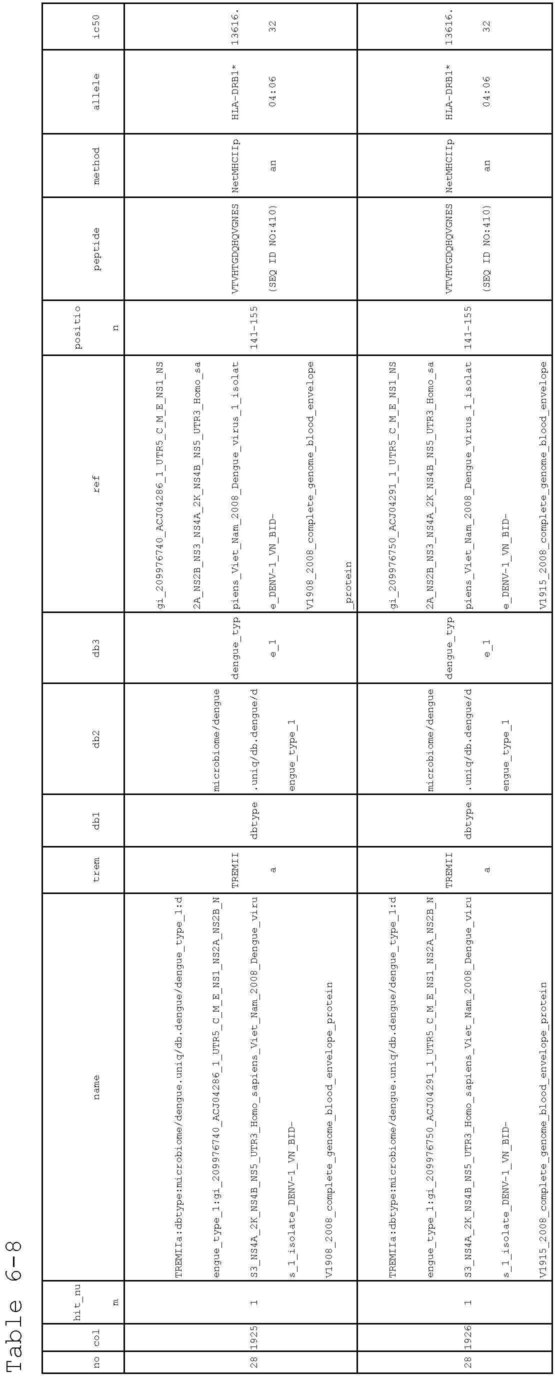

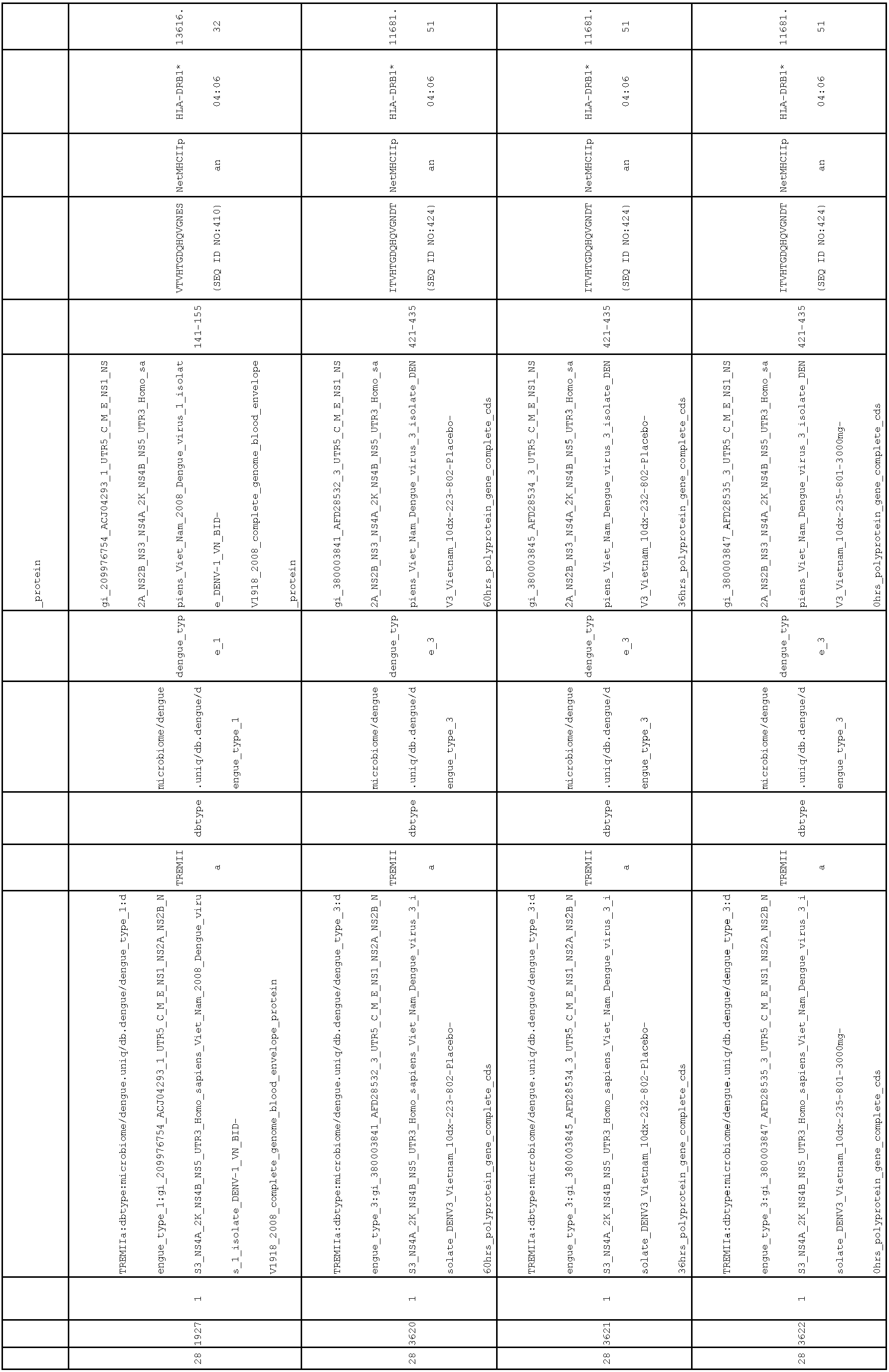

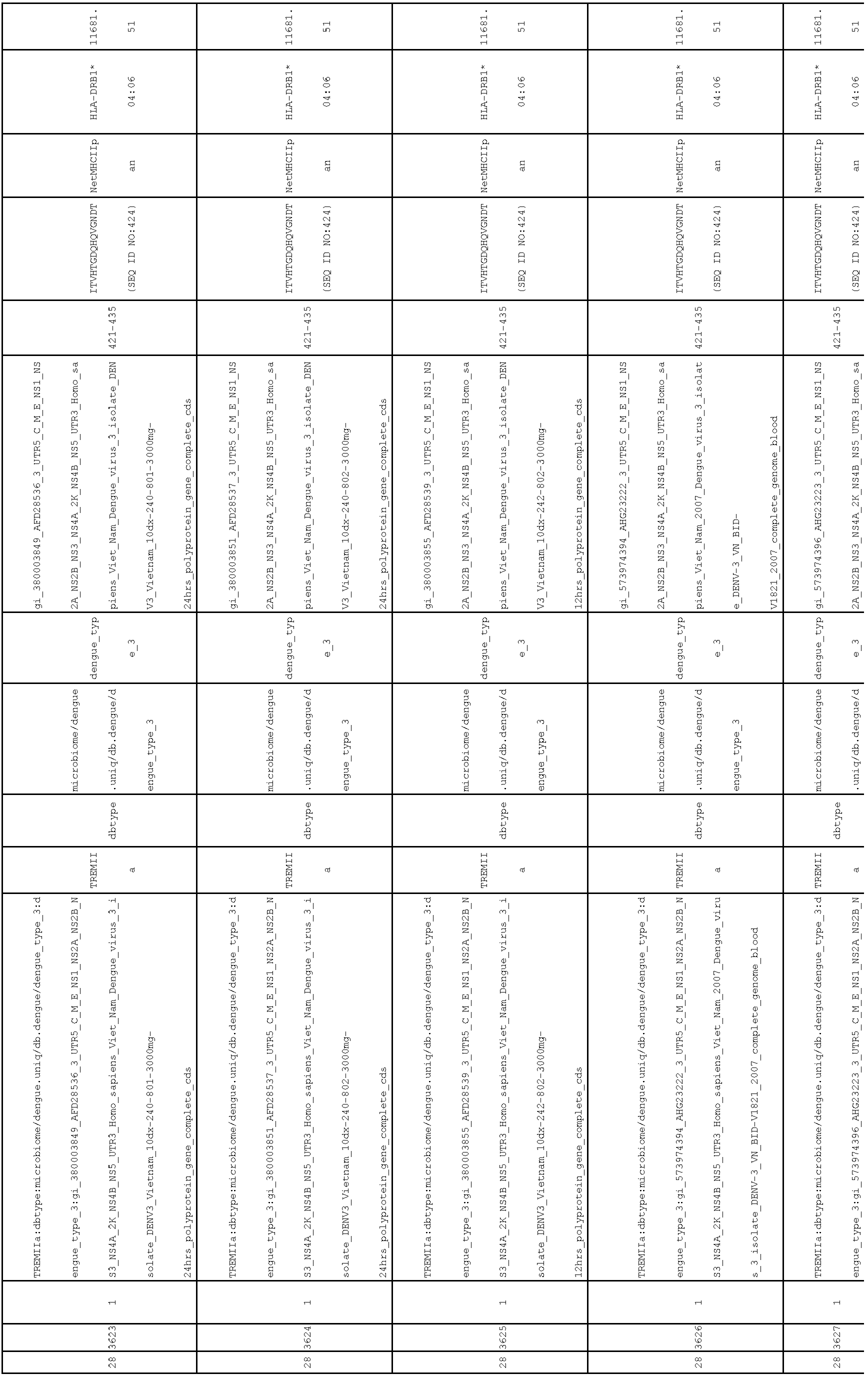

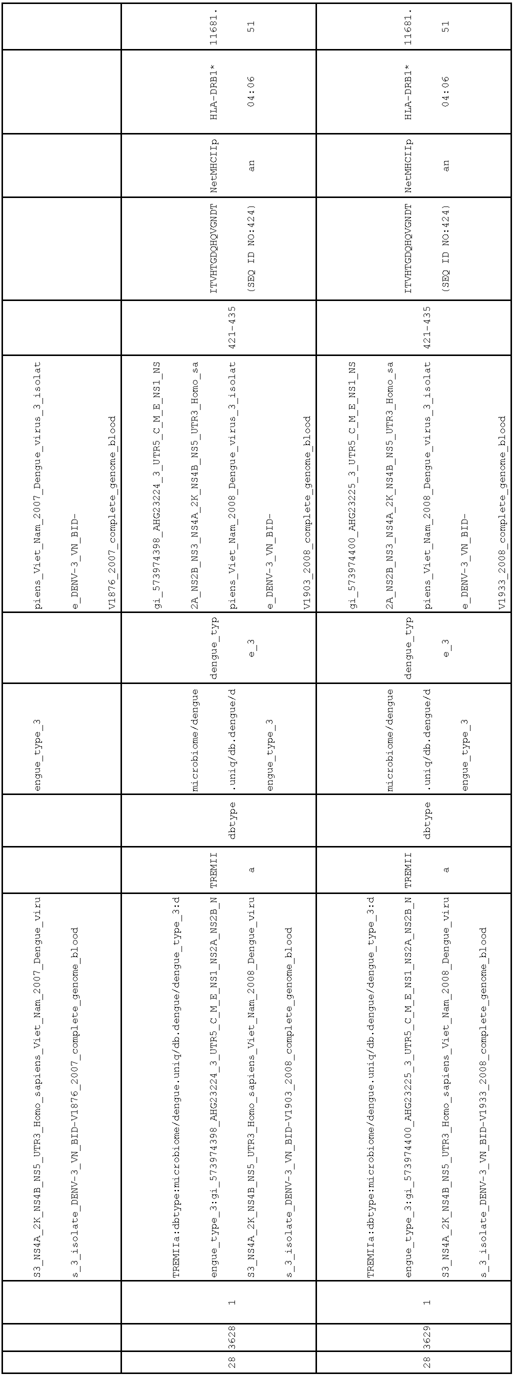

### (Example 7: Narrowing down of Peptides Based on MHC (HLA) Type)

In this example, peptides were narrowed down based on the HLA type. Specifically, among the peptide sequences obtained in Example 6, peptides based on the HLA type were narrowed down. Specifically, in the example of the dengue fever patient 1, peptide sequence information was extracted for HLA-A*02:01, HLA-A*24:02, HLA-B*07:02, HLA-B*46:01, HLA-C*01:02, HLA-C*07:02 in MHC class I. Meanwhile, peptide sequence information was extracted for DRB1*14:54, DRB1*15:01, DRB3*02:02, DRB5*01:01, DPA1*01:03/DPB1*02:02, DPA1*01:03/DPB1*04:01, DPA1*02:02/DPB1*02:02, DPA1*02:02/DPB1*04:01, DQA1*01:02/DQB1*05:02, DQA1*01:02/DQB1*06:02, DQA1*01:04/DQB1*05:02, DQA1*01:04/DQB1*06:02 in MHC class II. Further, the peptide sequence information was narrowed down by specifying the threshold value of the IC50 value to 5000, and an HLA typespecific peptide with a TREM sequence common between a repertoire clone sequence and a reference sequence was obtained (Step 7).

### (Results)

The results are shown in Tables 7-1 to 7-2. An HLA-specific peptide with a common TREM between the repertoire and the reference was obtained.

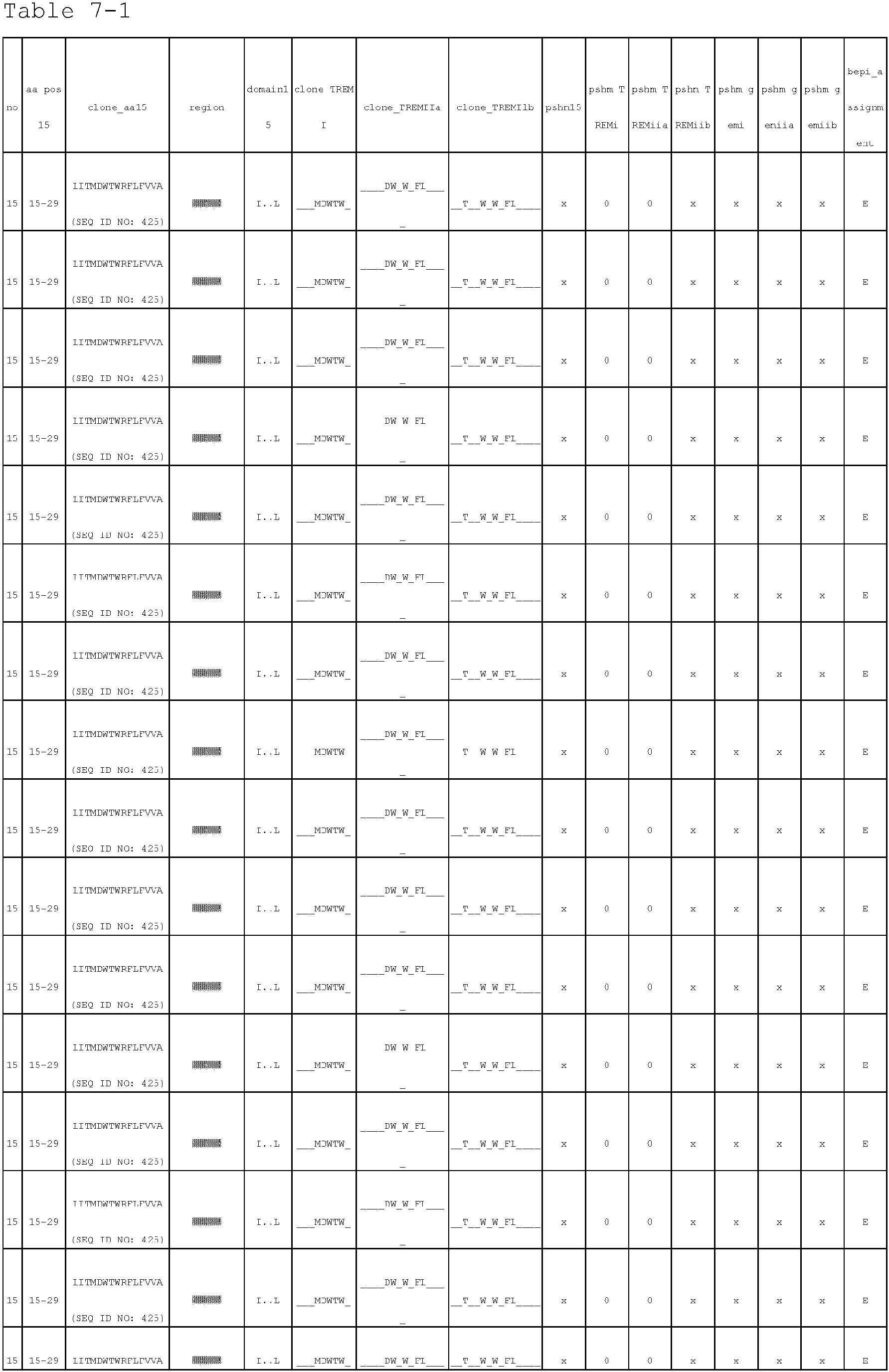

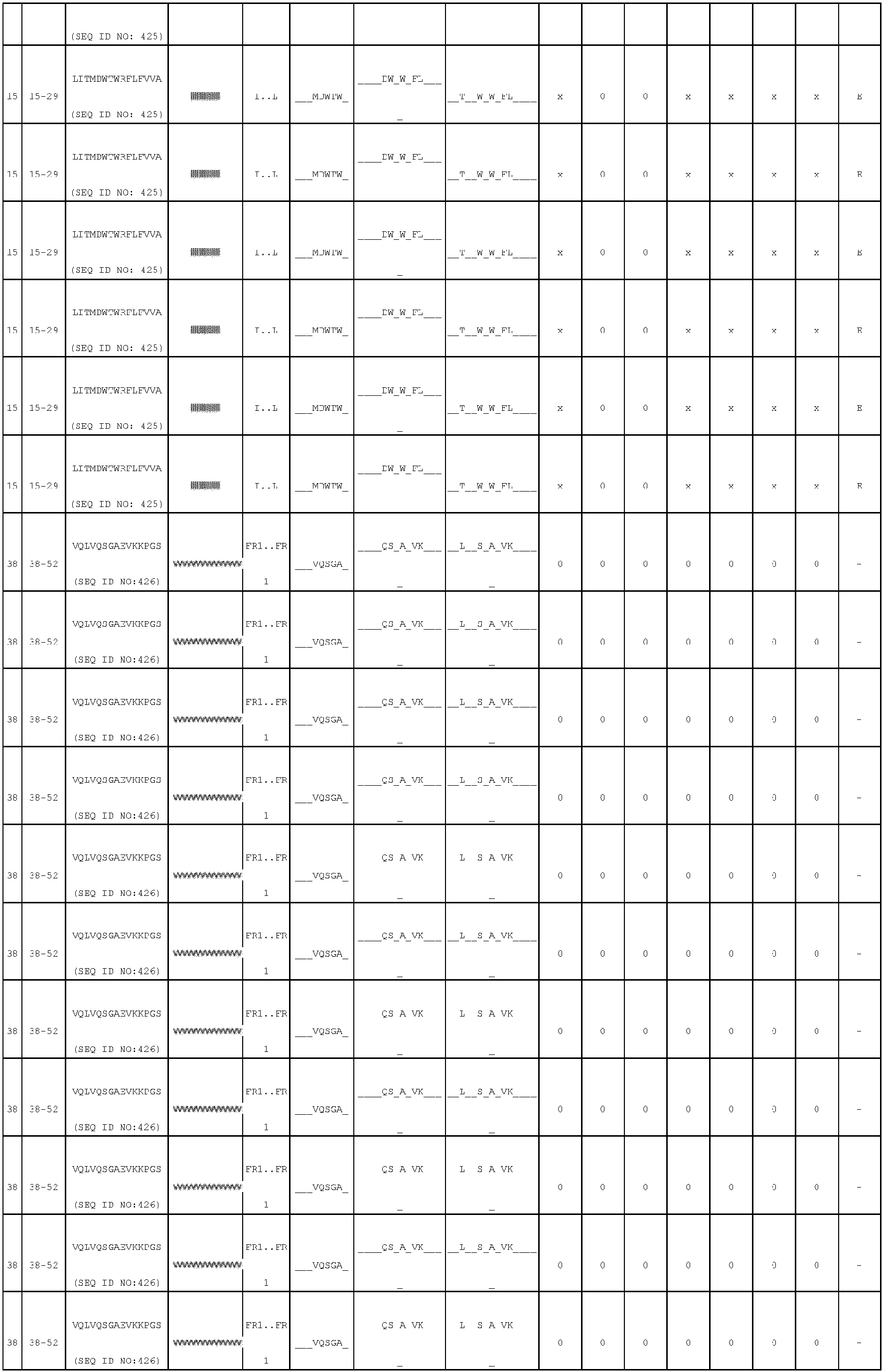

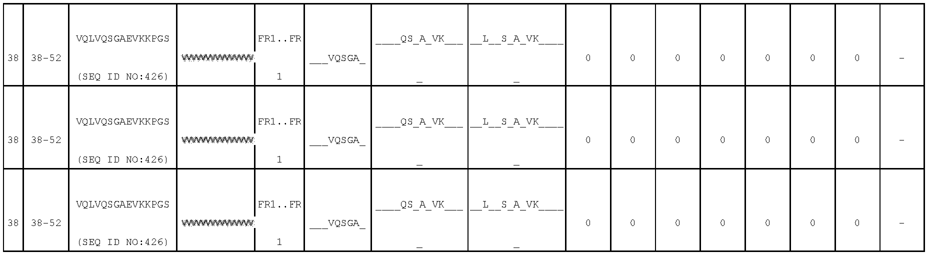

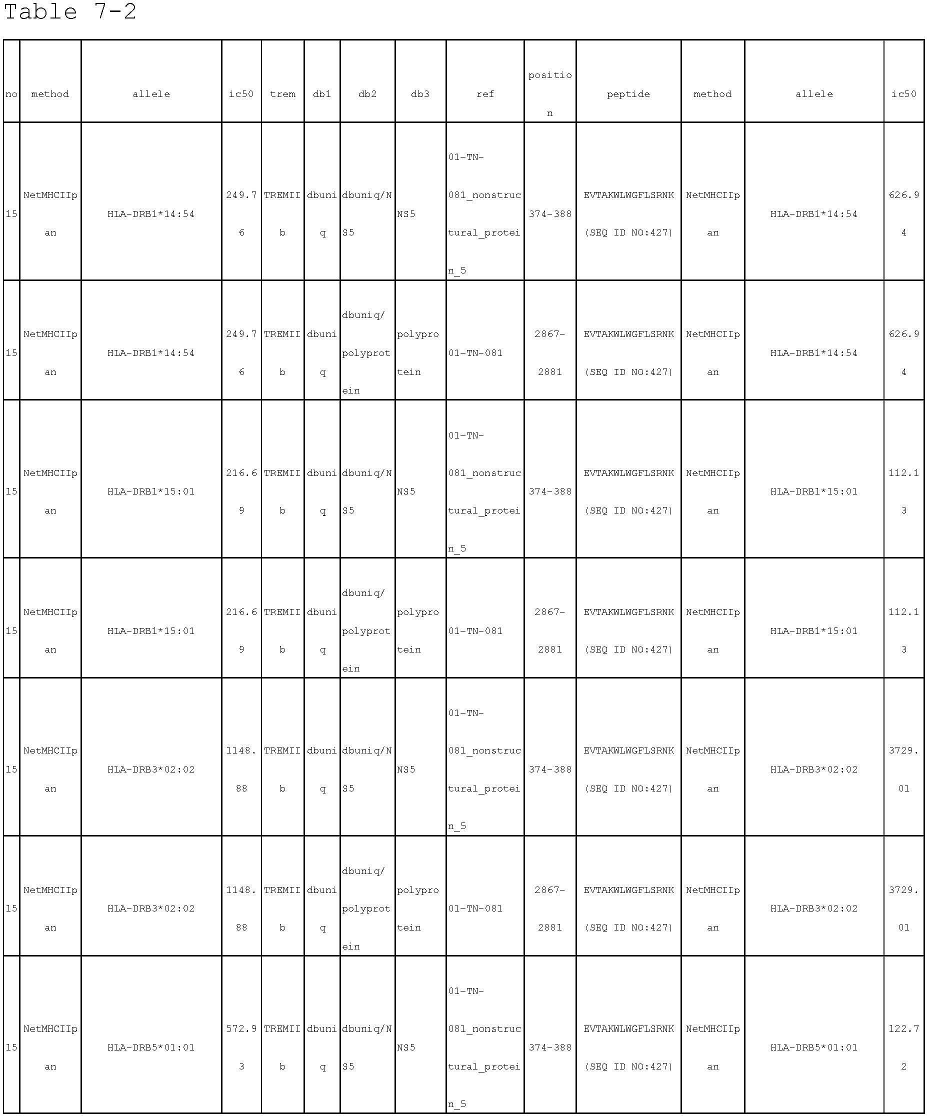

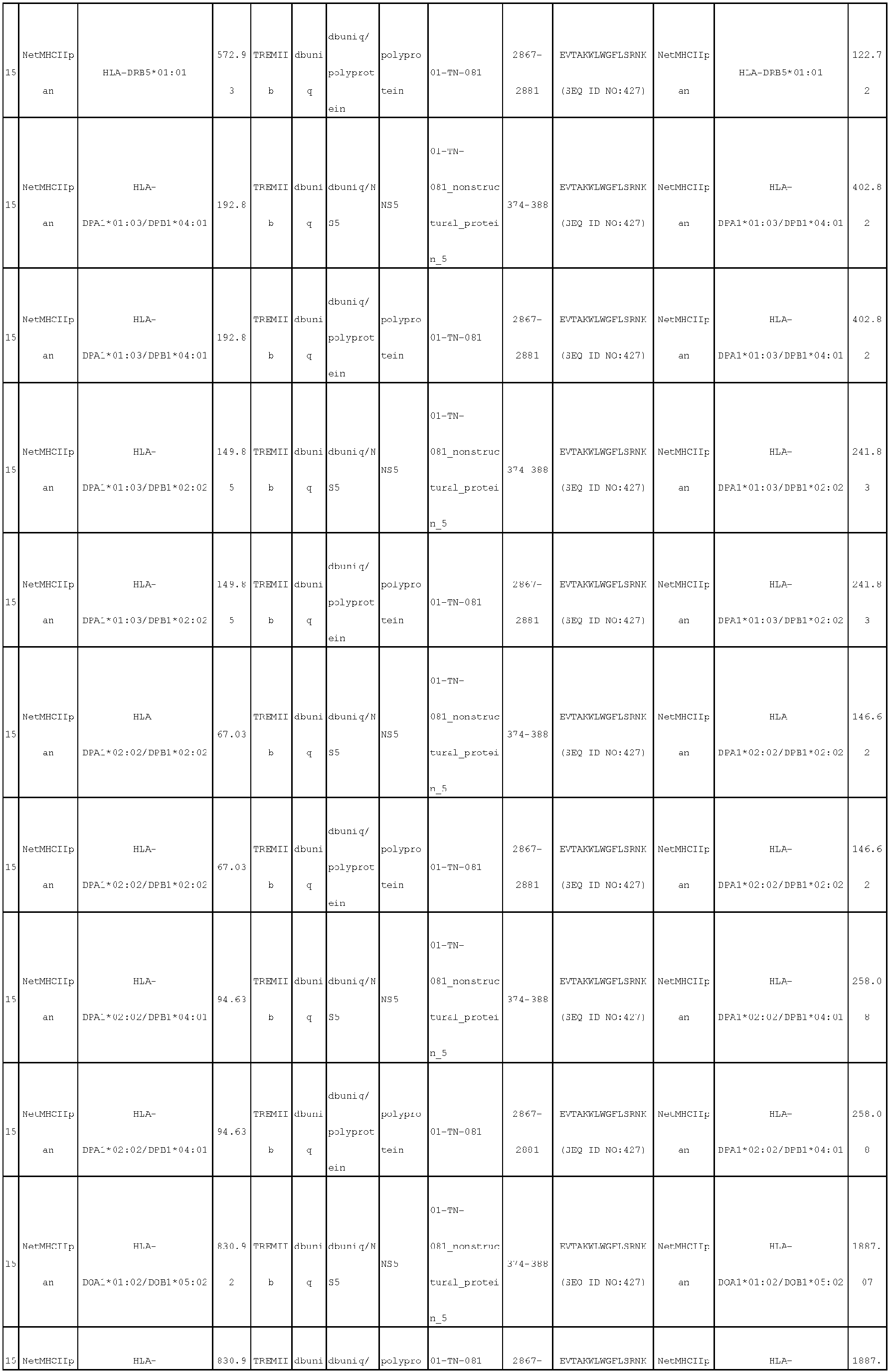

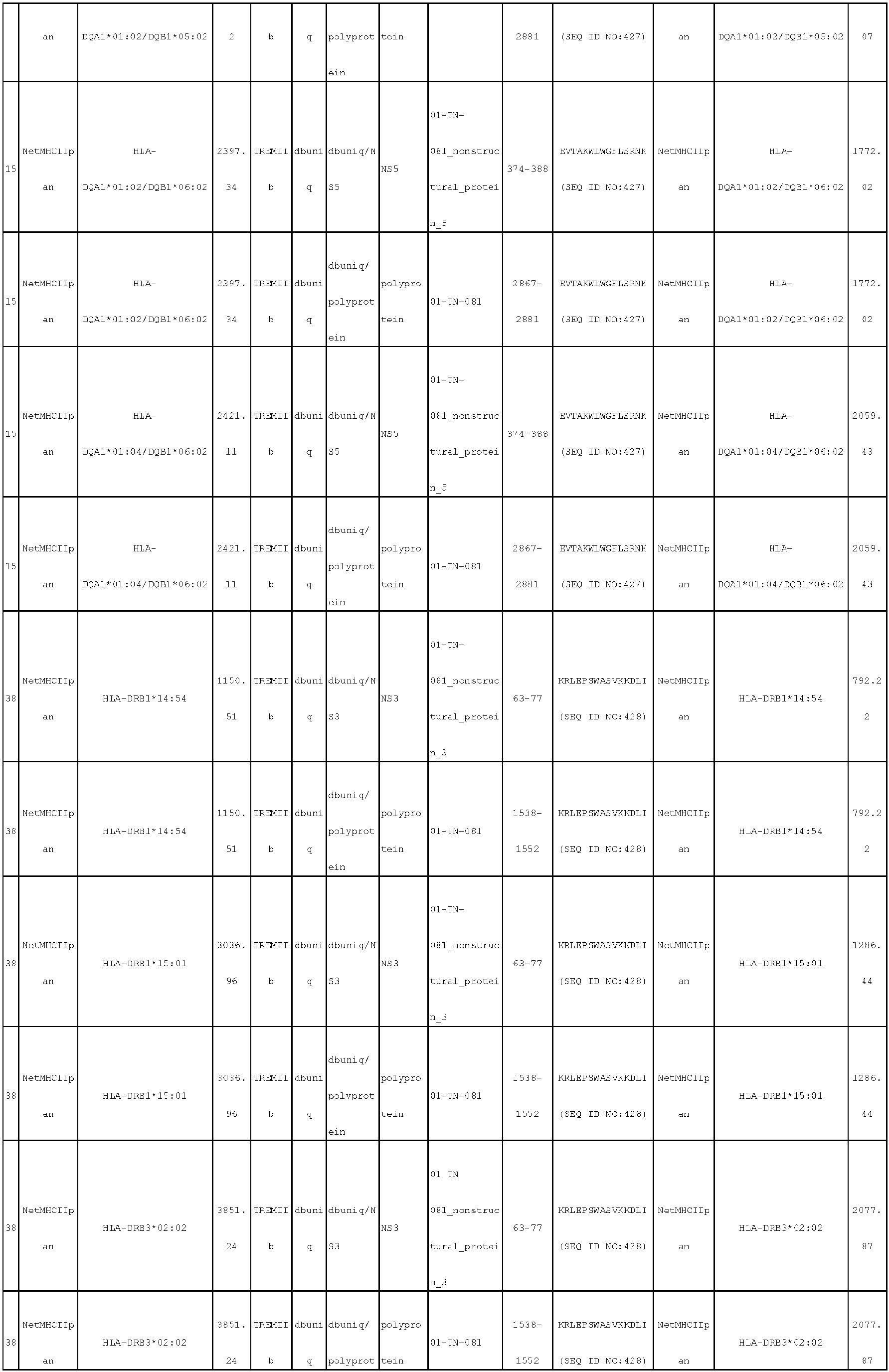

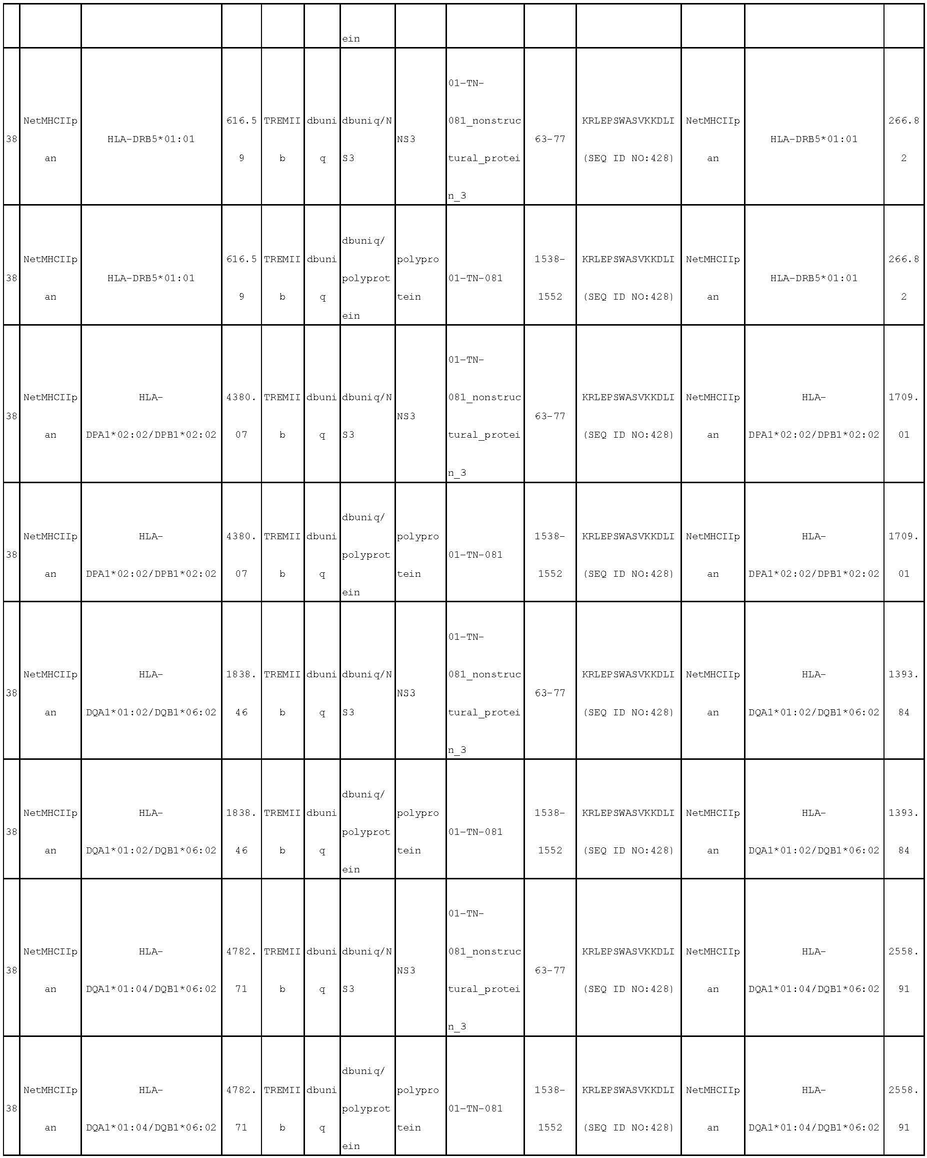

### (Example 8: Narrowing down Based on Commonality)

In this example, peptides were narrowed down from a plurality of specimens, and a highly versatile peptide sequence candidate was determined. Specifically, among the types of peptide sequences obtained in Example 7, peptides with high commonality among the specimens were narrowed down. Specifically, in the case of dengue fever infection, 49 types of TREM sequences obtained in all 18 patients were identified. Among them, 24 types of TREM sequences common to 2 or more patients were found, and 25 types of patient-specific (patient-unique) TREM sequences were found.
Further, from 24 types of TREM sequences common to 2 or more patients, the number of types of TREM sequences common to 5 or more patients was narrowed down to 14. The reference peptide sequences including these 14 types of TREM sequences were common among the 1 to 51 reference sequences to the TREM sequence. For each TREM sequence, the most common peptide sequence among the reference sequences was selected. Next, among these 14 types o pf reference peptide sequences, peptides having high compatibility (high versatility) with as many MHC types as possible were selected. Specifically, the intensity of binding affinity for a plurality of HLA types was analyzed. Among them, peptide sequences showing a moderate or higher binding affinity for as many HLA types as possible were selected. In this regard, it was determined that there was high binding affinity when IC 50 < 50, moderate binding affinity when IC 50 < 500, and low binding affinity when IC 50 < 5000. Here, 2924 types of HLA types were used for evaluating the binding affinity for class I HLA type, and 5620 types of HLA types were used for evaluating the binding affinity for class II HLA type. Among the 14 peptide sequences, peptide sequences with high binding affinity (IC 50 ≤ 500) in the HLA-DRB type were selected. Further, peptide sequences having gene fragment information of C gene were excluded from the candidate peptides. As a result, the peptide sequences were narrowed down to 12 peptide sequences having high versatility as epitope candidates.

### (Results)

The results are shown in Table 8. 12 epitope candidate sequences having a common TREM among specimens and having high compatibility with a large number of HLA types were obtained. A plus "+" indicates high versatility.

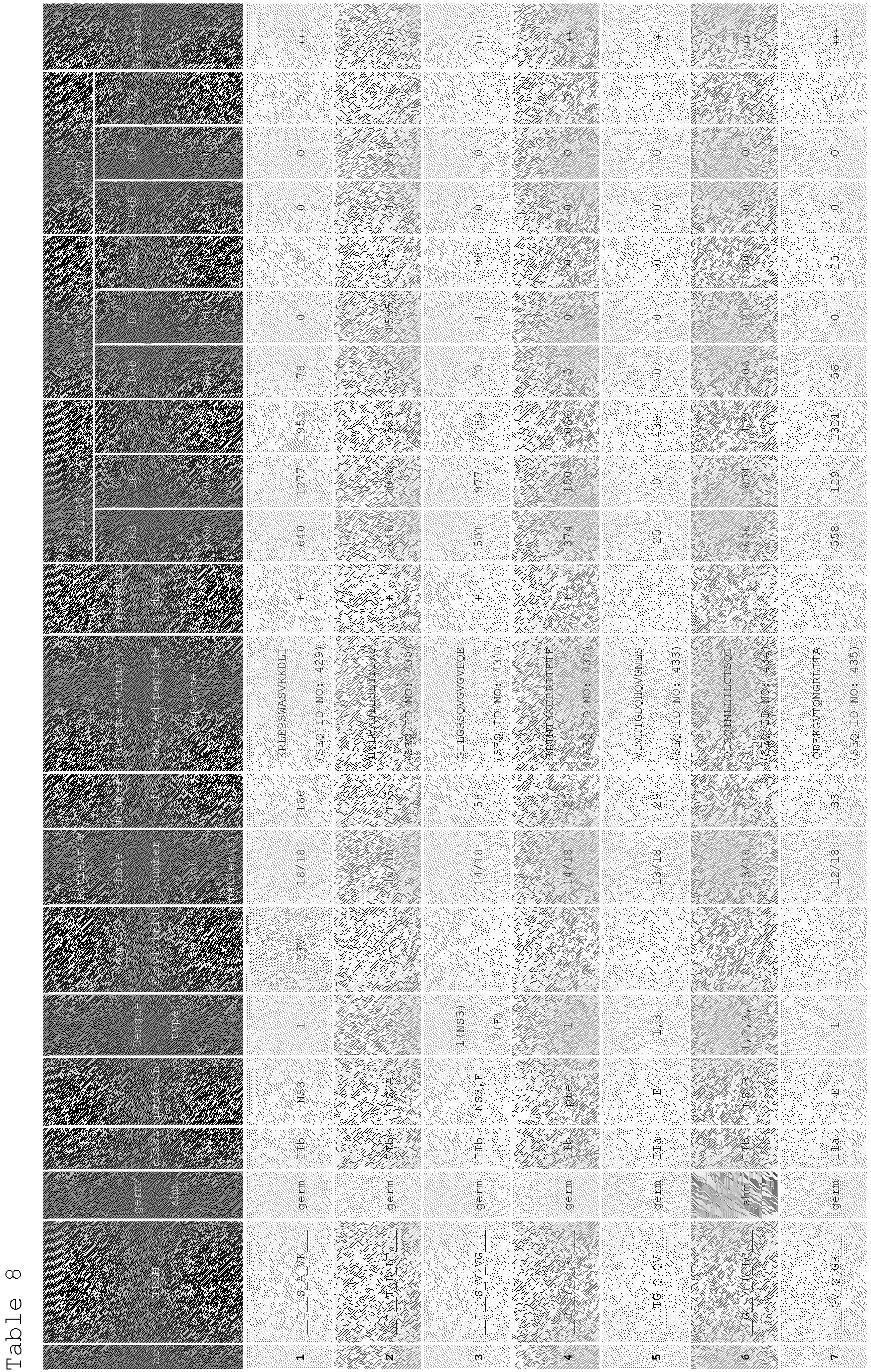

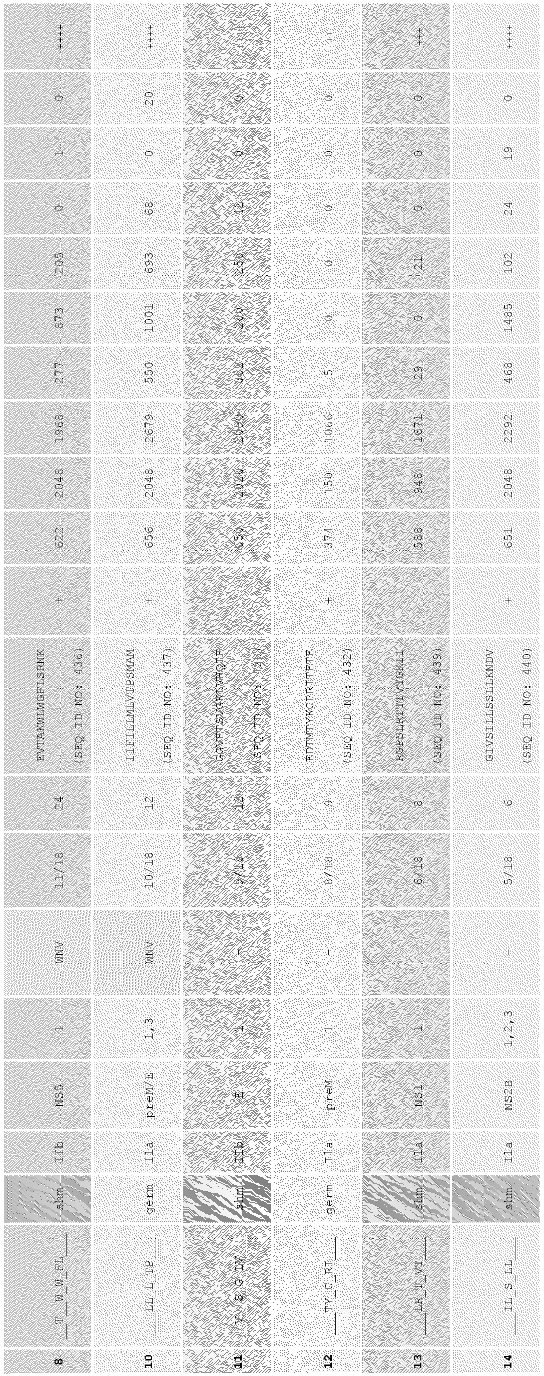

### (Example 9: Assessment Based on Experimentation)

There was performed an assay for specifying a peptide that could actually be an antigen from the peptide candidate list obtained up to Example 8. In this example, it was confirmed whether the sequence of the peptide obtained as described above could function as a T cell epitope. Of the 12 highly versatile epitope candidates obtained above, 8 epitope candidates were subjected to an assay using peptides.

### (Peptide Synthesis)

Of the 12 epitope candidates obtained above, 8 epitope candidates were synthesized by a peptide synthesizer. The synthesis was performed by outsourcing to GenScript (Tokyo, Japan).

### (Sample)

Human peripheral blood monocyte cells carrying HLA types and showing high binding affinity with 8 peptide sequences in Example 8 were used. Specifically, human peripheral blood monocyte cells carrying the following 2 types of HLA types were purchased from Lonza K.K. (Tokyo, Japan) and used. The HLA types of the first type of human peripheral blood monocyte cells used were HLA-DPA1*01:03,02:02, HLA-DPB1*04:01,05:01, HLA-DQA1*01:02,02:01, HLA-DQB1*03:03,05:02, and HLA-DRB1*07:01,15:02. The HLA types of the second type of human peripheral blood monocyte cells were HLA-DPA1*01:03, HLA-DPB1*04:01,04:02, HLA-DQA1*01:03,05:05, HLA-DQB1*03:01,06:03, and HLA-DRB1*13:01,14:06.

The IC50 values for the HLA types of the cells used in each peptide sequence are shown in Table 9.

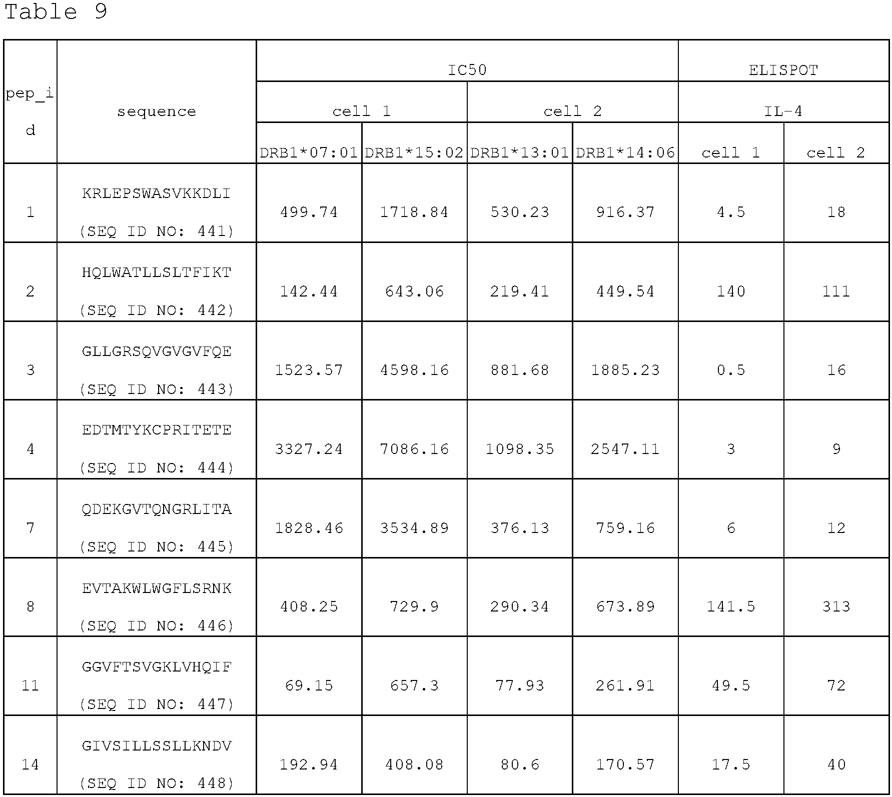

### (Culture of Cells)

Cells were cultured in the following manner. Cells were rested overnight in RPMI 1640 medium with 10% FBS after thawing. Then, IL-2 and synthetic peptides at final concentrations of 10 U/ml and 1 µg/ml were added to different wells, respectively, and cultured in a 5% CO₂ incubator at 37°C for 13 days. During the course of culture, 3 days, 6 days and 9 days after the culture, a half amount of the medium was removed, and the same amount of medium and IL-2 and synthetic peptides at final concentrations of 10 U/ml and 1 µg/ml were added.

### (ELISPOT Assay)

ELISPOT assays for interferon-γ and interleukin-4 were conducted. The assays were conducted according to the protocol attached to the ELISPOT kit (product number: hIFNgIL4-2 M/2) manufactured by C.T.L. (Ohio, U.S.A.). Specifically, each of the assays was performed in the following manner. (1) PVDF membranes on 96 well plates for ELISOPT were pretreated with 70% ethanol and washed with PBS. The antibody solution was added and incubated in a humidified container at 4°C overnight. (2) After washing with PBS, 4 × 10⁵ cells cultured as described above were added to each well, peptides were added at a final concentration of 10 µg/ml, or anything was not added as a control, and the wells were left to stand still in a CO₂ incubator at 37°C for 48 hours. (3) The cells were removed and washed with PBS + 0.05% Tween-20, then a detection solution was added thereto, and the mixture was incubated at room temperature for 2 hours. (4) After washing with PBS + 0.05% Tween-20, a strep-AP solution was added, and the mixture was incubated at room temperature for 30 minutes. (5) After washing with PBS + 0.05% Tween-20, a developer solution was added, and the mixture was incubated at room temperature for 10 to 20 minutes. The number of spots of the color-developed plate was measured by a contract analysis service of M&S TechnoSystems, Inc. (Osaka, Japan).

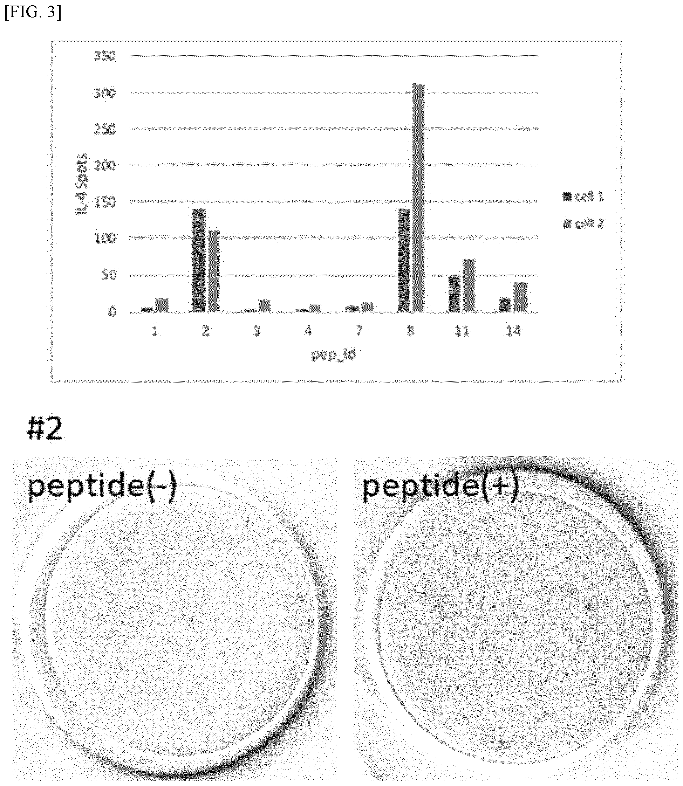

The results of the ELISPOT assay are shown in Table 9 and FIG. 3. It was found that 4 out of 8 peptides verified this time, i.e. 50%, had an IL-4 positive response to ELISPOT. Candidates for T-cell epitopes, which may be useful in as many as 50% examples, have been found. This is not the technique of the related art, but can be said to be a remarkable effect.

### (Comparative Example 1: Comparison with Method Using Classification According to Frequency of TCEM Sequence Use)

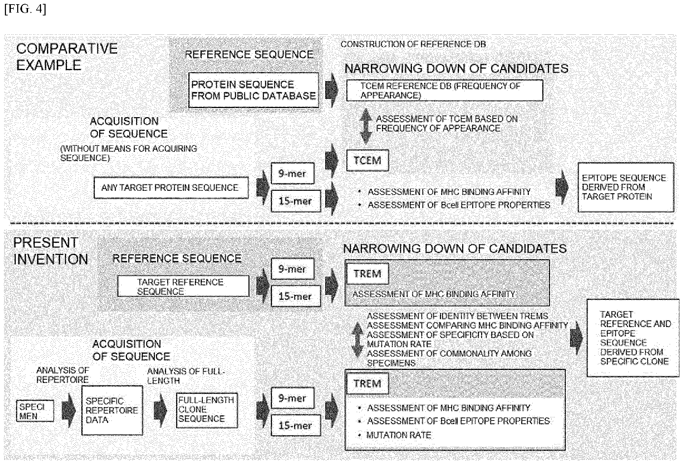

In this comparative example, the method of the present disclosure as described above was compared with a method using classification according to the frequency of T-cell exposed motif (TCEM) sequence use (FIG. 4). Note that the TCEM sequence is a continuous or discontinuous motif sequence composed of about 5-mer amino acids, similarly to the TREM sequence of the present invention. In the method used as the comparative example, a T cell epitope sequence derived from a target protein can be obtained as follows. This method is a technique for identifying a peptide having a TCEM sequence with a frequency of appearance based on the TCEM database from the target sequence.

### (Target sequence, Sequence Acquisition)

In the comparative example, the target sequence is any existing protein sequence, and no sequence acquisition method is included. Further, the target sequence is not limited to repertoire clone sequences.

### (Reference Sequence, Database)

In the comparative example, 40000 or more types of known protein sequences derived from a public database or the like are used as reference sequences, and 9-mer and 15 mer peptide sequences are created from these protein sequences. Further, a TCEM sequence is extracted from these peptide sequences, the number of times (frequency of appearance) each TCEM sequence appears in these protein sequences is calculated, and a database of the frequency of appearance of the TCEM sequence is constructed. Reference proteins used are selected from immunoglobulin proteins, human proteomes (other than immunoglobulins), allergen proteins, microbial proteins. Also, means for obtaining a reference sequence is not included.

### (TCEM Sequence)

In the comparative example, the TCEM sequence conforms to Rudolph et al. (2006) and the like, similarly to the present invention, and is a continuous (TCEMI) or discontinuous (TCEMIIa, TCEMIIb) motif sequence composed of 5-mer amino acids, is a sequence (TCEMI) composed of amino acids present at positions P4, P5, P6, P7 and P8 in the 9-mer amino acid sequence (with position numbers P1 to P9), and include sequences (TCEMIIa and TCEMIIb, respectively) composed of amino acids present at positions P2, P3, P5, P7 and P8, or positions P-1, P3, P5, P7 and P8 in the 15-mer amino acid sequence (with position numbers P-3 to P12).

### (Method for Identifying Epitope Sequence 1)

In the comparative example, 9-mer and 15-mer peptide sequences are created from a target sequence, a TCEM sequence is extracted from each of the peptide sequences, the peptide sequences are classified based on the frequency of appearance of TCEM sequence in the TCEM database, and a peptide sequence with known frequency of appearance of the TCEM in the database is selected from the target sequence and identified. Further, MHC binding affinity in these peptide sequences is calculated.

### (Method for Identifying Epitope Sequence 2)

It is determined whether the frequency of appearance of the TCEM contained in the target sequence is 1/64 or more, 1/1024 or more, or less, the TCEM sequence of the target sequence is ranked by the frequency of appearance, and the immunogenicity of the target sequence is characterized.

In the method of the comparative example as described above, an epitope sequence search is performed only based on existing information, and thus an efficient epitope candidate cannot be identified. However, the method of the present invention includes: obtaining and using a reference sequence suitable for a purpose; obtaining and using a specific repertoire clone as a target sequence; and further including the identity between these TCEM sequences and the MHC binding affinity between both the sequences, as a result of which a specific T cell epitope can be identified more reliably.

### (Target Sequence to Be Analyzed)

In the comparative example, the target sequence to be analyzed is any existing protein sequence. Meanwhile, in the present invention, a repertoire clone sequence generated by a specific immune response is used as a sequence to be analyzed, and this makes it possible to identify an antigen that has induced the specific immune response.

### (Acquisition of Target Sequence)

The comparative example does not include a method for acquiring a target sequence. Meanwhile, in the present invention, a sequence of a unique repertoire clone generated by a specific immune response is acquired by performing repertoire analysis and full-length sequence analysis of the repertoire clone. Since the repertoire analysis and the full-length sequence analysis of the repertoire clone are independently established analysis systems, the sequence of the repertoire clone can be acquired by combining the systems. In particular, the sequence of the BCR clone has a unique mutation generated by somatic hypermutation, and this mutation can be identified only by the full-length sequence analysis of the present invention. This identification cannot be performed in the comparative example without the means for acquiring a sequence.

### (Reference Sequence)

In the comparative example, a database of TCEM sequences constructed from 40000 or more known protein sequences is constructed and used as a reference sequence. Meanwhile, in the present invention, focusing on a specific immune response, analysis targeting the specific immune response can be performed by using a reference sequence that causes the specific immune response.

### (Method for Identifying Epitope Candidate 1)

In the comparative example, adopted is a method in which a database including a TCEM sequence present in an existing protein sequence is constructed, and the TCEM sequence is classified based on the frequency of appearance (the number of times a certain TCEM sequence appears in the reference sequence group). In this system of classifying the frequency of appearance, a case where the frequency of appearance is low (such as 1/1024 or less) is classified as a case where the possibility of the T cell epitope is high. Meanwhile, in the present invention, adopted is a method for identifying a T cell epitope from the identity between a TREM sequence present in an idiotope of TCR and BCR: antigen receptors and a TREM sequence present in a reference sequence based on the immune network theory (Niels K. Jerne, 1974). Particularly, in the present invention, focusing on a specific immune response, the TCR and BCR of the clone generated by the specific reaction and the reference sequence causing the TCR and BCR are used for analysis, an epitope with high specificity can be identified.

### (Method for Identifying Epitope Candidate 2)

In the comparative example, MHC binding affinity is assessed only for a peptide sequence obtained from a target sequence. Meanwhile, in the present invention, highly accurate epitope identification can be realized by evaluating MHC binding affinity of both a target peptide sequence obtained from the TCR and BCR and a reference sequence based on the immune network theory (Niels K. Jerne, 1974).

### (Method for Identifying Epitope Candidate 3)

In the method of the present disclosure, a highly versatile T cell epitope can be identified by focusing on commonality. Specifically, a plurality of specimens of the same disease example is compared and analyzed to identify a TREM sequence with high commonality, and a peptide sequence having binding affinity in common to a wide range of MHC types (HLA types) is identified. Accordingly, it is possible to identify a highly versatile epitope. This method was not performed in the comparative example.

### (Example 10: Applied Example)

In this example, it was confirmed whether or not the peptide obtained by the method of the present invention was actually an epitope.

For the 49 types of TREM sequences identified from dengue virus-infected patients in Example 8, it was confirmed that peptides including the TREM sequences thereof were demonstrated as T cell epitopes. As a result, among the 49 types of TREM sequences, 19 types of TREM sequences corresponding to 38.7% had already been revealed as T cell epitopes inducing the production of interferon-γ on T cells. The results are shown in Tables 10-1 to 10-2.

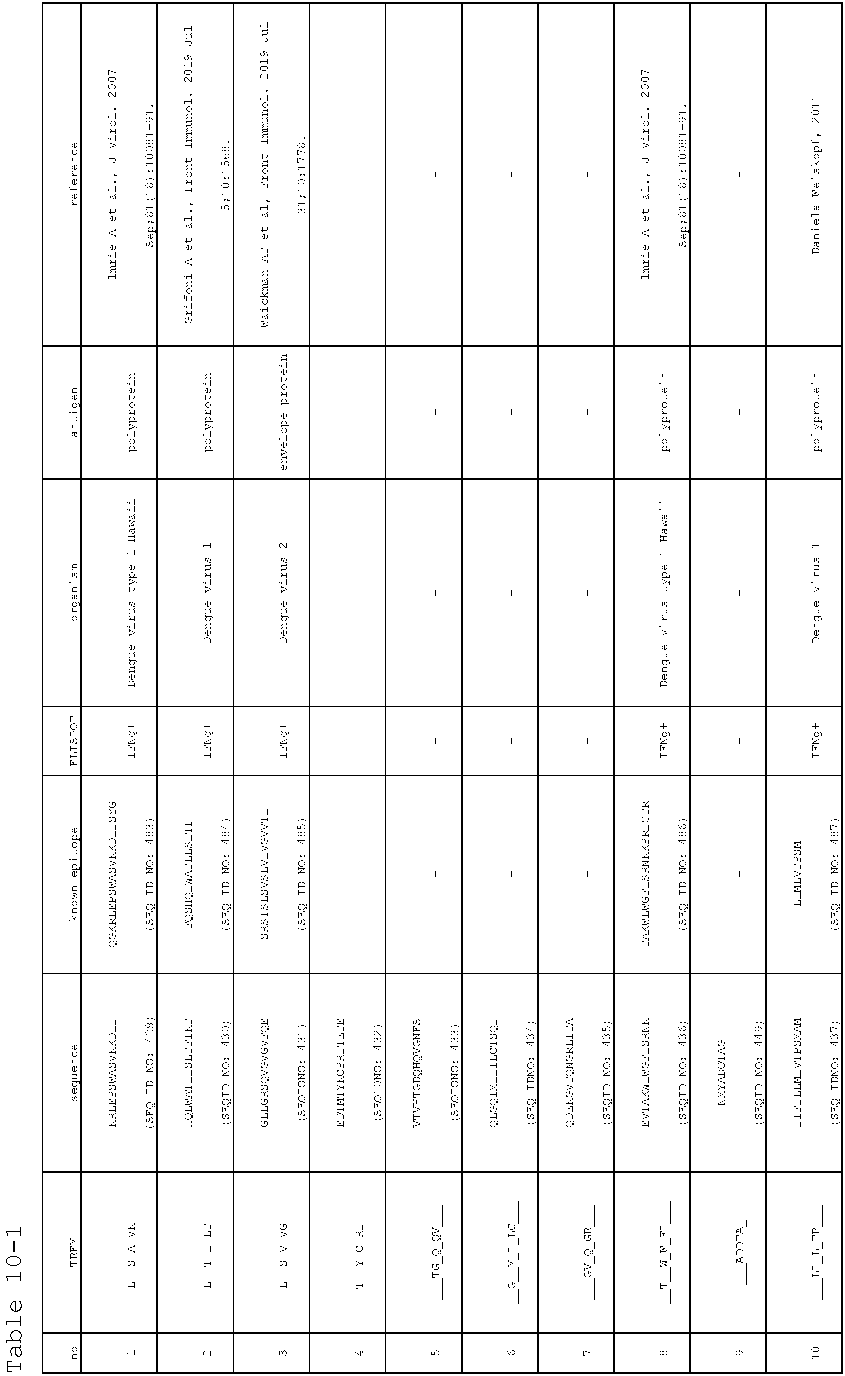

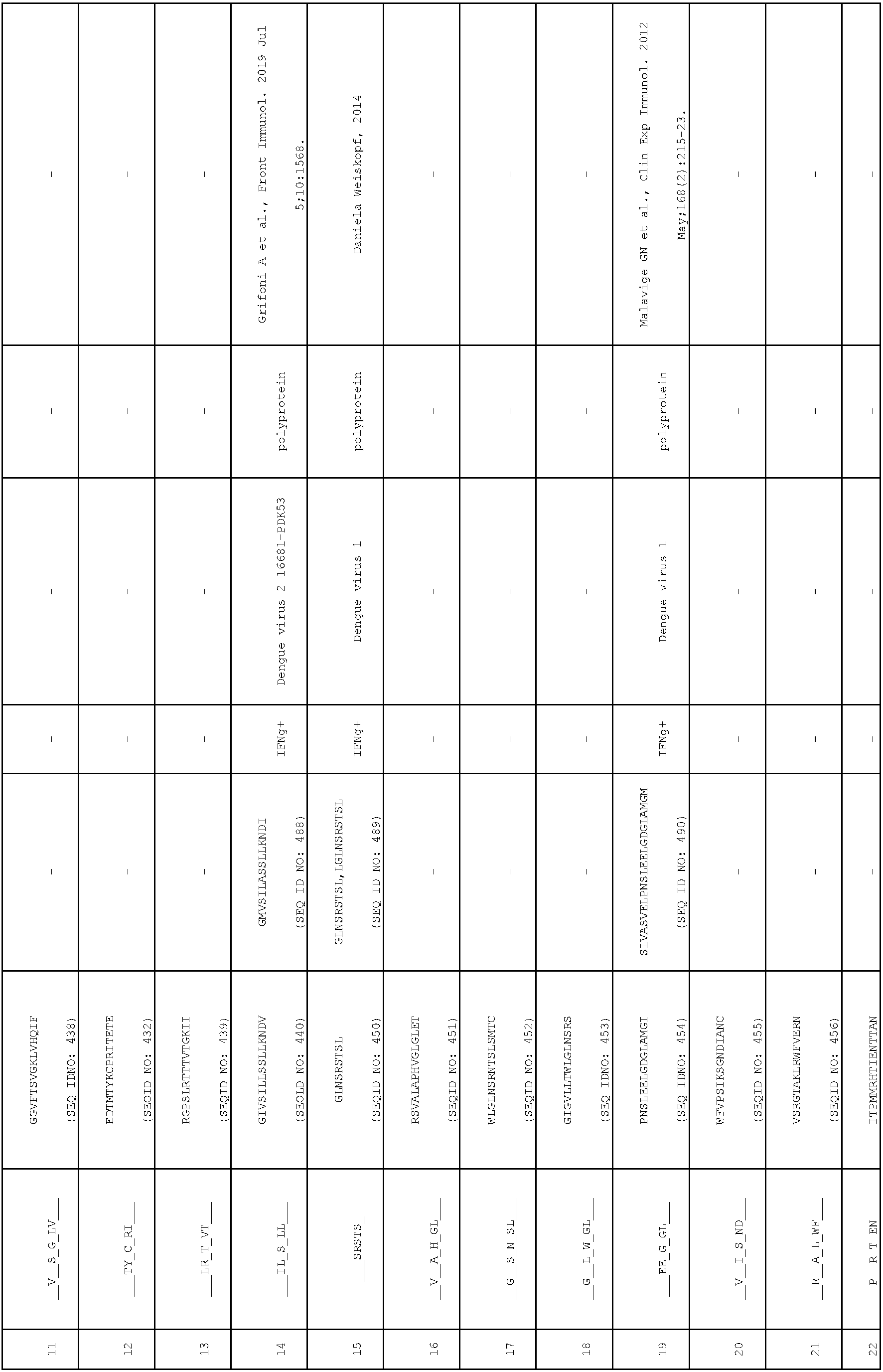

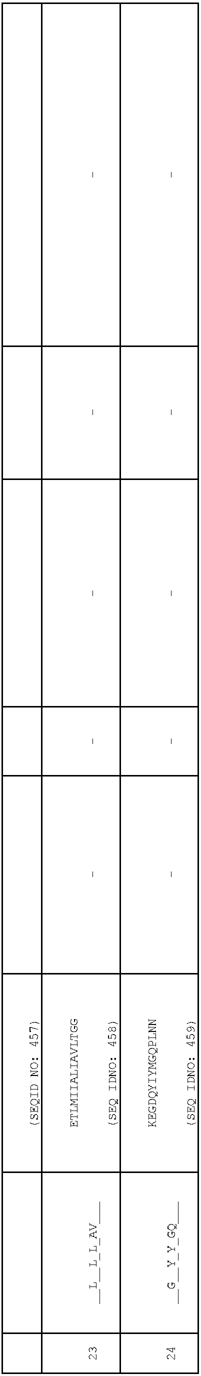

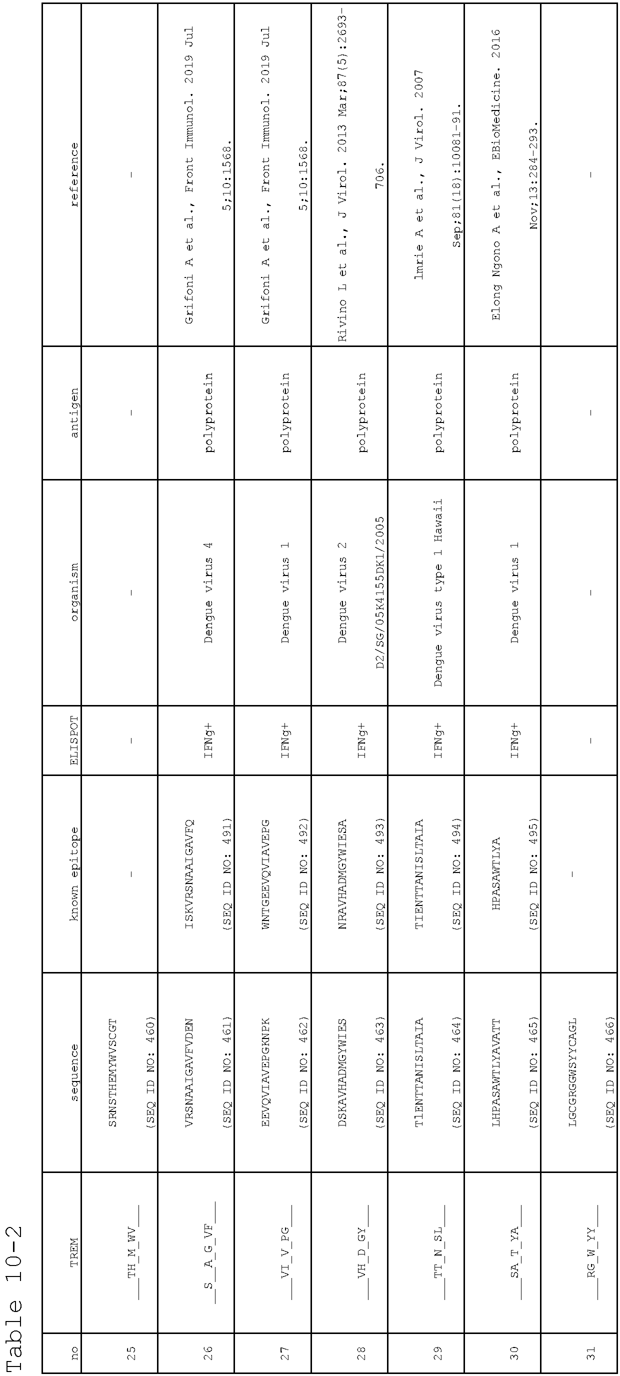

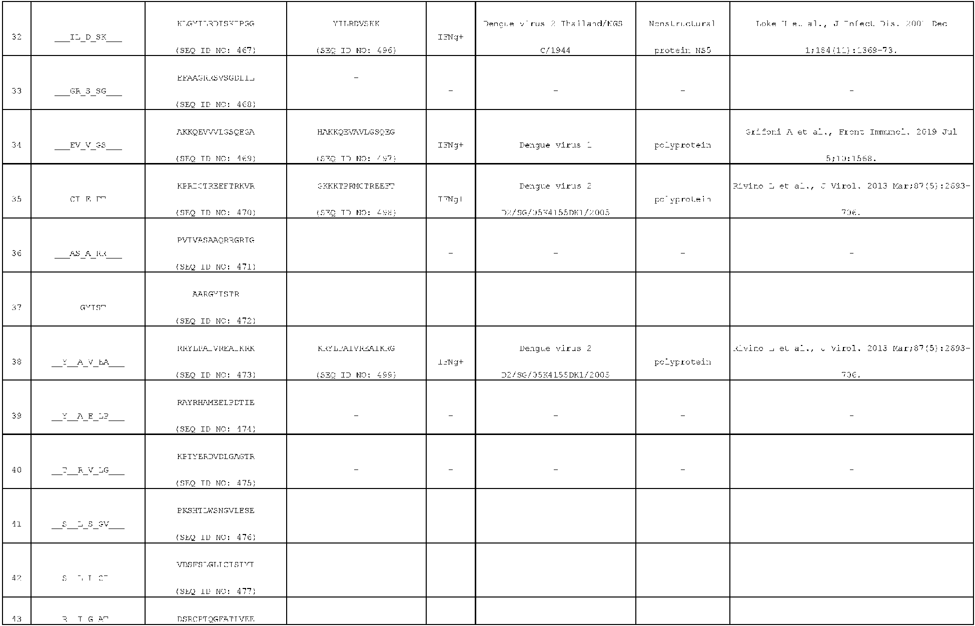

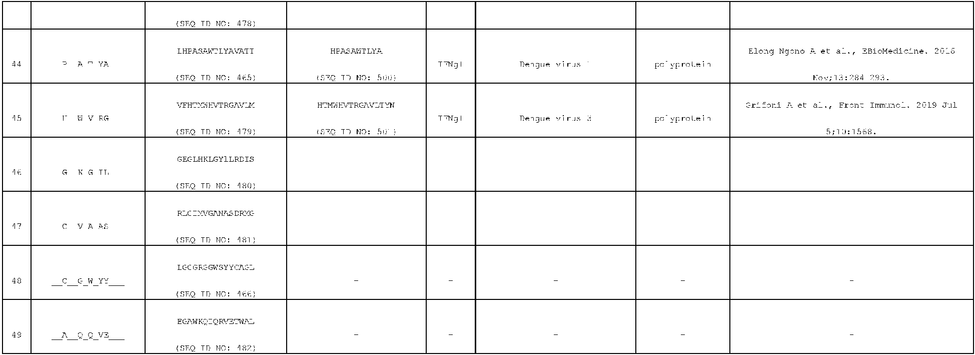

### (Example 11: Example in Tumor)

The present invention can be applied to any immune response associated with a specific physiological state. For example, a T cell epitope can also be identified for a tumor using as follows. Repertoire analysis of TCR and BCR is performed using a tumor tissue as a specimen, and a repertoire clone present in the tumor tissue is specified. The full-length sequence of the clone is obtained by the full-length analysis, and 9-mer and 15-mer peptide sequences are created in the same manner as in the Examples descried above. TREM sequences are extracted from the peptide sequences. These TREM sequences are compared with a reference sequence to identify a common TREM sequence. Here, as the reference sequence, a variant protein sequence specific to the tumor tissue is used. This reference sequence can be obtained by performing neoepitope analysis (analysis combining RNA and EXOME sequences, mutation search) on the tumor tissue. For the peptide sequence including a common TREM sequence, the binding affinity for the patient's HLA type is calculated. As a result, the peptide sequence with high binding affinity thus obtained can be a T cell epitope candidate in tumor tissue.

### (Example 12: Assessment of Commonality among Patients, Specimens and Clones)

In the present invention, as shown in Tables 11-1 to 11-4, 49 types of TREM sequences were identified from the analysis examples of 18 dengue virus patients. Among them, 24 types were common to at least 2 persons or more. Further, the 24 types of TREM sequences were not only common among patients, but also common among clones of the same or different specimens. Actually, 50% of the 8 peptide sequences selected from these 24 sequences induced T-cell activity. Therefore, a highly versatile peptide candidate can be obtained by selecting a sequence with high commonality. Meanwhile, among the 49 types, 25 types were patient-unique sequences. Consequently, these sequences can be candidates for patient-specific peptides.

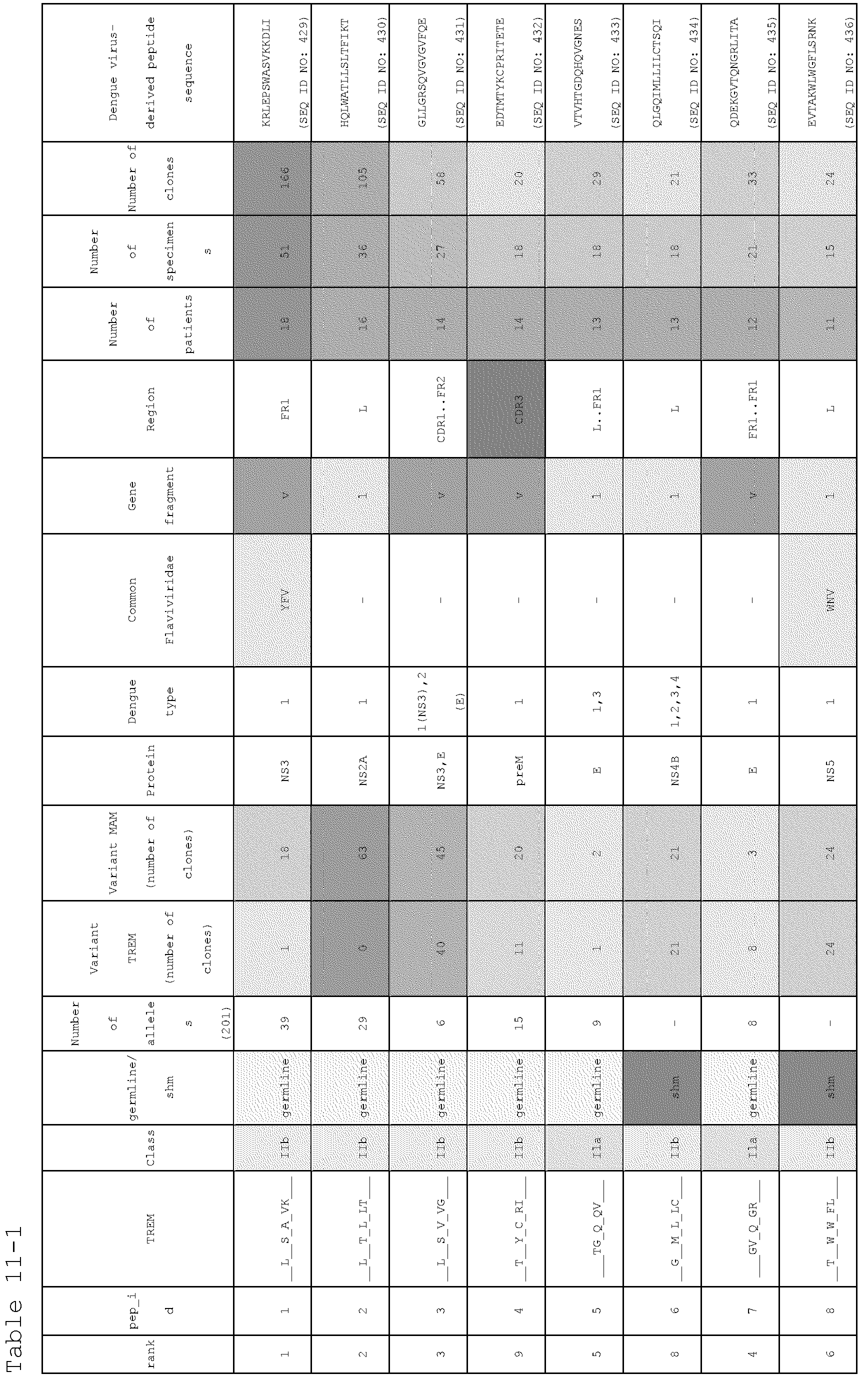

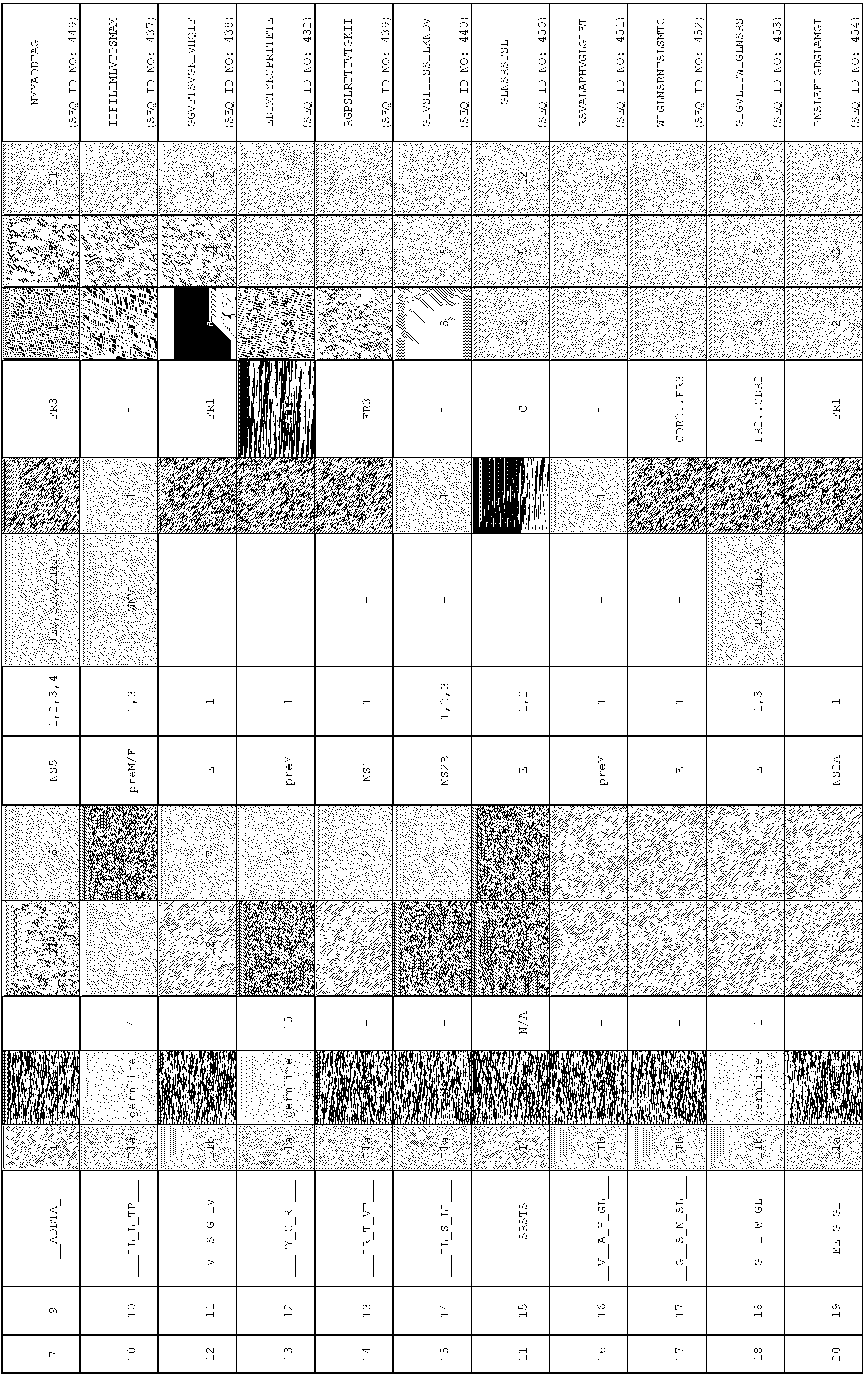

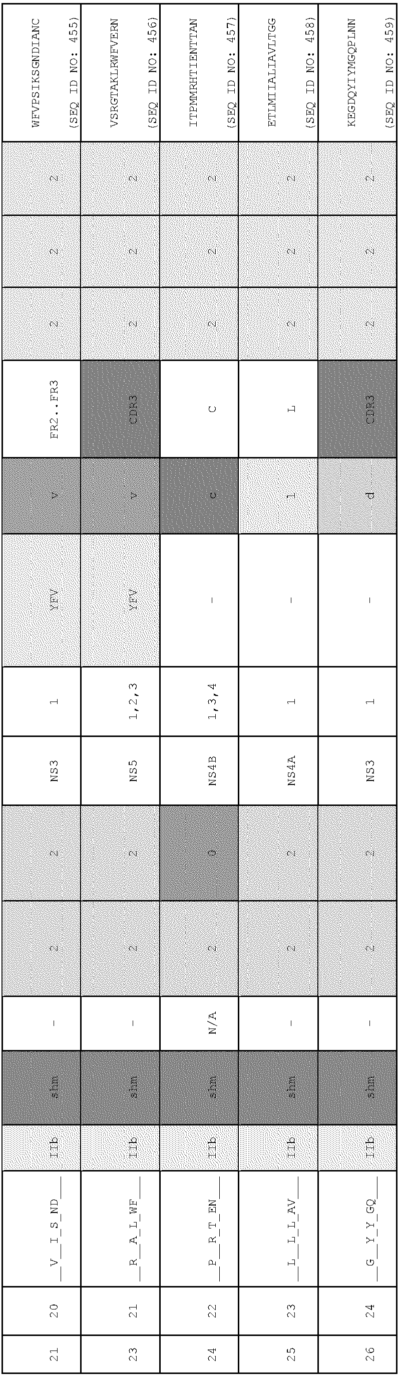

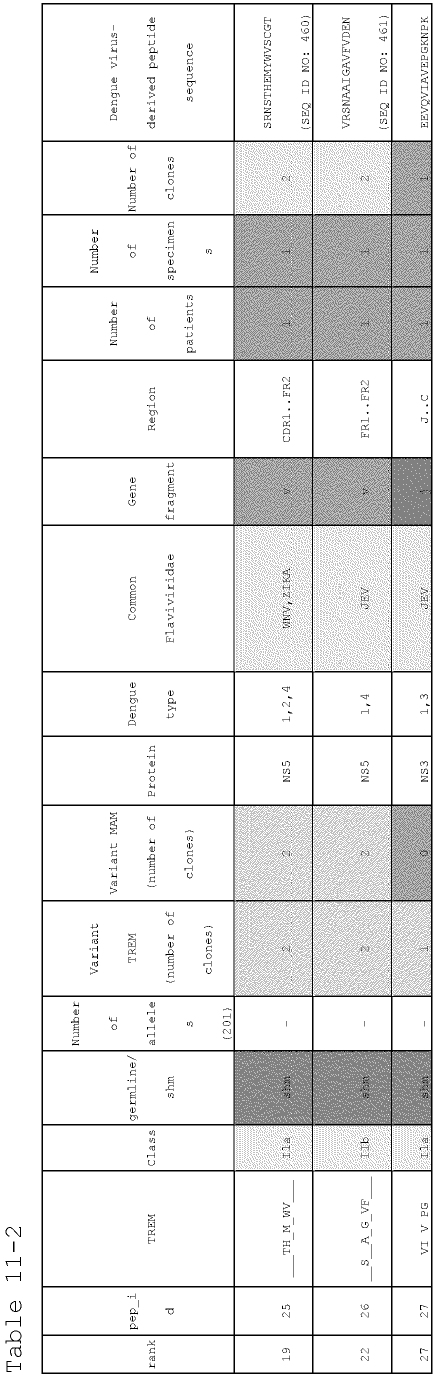

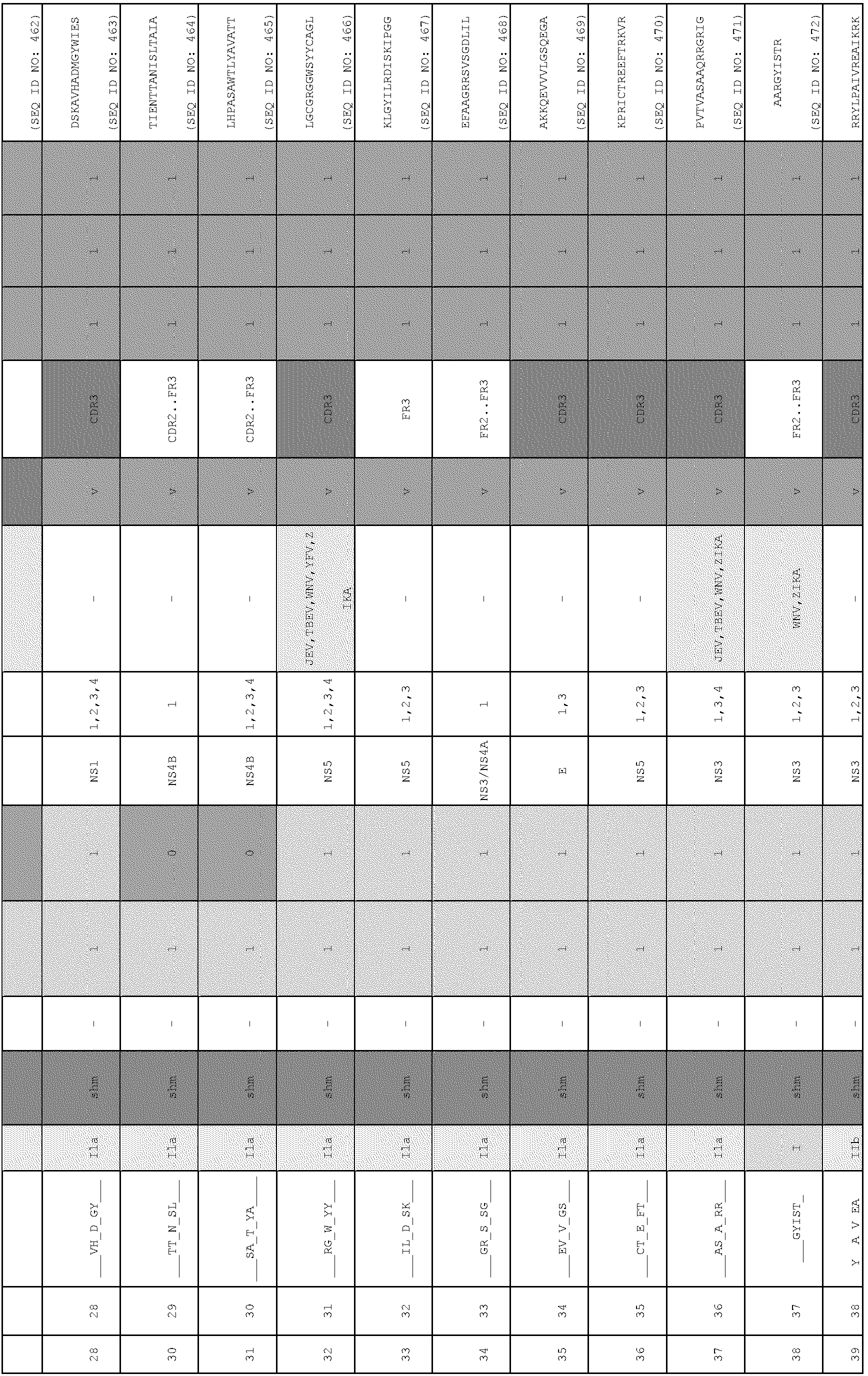

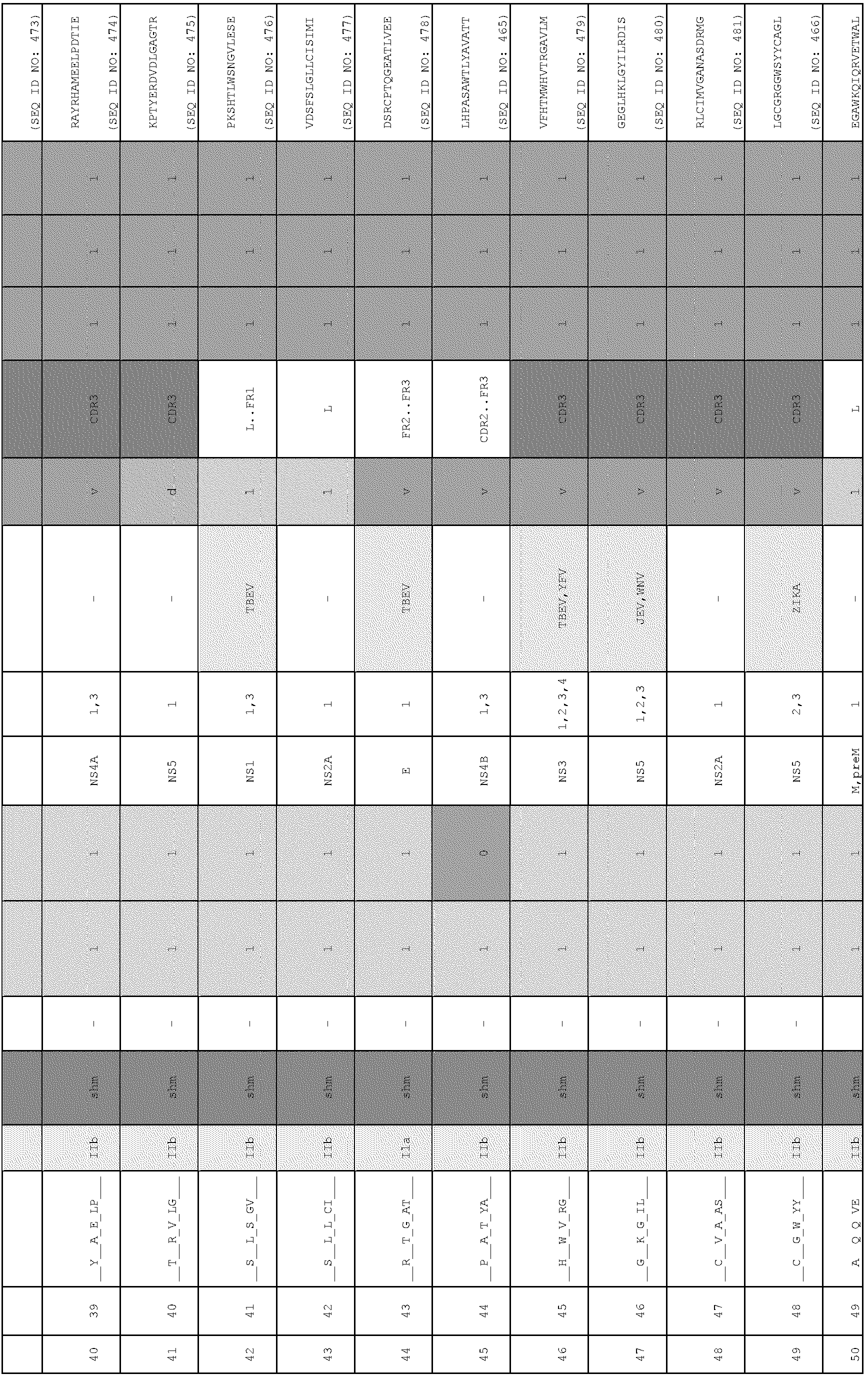

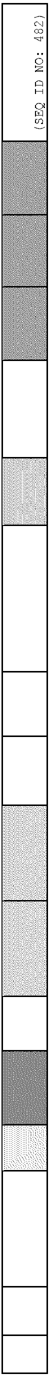

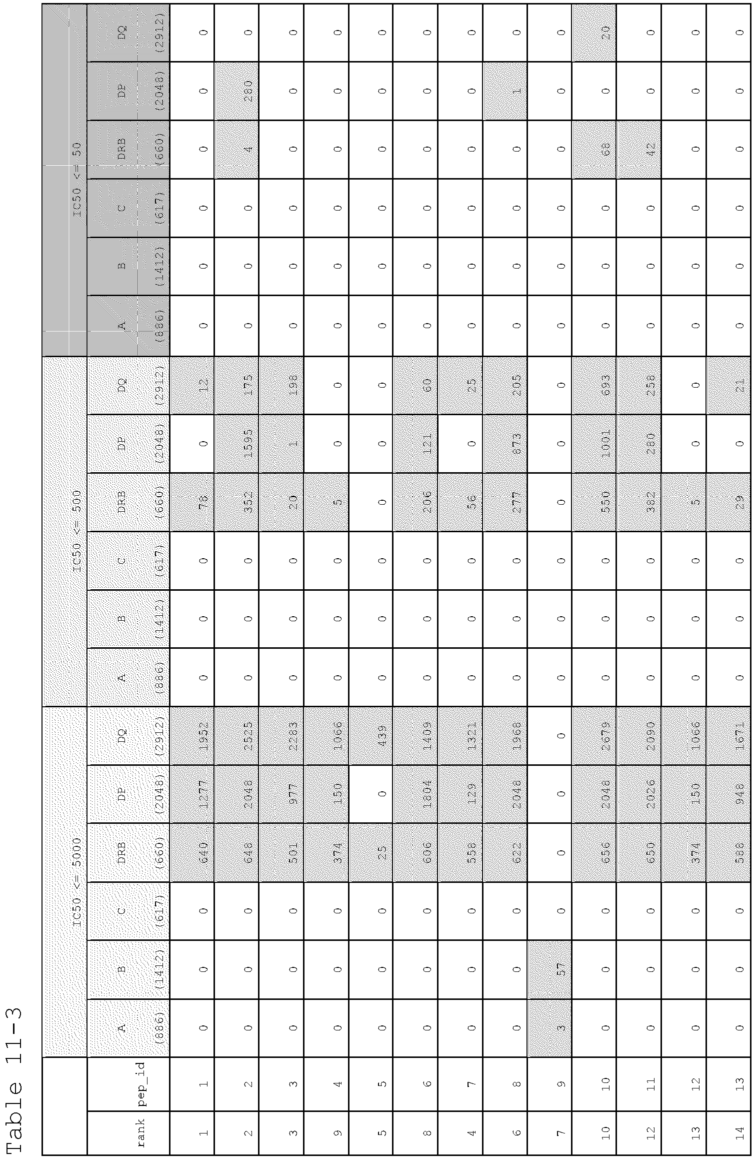

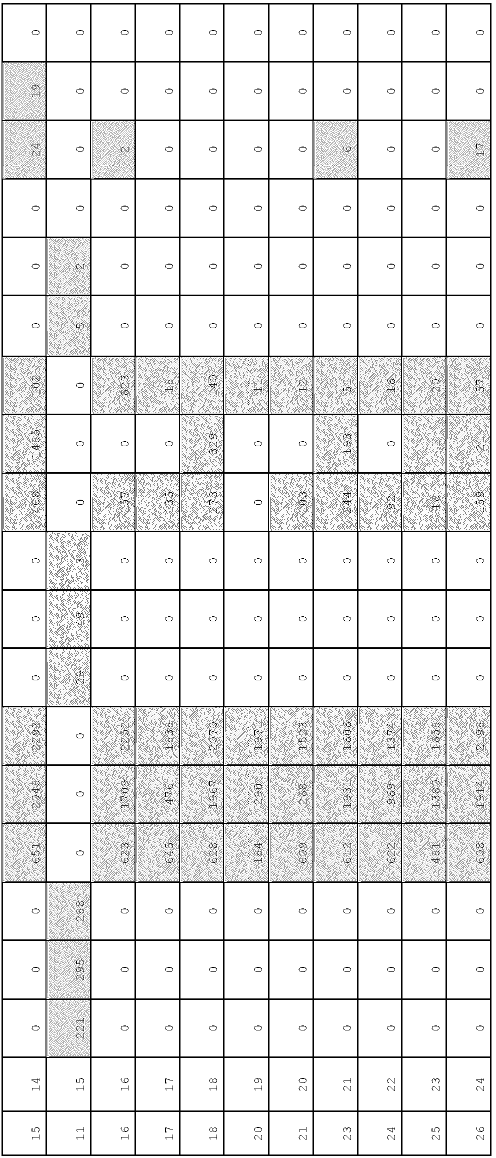

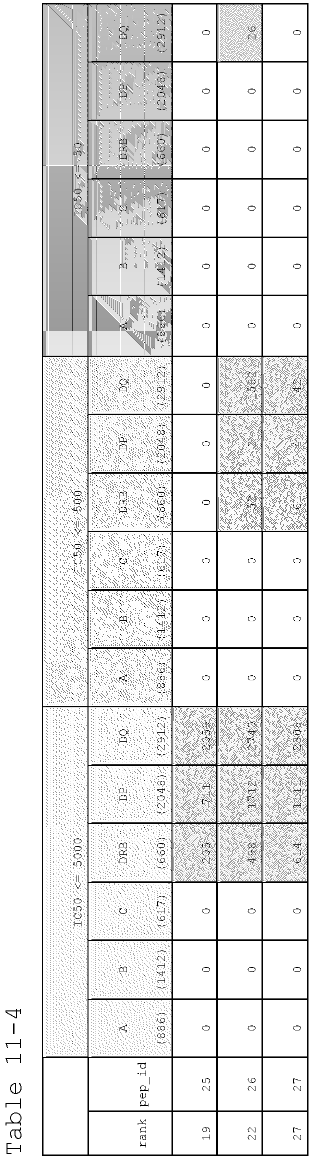

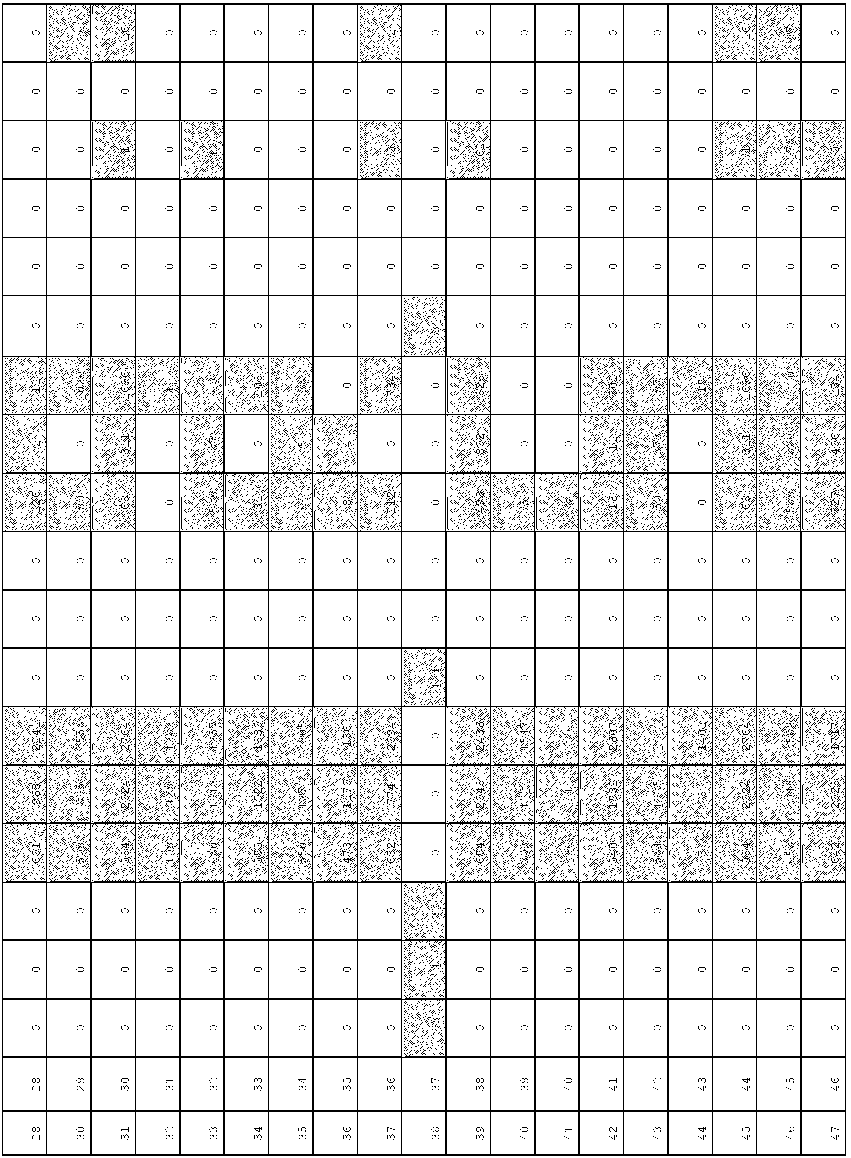

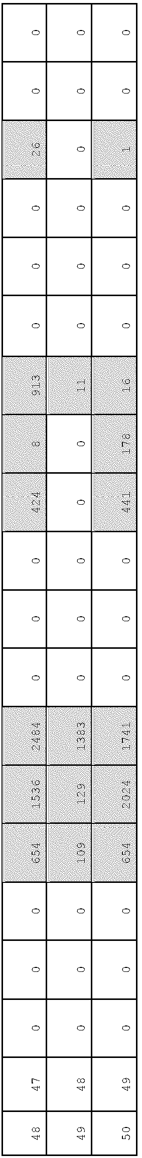

Many of the 49 types of identified TREM sequences were mutated, 40 types were mutated SHM type TREM sequences, and 9 types were unmutated germline type TREM sequences. When a mutation is present in a TREM sequence, the sequence of the idiotope changes to match the sequence of the dengue virus, and thus the mutation is a TREM sequence specifically generated by the immune response. Meanwhile, it is considered that the germline type TREM sequence has been detected in the immune response because its TREM sequence pattern is identical to that of the dengue virus. Among the germline type TREM sequences, a mutation may be present in the MAM sequence, and it is considered that this mutation has been detected as a specific clone in the immune response of the patient. For example, in the second most common TREM sequence, no mutation is present in the TREM sequence, but mutation occurs in the clone of 63/105 = 60% in the MAM sequence. Further, the third most common TREM sequence shows a germline type, but it is found that mutation occurs in the clone of 40/58 = 69% to cause a germline type, resulting in the same TREM sequence as that of the dengue virus. Therefore, in the present invention, these sequences can be effectively detected by full-length analysis of repertoire clone.

Many of the 49 types of identified TREM sequences were present in the V region. Specifically, the number of TREM sequences in the V region (including the L region) was 44, the number of TREM sequences in the D region was 2, the number of TREM sequences in the J region was 1, and the number of TREM sequences in the C region was 2. Further, the number of 24 types of common TREM sequences present in the CDR3 region was 4, and many of the TREM sequences were present in regions other than CDR3. Meanwhile, 48% (12/25 types) of patient-unique TREM sequences were present in the CDR3 region. Therefore, it is found that the full-length analysis technique of the repertoire clone established in the present invention is effective in identifying these TREM sequences.

In the present invention, it is possible to designate a protein functional domain, a module, a motif, a specific pathogenic species or the like in the reference sequence by setting a subordinate condition of the reference sequence in the comparison of the TREM sequence in Step 5, and it is possible to specify at which site the identified TREM sequence is present. For example, as shown in Table 11, the serotypes and proteins of dengue virus with 49 types of identified TREM sequences could be identified. Further, the use of these functions made it possible to search whether the TREM sequences were commonly present in other viruses of the same Flaviviridae family as that of the dengue virus, and to identify that some of the TREM sequences were commonly present in yellow fever virus and West Nile as shown in Table 11.

### (Example 13: Other Applied Examples)

The present invention can also identify a TREM sequence serving as a T cell antigen epitope from TCR repertoire analysis data. TCR repertoire analysis was performed on 18 dengue virus patients in the same manner as in the Examples in BCR described above, and then T cell antigen epitope analysis was performed using the resultant data. The results are shown in Table 12. 26 types of TREM sequences were identified from repertoire analysis data of TCRβ from 18 dengue virus patients. Among these sequences, for 6 types of TREM sequences, it has been revealed that a peptide sequence including the TREM sequence activates T cells to induce the production of interferon-γ. Therefore, in the present invention, not only BCR repertoire analysis but also TCR repertoire analysis can identify an epitope sequence serving as an antigen in a specific immune response.

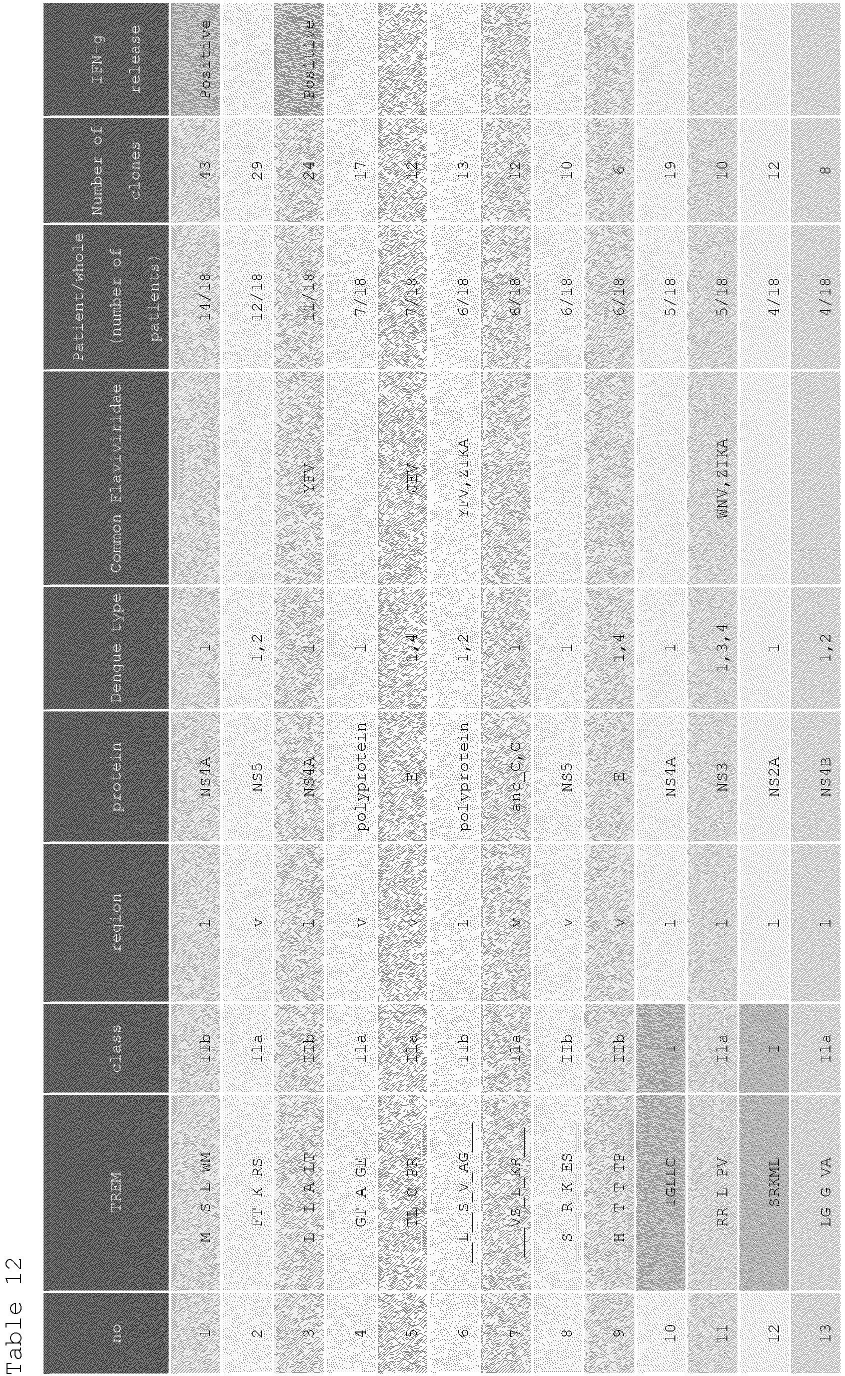

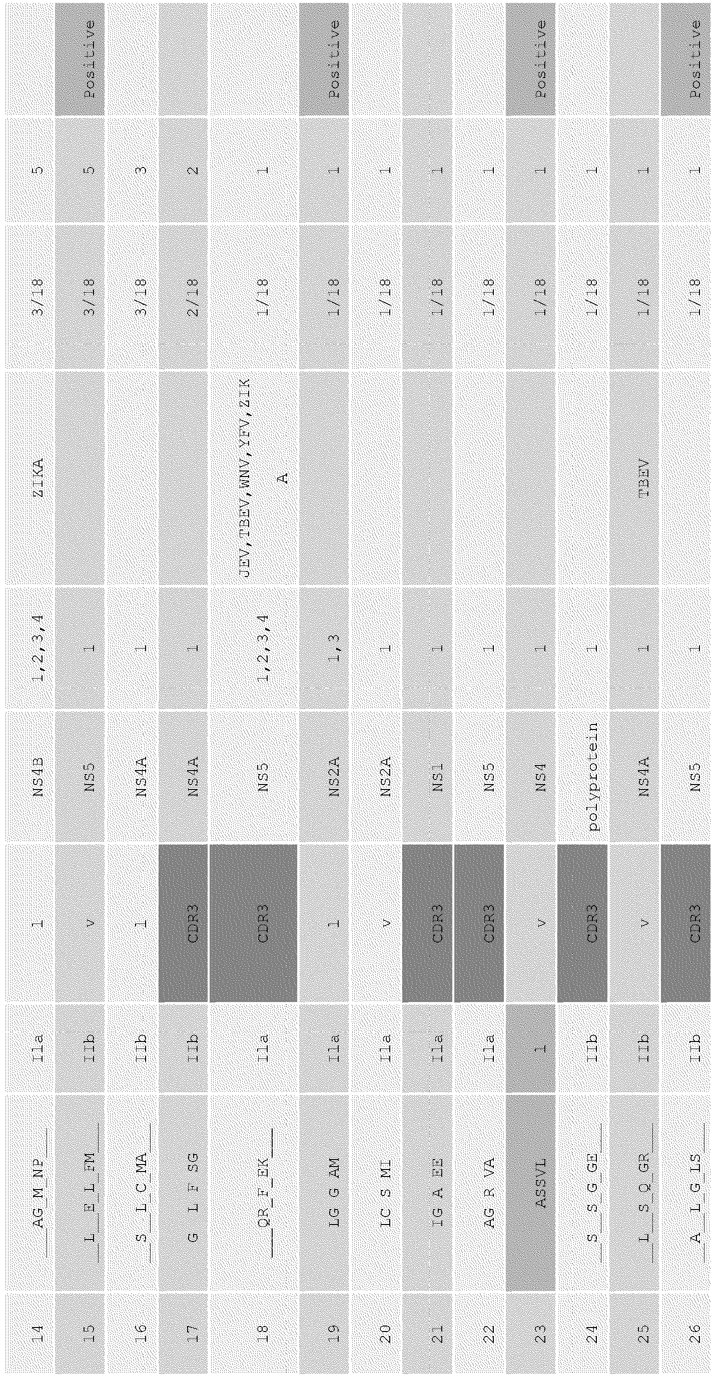

### (Example A: Application in BIO in vitro)

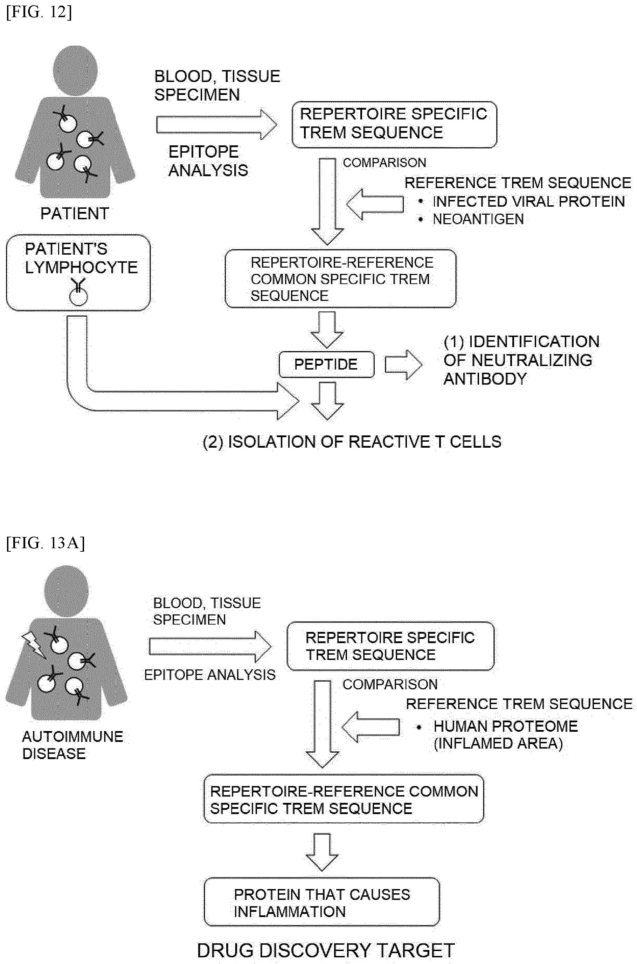

Examples of application in BIO in vitro using the method of the present disclosure include the following. For example, as shown in FIG. 12, in a case where epitope analysis is performed by the method of the present disclosure using the blood or tissue of a patient infected with a virus or a cancer patient as a specimen, the obtained repertoire specific TREM sequence is compared with a designated reference sequence (e.g. a viral protein for a viral infection and a neoantigen for a tumor), thereby obtaining a repertoire-reference common specific TREM sequence. A process of synthesizing a peptide sequence containing this repertoire-reference common specific TREM sequence and (1) searching for an antibody recognizing the peptide makes it possible to identify a neutralizing antibody against a target viral protein or a variant protein specifically expressing cancer cells. Further, (2) searching for T cells from patient-derived lymphocyte cells using the synthesized peptide makes it possible to isolate T cells reactive to the target viral protein or the variant protein specifically expressing cancer cells.

### (Example B: Application in Drug Discovery)

Examples of application in drug discovery using the method of the present disclosure include the following. For example, as shown in FIG. 13 A, in an autoimmune disease, in a case where epitope analysis is performed by the method of the present disclosure using the blood or tissue of a patient as a specimen, the obtained repertoire specific TREM sequence is compared with a proteome derived from an inflamed area as a reference sequence, thereby obtaining a repertoire-reference common specific TREM sequence. Since this repertoire-reference common specific TREM sequence is present in a protein that causes inflammation, this protein becomes a drug discovery target agent for autoimmune diseases.

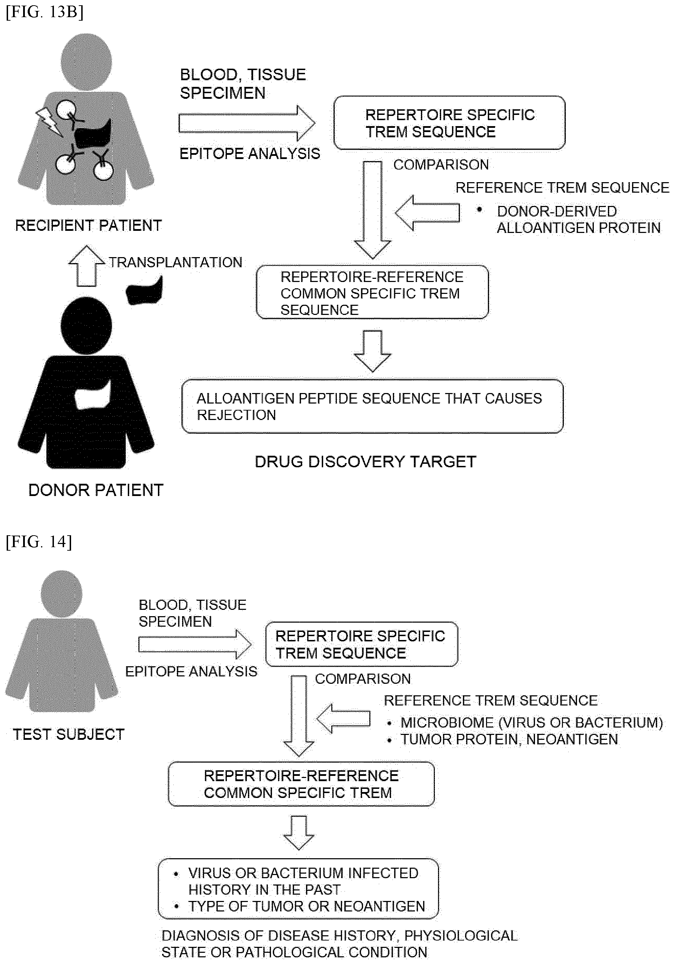

For example, in allograft, as shown in FIG. 13B, when epitope analysis is performed by the method of the present disclosure using the blood or tissue of a patient as a specimen, the obtained repertoire specific TREM sequence is compared with an alloantigen protein derived from a donor as a reference sequence, thereby obtaining a repertoire-reference common specific TREM sequence. Since this repertoire-reference common specific TREM sequence is present at the alloantigen site that causes rejection, this alloantigen peptide sequence becomes a drug discovery target agent for rejection in allograft.

### (Example C: Application in Diagnosis Example)

Examples of application in diagnosis example using the method of the present disclosure include the following. For example, as shown in FIG. 14, in a case where epitope analysis is performed by the method of the present disclosure using the blood or tissue of a test subject as a specimen, the obtained repertoire specific TREM sequence is compared with a specified reference sequence (e.g. a microbiome such as a virus or bacterium for an infection, and a tumor protein or neoantigen for a tumor), thereby obtaining a repertoire-reference common specific TREM sequence. Since the reference sequence includes a characteristic TREM sequence, it is examined what type of TREM sequence the repertoire-reference common specific TREM sequence shows, and this makes it possible to specify the virus, bacterium, and the like having been infected in the past, or to specify the type of tumor affected, neoantigen expressing cancer cells, and the like. Thus, the disease history, physiological state or pathological condition can be diagnosed.

### (Example D: Application in Diagnosis Example)

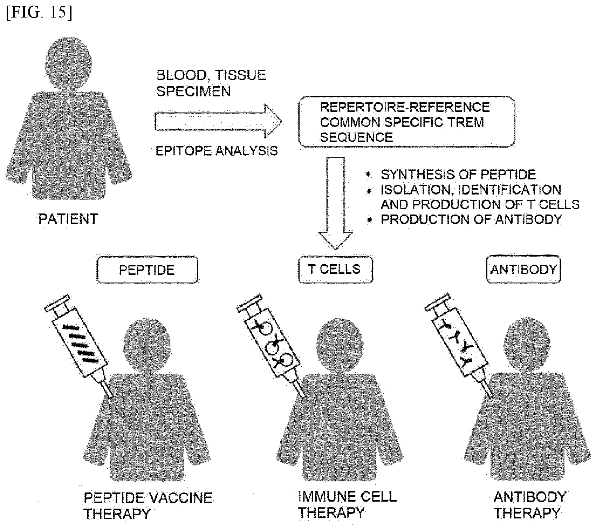

Examples of application in therapy using the method of the present disclosure include the following. For example, as shown in FIG. 15, in a case where epitope analysis is performed by the method of the present disclosure using the blood or tissue of a patient as a specimen, a repertoire-reference common specific TREM sequence can be obtained. As shown in Example A, this repertoire-reference common specific TREM sequence can be used in (1) synthesizing a peptide, (2) isolating, identifying or producing reactive T cells, or (3) producing an antibody recognizing the peptide. These processes can be used in (1) peptide vaccine therapy in the case of peptides, (2) immune cell therapy in the case of T cells, or (3) antibody therapy in the case of antibodies.

### (Note)

As described above, the present disclosure has been illustrated using preferred embodiments of the present disclosure; however it is understood that the scope of the present disclosure should be interpreted only by the claims. It is understood that the contents of patents, patent applications, and other literatures cited herein should be incorporated by reference in the same way that the contents themselves are specifically described in the present specification. The present application claims priority to Japanese Patent Application No. 2021-105709 (filed on June 25, 2021) filed with the Japan Patent Office, the entire contents of which are incorporated herein by reference in the same manner as all of them are described in the present specification.

### Industrial Applicability

According to the present disclosure, based on BCR repertoire and/or TCR repertoire data associated with a specific physiological state of a test subject, an epitope sequence associated with the specific physiological state of the test subject can be identified, which is useful in the medical field and the like.

### Sequence Listing Free Text

SEQ ID NOs: 1 to 172: peptide sequences shown in Tables 1-1 to 1-4
SEQ ID NOs: 173 to 192: peptide sequences shown in Table 2
SEQ ID NOs: 193 to 292: peptide sequences shown in Tables 3-1 to 3-2
SEQ ID NOs: 293 to 402: peptide sequence shown in Table 5
SEQ ID NOs: 403 to 424: peptide sequences shown in Tables 6-1 to 6-8
SEQ ID NOs: 425 to 428: peptide sequences shown in Tables 7-1 to 7-2
SEQ ID NOs: 429 to 440: peptide sequences shown in Table 8
SEQ ID NOs: 441 to 448: peptide sequences shown in Table 9
SEQ ID NOs: 449 to 501: peptide sequences shown in Tables 10-1 to 10-2

### Reference Signs List

- 100: computer system
- 110: interface unit
- 120: processor unit
- 130: memory unit
- 200: database unit
- 300: terminal device
- 400: network
- 1401: CPU
- 1403: RAM
- 1405: external storage device
- 1407: output device
- 1409: input device
- 1411: communication device
- 1420: system bus
- 1425: input/output I/F
- 1430: information database storage unit
- 1440: program storage unit

### Sequence Listing

International Application under the Patent Cooperation Treaty: EC011PCTJP22025376_28.app

## Claims

1. A method for identifying an epitope sequence associated with a specific physiological state of a test subject,
the method comprising the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence; and
E) determining, based on the repertoire-reference common specific TREM sequence, whether a peptide sequence including the repertoire-reference common specific TREM sequence is an epitope sequence associated with the specific physiological state of the test subject.

2. The method according to claim 1, wherein the step of obtaining BCR repertoire and/or TCR repertoire data includes a step of obtaining a full-length sequence of BCR and/or TCR.

3. The method according to claim 1 or 2, further comprising a step of determining a full-length sequence of an immunoglobulin variable section of BCR or TCR clone specific to the physiological state.

4. The method according to any one of claims 1 to 3, wherein the BCR repertoire and/or TCR repertoire data is obtained by analyzing a specimen obtained from the test subject.

5. The method according to any one of claims 1 to 3, wherein the BCR and/or TCR repertoire data is obtained from an existing database.

6. The method according to any one of claims 1 to 5, further comprising a step of analyzing BCR and/or TCR repertoire in the specimen from the test subject to determine a clone specifically reacting to the specific physiological state.

7. The method according to any one of claims 1 to 6, further comprising a step of determining a gene region in which the repertoire-specific TREM sequence is present.

8. The method according to claim 7, wherein the gene region is selected from the group consisting of an L region, a V region, a D region, a J region or a C region, FR1, 2 or 3, and CDR1, 2 or 3.

9. The method according to any one of claims 1 to 8, further comprising a step of producing an about 8- to about 10-mer and/or about 13- to about 17-mer amino acid sequence from the sequence of BCR or TCR clone specific to the physiological state and extracting the TREM sequence.

10. The method according to any one of claims 1 to 9, further comprising a step of producing an about 9- and/or about 15-mer amino acid sequence from the sequence of BCR or TCR clone specific to the physiological state and extracting the TREM sequence.

11. The method according to any one of claims 1 to 10, wherein, in a 9-mer amino acid sequence, the TREM sequence includes at least one of positions 4, 5, 6, 7 and 8 from the N-terminus of the 9-mer amino acid sequence.

12. The method according to any one of claims 1 to 11, wherein, in a 15-mer amino acid sequence, the TREM sequence includes at least one of positions 5, 6, 8, 10 and 11, or at least one of positions 3, 6, 8, 10 and 11 from the N-terminus of the 15-mer amino acid sequence.

13. The method according to any one of claims 1 to 12, further comprising a step of determining MHC I/II binding affinity of an about 8- to about 10-mer and/or about 13- to about 17-mer amino acid sequence including the TREM sequence.

14. The method according to any one of claims 1 to 13, further comprising a step of identifying a variant TREM.

15. The method according to any one of claims 1 to 14, further comprising a step of evaluating and specifying a peptide with B-cell epitope properties.

16. The method according to any one of claims 1 to 15, wherein the step of determining whether a peptide sequence is an epitope sequence associated with the specific physiological state includes testing HLA (MHC) binding affinity of the peptide sequence including the repertoire-reference common specific TREM sequence to determine whether the peptide sequence is an epitope sequence associated with the specific physiological state of the test subject.

17. The method according to claim 16, wherein the method includes, in the test of HLA (MHC) binding affinity, a step of selecting an epitope sequence with an IC50 of about 5000 or less as the epitope sequence associated with the specific physiological state of the test subject.

18. The method according to claim 16 or 17, wherein the test of HLA (MHC) binding affinity includes estimating the HLA (MHC) binding affinity.

19. The method according to any one of claims 1 to 18, wherein the reference TREM sequence is a TREM sequence of one selected from the group consisting of a cancer-associated protein, an infection-associated protein, an autoimmunityassociated protein, an allergy-associated protein, a microbiome and an alloreaction/xenoreaction-associated protein.

20. The method according to any one of claims 1 to 19, wherein the reference TREM sequence is a TREM sequence of one selected from the group consisting of a variant protein, a neoantigen, a tumor protein, a proteome in an inflamed area, a virus-associated protein, a bacteria-associated protein, a fungus-associated protein, an alloantigen, a xenoantigen, an allergen protein and a microbiome.

21. The method according to any one of claims 1 to 20, wherein the reference TREM sequence is obtained from the specimen from the test subject.

22. The method according to any one of claims 1 to 20, wherein the reference TREM sequence is obtained from an existing database.

23. The method according to any one of claims 1 to 22, wherein the reference TREM sequence contains an artificial peptide.

24. The method according to any one of claims 1 to 23, further comprising a step of comparing the sequence of the BCR or TCR clone with any reference sequence to analyze whether an identical TREM sequence is present.

25. The method according to claim 24, wherein the any reference sequence includes a reference sequence unique to the physiological state.

26. The method according to any one of claims 1 to 25, comprising a step of determining the about 9-mer and/or about 15-mer amino acid sequence with a common TREM sequence between the sequence of the BCR or TCR clone and the reference sequence, as a candidate T-cell antigen epitope to be presented to T cells.

27. The method according to claim 26, wherein the candidate T-cell antigen epitope is determined by evaluating identity between the repertoire specific TREM sequence and the reference TREM sequence, extracting the repertoire specific TREM sequence having TREM with identity and/or an about 9-mer or about 15-mer peptide sequence in the reference TREM sequence, and calculating HLA (MHC) binding affinity of the extracted peptide sequence.

28. The method according to any one of claims 1 to 27, comprising a step of extracting a reference sequence peptide in which the TREM matches with the BCR repertoire and/or TCR repertoire.

29. The method according to any one of claims 1 to 28, further comprising a step of narrowing down peptides based on the HLA type to determine an HLA-specific peptide with a common TREM between the BCR and/or TCR repertoire and the reference TREM sequence.

30. The method according to any one of claims 1 to 29, wherein the step of determining whether a peptide sequence is an epitope sequence includes a step of evaluating binding affinity for a plurality of different MHCs.

31. A method for producing an antigen containing an epitope sequence associated with a specific physiological state of a test subject,
the method comprising the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence;
E) determining, based on the repertoire-reference common specific TREM sequence, whether a peptide sequence including the repertoire-reference common specific TREM sequence is an epitope sequence associated with the specific physiological state of the test subject; and
F) generating a peptide containing the epitope sequence.

32. The method according to claim 31, wherein the step of obtaining BCR repertoire and/or TCR repertoire data includes a step of obtaining a full-length sequence of BCR and/or TCR.

33. The method according to claim 31 or 32, further comprising a step of determining a full-length sequence of an immunoglobulin variable section of BCR or TCR clone specific to the physiological state.

34. The method according to any one of claims 31 to 33, wherein the BCR repertoire and/or TCR repertoire data is obtained by analyzing a specimen obtained from the test subject.

35. The method according to any one of claims 31 to 33, wherein the BCR and/or TCR repertoire data is obtained from an existing database.

36. The method according to any one of claims 31 to 35, further comprising a step of analyzing BCR and/or TCR repertoire in the specimen from the test subject to determine a clone specifically reacting to the specific physiological state.

37. The method according to any one of claims 31 to 36, further comprising a step of determining a gene region in which the repertoire-specific TREM sequence is present.

38. The method according to claim 37, wherein the gene region is selected from the group consisting of an L region, a V region, a D region, a J region or a C region, FR1, 2 or 3, and CDR1, 2 or 3.

39. The method according to any one of claims 31 to 38, further comprising a step of producing an about 8- to about 10-mer and/or about 13- to about 17-mer amino acid sequence from the sequence of BCR or TCR clone specific to the physiological state and extracting the TREM sequence.

40. The method according to any one of claims 31 to 39, further comprising a step of producing an about 9- to about 15-mer amino acid sequence from the sequence of BCR or TCR clone specific to the physiological state and extracting the TREM sequence.

41. The method according to any one of claims 31 to 40, wherein, in a 9-mer amino acid sequence, the TREM sequence includes at least one of positions 4, 5, 6, 7 and 8 from the N-terminus of the 9-mer amino acid sequence.

42. The method according to any one of claims 31 to 41, wherein, in a 15-mer amino acid sequence, the TREM sequence includes at least one of positions 5, 6, 8, 10 and 11, or at least one of positions 3, 6, 8, 10 and 11 from the N-terminus of the 15-mer amino acid sequence.

43. The method according to any one of claims 31 to 42, further comprising a step of determining MHC I/II binding affinity of an about 8- to about 10-mer and/or about 13- to about 17-mer amino acid sequence including the TREM sequence.

44. The method according to any one of claims 31 to 43, further comprising a step of identifying a variant TREM.

45. The method according to any one of claims 31 to 44, further comprising a step of evaluating and specifying a peptide with B-cell epitope properties.

46. The method according to any one of claims 31 to 45, wherein the step of determining whether a peptide sequence is an epitope sequence associated with the specific physiological state includes testing HLA (MHC) binding affinity of the peptide sequence including the repertoire-reference common specific TREM sequence to determine whether the peptide sequence is an epitope sequence associated with the specific physiological state of the test subject.

47. The method according to claim 46, wherein the method includes, in the test of HLA (MHC) binding affinity, a step of selecting an epitope sequence with an IC50 of about 5000 or less as the epitope sequence associated with the specific physiological state of the test subject.

48. The method according to claim 46 or 47, wherein the test of HLA (MHC) binding affinity includes estimating the HLA (MHC) binding affinity.

49. The method according to any one of claims 31 to 48, wherein the reference TREM sequence is a TREM sequence of one selected from the group consisting of a cancer-associated protein, an infection-associated protein, an autoimmunityassociated protein, an allergy-associated protein, a microbiome and an alloreaction/xenoreaction-associated protein.

50. The method according to any one of claims 31 to 49, wherein the reference TREM sequence is a TREM sequence of one selected from the group consisting of a variant protein, a neoantigen, a tumor protein, a proteome in an inflamed area, a virus-associated protein, a bacteria-associated protein, a fungus-associated protein, an alloantigen, a xenoantigen, an allergen protein and a microbiome.

51. The method according to any one of claims 31 to 50, wherein the reference TREM sequence is obtained from the specimen from the test subject.

52. The method according to any one of claims 31 to 50,
wherein the reference TREM sequence is obtained from an existing database.

53. The method according to any one of claims 31 to 52, wherein the reference TREM sequence contains an artificial peptide.

54. The method according to any one of claims 31 to 53, further comprising a step of comparing the sequence of the BCR or TCR clone with any reference sequence to analyze whether an identical TREM sequence is present.

55. The method according to claim 54, wherein the any reference sequence includes a reference sequence unique to the physiological state.

56. The method according to any one of claims 31 to 55, comprising a step of determining the about 9-mer and/or about 15-mer amino acid sequence with a common TREM sequence between the sequence of the BCR or TCR clone and the reference sequence, as a candidate T-cell antigen epitope to be presented to T cells.

57. The method according to claim 56, wherein the candidate T-cell antigen epitope is determined by evaluating identity between the repertoire specific TREM sequence and the reference TREM sequence, extracting the repertoire specific TREM sequence having TREM with identity and/or an about 9-mer or about 15-mer peptide sequence in the reference TREM sequence, and calculating HLA (MHC) binding affinity of the extracted peptide sequence.

58. The method according to any one of claims 31 to 57, comprising a step of extracting a reference sequence peptide in which the TREM matches with the BCR repertoire and/or TCR repertoire.

59. The method according to any one of claims 31 to 58, further comprising a step of narrowing down peptides based on the HLA type to determine an HLA-specific peptide with a common TREM between the BCR and/or TCR repertoire and the reference TREM sequence.

60. The method according to any one of claims 31 to 59,
wherein the step of determining whether a peptide sequence is an epitope sequence includes a step of evaluating binding affinity for a plurality of different MHCs.

61. A program for causing a computer to execute processing of a method for identifying an epitope sequence associated with a specific physiological state of a test subject,
the method comprising the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence; and
E) causing the computer to determine, based on the repertoire-reference common specific TREM sequence, whether a peptide sequence including the repertoire-reference common specific TREM sequence is an epitope sequence associated with the specific physiological state of the test subject.

62. A recording medium that stores a program for causing a computer to execute processing of a method for identifying an epitope sequence associated with a specific physiological state of a test subject,
the method comprising the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence; and
E) causing the computer to determine, based on the repertoire-reference common specific TREM sequence, whether a peptide sequence including the repertoire-reference common specific TREM sequence is an epitope sequence associated with the specific physiological state of the test subject.

63. A system for identifying an epitope sequence associated with a specific physiological state of a test subject,
the system comprising:
A) means for obtaining BCR and/or TCR repertoire data associated with the specific physiological state of the test subject;
B) means for specifying a TREM sequence (repertoire specific TREM sequence) from the BCR and/or TCR repertoire data;
C) means for obtaining a reference TREM sequence associated with the test subject;
D) means for specifying a repertoire-reference common specific TREM sequence from the reference TREM sequence and the repertoire specific TREM sequence; and
E) means for determining, based on the repertoire-reference common specific TREM sequence, whether a peptide sequence including the repertoire-reference common specific TREM sequence is an epitope sequence associated with the specific physiological state of the test subject.

64. A computer program for causing a computer to execute processing of a method for producing an antigen containing an epitope sequence associated with a specific physiological state of a test subject,
the method comprising the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence;
E) causing the computer to determine, based on the repertoire-reference common specific TREM sequence, whether a peptide sequence including the repertoire-reference common specific TREM sequence is an epitope sequence associated with the specific physiological state of the test subject; and
F) causing the computer to generate a peptide containing the epitope sequence.

65. A recording medium that stores a computer program for causing a computer to execute processing of a method for producing an antigen containing an epitope sequence associated with a specific physiological state of a test subject,
the method comprising the steps of:
A) causing the computer to obtain BCR and/or TCR repertoire data associated with the specific physiological state of the test subject;
B) causing the computer to specify a TREM sequence (repertoire specific TREM sequence) from the BCR and/or TCR repertoire data;
C) causing the computer to obtain a reference TREM sequence associated with the test subject;
D) causing the computer to specify a repertoire-reference common specific TREM sequence from the reference TREM sequence and the repertoire specific TREM sequence;
E) causing the computer to determine, based on the repertoire-reference common specific TREM sequence, whether a peptide sequence including the repertoire-reference common specific TREM sequence is an epitope sequence associated with the specific physiological state of the test subject; and
F) causing the computer to generate a peptide containing the epitope sequence.

66. A system for producing an antigen containing an epitope sequence associated with a specific physiological state of a test subject,
the system comprising:
A) means for obtaining BCR and/or TCR repertoire data associated with the specific physiological state of the test subject;
B) means for specifying a TREM sequence (repertoire specific TREM sequence) from the BCR and/or TCR repertoire data;
C) means for obtaining a reference TREM sequence associated with the test subject;
D) means for specifying a repertoire-reference common specific TREM sequence from the reference TREM sequence and the repertoire specific TREM sequence;
E) means for determining, based on the repertoire-reference common specific TREM sequence, whether a peptide sequence including the repertoire-reference common specific TREM sequence is an epitope sequence associated with the specific physiological state of the test subject; and
F) means for generating a peptide containing the epitope sequence.

67. A peptide comprising an epitope sequence identified by the method described in any one of claims 1 to 30.

68. An antigen peptide produced by the method described in any one of claims 31 to 60.

69. A peptide library comprising two or more of the peptides described in claim 67 or 68.

70. A peptide library comprising two or more peptides containing an epitope sequence identified by the method described in any one of claims 1 to 30.

71. A peptide library comprising two or more antigen peptides produced by the method described in any one of claims 31 to 60.

72. A method for generating a peptide library of peptides containing an epitope sequence associated with a specific physiological state of a test subject,
the method comprising the steps of:
A) obtaining BCR and/or TCR repertoire data associated with the specific physiological state of the test subject;
B) specifying a TREM sequence (repertoire specific TREM sequence) from the BCR and/or TCR repertoire data;
C) obtaining a reference TREM sequence associated with the test subject;
D) specifying a repertoire-reference common specific TREM sequence from the reference TREM sequence and the repertoire specific TREM sequence;
E) determining, based on the repertoire-reference common specific TREM sequence, whether a peptide sequence including the repertoire-reference common specific TREM sequence is an epitope sequence associated with the specific physiological state of the test subject;
F) generating a peptide containing the epitope sequence; and
G) combining two or more of the peptides generated by the step F to generate a peptide library.
